# EUROPEAN PATENT SPECIFICATION

(11) **EP 3 099 300 B1**
(45) Date of publication and mention of the grant of the patent: **05.09.2018**
(21) Application number: 14704047.1
(22) Date of filing: 29.01.2014
(51) Int. Cl.: A61K 31/4375, C07D 471/04, A61P 25/02, A61P 25/16, A61P 25/28

(54) **ARYL LACTAM KINASE INHIBITORS**
ARYLLACTAMKINASEINHIBITOREN
INHIBITEURS D'ARYL-LACTAME KINASE

(43) Date of publication of application: 07.12.2016
(73) Proprietor: Bristol-Myers Squibb Company, Princeton, NJ 08543 (US)
(72) Inventor: VRUDHULA, Vivekananda, M., Brier, Washington 98036 (US); PAN, Senliang, Woodbridge, Connecticut 06525 (US); RAJAMANI, Ramkumar, Wallingford, Connecticut 06492 (US); NARA, Susheel, Jethanand, Bangalore Karnataka 560099 (IN); KARATHOLUVHU, Maheswaran, Sivasamban, Yelahanka Bangalore 560064 Karnataka (IN); MAISHAL, Tarun, Kumar, Bangalore Karnataka 560099 (IN); DITTA, Jonathan, L., Wallingford, Connecticut 06492 (US); DZIERBA, Carolyn, Diane, Wallingford, Connecticut 06492 (US); BRONSON, Joanne, J., Wallingford, Connecticut 06492 (US); MACOR, John, E., Wallingford, Connecticut 06492 (US)
(74) Representative: Kling, Edouard
(86) International application number: PCT/US2014/013539
(87) International publication number: WO 2015/116060

(56) References cited:
- WO-A1-2013/134036
- WO-A1-2014/022167
- US-A1- 2005 171 101
- US-A1- 2011 118 299

## Description

The present disclosure is generally directed to compounds which can inhibit adaptor associated kinase 1 (AAK1), compositions comprising such compounds, and methods for inhibiting AAK1.

Adaptor associated kinase 1 (AAK1) is a member of the Ark1/Prk1 family of serine/threonine kinases. AAK1 mRNA exists in two splice forms termed short and long. The long form predominates and is highly expressed in brain and heart (Henderson and Conner, Mol. Biol. Cell. 2007, 18, 2698-2706). AAK1 is enriched in synaptosomal preparations and is co-localized with endocytic structures in cultured cells. AAK1 modulates clatherin coated endocytosis, a process that is important in synaptic vesicle recycling and receptor-mediated endocytosis. AAK1 associates with the AP2 complex, a hetero-tetramer which links receptor cargo to the clatherin coat. The binding of clatherin to AAK1 stimulates AAK1 kinase activity (Conner et. al., Traffic 2003, 4, 885-890; Jackson et. al., J. Cell. Biol. 2003, 163, 231-236). AAK1 phosphorylates the mu-2 subunit of AP-2, which promotes the binding of mu-2 to tyrosine containing sorting motifs on cargo receptors (Ricotta et. al., J. Cell Bio. 2002, 156, 791-795; Conner and Schmid, J. Cell Bio. 2002, 156, 921-929). Mu2 phosphorylation is not required for receptor uptake, but phosphorylation enhances the efficiency of internalization (Motely et. al., Mol. Biol. Cell. 2006, 17, 5298-5308).

AAK1 has been identified as an inhibitor of Neuregulin-1/ErbB4 signaling in PC12 cells. Loss of AAK1 expression through RNA interference mediated gene silencing or treatment with the kinase inhibitor K252a (which inhibits AAK1 kinase activity) results in the potentiation of Neuregulin-1 induced neurite outgrowth. These treatments result in increased expression of ErbB4 and accumulation of ErbB4 in or near the plasma membrane (Kuai et. al., Chemistry and Biology 2011, 18, 891-906). NRG1 and ErbB4 are putative schizophrenia susceptibility genes (Buonanno, Brain Res. Bull. 2010, 83, 122-131). SNPs in both genes have been associated with multiple schizophrenia endophenotypes (Greenwood et. al., Am. J. Psychiatry 2011, 168, 930-946). Neuregulin 1 and ErbB4 KO mouse models have shown schizophrenia relevant morphological changes and behavioral phenotypes (Jaaro-Peled et. al., Schizophrenia Bulletin 2010, 36, 301-313; Wen et. al., Proc. Natl. Acad. Sci. USA. 2010, 107, 1211-1216). In addition, a single nucleotide polymorphism in an intron of the AAK1 gene has been associated with the age of onset of Parkinson's disease (Latourelle et. al., BMC Med. Genet. 2009, 10, 98). These results suggest that inhibition of AAK1 activity may have utility in the treatment of schizophrenia, cognitive deficits in schizophrenia, Parkinson's disease, neuropathic pain, bipolar disorder, and Alzheimer's disease.

Document WO 2013/134036 discloses chromeno[3,4-c]pyridine kinase inhibitors. Document US2005/171101 and US 2011/118299 disclose phenanthridinones and benzo[c][2,7]naphtyridinones.

In a first aspect the present disclosure provides a compound of formula (I) 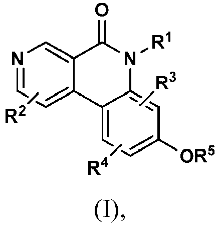 or a pharmaceutically acceptable salt thereof, wherein:
R¹ is selected from hydrogen, C₂-C₄alkenyl, C₁-C₃alkoxy, C₁-C₃alkoxyC₁-C₃alkyl, C₁-C₃alkyl, C₁-C₃alkylamino, arylC₁-C₃alkyl, C₃-C₆cycloalkyl, C₃-C₆cycloalkylC₁-C₃alkyl, C₁-C₃haloalkyl, and C₁-C₃hydroxyalkyl;
R² is selected from hydrogen, C₁-C₃alkoxy, C₁-C₃alkoxycarbonylamino, C₁-C₃alkyl, C₁-C₃alkylamino, C₁-C₃alkylcarbonylamino, amino, arylamino, arylcarbonylamino, C₃-C₆cycloalkylamino, C₃-C₆cycloalkylcarbonylamino, C₃-C₆cycloalkyloxy, halo, C₁-C₃haloalkoxy, C₁-C₃haloalkyl, C₂-C₃haloalkylamino, C₂-C₃haloalkylcarbonylamino, hydroxy, and phenylC₁-C₃alkylamino, wherein the phenyl is optionally substituted with a C₁-C₃alkoxy group;
R³ and R⁴ are independently selected from hydrogen, C₁-C₄alkoxy, C₁-C₃alkoxyC₁-C₃alkyl, C₁-C₃alkoxycarbonyl, C₁-C₄alkyl, C₁-C₃alkylsulfonyl, aminocarbonyl, cyano, C₃-C₆cycloalkyl, di(C₁-C₃alkyl)aminocarbonyl, halo, C₁-C₃haloalkoxy, C₁-C₃haloalkyl, heteroaryl, hydroxy, C₁-C₃hydroxyalkyl, and phenyl optionally substituted with a halo group;
R⁵ is C₁-C₃alkyl-Y or C₂-C₈alkyl, wherein the C₂-C₈alkyl is optionally substituted with one, two, three, or four groups independently selected from C₁-C₃alkoxy, C₁-C₃alkylamino, C₁-C₃alkoxyC₂-C₃alkylamino, amino, aryl, di(C₁-C₃alkyl)amino, halo, C₁-C₃haloalkylamino, C₁-C₃haloalkylcarbonylamino, hydroxy,-NR^{x}R^{y}, and C₃-C₈cycloalkyl, wherein the cycloalkyl is further optionally substituted with one, two, or three groups independently selected from C₁-C₃alkoxy, C₁-C₃alkyl, C₁-C₃alkylamino, C₁-C₃alkoxyC₂-C₃alkylamino, amino, aryl, arylC₁-C₃alkyl, halo, C₁-C₃haloalkyl, C₁-C₃haloalkylamino and hydroxy;
R^{x} and R^{y}, together with the nitrogen atom to which they are attached, form a three- to six-membered ring; and
Y is selected from 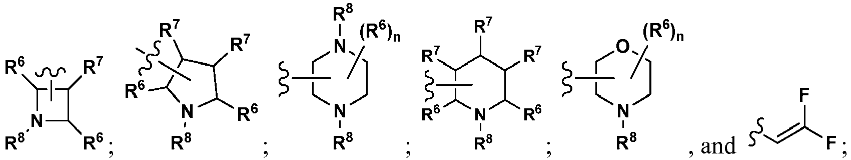 wherein
   n is 0, 1, 2, or 3;
      each R⁶ is independently selected from hydrogen, C₁-C₆alkyl, aryl, arylC₁-C₃alkyl, C₃-C₆cycloalkyl, halo, and C₁-C₃haloalkyl;
   each R⁷ is independently selected from hydrogen, C₁-C₃alkoxy and hydroxy; and
   R⁸ is selected from hydrogen, C₁-C₆alkyl, C₃-C₆cycloalkyl, and C₁-C₆alkylcarbonyl.

In a first embodiment of the first aspect the present disclosure provides a compound of formula (I) wherein R⁵ is C₂-C₈alkyl, wherein the C₂-C₈alkyl is optionally substituted with one, two, three, or four groups independently selected from C₁-C₃alkoxy, amino, C₁-C₃alkylamino, di(C₁-C₃alkyl)amino, halo, and C₃-C₈cycloalkyl; or R⁵ is C₁-C₃alkyl-Y wherein Y is 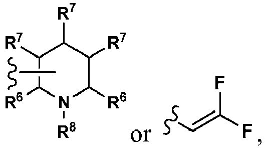 wherein R⁶, R⁷, and R⁸ are hydrogen.

In a second embodiment of the first aspect the present disclosure provides a compound of formula (I) wherein R⁵ is C₂-C₈alkyl optionally substituted with one, two, three, or four groups independently selected from amino and halo.

In a third embodiment of the first aspect the present disclosure provides a compound of formula (I) wherein R¹ is selected from hydrogen, C₂-C₄alkenyl, C₁-C₃alkoxyC₁-C₃alkyl, C₁-C₃alkyl, arylC₁-C₃alkyl, C₃-C₆cycloalkylC₁-C₃alkyl, C₁-C₃haloalkyl, and C₁-C₃hydroxyalkyl;
R² is selected from hydrogen, C₁-C₃alkoxy, C₁-C₃alkyl, C₁-C₃alkylcarbonylamino, amino, halo, C₁-C₃haloalkyl, and phenylC₁-C₃alkylamino, wherein the phenyl is optionally substituted with a C₁-C₃alkoxy group;
R³ and R⁴ are independently selected from hydrogen, C₁-C₄alkoxy, C₁-C₃alkoxyC₁-C₃alkyl, C₁-C₃alkoxycarbonyl, C₁-C₄alkyl, C₁-C₃alkylsulfonyl, aminocarbonyl, cyano, C₃-C₆cycloalkyl, di(C₁-C₃alkyl)aminocarbonyl, halo, C₁-C₃haloalkoxy, C₁-C₃haloalkyl, heteroaryl, hydroxy, C₁-C₃hydroxyalkyl, and phenyl optionally substituted with a halo group;
R⁵ is C₁-C₃alkyl-Y or C₂-C₈alkyl, wherein the C₂-C₈alkyl is optionally substituted with one, two, three, or four groups independently selected from C₁-C₃alkoxy, C₁-C₃alkylamino, amino, di(C₁-C₃alkyl)amino, halo, and C₃-C₈cycloalkyl; and wherein Y is selected from 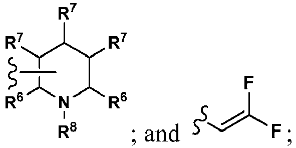 wherein R⁶, R⁷, and R⁸ are hydrogen.

In a second aspect the present disclosure provides a compound of formula (II) 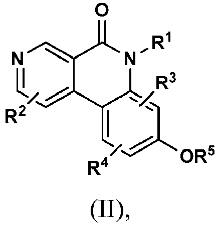 or a pharmaceutically acceptable salt thereof, wherein:
R¹ is selected from hydrogen, C₂-C₄alkenyl, C₁-C₃alkoxy, C₁-C₃alkoxy C₁-C₃alkyl, C₁-C₃alkyl, C₁-C₃alkylamino, arylC₁-C₃alkyl, C₃-C₆cycloalkyl, C₃-C₆cycloalkylC₁-C₃alkyl, and C₁-C₃haloalkyl;
R² is selected from hydrogen, C₁-C₃alkoxy, C₁-C₃alkoxycarbonylamino, C₁-C₃alkyl, C₁-C₃alkylamino, C₁-C₃alkylcarbonylamino, amino, arylamino, arylcarbonylamino, C₃-C₆cycloalkylamino, C₃-C₆cycloalkylcarbonylamino, C₃-C₆cycloalkyloxy, halo, C₁-C₃haloalkoxy, C₁-C₃haloalkyl, C₂-C₃haloalkylamino, C₂-C₃haloalkylcarbonylamino, and hydroxy;
R³ and R⁴ are independently selected from hydrogen, C₁-C₄alkyl, cyano, C₃-C₆cycloalkyl, halo, C₁-C₃haloalkyl, and hydroxy;
R⁵ is C₁-C₃alkyl-Y or C₂-C₈alkyl, wherein the C₂-C₈alkyl is optionally substituted with one, two, or three groups independently selected from C₁-C₃alkoxy, C₁-C₃alkylamino, C₁-C₃alkoxyC₂-C₃alkylamino, amino, aryl, halo, C₁-C₃haloalkylamino, C₁-C₃haloalkylcarbonylamino, hydroxy, -NR^{x}R^{y}, and C₃-C₈cycloalkyl, wherein the cycloalkyl is further optionally substituted with one, two, or three groups independently selected from C₁-C₃alkoxy, C₁-C₃alkyl, C₁-C₃alkylamino, C₁-C₃alkoxyC₂-C₃alkylamino, amino, aryl, arylC₁-C₃alkyl, halo, C₁-C₃haloalkyl, C₁-C₃haloalkylamino and hydroxy;
R^{x} and R^{y}, together with the nitrogen atom to which they are attached, form a three- to six-membered ring; and
Y is selected from 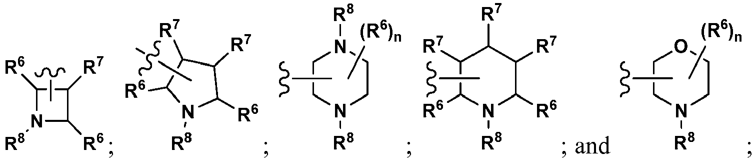 wherein n is 0, 1, 2, or 3;
   each R⁶ is independently selected from hydrogen, C₁-C₆alkyl, aryl, arylC₁-C₃alkyl, C₃-C₆cycloalkyl, halo, and C₁-C₃haloalkyl;
   each R⁷ is independently selected from hydrogen, C₁-C₃alkoxy and hydroxy; and
   R⁸ is selected from hydrogen, C₁-C₆alkyl, C₃-C₆cycloalkyl, and C₁-C₆alkylcarbonyl.

In a first embodiment of the second aspect the present disclosure provides a compound of formula (I), or a pharmaceutically acceptable salt thereof, wherein R⁵ is C₂-C₈alkyl optionally substituted with one, two, or three groups independently selected from C₁-C₃alkoxy, C₁-C₃alkylamino, C₁-C₃alkoxyC₂-C₃alkylamino, amino, aryl, halo, C₁-C₃haloalkylamino, C₁-C₃haloalkylcarbonylamino, hydroxy, -NR^{x}R^{y}, and C₃-C₈cycloalkyl, wherein the cycloalkyl is further optionally substituted with one, two, or three groups independently selected from C₁-C₃alkoxy, C₁-C₃alkyl, C₁-C₃alkylamino, C₁-C₃alkoxyC₂-C₃alkylamino, amino, aryl, arylC₁-C₃alkyl, halo, C₁-C₃haloalkyl, C₁-C₃haloalkylamino and hydroxy. In a second embodiment of the second aspect R⁵ is C₂-C₈alkyl optionally substituted with one, two, or three groups independently selected from amino and halo.

In a third embodiment of the second aspect the present disclosure provides a compound of formula (I), or a pharmaceutically acceptable salt thereof, wherein R¹ is selected from hydrogen, C₂-C₄alkenyl, C₁-C₃alkoxyC₁-C₃alkyl, C₁-C₃alkyl, arylC₁-C₃alkyl, and C₃-C₆cycloalkylC₁-C₃alkyl;
R² is selected from hydrogen, C₁-C₃alkyl, C₁-C₃alkylcarbonylamino, and amino;
R³ and R⁴ are independently selected from hydrogen, C₁-C₄alkyl, cyano, C₃-C₆cycloalkyl, halo, and hydroxy; and
R⁵ is C₂-C₈alkyl optionally substituted with one, two, or three groups independently selected from amino and halo.

In a third aspect the present disclosure provides composition comprising a pharmaceutically acceptable amount of a compound of formula (I), or a pharmaceutically acceptable salt thereof, and a pharmaceutically acceptable carrier.

In a fourth aspect the present disclosure provides a method of inhibiting adaptor associated kinase 1 (AAK1) activity, comprising contacting AAK1 with a compound of formula (I), or a pharmaceutically acceptable salt thereof.

In a fifth aspect the present disclosure provides a method for treating or managing a disease or a disorder mediated by AAK1 activity, the method comprising administering to a patient in need thereof a therapeutically effective amount of a compound of formula (I), or a pharmaceutically acceptable salt thereof. In a first embodiment of the fifth aspect the disease or disorder is selected from Alzheimer's disease, bipolar disorder, pain, Parkinson's disease, and schizophrenia. In a second embodiment of the fifth aspect the pain is neuropathic pain. In a third embodiment of the fifth aspect the neuropathic pain is fibromyalgia or peripheral neuropathy.

Other aspects of the present disclosure may include suitable combinations of embodiments disclosed herein.

Yet other aspects and embodiments may be found in the description provided herein.

### BRIEF DESCRIPTION OF THE FIGURES

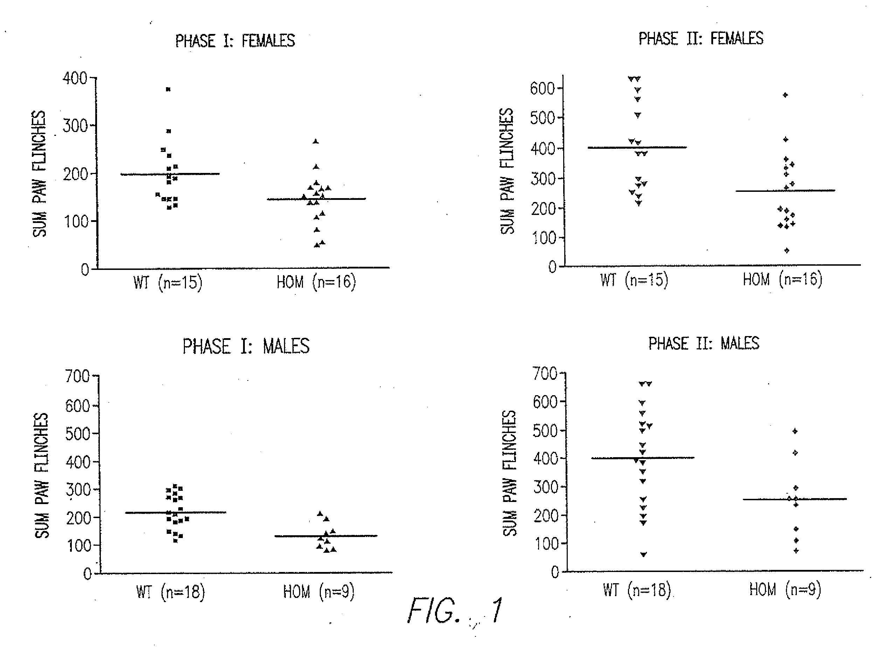

Aspects of the disclosure are illustrated in Figure 1, which shows results obtained from a formalin pain model using AAK1 homozygous (-/-) knockout mice and their wild-type (+/+) littermates. The AAK1 homozygous (-/-) knockout mice show a clear reduction in both acute and tonic pain response as compared to their wild-type (+/+) littermates.

This disclosure is based, in part, on the discovery that AAK1 knockout mice exhibit a high resistance to pain. That discovery prompted research that ultimately led to the discovery of AAK1 inhibitors, compositions comprising them, and methods of their use.

The description of the present disclosure herein should be construed in congruity with the laws and principals of chemical bonding. In some instances it may be necessary to remove a hydrogen atom in order to accommodate a substituent at any given location.

It should be understood that the compounds encompassed by the present disclosure are those that are suitably stable for use as pharmaceutical agent.

It is intended that the definition of any substituent or variable at a particular location in a molecule be independent of its definitions elsewhere in that molecule. For example, when n is 2, each of the two R⁶ groups may be the same or different.

As used in the present specification, the following terms have the meanings indicated:
As used herein, the singular forms "a", "an", and "the" include plural reference unless the context clearly dictates otherwise.

In some instances, the number of carbon atoms in any particular group is denoted before the recitation of the group. For example, the term "C₁₋₆ alkyl" denotes an alkyl group containing one to six carbon atoms. Where these designations exist they supercede all other definitions contained herein.

The term "alkenyl," as used herein, refers to a straight or branched chain group containing at least one carbon-carbon double bond.

The term "alkoxy," as used herein, refers to an alkyl group attached to the parent molecular moiety through an oxygen atom.

The term "alkoxyalkyl," as used herein, refers to an alkyl group substituted with one, two, or three alkoxy groups.

The term "alkoxyalkylamino," as used herein, refers to -NHR wherein R is an alkoxyalkyl group.

The term "alkoxycarbonyl," as used herein, refers to an alkoxy group attached to the parent molecular moiety through a carbonyl group.

The term "alkoxycarbonylamino," as used herein, refers to an -NHR wherein R is an alkoxycarbonyl group.

The term "alkyl," as used herein, refers to a group derived from a straight or branched chain saturated hydrocarbon.

The term "alkylamino," as used herein refers to -NHR, wherein R is an alkyl group.

The term "alkylcarbonyl," as used herein, refers to an alkyl group attached to the parent molecular moiety through a carbonyl group.

The term "alkylcarbonylamino," as used herein, refers to -NHR wherein R is an alkylcarbonyl group.

The term "alkylsulfonyl," as used herein, refers to an alkyl group attached to the parent molecular moiety through a sulfonyl group.

The term "amino," as used herein, refers to -NH₂.

The term "aminocarbonyl," as used herein, refers to -C(O)NH₂.

The term "aryl," as used herein, refers to a phenyl group, or a bicyclic fused ring system wherein one or both of the rings is a phenyl group. Bicyclic fused ring systems consist of a phenyl group fused to a four- to six-membered aromatic or nonaromatic carbocyclic ring. The aryl groups of the present invention can be attached to the parent molecular moiety through any substitutable carbon atom in the group. Representative examples of aryl groups include, but are not limited to, indanyl, indenyl, naphthyl, phenyl, and tetrahydronaphthyl.

The term "arylalkyl," as used herein, refers to an alkyl group substituted with one, two, or three aryl groups.

The term "arylamino," as used herein, refers to -NHR wherein R is an aryl group.

The term "arylcarbonyl," as used herein, refers to an aryl group attached to the parent molecular moiety through a carbonyl group.

The term "arylcarbonylamino," as used herein refers to -NHR wherein R is an arylcarbonyl group.

The term "carbonyl," as used herein, refers to -C(O)-.

The term "cyano," as used herein, refers to -CN.

The term "cycloalkyl," as used herein, refers to a saturated monocyclic hydrocarbon ring system having zero heteroatoms. Representative examples of cycloalkyl groups include, but are not limited to, cyclopropyl, cyclopentyl, and cyclohexyl.

The term "(cycloalkyl)alkyl," as used herein, refers to an alkyl group substituted with one, two, or three cycloalkyl groups.

The term "cycloalkylamino," as used herein, refers to -NHR wherein R is a cycloalkyl group.

The term "cycloalkylcarbonyl," as used herein, refers to a cycloalkyl group attached to the parent molecular moiety through a carbonyl group.

The term "cycloalkylcarbonylamino," as used herein, refers to -NHR wherein R is a cycloalkylcarbonyl group.

The term "cycloalkyloxy," as used herein, refers to a cycloalkyl group attached to the parent molecular moiety through an oxygen atom.

The term "dialkylamino," as used herein, refers to -NR₂ wherein R is alkyl. The two R groups may be the same or different.

The term "dialkylaminocarbonyl," as used herein, refers to -C(O)NR₂, wherein R is alkyl. The two R groups may be the same or different.

The term "halo," as used herein, refers to Br, Cl, F, and/or I.

The term "haloalkoxy," as used herein, refers to a haloalkyl group attached to the parent molecular moiety through an oxygen atom.

The term "haloalkyl," as used herein, refers to an alkyl group substituted by one, two, three, or four halogen atoms.

The term "haloalkylamino," as used herein, refers to -NHR wherein R is a haloalkyl group.

The term "haloalkylcarbonyl," as used herein, refers to a haloalkyl group attached to the parent molecular moiety through a carbonyl group.

The term "haloalkylcarbonylamino," as used herein, refers to -NHR wherein R is a haloalkylcarbonyl group.

The term "heteroaryl," as used herein, refers to a five- or six-membered aromatic ring containing one or two heteroatoms independently selected from nitrogen, oxygen, and sulfur.

The term "hydroxy," as used herein, refers to -OH.

The term "hydroxyalkyl," as used herein, refers to an alkyl group substituted with one, two, or three hydroxy groups.

The term "phenylalkylamino" as used herein, refers to -NHR, wherein R is a phenylalkylgroup.

The term "sulfonyl," as used herein, refers to -SO₂.

Asymmetric centers may exist in the compounds of the present disclosure. It should be understood that the disclosure encompasses all stereochemical isomeric forms, or mixtures thereof, which possess the ability to inhibit AAK1. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, or direct separation of enantiomers on chiral chromatographic columns. Starting compounds of particular stereochemistry are either commercially available or can be made and resolved by techniques known in the art.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

The term "compounds of the present disclosure", and equivalent expressions, are meant to embrace compounds of formula (I), and pharmaceutically acceptable enantiomers, diastereomers, and salts thereof. Similarly, references to intermediates are meant to embrace their salts where the context so permits.

The present disclosure is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the disclosure can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use. The salts can be prepared during the final isolation and purification of the compounds or separately by reacting a suitable nitrogen atom with a suitable acid. Representative acid addition salts include acetate, adipate, alginate, citrate, aspartate, benzoate, benzenesulfonate, bisulfate, butyrate, camphorate, camphorsulfonate; digluconate, dihydrobromide, diydrochloride, dihydroiodide, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, and N,N'-dibenzylethylenediamine. Other representative organic amines useful for the formation of base addition salts include ethylenediamine, ethanolamine, diethanolamine, piperidine, and piperazine.
One embodiment of this disclosure encompasses methods of inhibiting adaptor associated kinase 1 (AAK1), both *in vitro* and *in vivo,* which comprise contacting AAK1 with a compound of formula I or a pharmaceutically acceptable salt thereof.

When it is possible that, for use in therapy, therapeutically effective amounts of a compound of formula (I), as well as pharmaceutically acceptable salts thereof, may be administered as the raw chemical, it is possible to present the active ingredient as a pharmaceutical composition. Accordingly, the disclosure further provides pharmaceutical compositions, which include therapeutically effective amounts of compounds of formula (I) or pharmaceutically acceptable salts thereof, and one or more pharmaceutically acceptable carriers, diluents, or excipients. Unless otherwise indicated, a "therapeutically effective amount" of a compound is an amount sufficient to provide a therapeutic benefit in the treatment or management of a disease or condition, or to delay or minimize one or more symptoms associated with the disease or condition. The term "therapeutically effective amount" can encompass an amount that improves overall therapy, reduces or avoids symptoms or causes of a disease or condition, or enhances the therapeutic efficacy of another therapeutic agent.

The term "therapeutically effective amount," as used herein, refers to an amount of a compound or compounds sufficient to provide a therapeutic benefit in the treatment or management of a disease or condition, or to delay or minimize one or more symptoms associated with the disease or condition. A "therapeutically effective amount" of a compound means an amount of therapeutic agent, alone or in combination with other therapies, that provides a therapeutic benefit in the treatment or management of the disease or condition. The term "therapeutically effective amount" can encompass an amount that improves overall therapy, reduces or avoids symptoms or causes of a disease or condition, or enhances the therapeutic efficacy of another therapeutic agent. When applied to an individual active ingredient, administered alone, the term refers to that ingredient alone. When applied to a combination, the term refers to combined amounts of the active ingredients that result in the therapeutic effect, whether administered in combination, serially, or simultaneously. The compounds of formula (I) and pharmaceutically acceptable salts thereof, are as described above. The carrier(s), diluent(s), or excipient(s) must be acceptable in the sense of being compatible with the other ingredients of the formulation and not deleterious to the recipient thereof. In accordance with another aspect of the present disclosure there is also provided a process for the preparation of a pharmaceutical formulation including admixing a compound of formula (I), or a pharmaceutically acceptable salt thereof, with one or more pharmaceutically acceptable carriers, diluents, or excipients. The term "pharmaceutically acceptable," as used herein, refers to those compounds, materials, compositions, and/or dosage forms which are, within the scope of sound medical judgment, suitable for use in contact with the tissues of patients without excessive toxicity, irritation, allergic response, or other problem or complication commensurate with a reasonable benefit/risk ratio, and are effective for their intended use.

Pharmaceutical formulations may be presented in unit dose forms containing a predetermined amount of active ingredient per unit dose. Dosage levels of between about 0.01 and about 250 milligram per kilogram ("mg/kg") body weight per day, preferably between about 0.05 and about 100 mg/kg body weight per day of the compounds of the present disclosure are typical in a monotherapy for the prevention and treatment of disease. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Treatment may be initiated with small dosages substantially less than the optimum dose of the compound. Thereafter, the dosage is increased by small increments until the optimum effect under the circumstances is reached. In general, the compound is most desirably administered at a concentration level that will generally afford effective results without causing any harmful or deleterious side effects.

When the compositions of this disclosure comprise a combination of a compound of the present disclosure and one or more additional therapeutic or prophylactic agent, both the compound and the additional agent are usually present at dosage levels of between about 10 to 150%, and more preferably between about 10 and 80% of the dosage normally administered in a monotherapy regimen.

Compounds of the disclosure may be administered in combination with one or more additional therapeutic or prophylactic agents. For example, when used for the treatment of pain, possible additional agents include immunosuppressive agents, anti-inflammatory agents, and/or other agents used in the treatment of pain.

Immunosuppressants suitable for use in the methods and compositions of this disclosure include those known in the art. Examples include aminopterin, azathioprine, cyclosporin A, D-penicillamine, gold salts, hydroxychloroquine, leflunomide, methotrexate, minocycline, rapamycin, sulfasalazine, tacrolimus (FK506), and pharmaceutically acceptable salts thereof. A particular immunosuppressant is methotrexate.

Additional examples of immunosuppressants include anti-TNF antibodies, such as adalimumab, certolizumab pegol, etanercept, and infliximab. Others include interleukin-1 blockers, such as anakinra. Others include anti-B cell (CD20) antibodies, such as rituximab. Others include T cell activation blockers, such as abatacept.

Other immunosuppressants include inosine monophosphate dehydrogenase inhibitors, such as mycophenolate mofetil (CellCept®) and mycophenolic acid (Myfortic®).

Anti-inflammatory drugs suitable for use in the methods and compositions of this disclosure include those known in the art. Examples include glucocorticoids and NSAIDs. Examples of glucocorticoids include aldosterone, beclometasone, betamethasone, cortisone, deoxycorticosterone, dexamethasone, fludrocortisones, hydrocortisone, methylprednisolone, prednisolone, prednisone, triamcinolone, and pharmaceutically acceptable salts thereof.

Examples of NSAID include salicylates (*e.g.,* aspirin, amoxiprin, benorilate, choline magnesium salicylate, diflunisal, faislamine, methyl salicylate, magnesium salicylate, salicyl salicylate, and pharmaceutically acceptable salts thereof), arylalkanoic acids (*e.g.,* diclofenac, aceclofenac, acemetacin, bromfenac, etodolac, indometacin, nabumetone, sulindac, tolmetin, and pharmaceutically acceptable salts thereof), arylpropionic acids (*e.g.,* ibuprofen, carprofen, fenbufen, fenoprofen, flurbiprofen, ketoprofen, ketorolac, loxoprofen, naproxen, oxaprozin, tiaprofenic acid, suprofen, and pharmaceutically acceptable salts thereof), arylanthranilic acids (*e.g.,* meclofenamic acid, mefenamic acid, and pharmaceutically acceptable salts thereof), pyrazolidine derivatives (*e.g.,* azapropazone, metamizole, oxyphenbutazone, phenylbutazone, sulfinprazone, and pharmaceutically acceptable salts thereof), oxicams (*e.g.,* lornoxicam, meloxicam, piroxicam, tenoxicam, and pharmaceutically acceptable salts thereof), COX-2 inhibitors (*e.g.,* celecoxib, etoricoxib, lumiracoxib, parecoxib, rofecoxib, valdecoxib, and pharmaceutically acceptable salts thereof), and sulphonanilides (*e.g.,* nimesulide and pharmaceutically acceptable salts thereof).

Other agents used in the treatment of pain (including but not limited to neuropathic and inflammatory pain) include, but are not limited to, agents such as pregabalin, lidocaine, duloxetine, gabapentin, carbamazepine, capsaicin, and other serotonin/norepinephrine/dopamine reuptake inhibitors, and opiates (such as oxycontin, morphine, and codeine).

In the treatment of pain caused by a known disease or condition, such as diabetes, infection (*e.g.,* herpes zoster or HIV infection), or cancer, compounds of the disclosure may be administered in combination with one or more additional therapeutic or prophylactic agents directed at the underlying disease or condition. For example, when used to treat diabetic neuropathy, compounds of the disclosure may be administered in combination with one or more anti-diabetic agents, antihyperglycemic agents, hypolipidemic/lipid lowering agents, anti-obesity agents, antihypertensive agents and appetite suppressants. Examples of anti-diabetic agents include biguanides (*e.g.,* metformin, phenformin), glucosidase inhibitors (*e.g.,* acarbose, miglitol), insulins (including insulin secretagogues and insulin sensitizers), meglitinides (*e.g.,* repaglinide), sulfonylureas (*e.g.,* glimepiride, glyburide, gliclazide, chlorpropamide, and glipizide), biguanide/glyburide combinations (*e.g.,* Glucovance), thiazolidinediones (*e.g.,* troglitazone, rosiglitazone, and pioglitazone), PPAR-alpha agonists, PPAR-gamma agonists, PPAR alpha/gamma dual agonists, glycogen phosphorylase inhibitors, inhibitors of fatty acid binding protein (aP2), glucagon-like peptide-1 (GLP-1) or other agonists of the GLP-1 receptor, dipeptidyl peptidase IV (DPP4) inhibitors, and sodium-glucose co-transporter 2 (SGLT2) inhibitors (*e.g.,* dapagliflozin, canagliflozin, and LX-4211).

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s). Oral administration or administration by injection are preferred.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of formula (I), and pharmaceutically acceptable salts thereof, can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of formula (I) and pharmaceutically acceptable salts thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research 1986, 3(6), 318.

Pharmaceutical formulations adapted for topical administration may be formulated as ointments, creams, suspensions, lotions, powders, solutions, pastes, gels, sprays, aerosols, or oils.

Pharmaceutical formulations adapted for rectal administration may be presented as suppositories or as enemas.

Pharmaceutical formulations adapted for nasal administration wherein the carrier is a solid include a course powder having a particle size for example in the range 20 to 500 microns which is administered in the manner in which snuff is taken, *i.e.,* by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

The term "patient" includes both human and other mammals.
Unless otherwise indicated, the terms "manage," "managing", and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

The term "treating" refers to: (i) preventing a disease, disorder or condition from occurring in a patient that may be predisposed to the disease, disorder, and/or condition but has not yet been diagnosed as having it; (ii) inhibiting the disease, disorder, or condition, i.e., arresting its development; and (iii) relieving the disease, disorder, or condition, i.e., causing regression of the disease, disorder, and/or condition.

This disclosure is intended to encompass compounds having Formula (I) when prepared by synthetic processes or by metabolic processes including those occurring in the human or animal body (*in vivo*) or processes occurring *in vitro.*

The abbreviations used in the present application, including particularly in the illustrative schemes and examples which follow, are well-known to those skilled in the art. Some of the abbreviations used are as follows: RT or rt or r.t. for room temperature or retention time (context will dictate); *t*_{R} for retention time; h or hr or hrs for hours; min or mins for minutes; MeOH for methanol; EtOH for ethanol; EtOAc or EtOAC for ethyl acetate; OAc for acetate; DCM for dichloromethane; DMA for *N,N-*dimethylacetamide; DMF for N,N-dimethylformamide; NMP for N-methylpyrrolidinone; MeCN or ACN for acetonitrile; AcCl for acetyl chloride; THF for tetrahydrofuran; DMSO for dimethylsulfoxide; MeOD for CD₃OD; m-CPBA for meta-chloroperoxybenzoic acid; DCM for dichloromethane; Me for methyl; Et for ethyl; Ac for acetyl; Ph for phenyl; BOC or Boc for *tert*-butoxycarbonyl; EDC for 1-ethyl-3-(3-dimethylaminopropyl)carbodiimide; DBU for 1,8-diazabicycloundec-7-ene; HOBT or HOBt for hydroxybenzotriazole; NCS for N-chlorosuccinimide; TEA or Et₃N for triethylamine; DIPEA, DIEA, or *i*-Pr₂NEt for diisopropylethylamine; DEA for diethylamine; DAST for diethylaminosulfur trifluoride; NBS for *N-*bromosuccinimide; TOSMIC for tosylmethyl isocyanide; DIBAL for diisobutylaluminum hydride; LDA for lithium diisopropylamide; TFA for trifluoroacetic acid; dppf or DPPF for 1,1'-bis(diphenylphosphino)ferrocene; dba for dibenzylidiendiacetone; n-BuLi for n-butyllithium; LHMDS or LiHMDS for lithium hexamethyldisilazide; TMS for trimethylsilyl; and PMB-Cl for para-methoxybenzyl chloride.

### EXAMPLES

The present disclosure will now be described in connection with certain embodiments which are not intended to limit its scope. On the contrary, the present disclosure covers all alternatives, modifications, and equivalents as can be included within the scope of the claims. Thus, the following examples, which include specific embodiments, will illustrate one practice of the present disclosure, it being understood that the examples are for the purposes of illustration of certain embodiments and are presented to provide what is believed to be the most useful and readily understood description of its procedures and conceptual aspects.

The compounds of the present disclosure may be prepared using the reactions and techniques described in this section as well as other synthetic methods known to those of ordinary skill in the art. The reactions are performed in solvents appropriate to the reagents and materials employed and suitable for the transformation being effected. Also, in the description of the synthetic methods described below, it is to be understood that all proposed reaction conditions, including choice of solvents, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents which are compatible with the reaction conditions will be readily apparent to one skilled in the art and alternate methods must then be used.

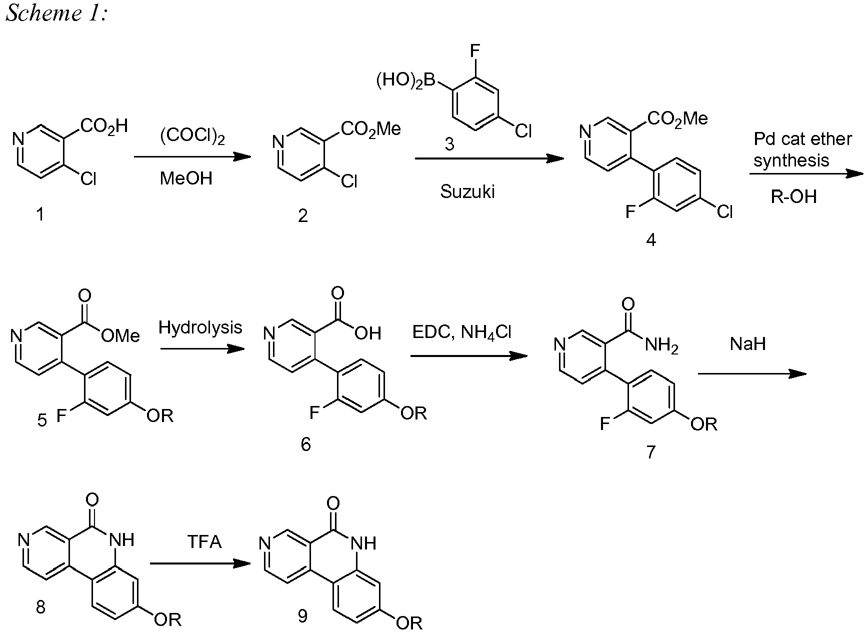

The compound of formula **9** is prepared by method outlined in Scheme 1. 4-Chloronicotinic acid can be subjected to esterification using standard conditions such as treatment with oxalyl chloride and methanol. The ester **2,** so obtained can be subjected to Suzuki cross coupling reaction with an appropriate coupling partner such as fluoroboronic acid **3,** under standard Suzuki conditions employing a base such as cesium carbonate and a catalyst such as Pd(PPh₃)₄ as described by Zhang, Lei et. al. (Journal of Medicinal Chemistry, 2011, 54, 1724 - 1739). The biaryl ester **4,** can then be subjected to palladium catalyzed ether synthesis including reaction conditions familiar to those skilled in the art following procedures such as those described by Gowrisankar, et. al. (J. Am. Chem. Soc. 2010, 132, 11592-11598). The reactions can be performed using appropriately protected, racemic or optically amino alcohols to afford racemic or optically pure ethers. The biaryl ester represented by formula **5,** can be subjected to hydrolysis to yield corresponding carboxylic acid **6** under standard saponification conditions using a base such as lithium hydroxide in a solvent such as water as described in Protective Groups in Organic Synthesis (Greene, Wuts; 3rd ed., 1999, John Wiley & Sons, Inc.). The acid **6** can be converted to amide **7** by using standard coupling conditions such as EDC, HOBt and ammonium chloride. The amide **7,** upon treatment with a hydride source such as sodium hydride in a solvent such as THF under inert atmosphere can afford the constrained lactam, **8.** The constrained lactam ether analog represented by **8** can be subjected to deprotection of the side chain amino group using appropriate conditions as described in Protective Groups in Organic Synthesis (Greene, Wuts; 3rd ed., 1999, John Wiley & Sons, Inc.) to yield compounds represented by formula **9.**

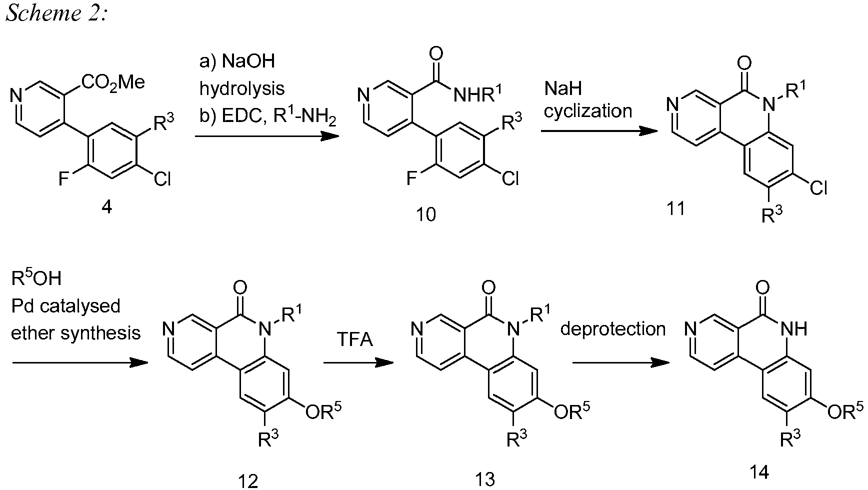

The compounds represented by formula **13** and **14** are prepared by methods outlined in Scheme 2. The biaryl ester **4,** prepared as described in Scheme 1, can be subjected to hydrolysis under standard saponification conditions using a base such as lithium or sodium hydroxide in a solvent such as water as described in Protective Groups in Organic Synthesis (Greene, Wuts; 3rd ed., 1999, John Wiley & Sons, Inc.) to yield corresponding carboxylic acids, which upon treatment with standard amide coupling conditions such as EDC, HOBt and ammonium chloride or substituted amine can be converted to amides **10.** Cyclization of amides **10** can be affected by treatment with a hydride source such as sodium hydride in a solvent such as THF under inert atmosphere to afford constrained lactam chloro cores **11.** Chloro cores **11** can be subjected to palladium catalyzed ether synthesis using reaction conditions familiar to those skilled in the art following procedures such as those described by Gowrisankar, et. al. (J. Am. Chem. Soc. 2010, 132, 11592-11598) to afford compounds represented by formula **12.** The reactions can be performed using appropriately protected, racemic or optically pure (*S*) or (*R*)-aminoalcohols to afford racemic or optically pure ethers **12.** The ether analogs can be subjected to deprotection of the side chain amino group using appropriate conditions as described in Protective Groups in Organic Synthesis (Greene, Wuts; 3rd ed., 1999, John Wiley & Sons, Inc.) to yield compounds of formula **13.** In cases where R² is serving as a protecting group, this group can be removed using appropriate conditions as described in Protective Groups in Organic Synthesis (Greene, Wuts; 3rd ed., 1999, John Wiley & Sons, Inc.).

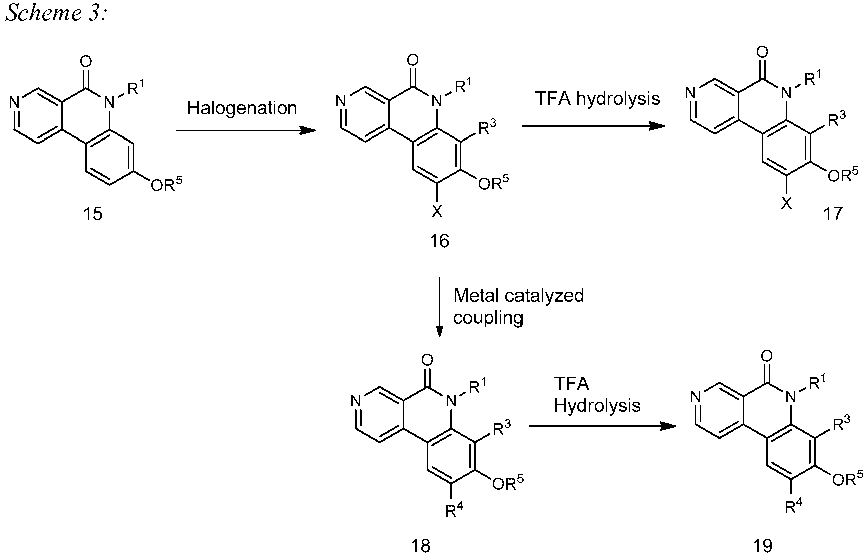

The compounds represented by formula **17** and **19** are prepared by methods outlined in Scheme 3. The constrained lactam ether, **15,** synthesized as shown in Scheme 2, can be subjected to halogenation employing N-halosuccinimide in polar aprotic solvents such as acetonitrile in the dark to yield mono-halo (X = halo, R³ = H) or dihalo-substituted (R³ = X = halo) ethers **16.** The side chain of ethers **16** can be subjected to deprotection using appropriate conditions as described in Protective Groups in Organic Synthesis (Greene, Wuts; 3rd ed., 1999, John Wiley & Sons, Inc.) to yield compounds of formula **17.** Alternatively, compound **16** can be subjected to a palladium coupling such as Suzuki cross coupling reaction with an appropriate coupling partner such as trimethylboroxine or cyclopropyl boronic acid under standard Suzuki conditions employing a base such as cesium carbonate and a catalyst such as Pd(PPh₃)₄ as described by Zhang, Lei et. al. (Journal of Medicinal Chemistry, 2011, 54, 1724 - 1739), or to a copper catalyzed cyanation reaction as described by Miroslav et.al. (Collection of Czechoslovak Chemical Communications, 1983, 48, 1765-1773) to install R⁴ of the compounds represented by formula **18.** Lactam **16** can be subjected to a copper catalyzed hydroxylation reaction using conditions such as those described by Punniyamurthy et. al. (Synthesis, 2010, 4268 - 4272) to provide **18** wherein R⁴ = OH. The ether analogs **18** can be subjected to deprotection of the side chain amino group using appropriate conditions as described *in* Protective Groups in Organic Synthesis (Greene, Wuts; 3rd ed., 1999, John Wiley & Sons, Inc.) to yield compounds of formula **19.**

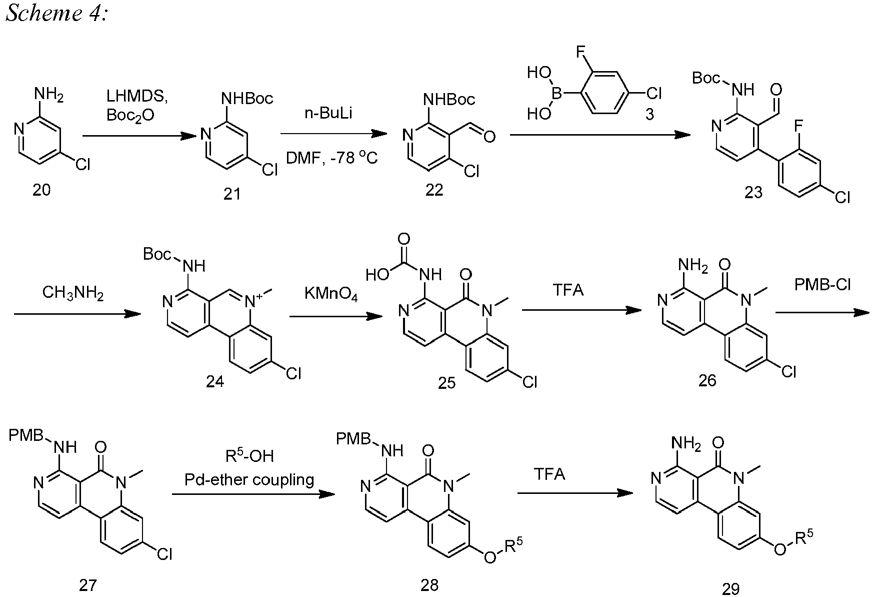

The compounds represented by formula **29** can be prepared as shown in Scheme 4. Boc-protection of **20** using base such as LiHMDS and Boc anhydride led to **21.** Directed *ortho*-metallation followed by treatment with dimethylformamide can furnish the pyridine aldehyde derivative **22** using methods such as those described by Charles et. al. (J. Med.Chem., 2010, 53, 3330 - 3348). The aldehyde can then be subjected to Suzuki cross coupling reaction with an appropriate coupling partner such as fluoroboronic acid **3,** under standard Suzuki conditions employing a base such as cesium carbonate and a catalyst such as Pd(PPh₃)₄ as described by Zhang, Lei et. al. (Journal of Medicinal Chemistry, 2011, 54, 1724 - 1739) to give biaryl aldehyde **23.** Treatment of biaryl aldehyde **23** with methyl amine can provide cyclic iminium fluoride **24,** which upon oxidation with reagents such as KMnO₄ give lactam **25.** Treatment of lactam **25** with trifluoroacetic acid can afford **26,** which can be protected with a group such as *p*-methoxy benzyl using appropriate conditions as described in Protective Groups in Organic Synthesis (Greene, Wuts; 3rd ed., 1999, John Wiley & Sons, Inc.) can afford lactam **27.** Lactam core **27** can be subjected to palladium catalyzed ether synthesis using reaction conditions familiar to those skilled in the art following procedures such as those described by Gowrisankar, et. al. (J. Am. Chem. Soc. 2010, 132, 11592-11598) to afford compound represented by formula **28.** The ether analog can be subjected to global deprotection of the side chain amino group using appropriate conditions as described in Protective Groups in Organic Synthesis (Greene, Wuts; 3rd ed., 1999, John Wiley & Sons, Inc.) to yield compound **29.**

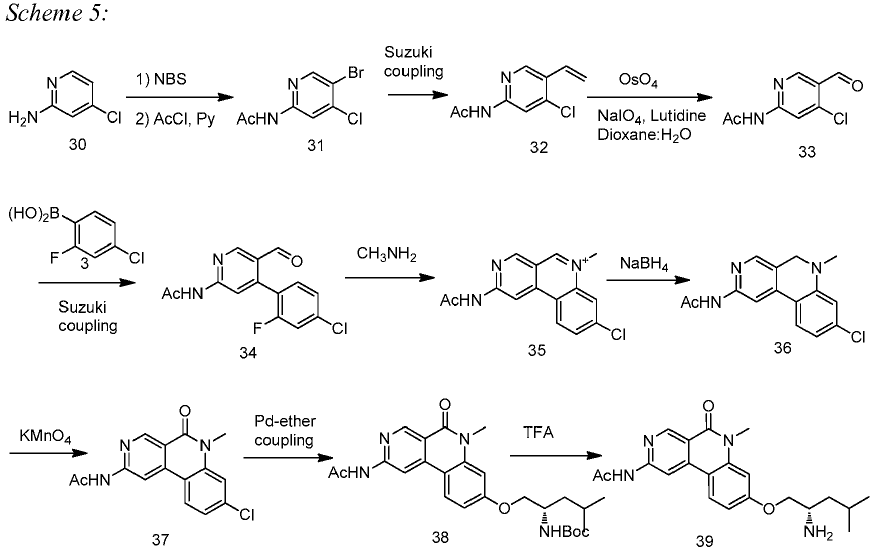

The compounds represented by the formula **39** can be prepared as shown in Scheme 5. Bromination of **30** with NBS followed by acylation with acetyl chloride and pyridine can furnish bromide **31.** Suzuki cross coupling of the bromide **31** with vinyl boronic acid anhydride pyridine complex under standard Suzuki conditions employing base such as sodium carbonate and a catalyst such as Pd(PPh₃)₄ as described by Zhang, Lei et. al. (Journal of Medicinal Chemistry, 2011, 54, 1724 - 1739) can furnish vinyl pyridine **32.** Vinyl pyridine **32** can be oxidized using osmium tetroxide and sodium metaperiodate to yield corresponding aldehyde **33,** which can then be subjected to Suzuki cross coupling reaction with an appropriate coupling partner such as fluoroboronic acid **3,** under standard Suzuki conditions employing a base such as cesium carbonate and a catalyst such as Pd(PPh₃)₄ as described by Zhang, Lei et. al. (Journal of Medicinal Chemistry, 2011, 54, 1724 - 1739) to give biaryl aldehyde **34.** Treatment of biaryl aldehyde **34** with methyl amine can lead to the cyclic iminium fluoride **35** which can be reduced to the corresponding constrained piperidine **36** by using an agent such as sodium borohydride. Constrained piperidine **36** upon oxidation with KMnO₄ can give lactam **37.** Lactam core **37** can be subjected to palladium catalyzed ether synthesis using reaction conditions familiar to those skilled in the art following procedures such as those described by Gowrisankar, et. al. (J. Am. Chem. Soc. 2010, 132, 11592-11598) to afford compound represented by formula **38.** The ether analog can be subjected to deprotection of the side chain amino group using appropriate conditions as described in Protective Groups in Organic Synthesis (Greene, Wuts; 3rd ed., 1999, John Wiley & Sons, Inc.) to yield compounds **39.**

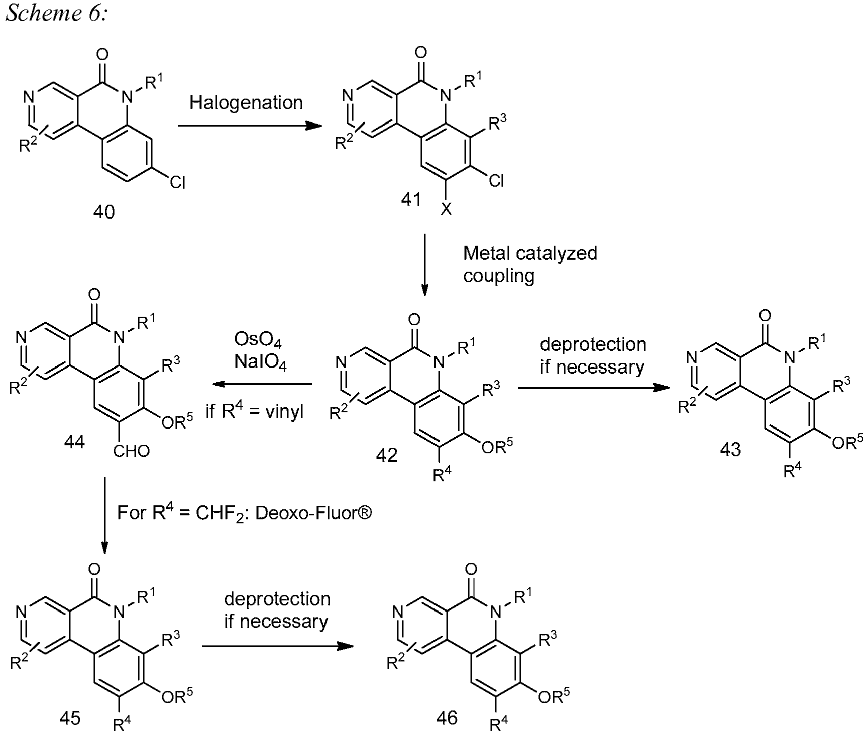

The compounds represented by formula **42 - 46** are prepared by methods outlined in Scheme 6. The constrained lactam ether, **40,** synthesized as shown in Scheme 2, can be subjected to halogenation by employing *N*-halosuccinimide in polar aprotic solvents such as acetonitrile in the dark to yield mono-halo (X = halo, R³ = H) or dihalo-substituted (R³ = X = halo) ethers **41.** Compound **41** can be subjected to a palladium coupling such as a Suzuki cross coupling reaction with an appropriate coupling partner such as trimethylboroxine, vinylboronic acid or cyclopropylboronic acid under standard Suzuki conditions employing a base such as cesium carbonate and a catalyst such as Pd(PPh₃)₄ as described by Zhang, Lei et. al. (Journal of Medicinal Chemistry, 2011, 54, 1724 - 1739), or to a copper catalyzed cyanation reaction as described by Miroslav et.al. (Collection of Czechoslovak Chemical Communications, 1983, 48, 1765-1773) to install R⁴ of the compounds represented by formula **42.** Lactam **41** can alternatively be subjected to a copper catalyzed hydroxylation reaction using conditions such as those described by Punniyamurthy et. al. (Synthesis, 2010, 4268 - 4272) to provide **42** wherein R⁴ = OH. In the cases of intermediates **42** where R⁴ = vinyl, oxidative cleavage of the vinyl group with reagents such as osmium tetroxide and sodium periodate can provide aldehydes **44.** The aldehyde can be converted to the difluoromethyl analog **45** by treatment with Deoxo-Fluor®. If R⁵ contains an amine group or another functional group that is protected, the protecting group in analogs **42** and **45** is removed by treating the substrate with the appropriate reagents as described in Protective Groups in Organic Synthesis (Greene, Wuts; 3rd ed., 1999, John Wiley & Sons, Inc.) to yield compounds of formula **43** and **46,** respectively.

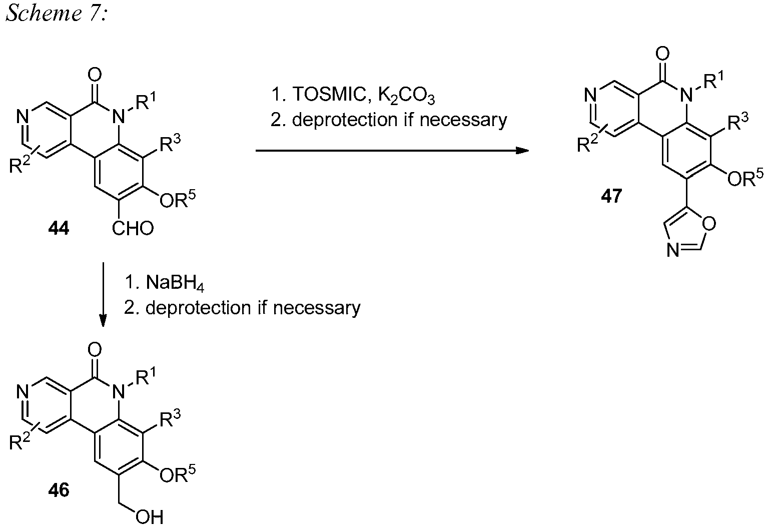

The compounds represented by formula **47** and **48** are prepared by methods outlined in Scheme 7. The aldehyde, **44,** synthesized as shown in Scheme 6, can be subjected to TOSMIC and a base such as potassium carbonate to afford oxazoles **47.** Alternatively, aldehydes **44** can be subjected to a reduction with reagents such as sodium borohydride or lithium borohydride in a solvent such as EtOH or MeOH to yield compounds represented by formula **48.** If R⁵ contains an amine group or another functional group that is protected, the protecting group is removed by treating the substrate with the appropriate reagents as described in Protective Groups in Organic Synthesis (Greene, Wuts; 3rd ed., 1999, John Wiley & Sons, Inc.) to provide deprotected compounds of the formula **47** and **48.**

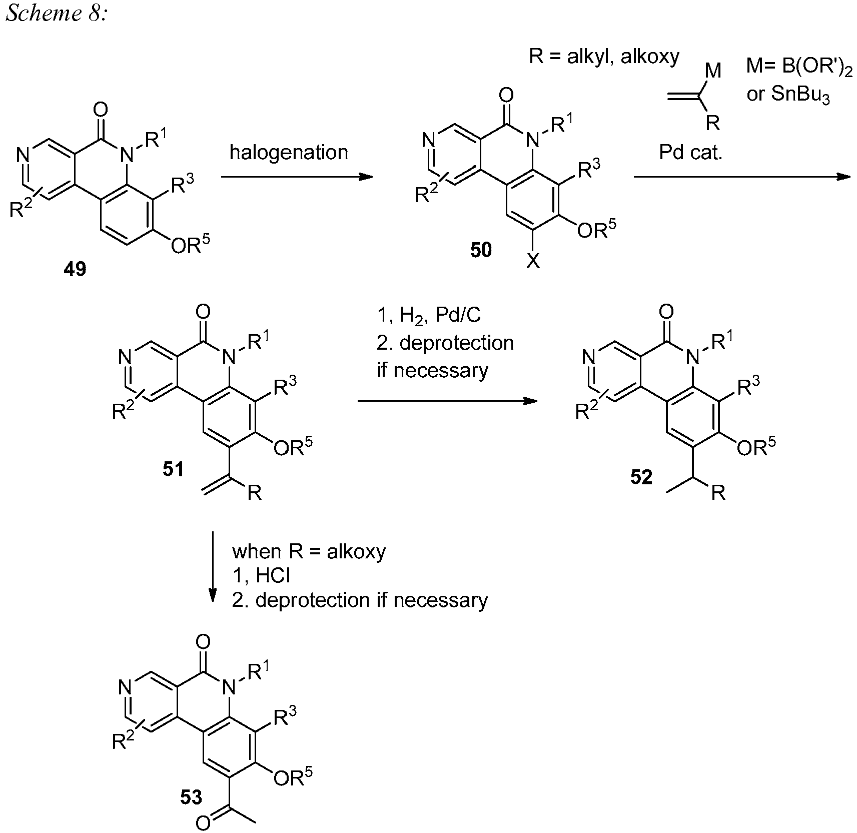

The compounds represented by formula **52 - 53** are prepared by methods outlined in Scheme 8. The constrained lactam ether, **49,** synthesized as shown in the above schemes, can be subjected to halogenation employing N-halosuccinimide in polar aprotic solvents such as acetonitrile in the dark to yield halo ethers **50.** Compound **50** can be subjected to a palladium coupling such as Suzuki cross coupling reaction with an appropriate coupling partner such as a vinylboronic acid under standard Suzuki conditions employing a base such as cesium carbonate and a catalyst such as Pd(PPh₃)₄ as described by Zhang, Lei et. al. (Journal of Medicinal Chemistry, 2011, 54, 1724 - 1739), or to a Stille coupling with an appropriate coupling partner such as a tributyl(1-ethoxyvinyl)stannane or under standard Stille conditions employing a catalyst such as tris(dibenzylidineacetone)dipalladium and a ligand such as DPPF at elevated temperatures to afford compounds represented by formula **51.** The vinyl group of **51** can be subjected to a hydrogenation in the presence of palladium on carbon to provide compounds represented by formula **52.** Alternatively, of R is an alkoxy group, **51** can be subjected to hydrolysis with an aqueous acid such as HCl to provide ketone compounds represented by formula **53.** If R¹ contains an amine group or another functional group that is protected, the protecting group is removed by treating the substrate with the appropriate reagents as described in Protective Groups in Organic Synthesis (Greene, Wuts; 3rd ed., 1999, John Wiley & Sons, Inc.) to provide deprotected compounds of the formula **52** and **53.**

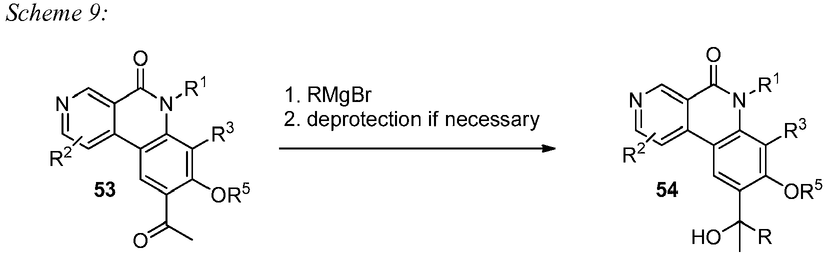

The compounds represented by formula **54** are prepared by methods outlined in Scheme 9. The constrained lactam ether, **53,** synthesized as shown in Scheme 8, can be treated with a Grignard reagent in a solvent such as diethyl ether or THF to provide compounds represented by formula **54.** If R⁵ contains an amine group or another functional group that is protected, the protecting group is removed by treating the substrate with the appropriate reagents as described in Protective Groups in Organic Synthesis (Greene, Wuts; 3rd ed., 1999, John Wiley & Sons, Inc.) to provide deprotected compounds of the formula **54.**

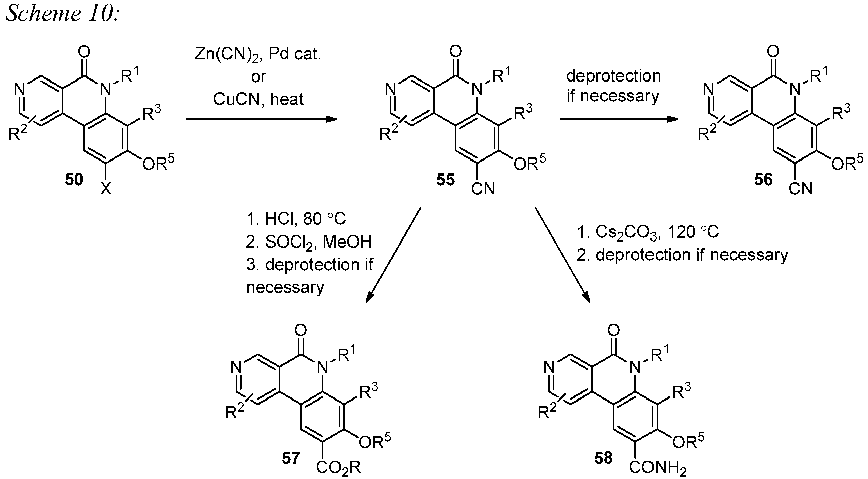

The compounds represented by formula **56 - 58** are prepared by methods outlined in Scheme 10. The constrained lactam ether, **50,** synthesized as shown in Scheme 8, can be subjected to cyanation employing a standard Negishi coupling using Zn(CN)₂ and a catalyst such as Pd(PPh₃)₄ in solvent such as DMF, or using CuCN at temperatures ranging from 50 to 120 °C to yield compounds represented by formula **55.** Nitrile compound **55** can be hydrolyzed with aqueous HCl at elevated temperatures followed by ester formation with thionyl or oxalyl chloride followed by quenching with an alcohol to afford compounds represented by formula **57.** Alternatively, nitrile compound **55** can be hydrolyzed to the amide by employing a base such as cesium carbonate in a solvent such as DMF at temperatures ranging from 100 -120 °C to provide compounds represented by formula **58.** If R⁵ contains an amine group or another functional group that is protected, the protecting group is removed by treating the substrate with the appropriate reagents as described in Protective Groups in Organic Synthesis (Greene, Wuts; 3rd ed., 1999, John Wiley & Sons, Inc.) to provide compounds of formula **56 - 58.**

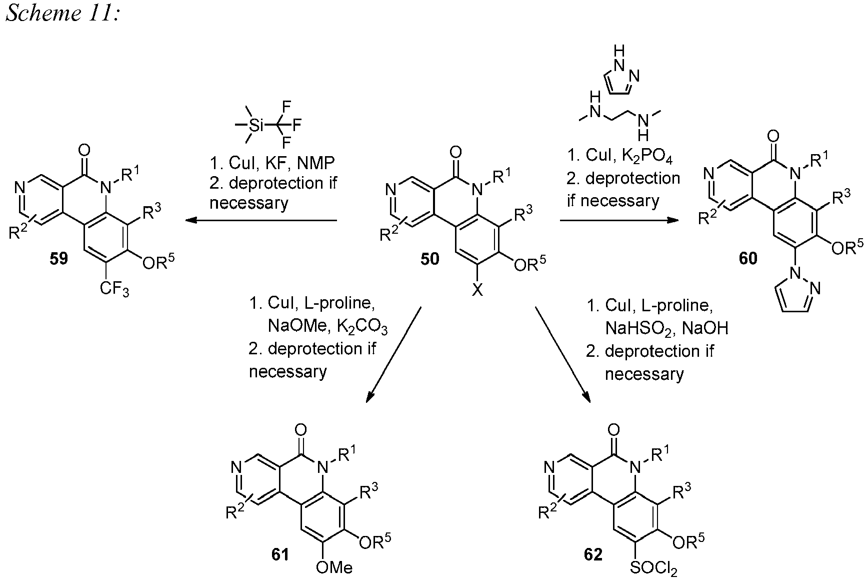

The compounds represented by formula **60 - 62** are prepared by methods outlined in Scheme 11. The constrained lactam ether, **50,** synthesized as shown in Scheme 8, can be subjected to copper catalyzed trifluoromethylation by employing copper(I) iodide and trimethyl(trifluoromethyl)silane in a solvent such as NMP at high temperatures to afford compounds represented by formula **59.** Alternatively, halo compound **50** can be subjected to copper-catalyzed coupling employing copper(I) iodide, 1*H*-pyrazole, *N*1, *N*2-dimethylethane-1,2-diamine, and a base such as potassium phosphate in a solvent such as DMF and 1,4-dioxane at high temperatures to afford compounds represented by formula **60.** Alternatively, halo compound **50** can be subjected to copper catalyzed methoxylation employing copper(I) iodide, L-proline, sodium methoxide, and a base such as potassium carbonate in a solvent such as DMSO at elevated temperatures to afford compounds represented by formula **61.** Alternatively, halo compound **50** can be subjected to copper catalyzed sulfoxylation employing copper(I) iodide, L-proline, methanesulfinic acid sodium salt, and a base such as sodium hydroxide in a solvent such as DMSO at elevated temperatures to afford compounds represented by formula **62.** If R⁵ contains an amine group or another functional group that is protected, the protecting group is removed by treating the substrate with the appropriate reagents as described in Protective Groups in Organic Synthesis (Greene, Wuts; 3rd ed., 1999, John Wiley & Sons, Inc.) to provide compounds of formula **60 - 62.**

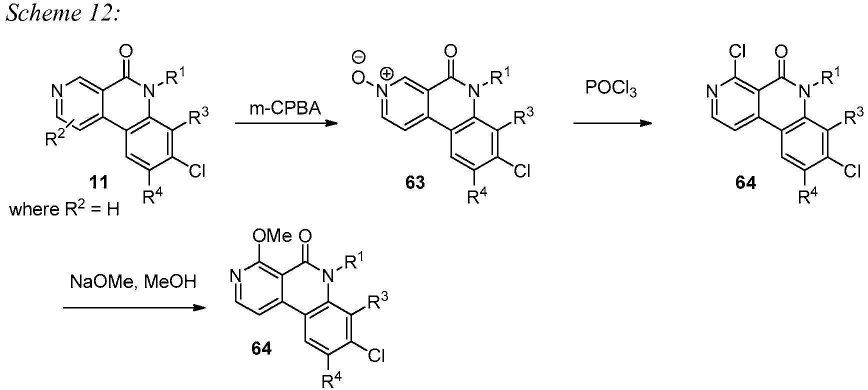

Intermediates represented by formula **65** are prepared by methods outlined in Scheme 12. The biaryl amide **11,** prepared as described in Scheme 2, can be subjected to oxidation with *m*-CPBA in a solvent such as DCM to afford compounds represented by formula **63.** Intermediates **63** can be treated with POCl₃ followed by sodium methoxide in methanol to afford intermediates represented by formula **65.**

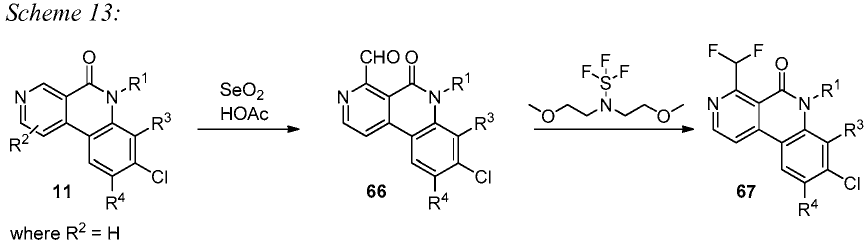

The intermediates represented by formula **67** are prepared by methods outlined in Scheme 13. The biaryl amides **11,** prepared as described in Scheme 2, can be subjected to oxidation with selenium dioxide in a solvent such as AcOH to afford compounds represented by formula **66.** Intermediates **66** can be treated with bis-(2-methoxyethyl)aminosulfur trifluoride in a solvent such as DCM to afford intermediates represented by formula **67.**

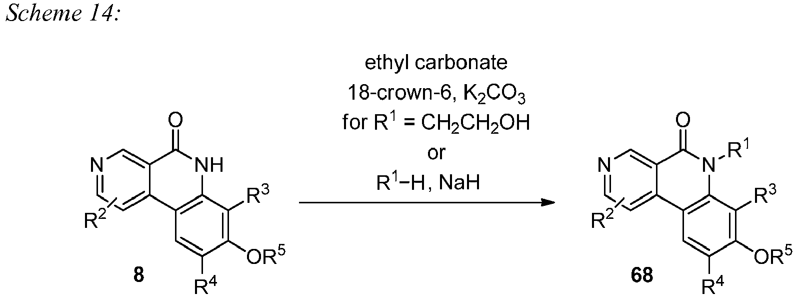

The intermediates represented by formula **68** are prepared by methods outlined in Scheme 14. Biaryl amides **8,** prepared as described in Scheme 1, can be subjected to alkylation with an alkyl halide in the presence of a base such as NaH or potassium carbonate in a solvent such as DMF, or in the case of R¹ = CH₂CH₂OH, the amide can be subjected to ethyl carbonate in the presence of 18-crown-6 and a base such as potassium carbonate to afford intermediates represented by formula **68.**

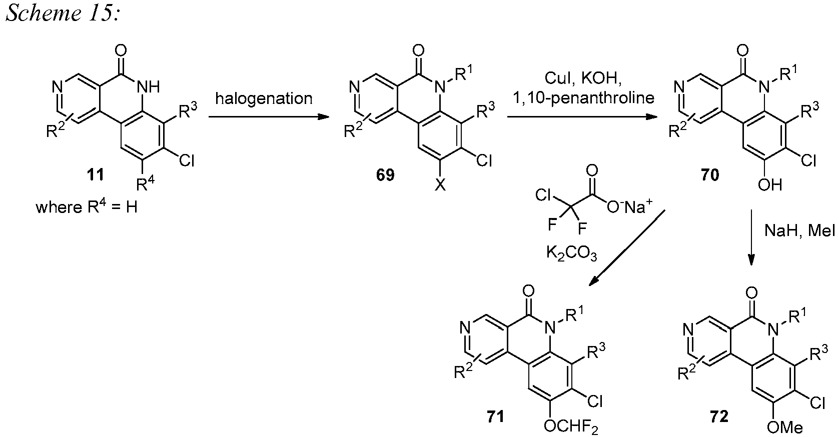

The intermediates represented by formula **71** and **72** are prepared by methods outlined in Scheme 15. The biaryl amide **11,** prepared as described in Scheme 2, can be subjected to halogenation by employing *N*-halosuccinimide in polar aprotic solvents such as acetonitrile in the dark to yield halides **69.** Compounds **69** can be treated with copper (I) iodide and potassium hydroxide in the presence of 1,10-phenanthroline to afforded intermediates represented by the formula **70.** Intermediates **70** can be treated with sodium 2-chloro-2,2-difluoroacetate and a base such as potassium carbonate to afford intermediated represented by the formula **71.** Alternatively, compounds **70** can be treated with a base such as sodium hydride and an alkylating agent such as methyl iodide in a solvent such as DMF to afforded intermediates represented by the formula **72.**

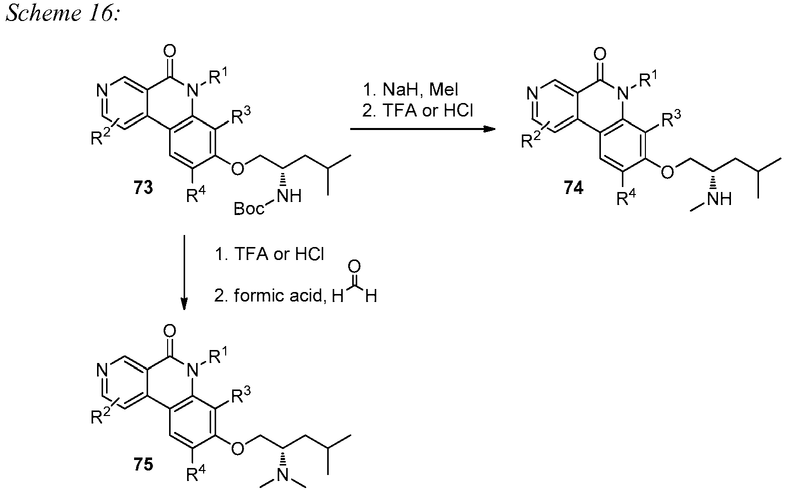

The compounds represented by formula **74** and **75** are prepared by methods outlined in Scheme 16. The intermediate **73,** prepared as described in Scheme 2, can be subjected to alkylation with methyl iodide and a base such as sodium hydride in a solvent such as DMF, followed by deprotection with TFA or HCl to afford compounds of the formula **74.** Alternatively, intermediate **73** can be deprotected with TFA or HCl, followed by treatment with formaldehyde in formic acid to afford compounds of the formula **75.**

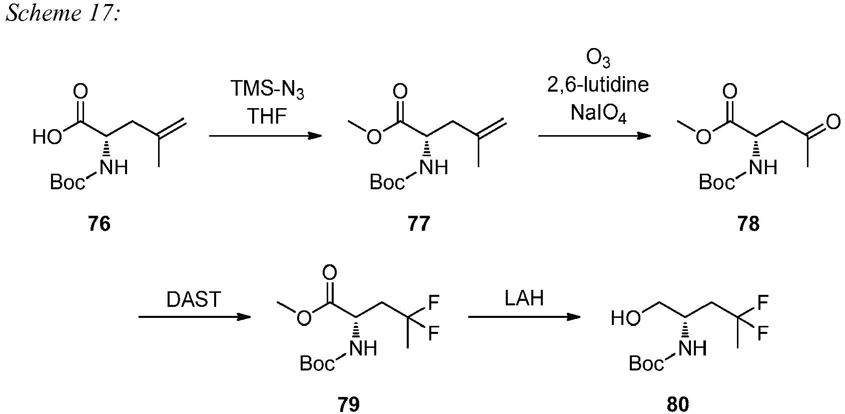

Intermediate **80** is prepared by methods outlined in Scheme 17. Intermediate **80** can be esterified by treatment with TMS-diazomethane in a solvent such as THF at low temperature, or by converting the acid to the acid chloride with thionyl chloride or oxalyl chloride and quenching with an alcohol such as MeOH or EtOH to provide ester **77.**

Intermediate **77** can be subjected to osmium tetroxide and sodium periodate in the presence of 2,6-lutidine in a solvent such as 1,4-dioxane and water to afford intermediate **78.** Intermediate **78** can be treated with DAST to provide intermediate **79.** Intermediate **79** can be subjected to reduction with a reagent such as lithium aluminum hydride or lithium borohydride in a solvent such as THF to provide intermediate **80.** 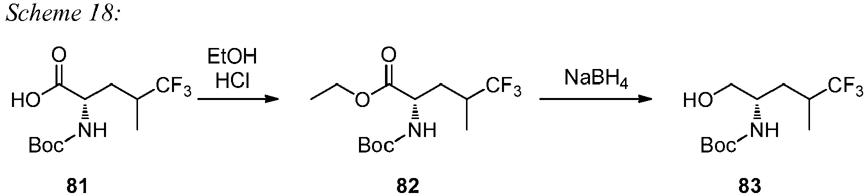 Intermediate **83** is prepared by methods outlined in Scheme 18. Intermediate **81** can be esterified by treatment with HCl in EtOH to provide ester **82.** Intermediate **82** can be subjected to reduction with a reagent such as lithium aluminum hydride or sodium borohydride in a solvent such as THF to provide intermediate **83.**

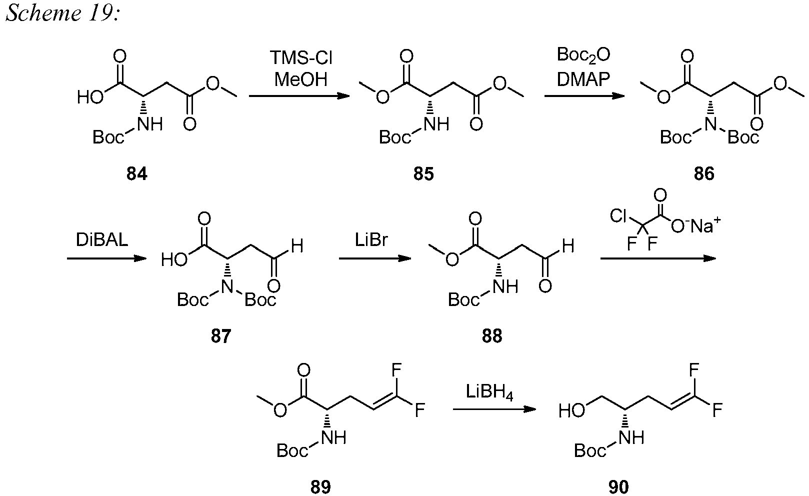

Intermediate **90** is prepared by methods outlined in Scheme 19. Intermediate **84** can be esterified by treatment with TMSCl in MeOH to provide ester **85.** Intermediate **85** can be treated with Boc₂O and DMAP in a solvent such as acetonitrile to afford intermediate **86.** Intermediate **86** can be subjected to reduction with a reagent such as DIBAL in a solvent such as THF or diethyl ether to provide intermediate **87.** Intermediate **87** can be mono-deprotected by treatment with LiBr in acetonitrile to afford intermediate **88.** Intermediate **88** can be subjected to sodium 2-chloro-2,2-difluoroacetate in DMF to afford intermediate **89.** Intermediate **89** can be reduced with LiBH₄ in THF to afford intermediate **90.**

Various analogues synthesized using Schemes 1-19 are listed in Table 1. AAK1 functional potency for select compounds are listed as IC₅₀ ranges where a = <1 nM; b = 1-10 nM; c = 10.01-100 nM; d = 100.01-2000 nM. The compounds prepared according to examples 108-111; 113; 115; 118; 121-126; 128-129; 131-135; 137; 140- 157 are according to the present invention.

**Table 1**

| | | | | | | | | | |
|---|---|---|---|---|---|---|---|---|---|
| 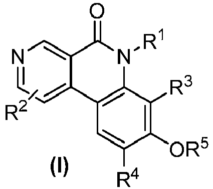 | | | | | | | | | |

| Ex. | stereo-chem | R¹ | R² | R³ | R⁴ | R⁵ | (M+H)⁺ | AAK1 IC₅₀ (nM) | cell IC₅₀ (nM) |
|---|---|---|---|---|---|---|---|---|---|
| **1** | *S* | H | H | H | H | 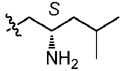 | 312.2 | 5.7 | 14 |
| **2** | *S* | Me | H | H | H | 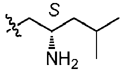 | 326.2 | b | 7.0 |
| **3** | *S* | Me | H | H | Br | 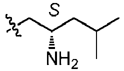 | 404.0 | 2.7 | 1.6 |
| **4** | *S* | Me | H | H | Me | 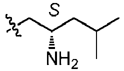 | 340.2 | b | 2.4 |
| **5** | *S* | Me | H | H | CN | 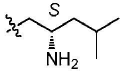 | 351.2 | b | 2.5 |
| **6** | *S* | Me | H | H | *cyc*Pr | 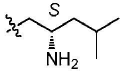 | 366.2 | b | 2.1 |
| **7** | *S* | Me | H | H | OH | 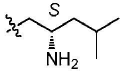 | 342.2 | 11 | -- |
| **8** | *S* | *i*-Pr | H | H | H | 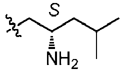 | 354.2 | c | -- |
| **9** | *S* | allyl | H | H | H | 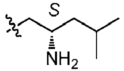 | 352.2 | 9.7 | 24 |
| **10** | *S* | Me | H | Cl | Cl | 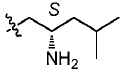 | 394.2 | c | 29 |
| **11** | *S* | Bn | H | H | H | 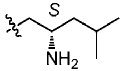 | 402.2 | 73 | -- |
| **12** | *S* | CH₂C H₂OM e | H | H | H | 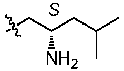 | 370.2 | 85 | -- |
| **13** | *S* | CH₂-*cyc*-Pr | H | H | H | 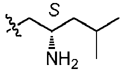 | 366.2 | c | -- |
| **14** | *S* | Me | H | H | F | 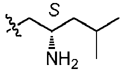 | 344.2 | 7.0 | b |
| **15** | *RS* | Me | H | H | H | 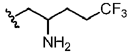 | 366.1 | c | c |
| **16** | *S* | Me | 4-Me | H | H | 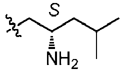 | 340.2 | c | c |
| **17** | *S* | Me | 4-NH₂ | H | H | 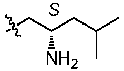 | 341.2 | 3.0 | b |
| **18** | *S* | Me | 2-NHAc | H | H | 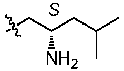 | 383.2 | d | -- |
| **19** | *R* | Me | 4-Me | H | H | 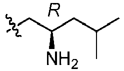 | 340.2 | c | c |
| **20** | *R* | Me | 4-Me | H | Cl | 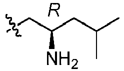 | 374.2 | b | b |
| **21** | *S* | Me | 4-Me | H | H | 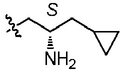 | 338.2 | 23 | -- |
| **22** | *S* | Me | 4-Me | H | H | 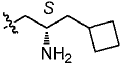 | 352.3 | 12 | -- |
| **23** | *S* | Me | 4-Me | H | Cl | 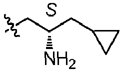 | 372.2 | 0.12 | b |
| **24** | *S* | Me | 4-Me | H | Cl | 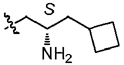 | 386.2 | b | b |
| **25** | *S* | Me | 4-Me | H | Me | 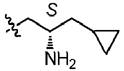 | 366.3 | 0.55 | b |
| **26** | *S* | Me | 4-Me | H | Me | 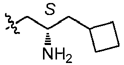 | 352.2 | 2.9 | b |
| **27** | *S* | Me | 4-Me | H | CHF₂ | 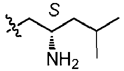 | 390.2 | a | 0.3 |
| **28** | *RS* | Me | H | H | H | 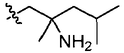 | 340.0 | 1.1 | -- |
| **29** | *RS* | Me | 4-Me | F | H | 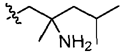 | 372.0 | d | -- |
| **30** | Dia-1 | Me | 4-Me | H | CH(OH)Me | 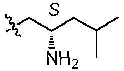 | 384.2 | 1.7 | -- |
| **31** | Dia-2 | Me | 4-Me | H | CH(OH)Me | 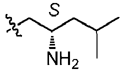 | 384.2 | c | -- |
| **32** | *RS* | Me | 4-Me | H | H | 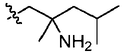 | 354.2 | 3.9 | c |
| **33** | Dia mix | Me | 4-Me | H | H | 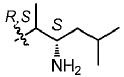 | 354.0 | c | 11 |
| **34** | *RS* | Me | 4-Me | H | OCH3 | 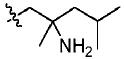 | 384.2 | 5.9 | -- |
| **35** | S | Me | 4-Me | H | C(OH) Me₂ | 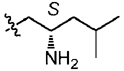 | 398.0 | 114 | -- |
| **36** | S | Me | 4-Me | H | Et | 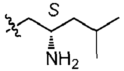 | 368.4 | 0.35 | -- |
| **37** | *Ent* -*1* | Me | 4-Me | H | H | 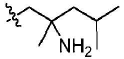 | 354.2 | 3.9 | c |
| **38** | *Ent* -*2* | Me | 4-Me | H | H | 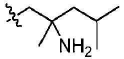 | 354.2 | 13 | -- |
| **39** | S | Me | H | H | H | 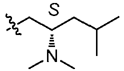 | 354.0 | c | -- |
| **40** | | Me | 4-Me | H | H | 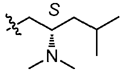 | 368.0 | 123 | -- |
| **41** | *RS* | Me | 4-Me | H | Cl | 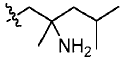 | 388.0 | 0.64 | 3.3 |
| **42** | S | Me | 4-OCH3 | H | H | 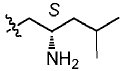 | 356.0 | d | -- |
| **43** | S | Me | H | H | Cl | 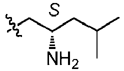 | 360.2 | b | 5.0 |
| **44** | S | CH₂C H₂OH | H | H | H | 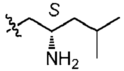 | 356.2 | 41 | -- |
| **45** | S | CH₂CF 3 | H | H | H | 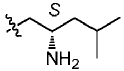 | 394.2 | 29 | -- |
| **46** | S | Me | H | H | Et | 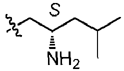 | 354.2 | b | 3.1 |
| **47** | S | Me | H | H | *i*Pr | 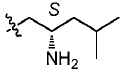 | 368.2 | 4.5 | c |
| **48** | S | Me | H | H | 5-oxazole | 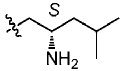 | 393.2 | b | 1.8 |
| **49** | *S* | H | 4-Me | H | Br | 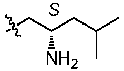 | 402.0 | b | 0.6 |
| **50** | S | Me | 4-NHPMB | H | H | 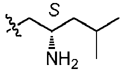 | 461.0 | 33 | -- |
| **51** | S | Me | H | H | 4-F-Ph | 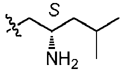 | 420.0 | d | -- |
| **52** | S | Me | 4-NH2 | H | F | 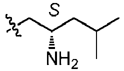 | 359.2 | b | 2.4 |
| **53** | S | Me | H | H | H | 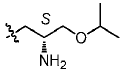 | 342.2 | 119 | -- |
| **54** | S | Me | H | H | H | 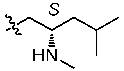 | 340.2 | c | -- |
| **55** | S | Me | H | H | H | 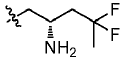 | 348.2 | c | -- |
| **56** | *RS* | Me | H | H | CN | 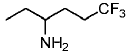 | 391.2 | b | b |
| **57** | *RS* | Me | H | H | H | 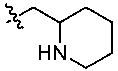 | 324.2 | 229 | -- |
| **58** | *Ent* -*1* | Me | H | H | H | 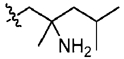 | 340.0 | 11 | c |
| **59** | *Ent* -*2* | Me | H | H | H | 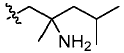 | 340.0 | b | c |
| **60** | S | Me | 4-CHF2 | H | H | 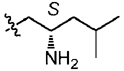 | 376.2 | d | -- |
| **61** | S | Me | 4-Me | Cl | H | 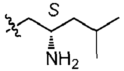 | 373.8 | 126 | -- |
| **62** | S | Me | 4-Me | H | Cl | 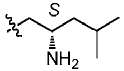 | 373.8 | b | b |
| **63** | S | Me | 4-Me | H | Me | 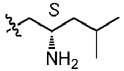 | 353.9 | b | b |
| **64** | S | Me | 4-Me | H | CN | 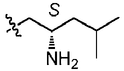 | 365.2 | a | 0.6 |
| **65** | S | Me | 4-Me | H | CN | 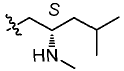 | 379.0 | a | b |
| **66** | S | Me | 2-Me | H | Br | 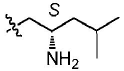 | 418.0 | d | -- |
| **67** | S | Me | 2-Me | H | H | 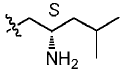 | 340.2 | 893 | -- |
| **68** | S | Me | 2-Me | H | Cl | 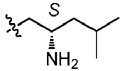 | 373.8 | c | -- |
| **69** | S | Me | H | H | H | 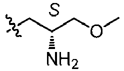 | 314.2 | c | -- |
| **70** | S | Me | 4-Me | H | F | 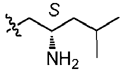 | 358.2 | b | b |
| **71** | S | Me | 4-Me | H | CH₂O H | 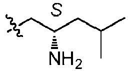 | 370.2 | b | -- |
| **72** | S | Me | 4-Me | H | 5-oxazole | 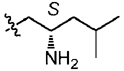 | 407.2 | a | a |
| **73** | *RS* | Me | 4-Me | H | CN | 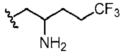 | 405.4 | b | b |
| **74** | S | Me | 4-Me | H | N-pyrazole | 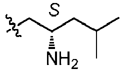 | 406.2 | b | -- |
| **75** | S | Me | 4-Me | H | CF₃ | 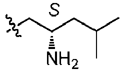 | 408.2 | b | b |
| **76** | S | Me | 4-Me | H | SO₂Me | 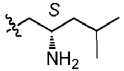 | 466.1 | d | -- |
| **77** | S | H | 4-Me | H | H | 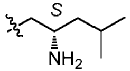 | 326.2 | b | -- |
| **78** | R | Me | 4-Me | H | CN | 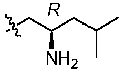 | 365.2 | 0.8 | b |
| **79** | S | CH₂C H₂OM e | 4-Me | H | Cl | 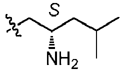 | 384.2 | b | -- |
| **80** | S | CH₂C H₂OM e | 4-Me | H | H | 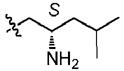 | 418.2 | 134 | -- |
| **81** | S | Me | 4-Me | F | H | 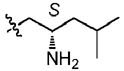 | 358.2 | d | -- |
| **82** | S | H | 4-Me | F | H | 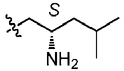 | 342.2 | 150 | -- |
| **83** | S | Me | 4-Me | H | 5-thiazole | 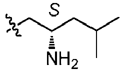 | 423.2 | b | b |
| **84** | S | Me | 4-Me | H | CO₂M e | 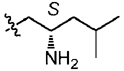 | 398.2 | b | -- |
| **85** | S | Me | 1-F | H | H | 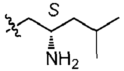 | 344.2 | c | -- |
| **86** | S | Me | 4-Me | H | 2-pyridyl | 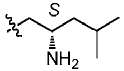 | 417.6 | c | c |
| **87** | *Dia* -*1* | Me | 4-Me | H | H | 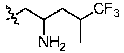 | 394.6 | b | -- |
| **88** | *Dia* -*2* | Me | 4-Me | H | H | 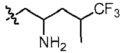 | 394.6 | b | c |
| **89** | S | Me | 4-Me | H | OMe | 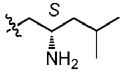 | 370.2 | b | -- |
| **90** | S | Me | 4-Me | H | OCHF ₂ | 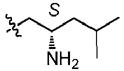 | 406.2 | c | -- |
| **91** | *Ent* -*1* | Me | 4-Me | H | H | 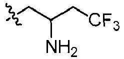 | 366.1 | c | -- |
| **92** | *Ent* -*2* | Me | 4-Me | H | H | 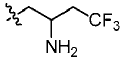 | 366.1 | b | -- |
| **93** | *RS* | Me | H | H | H | 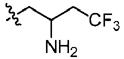 | 352.0 | c | -- |
| **94** | S | Me | 4-Me | H | CONH2 | 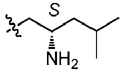 | 383.2 | c | -- |
| **95** | S | Me | 4-Me | H | H | 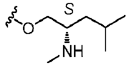 | 354.0 | c | -- |
| **96** | S | Me | 4-Me | H | CONMe₂ | 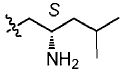 | 411.2 | d | -- |
| **97** | *RS* | Me | 1-F | H | H | 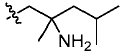 | 358.2 | b | c |
| **98** | S | Me | 1-OCH₃ | H | H | 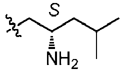 | 356.0 | d | -- |
| **99** | S | H | 4-Me | OCH3 | H | 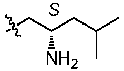 | 356.2 | d | -- |
| **100** | S | H | 4-Me | F | Br | 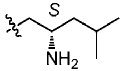 | 423.0 | b | -- |
| **101** | S | H | 4-Me | H | F | 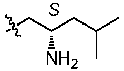 | 344.0 | b | 5.2 |
| **102** | *RS* | H | 4-Me | H | F | 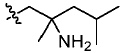 | 358.0 | b | -- |
| **103** | *S* | H | 4-Me | F | Cl | 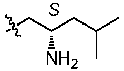 | 378.0 | b | c |
| **104** | *S* | Me | 4-Me | H | CH₂O Me | 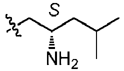 | 384.2 | c | -- |
| **105** | *RS* | H | 4-Me | F | H | 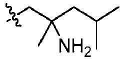 | 358.0 | c | -- |
| **106** | *S* | Me | 4-Me | H | CN | 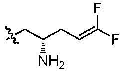 | 385.3 | a | a |
| **107** | *S* | Me | 4-Me | F | Cl | 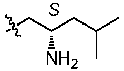 | 392.2 | b | -- |
| **108** | *S* | H | H | H | Br | 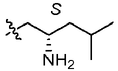 | 390.0 | b | b |
| **109** | *S* | H | H | H | Me | 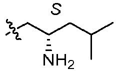 | 326.2 | b | b |
| **110** | *S* | Me | H | H | *iso*-Bu | 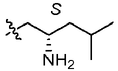 | 382.2 | c | -- |
| **111** | *Ent 1* | Me | H | H | H | 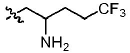 | 366.2 | b | c |
| **112** | *Ent 2* | Me | H | H | H | 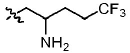 | 366.2 | b | c |
| **113** | *RS* | Me | H | H | Br | 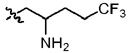 | 444.0 | b | b |
| **114** | *RS* | Me | H | H | 5-oxazole | 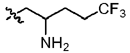 | 433.2 | b | c |
| **115** | *RS* | Me | H | H | Cl | 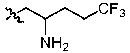 | 400.6 | b | b |
| **116** | *Ent 1* | Me | H | H | Cl | 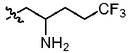 | 400.0 | c | c |
| **117** | *Ent 2* | Me | H | H | Cl | 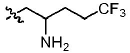 | 400.0 | b | b |
| **118** | *RS* | Me | 4-NH₂ | H | H | 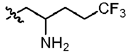 | 381.2 | b | c |
| **119** | *Ent 1* | Me | 4-NH₂ | H | H | 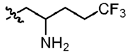 | 381.2 | b | b |
| **120** | *Ent 2* | Me | 4-NH₂ | H | H | 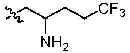 | 381.2 | b | b |
| **121** | *S* | Me | H | H | CHF₂ | 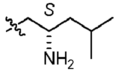 | 376.2 | b | b |
| **122** | *S* | Me | 4-Me | H | Br | 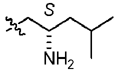 | 418.0 | b | b |
| **123** | *RS* | Me | 4-Me | H | H | 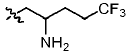 | 380.2 | c | -- |
| **124** | *RS* | Me | 4-Me | H | Cl | 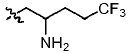 | 414.0 | a | -- |
| **125** | *S* | Me | 4-Me | H | *cyc*-Pr | 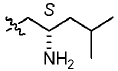 | 379.9 | b | -- |
| **126** | *Ent 1* | Me | H | H | CN | 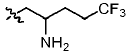 | 391.0 | 16 | 56 |
| **127** | *Ent 2* | Me | H | H | CN | 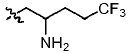 | 391.0 | b | b |
| **128** | *RS* | Me | 4-Me | H | Br | 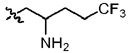 | 458.4 | a | -- |
| **129** | *S* | Me | 4-Me | H | *i*-Pr | 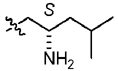 | 382.2 | b | b |
| **130** | *S* | CH₂-CH₂-OH | 4-Me | H | H | 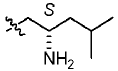 | 370.2 | c | -- |
| **131** | *S* | CH₂-CH₂-OMe | 4-Me | H | Br | 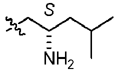 | 463.2 | c | -- |
| **132** | *S* | CH₂-CH₂-OH | 4-Me | H | Cl | 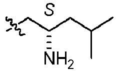 | 404.2 | a | -- |
| **133** | *R* | Me | 4-Me | H | Br | 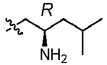 | 418.2 | 0.4 | 2.3 |
| **134** | *S* | Me | 4-Me | F | Br | 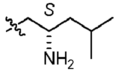 | 436.0 | c | -- |
| **135** | *Ent 1* | Me | H | H | H | 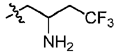 | 352.2 | c | -- |
| **136** | *Ent 2* | Me | H | H | H | 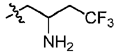 | 352.2 | b | -- |
| **137** | *RS* | Me | 4-Me | H | Br | 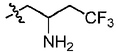 | 443.9 | b | -- |
| **138** | *Ent 1* | Me | 4-Me | H | Br | 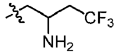 | 443.9 | b | c |
| **139** | *Ent 2* | Me | 4-Me | H | Br | 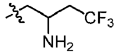 | 443.9 | a | b |
| **140** | *RS* | Me | H | H | Br | 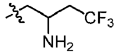 | 429.9 | b | c |
| **141** | *RS* | Me | H | H | Cl | 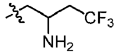 | 386.0 | b | c |
| **142** | *S* | H | 4-Me | H | Cl | 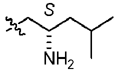 | 346.0 | a | b |
| **143** | *S* | CH₂-CH₂-OH | 4-Me | H | Br | 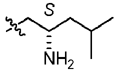 | 448.0 | b | b |
| **144** | | Me | 4-Me | H | H | OMe | 255.2 | d | -- |
| **145** | *S* | Me | 4-NHC(O)N HMe | H | F | 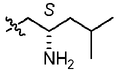 | 416.2 | c | c |
| **146** | *RS* | Me | H | H | H | 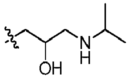 | 342.2 | d | -- |
| **147** | *RS* | Me | 4-Me | H | H | 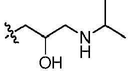 | 356.2 | 1330 | -- |
| **148** | *RS* | H | 4-Me | H | Br | 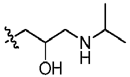 | 420.0 | d | d |
| **149** | *RS* | Me | 4-Me | H | Br | 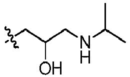 | 434.0 | c | -- |
| **150** | *RS* | Me | H | H | H | 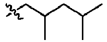 | 325.2 | c | -- |
| **151** | | Me | H | H | H | 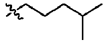 | 311.2 | c | -- |
| **152** | *S* | Me | 4-Me | H | H | 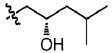 | 341.0 | c | -- |
| **153** | *RS* | Me | 4-Me | H | H | 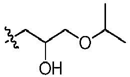 | 357.1 | d | -- |
| **154** | *RS* | Me | H | H | Br | 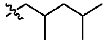 | 402.1 | c | -- |
| **155** | *RS* | Me | 4-Me | H | H | 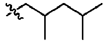 | 339.0 | d | -- |
| **156** | | Me | 4-Me | H | H | 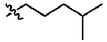 | 325.0 | c | -- |
| **157** | *Ent 1* | Me | H | H | H | 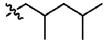 | 325.0 | c | -- |
| **158** | *Ent 2* | Me | H | H | H | 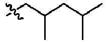 | 325.0 | c | -- |

### DESCRIPTION OF THE SPECIFIC EMBODIMENTS

In the following examples, proton NMR spectra were recorded on either a Bruker 400 or 500 MHz NMR spectrometer. Chemical shifts are reported in δ values relative to tetramethylsilane. LC/MS were run on a Shimadzu LC coupled to a Waters Micromass ZQ. HPLC retention times were obtained using at least one of the following methods:
LC-MS methods:
   LC/MS Method A: Column: PUROSPHER@star RP-18 (4X55mm), 3µm; Buffer : 20mM NH₄OAC IN WATER; Mphase A : Buffer + ACN(90+10); Mphase B : Buffer + MeCN(10+90); Flow : 2.5 mL/min)
   LC/MS Method B: Column : ZORBAX SB C18 (46X50mm), 5µm; Positive mode Mphase A : 10% MeOH - 90% H₂O - 0.1% TFA; Mphase B : 90% MeOH - 10% H₂O - 0.1% TFA; Flow : 5 mL/min).
   LC/MS Method C: Column - Ascentis Express C8 (5X2.1mm), 2.7µm; Mphase A : 2%MeCN - 98%H₂O - 10mM NH₄COOH; Mphase B : 98%ACN - 2%H2O - 10 mM NH₄COOH; Flow : 1 mL/min).
   LC/MS Method D: Column -ACQUITY UPLC BEH C18 (2.1 X 50 mm), 1.7µm; Mphase A :0.1% TFA in water; Mphase B : 1% TFA in ACN; Flow : 1/min) LC/MS Method E: Column -ACQUITY UPLC BEH C18 (2.1 X 50 mm), 1.7µm; Mphase A : 5 mM NH₄OAc:ACN (95:5); Mphase B : 5 mM NH₄OAc: ACN (5:95); Flow : 1/min).
   LC/MS Method F: Column : X-Bridge BEH C18 (50 X 2.1 mm), 2.5µm; Mphase A : ACN + H₂O (2+98) + 0.1% TFA; Mphase B : ACN + H₂O (98+2) + 0.05% TFA; Flow : 1.2 mL/min.
   LC-MS method G: Column : Kinetex C18 (50 X 2.1 mm), 2.6um: Mphase A: 2%ACN - 98%H₂O - 10mM; Mphase B : 98%ACN - 2%H₂O - 10mM NH₄COOH; Flow : 1/min).
   LC-MS method H: Column : BEH C18 (50 X 3.0 mm), 1.7 µm: Mphase A: 5%ACN - 95%H₂O - 10mM; Mphase B : 95%ACN - 5%H₂O - 10mM NH₄COOH; Flow : 1.2/min).
   LC-MS method I: Column : Ace Excel 2 C18 (50 X 3.0 mm), 2.0 µm: Mphase A: 2%ACN - 98%H₂O - 10mM; Mphase B : 98%ACN - 2%H₂O - 10mM NH₄COOH; Flow : 1.2/min).
Chiral HPLC methods:
   Method A: CHIRALCEL OJH (250x4.6) mm 5 micron Mob. phase: 0.2% DEA in n-hexane : ethanol (80:20)
   Method B: CHIRALPAK AD-H (250x4.6) mm 5 micron Mob. Phase A: 0.2% DEA in n-hexane (70) B: ethanol (30)
   Method C: CHIRALPAK- ASH (250x4.6) mm 5 micron Mob. Phase A: 0.2% DEA in n-hexane : ethanol (90:10)
Analytical HPLC methods:
   Method A: Waters analytical C18 sunfire column (4.6 x 150 mm, 3.5 µm); mobile phase:
      Buffer: 0.05% TFA in H₂O pH = 2.5 adjusted with ammonia
      A = buffer and acetonitrile (95:5), B = acetonitrile and buffer (95:5); 0 - 15 min, 0%
      B → 50% B; 15 - 18 min, 50% B → 100% B; 18 - 23 min, 100% B; flow rate = 1 mL/min; λ = 254 nm and 220 nm; run time = 28 min.
   Method B: Waters analytical phenyl xbridge column (4.6 x 150 mm, 3.5 µm), mobile phase: Buffer: 0.05% TFA in H₂O pH = 2.5 adjusted with ammonia
      A = buffer and acetonitrile (95:5), B = acetonitrile and buffer (95:5); 0 - 15 min, 0%
      B → 50% B; 15 - 18 min, 50% B → 100% B; 18 - 23 min, 100% B; flow rate = 1 mL/min; λ = 254 nm and 220 nm; run time = 28 min.
   Method C: Waters analytical C18 sunfire column (3.0 x 150 mm, 3.5 µm); mobile phase: A = 10mM amm. bicarbonate (pH=9.5)/95% H2O/5% methanol, B = 10mM amm. bicarbonate (pH=9.5)/5% H₂O/95% methanol; 0 - 15 min, 0% B → 100% B; 15 - 18 min, 100% B; flow rate = 1 mL/min; λ = 254 nm; run time = 18 min.
   Method D: Waters analytical phenyl xbridge column (3.0 x 150 mm, 3.5 µm), mobile phase: A = 10mM amm. bicarbonate (pH=9.5)/95% H2O/5% methanol, B = 10mM amm. bicarbonate (pH=9.5)/5% H2O/95% methanol; 0 - 15 min, 0% B → 100% B; 15 - 18 min, 100% B; flow rate = 1 mL/min; λ = 254 nm; run time = 18 min.

### Example 1

### (S)-8-(2-amino-4-methylpentyloxy)benzo[c][2,7]naphthyridin-5(6H)-one

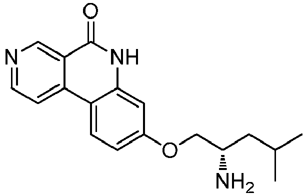

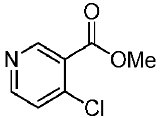

### Part A. Methyl 4-chloronicotinate

A solution of 4-chloronicotinic acid (20 g, 127 mmol) in dichloromethane (600 mL) and DMF (15 mL), cooled to 0 °C, was treated with oxalyl chloride (27.8 mL, 317 mmol) dropwise. After addition, the mixture was stirred for another 2 h at RT. The mixture was then cooled back to 0 °C and MeOH (30 mL) was added slowly. The reaction was warmed to RT and stirred for 0.5 h. The mixture was diluted with dichloromethane (500 mL) and saturated aqueous NaHCO₃ (100 mL) then extracted with DCM (3x1000 mL). The DCM layer was washed with brine (2x100 mL), dried over sodium sulfate and concentrated under reduced pressure. The crude product obtained as yellow oil (20 g, 92 % yield) was taken to the next step without further purification. LC/MS (ESI) *m*/*e* 172.0 [(M+H)⁺, calcd for C₇H₇ClNO₂: 172.6]; LC/MS retention time (method B): *t*_{R} = 1.21 min. ¹H NMR (400 MHz, *CDCl₃*) δ ppm 9.27 (s, 1H), 8.86 (bs, 1H), 7.98 (bs, 1H), 4.05 (s, 3H).

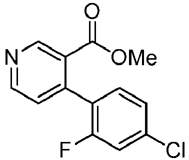

### Part B. Methyl 4-(4-chloro-2-fluorophenyl)nicotinate

Methyl 4-chloronicotinate (20 g, 117 mmol), (4-chloro-2-fluorophenyl)boronic acid (22.4 g, 128 mmol), Pd(Ph₃P)₄ (9.4 g, 8.16 mmol) and Cs₂CO₃ (114 g, 350 mmol) were taken in a solvent mixture of 1,4-dioxane (400 mL) and water (30 mL) and purged with nitrogen for 5 min and heated to 85°C. After overnight stirring at this temperature the reaction mixture was diluted with ethyl acetate and filtered through a bed of diatomaceous earth (Celite®). The bed was further washed with ethyl acetate. Combined filtrate was washed with water (1x100 mL), dried (Na₂SO₄) and concentrated under reduced pressure to afford the crude product which was purified via combi flash (gradient of methanol and chloroform) to afford methyl 4-(4-chloro-2-fluorophenyl)nicotinate (13 g, 48.9 mmol, 42%, with purity of 77% by LC/MS) as a brown solid. LC/MS (ESI) *m*/*e* 266.0 [(M+H)⁺, calcd for C₁₃H₁₀ClFNO₂ 266.1]; LC/MS retention time (method B): *t*_{R} = 1.69 min.

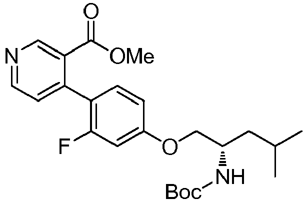

### Part C. (S)-methyl 4-(4-((2-((tert-butoxycarbonyl)amino)-4-methylpentyl)oxy)-2-fluorophenyl)nicotinate

Methyl 4-(4-chloro-2-fluorophenyl)nicotinate (50 mg, 0.188 mmol) in toluene (1 mL) was treated with cesium carbonate (92 mg, 0.282 mmol) and *N*-Boc-L-Leucinol (121 mg, 0.565 mmol). The mixture was purged with nitrogen gas for 5 min and treated with di-*tert*-butyl(2',4',6'-triisopropyl-[1,1'-biphenyl]-2-yl)phosphine (48.0 mg, 0.113 mmol). After purging the mixture with nitrogen for another 5 min, palladium(II)acetate (42.3 mg, 0.188 mmol) was added and nitrogen bubbled through for another 10 min. The mixture was sealed tightly and heated at 80 °C for 12 h. The mixture was filtered through a bed of diatomaceous earth (Celite®) and washed with ethyl acetate (2x3 mL). The washings were concentrated under reduced pressure. The residue was purified by combi flash (ethyl acetate and petroleum ether) to afford (*S*)-methyl 4-(4-((2-((*tert*-butoxycarbonyl)amino)-4-methylpentyl)oxy)-2-fluorophenyl)nicotinate (50 mg, 0.112 mmol, 60% yield). LC/MS (ESI) *m*/*e* 447.2 [(M+H)⁺, calcd for C₂₄H₃₂FN₂Os 447.2]; LC/MS retention time (method C): *t*_{R} = 2.17 min.

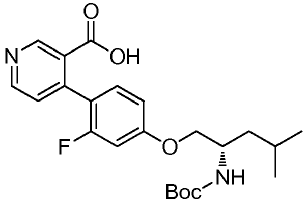

### Part D. (S)-4-(4-((2-((tert-butoxycarbonyl)amino)-4-methylpentyl)oxy)-2-fluorophenyl)nicotinic acid

(*S*)-methyl-4-(4-((2-((*tert*-butoxycarbonyl)amino)-4-methylpentyl)oxy)-2 fluorophenyl)nicotinate (50.0 mg, 0.112 mmol) was taken in a mixture of tetrahydrofuran (1 mL), methanol (1 mL) and water (1 mL). To the solution was added lithium hydroxide (8.05 mg, 0.336 mmol) and the reaction mixture was stirred at RT for 2 h. The reaction mixture was concentrated under reduced pressure and the residue was diluted with water and the pH adjusted to 4 using 1.5N aqueous HCl. The crude product from the aqueous layer was extracted with ethyl acetate (3x5 mL). The combined organic layers were washed with brine (1x5 mL), dried (Na₂SO₄) and concentrated under reduced pressure to afford (*S*)-4-(4-((2-((*tert-*butoxycarbonyl)amino)-4-methylpentyl)oxy)-2-fluorophenyl)nicotinic acid (30 mg, 0.060 mmol, 53% yield) as an off-white solid. LC/MS (ESI) *m*/*e* 433.2 [(M+H)⁺, calcd for C₂₃H₃₀FN₂O₅ 433.2]; LC/MS retention time (method C): *t*_{R} = 1.74 min.

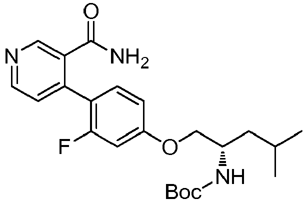

### Part E. (S)-tert-butyl (1-(4-(3-carbamoylpyridin-4-yl)-3-fluorophenoxy)-4-methylpentan-2-yl)carbamate

(*S*)-4-(4-((2-((*tert*-butoxycarbonyl)amino)-4-methylpentyl)oxy)-2-fluorophenyl)nicotinic acid (75 mg, 0.173 mmol) was taken in DMF (2 mL) and cooled to 0 °C. To the solution was added EDC (49.9 mg, 0.260 mmol) and HOBT (53.1 mg, 0.347 mmol). After stirring the reaction mixture for 5 min, ammonium chloride (55.7 mg, 1.040 mmol) was added and the resultant mixture was stirred for 5 min. Diisopropylethylamine (0.121 mL, 0.694 mmol) was added and the mixture was allowed to stir at room temperature for an additional 12 h. The mixture was then quenched with ice and the residue was filtered and dried under vacuum to afford (*S*)-*tert*-butyl (1 -(4-(3 -carbamoylpyridin-4-yl)-3 -fluorophenoxy)-4-methylpentan-2-yl)carbamate (50 mg, 0.098 mmol, 57% yield) as a light yellow solid. LC/MS (ESI) *m*/*e* 432.2 [(M+H)⁺, calcd for C₂₃H₃₁FN₃O₄ 432.2]; LC/MS retention time (method C): *t*_{R} = 1.83 min.

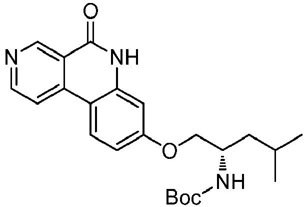

### Part F. (S)-tert-butyl (4-methyl-1-((5-oxo-5,6-dihydrobenzo[c][2,7]naphthyridin-8-yl)oxy)pentan-2-yl)carbamate

Sodium hydride (5.56 mg, 0.232 mmol, 60% in mineral oil) was taken in tetrahydrofuran (1 mL) and cooled to 0 °C. The suspension was treated with (*S*)-*tert-*butyl (1-(4-(3-carbamoylpyridin-4-yl)-3-fluorophenoxy)-4-methylpentan-2-yl)carbamate (50 mg, 0.116 mmol) in THF (1 mL) dropwise and the temperature was maintained at 0 °C for 30 min. The reaction mixture was then allowed to warm to room temperature and stirred for 2 h. The reaction was quenched with ice and extracted with ethyl acetate (3x5 mL). The combined organic layers were washed with brine (1x5 mL), dried (Na₂SO₄) and concentrated under reduced pressure to afford (*S*)-*tert*-butyl (4-methyl-1-((5-oxo-5,6-dihydrobenzo[*c*][2,7]naphthyridin-8-yl)oxy)pentan-2-yl)carbamate (40 mg, 0.097 mmol, 84% yield) as a colorless solid. LC/MS (ESI) *m*/*e* 412.2 [(M+H)⁺, calcd for C₂₃H₃₀N₃O₄ 412.2]; LC/MS retention time (method C): *t*_{R} = 1.97 min.

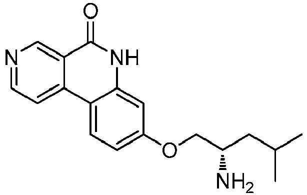

### Part G. (S)-8-(2-amino-4-methylpentyloxy)benzo[c][2,7]naphthyridin-5(6H)-one

(*S*)-*tert*-butyl (4-methyl-1-((5-oxo-5,6-dihydrobenzo[*c*][2,7]naphthyridin-8-yl)oxy)pentan-2-yl)carbamate (10 mg, 0.024 mmol) in dichloromethane (1 mL) was cooled to 0 °C. To the solution was added hydrogen chloride (0.886 mg, 0.012 mL, 0.024 mmol, 2M in diethyl ether) dropwise. The temperature of the reaction mixture was maintained at 0 °C for 30 min and then the reaction mixture was allowed to warm to room temperature and stirred for 2 h. The reaction mixture was then concentrated under reduced pressure to afford (*S*)-8-((2-amino-4-methylpentyl)oxy)benzo[*c*][2,7]naphthyridin-5(6*H*)-one (4 mg, 0.012 mmol, 49% yield) as a pale yellow solid. LC/MS (ESI) *m*/*e* 312.2 [(M+H)⁺, calcd for C₁₈H₂₂N₃O₂ 312.2]; LC/MS retention time (method A): *t*_{R} = 1.1 min; HPLC retention time (method A): *t*_{R} = 6.85 min; HPLC retention time (method B): *t*_{R} = 7.51 min ¹H NMR (400 MHz, *D₂O*) δ ppm 9.30 (br. s., 1H), 8.77 (br. s., 1H), 8.36 (br. s., 1H), 8.07 (br. s., 1H), 6.95 (br. s., 1H), 6.72 (br. s., 1H), 4.41 (d, *J* = 9.03 Hz, 1H), 4.26 (br. s., 1H), 3.89 (br. s., 1H), 3.38 (s, 1H), 1.72 - 1.88 (m, 2H), 1.05 (d, *J* = 2.51 Hz, 6H).

### Example 2

### (S)-8-((2-amino-4-methylpentyl)oxy)-6-methylbenzo[c][2,7]naphthyridin-5(6H)-one

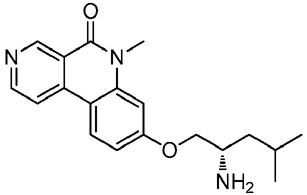

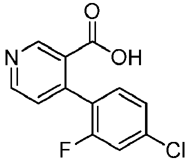

### Part A. 4-(4-chloro-2-fluorophenyl)nicotinic acid

To a solution of methyl 4-(4-chloro-2-fluorophenyl)nicotinate (15 g, 56.5 mmol) (as prepared in Ex 1, Part B) in a solvent mixture of methanol (150 mL) and water (150 mL) cooled to 0° C was added sodium hydroxide (9.03 g, 226 mmol) and the mixture was warmed to room temperature and stirred for 1 h. The reaction mixture was concentrated under reduced pressure and pH of the resultant aqueous solution was adjusted to pH = 4 using 1.5 N Hydrochloric acid during which time the product crashed out as a solid. Filtration of the mixture provided 4-(4-chloro-2-fluorophenyl)nicotinic acid (9 g, 35.8 mmol, 63% yield) as an off white solid. LC/MS (ESI) *m*/*e* 252.0 [(M+H)⁺, calcd for C₁₂H₈ClFNO₂ 252.1]; LC/MS retention time (method A): *t*_{R} = 0.97 min.

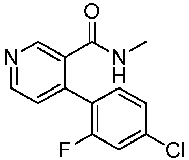

### Part B. 4-(4-chloro-2-fluorophenyl)-N-methylnicotinamide

To a solution of 4-(4-chloro-2-fluorophenyl)nicotinic acid (2 g, 7.95 mmol in dichloromethane (40 mL) cooled to 0 °C was added oxalyl chloride (2.09 mL, 23.8 mmol) followed by DMF (2 mL). The reaction mixture was allowed to warm to room temperature and stirred for 2 h. The reaction mixture was concentrated under reduced pressure and treated with dichloromethane (25 mL). This solution was added to a mixture of methylamine hydrochloride (5.37 g, 79 mmol) and triethylamine (11.08 mL, 79 mmol) in dichloromethane (25 mL) cooled to 0 °C. The resultant mixture was stirred at room temperature for 3 h and then washed with a saturated solution of sodium bicarbonate. The organic layer was separated, dried (Na₂SO₄) and concentrated under reduced pressure to afford 4-(4-chloro-2-fluorophenyl)-*N-*methylnicotinamide (1.3 g, 4.91 mmol, 62%) as an off-white solid. LC/MS (ESI) *m*/*e* 265.1 [(M+H)⁺, calcd for C₁₃H₁₁ClFN₂O 265.1]; LC/MS retention time (method C): *t*_{R} = 1.69 min.

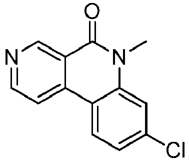

### Part C. 8-chloro-6-methylbenzo[c][2,7]naphthyndm-5(6H)-one

Sodium hydride (80 mg, 3.32 mmol) was taken in THF (1 mL) and cooled to 0 °C. To the suspension, 4-(4-chloro-2-fluorophenyl)-*N*-methylnicotinamide (440 mg, 1.66 mmol) in THF (1 mL) was added dropwise and the temperature was maintained at 0 °C for 30 min. The reaction mixture was then warmed to room temperature and stirred for 2 h. The reaction mixture was quenched with ice and extracted with ethyl acetate (3 x 5 mL). The combined organic layers were washed with brine (1 x 5 mL) dried (Na₂SO₄) and concentrated under reduced pressure to afford 8-chloro-6-methylbenzo[*c*][2,7]naphthyridin-5(6*H*)-one (440 mg, 1.798 mmol) as a light brown oil. LC/MS (ESI) *m*/*e* 245.1 [(M+H)⁺, calcd for C₁₃H₁₀ClN₂O 245.1]; LC/MS retention time (method C): *t*_{R} = 1.79 min.

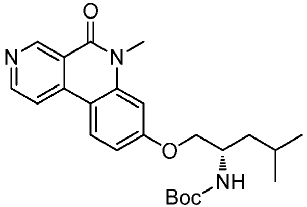

### Part D. (S)-tert-butyl (4-methyl-1-((6-methyl-5-oxo-5,6 dihydrobenzo[c][2,7]naphthyridin-8-yl)oxy)pentan-2-yl)carbamate

To a solution of 8-chloro-6-methylbenzo[*c*][2,7]naphthyridin-5(6*H*)-one (440 mg, 1.80 mmol) in toluene (8 mL) at room temperature, was added cesium carbonate (879 mg, 2.70 mmol) and di-*tert*-butyl(2',4',6'-triisopropyl-[1,1'-biphenyl]-2-yl)phosphine (458 mg, 1.079 mmol) and the mixture was degassed for 5 min. The mixture was then treated with *N*-Boc L-Leucinol (1160 mg, 5.39 mmol) followed by palladium(II)acetate (121 mg, 0.54 mmol) and the mixture degassed for another 10 min. The reaction mixture was sealed and heated at 80 °C. After overnight stirring the reaction mixture was cooled to room temperature and filtered through diatomaceous earth (Celite®). The bed was washed with ethyl acetate and the combined filtrate was concentrated under reduced pressure to afford the crude product which was purified by combi flash (gradient of ethyl acetate and petroleum ether) to afford (*S*)-*tert*-butyl (4-methyl-1-((6-methyl-5-oxo-5,6 dihydrobenzo[*c*][2,7]naphthyridin-8-yl)oxy)pentan-2-yl)carbamate (190 mg, 0.34 mmol, 19% yield) as an off-white solid. LC/MS (ESI) *m*/*e* 426.2 [(M+H)⁺, calcd for C₂₄H₃₂N₃O₄ 426.2]; LC/MS retention time (method C): *t*_{R} = 2.10 min.

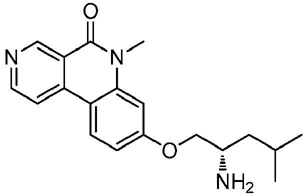

### Part E. (S)-8-((2-amino-4-methylpentyl)oxy)-6-methylbenzo[c][2,7]naphthyridin-5(6H)-one

To a solution of (*S*)-*tert*-butyl (4-methyl-1-((6-methyl-5-oxo-5,6-dihydrobenzo[*c*][2,7]naphthyridin-8-yl)oxy)pentan-2-yl)carbamate (190mg, 0.447 mmol) in dichloromethane (4 mL) at 0 °C was added hydrogen chloride (81 mg, 0.558 mL, 2.233 mmol) in 1,4-dioxane (4 M) dropwise and the reaction mixture was stirred at 0 °C for 30 min then warmed to room temperature and allowed to stir for 2 h. The solvents were removed under reduced pressure to afford crude product which was purified by preparative HPLC (0.1% TFA in water) to afford (*S*)-8-((2-amino-4-methylpentyl)oxy)-6-methylbenzo[*c*][2,7]naphthyridin-5(6*H*)-one (51 mg, 0.157 mmol, 35% yield) as a yellow solid. LC/MS (ESI) *m*/*e* 326.2 [(M+H)⁺, calcd for C₁₉H₂₄N₃O₂ 326.2]; LC/MS retention time (method A): *t*_{R} = 1.21 min; HPLC retention time (method A): *t*_{R} = 7.39 min; HPLC retention time (method B): *t*_{R} = 7.40 min. ¹H NMR (400 MHz, *CD₃OD*) δ ppm 9.48 (s, 1H), 8.77 (d, *J* = 5.77 Hz, 1H), 8.43 (d, *J* = 8.78 Hz, 1H), 8.26 (d, *J* = 5.77 Hz, 1H), 7.09 - 7.16 (m, 2H), 4.20 (dd, *J* = 9.41, 3.89 Hz, 1H), 4.02 (dd, *J* = 9.29, 7.03 Hz, 1H), 3.81 (s, 3H), 3.35 - 3.39 (m, 1H), 1.81 - 1.91 (m, 1H), 1.41 - 1.56 (m, 2H), 0.98 - 1.05 (m, 6H).

### Example 3

### (S)-8-(2-amino-4-methylpentyloxy)-9-bromo-6-methylbenzo[c][2,7]naphthyridin-5(6H)-one

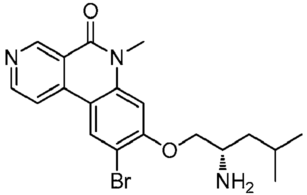

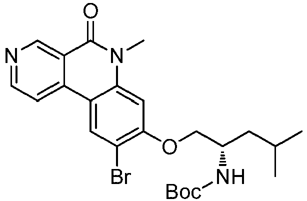

### Part A. (S)-tert-butyl (1-((9-bromo-6-methyl-5-oxo-5,6-dihydrobenzo[c][2,7]naphthyridin-8-yl)oxy)-4-methylpentan-2-yl)carbamate

To a solution of (*S*)-*tert*-butyl (4-methyl-1-((6-methyl-5-oxo-5,6-dihydrobenzo[*c*][2,7]naphthyridin-8-yl)oxy)pentan-2-yl)carbamate (0.100 g, 0.235 mmol) (as prepared in Ex. 2, Part D) in anhydrous acetonitrile (5 mL) was added *N-*bromosuccinimide (0.042 g, 0.235 mmol) and the mixture was heated at 85 °C for 5 h. The reaction mixture was diluted with water (10 mL), extracted with ethyl acetate (2x20 mL). The combined organic layers were dried over sodium sulfate and evaporated under reduced pressure to afford crude product which was purified by preparative TLC (gradient of ethyl acetate and petroleum ether) to afford (*S*)-*tert-*butyl (1-((9-bromo-6-methyl-5-oxo-5,6-dihydrobenzo[*c*] [2,7]naphthyridin-8-yl)oxy)-4-methylpentan-2-yl)carbamate (80 mg, 0.159 mmol, 68% yield) as an off-white solid. LC/MS (ESI) *m*/*e* 504.2 [(M+H)⁺, calcd for C₂₄H₃₁BrN₃O₄ 504.1]; LC/MS retention time (method A): *t*_{R} = 2.23 min.

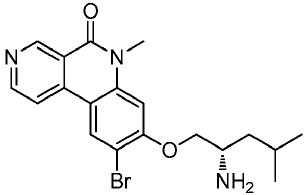

### Part B. (S)-8-((2-amino-4-methylpentyl)oxy)-9-bromo-6-methylbenzo[c][2,7]naphthyridm-5(6H)-one

To a solution of (*S*)-*tert*-butyl (1-((9-bromo-6-methyl-5-oxo-5,6-dihydrobenzo[*c*][2,7]naphthyridin-8-yl)oxy)-4-methylpentan-2-yl)carbamate (80 mg, 1.586 mmol) in anhydrous methanol (5 mL) was added a 4 M solution of HCl in 1,4-dioxane (2 mL, 8 mmol) dropwise at 0° C. The reaction mixture was warmed to room temperature and stirred for 2 h. The mixture was then was concentrated under reduced pressure to afford crude product which was purified by preparative HPLC (10 mM ammonium acetate in water ; acetonitrile) to afford (*S*)-8-((2-amino-4-methylpentyl)oxy)-9-bromo-6-methylbenzo[*c*][2,7]naphthyridin-5(6*H*)-one (20 mg, 0.049 mmol, 30% yield) as an off-white solid. LC/MS (ESI) *m*/*e* 404.1 [(M+H)⁺, calcd for C₁₉H₂₃BrN₃O₂ 404.1]; LC/MS retention time (method A): *t*_{R} = 1.47 min; HPLC retention time (method A): *t*_{R} = 9.55 min; HPLC retention time (method B): *t*_{R} = 10.23 min; ¹H NMR (400 MHz, *CD₃OD*) δ ppm 9.48 (s, 1H), 8.80 (d, *J* = 5.77 Hz, 1H), 8.67 (s, 1 H), 8.26 (d, *J* = 5.77 Hz, 1H), 7.15 (s, 1H), 4.37 (dd, *J* = 9.54, 3.76 Hz, 1H), 4.18 (dd, *J* = 9.66, 6.40 Hz, 1H), 3.83 (s, 3H), 3.48 - 3.56 (m, 1H), 1.89 (m, 1H), 1.51 - 1.72 (m, 2H), 1.05 (t, *J* = 6.27 Hz, 6H).

### Example 4

### (S)-8-((2-amino-4-methylpentyl)oxy)-6,9-dimethylbenzo[c][2,7]naphthyridin-5(6H)-one

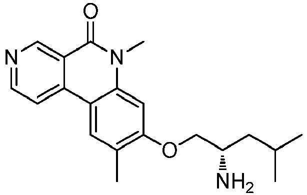

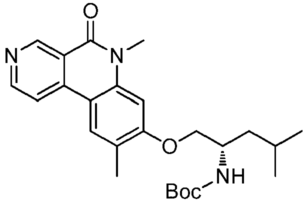

### Part A. (S)-tert-butyl (1-((6,9-dimethyl-5-oxo-5,6-dihydrobenzo[c][2,7]naphthyridin-8-yl)oxy)-4-methylpentan-2-yl)carbamate

In a 10 mL round-bottomed flask, (*S*)-*tert*-butyl (1-((9-bromo-6-methyl-5-oxo-5,6-dihydrobenzo[*c*][2,7]naphthyridin-8-yl)oxy)-4-methylpentan-2-yl)carbamate (as prepared in Ex. 3, Part A) (150 mg, 0.297 mmol), 2,4,6-trimethyl-1,3,5,2,4,6-trioxatriborinane (41.1 mg, 0.327 mmol), Cs₂CO₃ (291 mg, 0.892 mmol) and PdCl₂(dppf) (21.76 mg, 0.030 mmol) were taken up in a mixture of 1,4-dioxane (2 mL) and water (0.1 mL). The reaction mixture was purged with nitrogen for 5 min and heated at 90° C for 15 h. 1,4-dioxane was removed under reduced pressure and the residue was dissolved in ethyl acetate (20 mL). The ethyl acetate layer was washed with water (10x2 mL) and dried over sodium sulfate. Removal of the solvent gave crude product (0.08 g), which was filtered through silica-gel column (24 g silica-gel, MeOH-CHCl₃ mixture). The product was isolated as off-white solid (0.05 g, 0.11 mmol, 38% yield). LC/MS (ES-API) *m*/*e* 440.2 [(M+H)⁺, calcd for C₂₅H₃₄N₃O₄, 440.3]; LC/MS retention time (method B): *t*_{R} = 1.95 min.

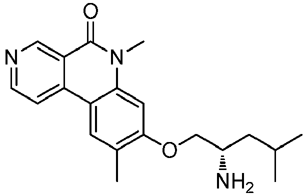

### Part B. (S)-8-((2-amino-4-methylpentyl)oxy)-6,9-dimethylbenzo[c][2,7]naphthyridin-5(6H)-one

In a 25 mL round-bottomed flask, (*S*)-*tert*-butyl (1-((6,9-dimethyl-5-oxo-5,6-dihydrobenzo[*c*][2,7]naphthyridin-8-yl)oxy)-4-methylpentan-2-yl)carbamate (80 mg, 0.182 mmol) was taken in MeOH (6 mL). The reaction mixture was cooled to 0 °C and a 4M solution of HCl in 1,4-dioxane (3.75 mL, 12 mmol) was added and the mixture stirred at RT for 2 h. The MeOH was removed and the crude product was dissolved in ethyl acetate (20 mL). The ethyl acetate layer was washed with water (10 mL) and dried over sodium sulfate. Removal of the solvent gave crude product which was purified by silica-gel column (MeOH-CHCl₃ mixture) to afford (*S*)-8-((2-amino-4-methylpentyl)oxy)-6,9-dimethylbenzo[*c*][2,7]naphthyridin-5(6*H*)-one (0.03 g, 0.09 mmol, 49% yield) as yellow solid. LC/MS (ESI) *m*/*e* 340.2 [(M+H)⁺, calcd for C₂₀H₂₆N₃O₂, 340.4]; LC/MS retention time (method B): *t*_{R} = 1.33 min. HPLC retention time (method A): *t*_{R} = 8.58 min; HPLC retention time (method B): *t*_{R} = 8.12 min. ¹H NMR (400 MHz, *CD₃OD*) δ ppm 9.51 (s, 1H), 8.79 (d, *J* = 6.02 Hz, 1H), 8.40 (d, *J* = 5.77 Hz, 1H), 8.33 (s, 1H), 7.10 (s, 1H), 4.50 (dd, *J* = 10.67, 3.14 Hz, 1H), 4.36 (dd, *J* = 10.67, 5.90 Hz, 1H), 3.77-3.87 (m, 4H), 2.46 (s, 3H), 1.79-1.92 (m, 2H), 1.66-1.77 (m, 1H), 1.08 (dd, *J* = 6.27, 4.02 Hz, 6H).

### Example 5

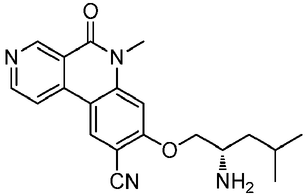

### (S)-8-((2-amino-4-methylpentyl)oxy)-6-methyl-5-oxo-5,6 dihydrobenzo[c][2,7]naphthyridine-9-carbonitrile

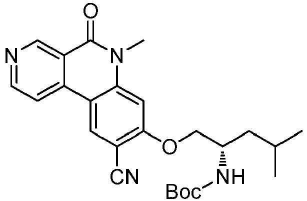

### Part A. (S)-tert-butyl (1-((9-cyano-6-methyl-5-oxo-5,6-dihydrobenzo[c][2,7]naphthyridin-8-yl)oxy)-4-methylpentan-2-yl)carbamate

In a 25 mL sealed tube, (*S*)-*tert*-butyl (1-((9-bromo-6-methyl-5-oxo-5,6-dihydrobenzo[*c*][2,7]naphthyridin-8-yl)oxy)-4-methylpentan-2-yl)carbamate (150 mg, 0.297 mmol) (as prepared in Ex. 3, Part A) was taken up in DMA (4 mL). To this mixture was added CuCN (53.3 mg, 0.595 mmol) and the mixture heated at 150° C for 24 h. The reaction mixture was cooled to room temperature and diluted with ethyl acetate (20 mL) and filtered through diatomaceous earth (Celite®). The ethyl acetate solution was washed with brine (10 mL) and water (10 mL). The organic layer was dried over sodium sulfate and concentrated under reduced pressure to afford the crude product as gummy solid which was carried forward without further purification. LC/MS (ESI) *m*/*e* 451.2 [(M+H)⁺, calcd for C₂₅H₃₁N₄O₄, 451.5]; LC/MS retention time (method B): *t*_{R} = 2.02 min.

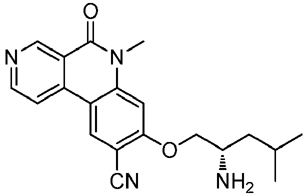

### Part B. (S)-8-((2-amino-4-methylpentyl)oxy)-6-methyl-5-oxo-5,6 dihydrobenzo[c][2,7]naphthyridine-9-carbonitrile

In a 25 mL round-bottomed flask, (*S*)-*tert*-butyl (1-((9-cyano-6-methyl-5-oxo-5,6-dihydrobenzo[*c*][2,7]naphthyridin-8-yl)oxy)-4-methylpentan-2-yl)carbamate (75 mg, 0.167 mmol) was taken up in MeOH (4 mL) and cooled to 0 °C. The mixture was treated with 4M solution of HCl in 1,4-dioxane (2 mL, 8 mmol) and stirred for 2 h at RT. The MeOH was removed and the crude product was dissolved in EtOAc (20 mL). The ethyl acetate layer was washed with water (2x10 mL) and dried over sodium sulfate. Removal of the solvent gave crude product which was purified by silica-gel column (MeOH-CHCl₃ mixture) to obtain (*S*)-8-((2-amino-4-methylpentyl)oxy)-6-methyl-5-oxo-5,6 dihydrobenzo[*c*][2,7]naphthyridine-9-carbonitrile (4 mg, 0.01 mmol, 7% yield over 2 steps) as a yellow solid. LC/MS (ESI) *m*/*e* 351.2. [(M+H)⁺, calcd for C₂₀H₂₃N₄O₂, 351.4]; LC/MS retention time (method B): *t*_{R} = 1.43 min. HPLC retention time (method A): *t*_{R} = 9.69 min; HPLC retention time (method B): *t*_{R} = 9.31 min. ¹H NMR (400 MHz, *CD₃OD*) δ ppm 9.56 (bs, 1H), 8.93 (s, 2H), 8.37 (d, *J* = 4.77 Hz, 1H), 7.25 (s, 1H), 4.63 (dd, *J* = 10.67, 3.14 Hz, 1H), 4.47 (dd, *J* = 10.79, 6.02 Hz, 1H), 3.81-3.89 (m, 4H), 1.83-1.91 (m, 2H), 1.72 (t, *J* = 6.53 Hz, 1H), 1.08 (d, *J* = 5.02 Hz, 6H).

### Example 6

### (S)-8-((2-amino-4-methylpentyl)oxy)-9-cyclopropyl-6-methylbenzo[c][2,7]naphthyridin-5(6H)-one

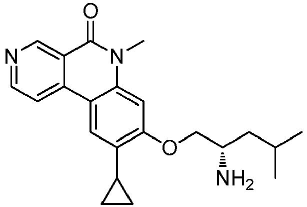

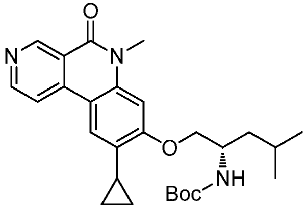

### Part A: (S)-tert-butyl (1-((9-cyclopropyl-6-methyl-5-oxo-5,6-dihydrobenzo[c][2,7]naphthyridin-8-yl)oxy)-4-methylpentan-2-yl)carbamate

A mixture of (*S*)-*tert*-butyl (1-((9-bromo-6-methyl-5-oxo-5,6-dihydrobenzo[*c*][2,7]naphthyridin-8-yl)oxy)-4-methylpentan-2-yl)carbamate (150 mg, 0.297 mmol) (as prepared in Ex. 3, Part A), cyclopropylboronic acid (28.1 mg, 0.327 mmol), tetrakis(triphenylphosphine)palladium (17.2 mg, 0.015 mmol) and Cs₂CO₃ (291 mg, 0.892 mmol) in toluene (5 mL) and water (0.43 mL) mixture was purged with nitrogen for 5 min then heated at 90°C overnight (16 h). After cooling, toluene was removed under reduced pressure and the product was extracted with ethyl acetate (20 mL). The organic phase was washed with brine (2x20 mL), dried over Na₂SO₄, and concentrated under reduced pressure. The residue was purified by silica-gel column chromatography (EtOAc-hexane) to afford (*S*)-*tert*-butyl (1-((9-cyclopropyl-6-methyl-5-oxo-5,6-dihydrobenzo[*c*][2,7]naphthyridin-8-yl)oxy)-4-methylpentan-2-yl)carbamate (70 mg, 0.150 mmol, 51% yield) as an off-white gummy solid. LC/MS (ESI) *m*/*z* 466.4 [(M+H)⁺, calcd for C₂₇H₃₆N₃O₄ 466.3]; LC/MS retention time (method E): t_{R} = 1.19 min.

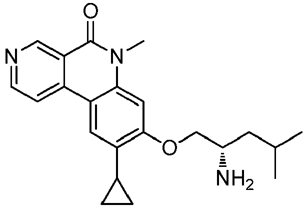

### Part B: (S)-8-((2-amino-4-methylpentyl)oxy)-9-cyclopropyl-6-methylbenzo[c][2,7]naphthyridin-5(6H)-one

To a solution of (*S*)-*tert*-butyl (1-((9-cyclopropyl-6-methyl-5-oxo-5,6-dihydrobenzo[*c*][2,7]naphthyridin-8-yl)oxy)-4-methylpentan-2-yl)carbamate (60 mg, 0.129 mmol) in MeOH (4 mL), at 0 °C was added a 4M solution of HCl in 1,4-dioxane (2 mL, 8 mmol). The resultant solution was stirred for 2 h at RT. The methanol was removed under reduced pressure and the residue was extracted with ethyl acetate (2x5 mL). The organic phase was washed with saturated aqueous NaHCO₃ (2x5 mL), dried over Na₂SO₄, and concentrated under reduced pressure. The residue was purified by silica-gel column (MeOH-CHCl₃) to afford (*S*)-8-((2-amino-4-methylpentyl)oxy)-9-cyclopropyl-6-methylbenzo[*c*][2,7]naphthyridin-5(6*H*)-one (8 mg, 0.022 mmol, 17% yield) as an off-white solid. LC/MS (ESI) *m*/*z* 366.2 [(M+H)⁺, calcd for C₂₂H₂₈N₃O₂ 366.2]; LC/MS retention time (method B): t_{R} = 1.34 min. HPLC retention time (method B): *t*_{R} = 4.87 min and HPLC retention time (method A): *t*_{R} = 9.18 min. ¹H NMR (400 MHz, *CDCl₃*) δ ppm 9.47 (s, 1H), 8.75 (d, *J* = 5.7 Hz, 1H), 8.28 (d, *J* = 5.7 Hz, 1H), 7.96 (s, 1H), 7.06 (s, 1H), 4.27 (m, 1H), 4.06-4.15 (m, 1H), 3.84 (s, 3H), 3.38-3.48 (m, 1H), 2.23-2.38 (m, 1H), 1.83-1.94 (m, 1H), 1.56-1.67 (m, 1H), 1.46-1.55 (m, 1H), 1.00-1.08 (m, 6H), 0.79-0.94 (m, 4H).

### Example 7

### (S)-8-(2-amino-4-methylpentyloxy)-9-hydroxy-6-methylbenzo[c][2,7]naphthyridin-5(6H)-one

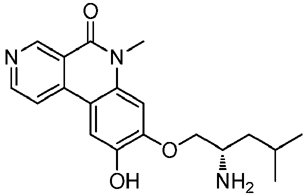

A mixture of (*S*)-*tert*-butyl (1-((9-bromo-6-methyl-5-oxo-5,6-dihydrobenzo[*c*][2,7]naphthyridin-8-yl)oxy)-4-methylpentan-2-yl)carbamate (0.05 g, 0.099 mmol) (as prepared in Ex. 3, Part A), *tetra*-*N*-butylammonium hydroxide.30 H₂O (0.463 g, 1.784 mmol) and water (1.5 mL) was added over 0.1 h to a stirred solution of copper(I) iodide (1.89 mg, 9.91 µmol) and 2-methyl 8-quinolinol (3.16 mg, 0.020 mmol) in DMSO (1 mL). The reaction mixture was heated to 120 °C and stirred for 14 h. The resulting mixture was cooled to room temperature. The crude product was purified by reverse phase HPLC (10 mM ammonium acetate) to afford a (*S*)-8-((2-amino-4-methylpentyl)oxy)-9-hydroxy-6-methylbenzo[*c*][2,7]naphthyridin-5(6*H*)-one (0.004 g, 0.011 mmol, 12% yield) as a brown solid. LC/MS (ESI) *m*/*e* 342.2 [(M+H)⁺, calcd for C₁₉H₂₄N₃O₃, 342.2]; HPLC retention time (method F): *t*_{R} = 1.62 min. HPLC retention time (method B): *t*_{R} = 5.88 min. ¹H NMR (400 MHz, *CD₃OD*) δ 9.39 (s, 1H), 8.62 (d, *J* = 6.00 Hz, 1H), 7.96 (d, *J* = 5.60 Hz, 1H), 7.56 (s, 1H), 6.73 (s, 1H), 3.80 (s, 3H), 3.67-3.70 (m, 1H), 3.49-3.59 (m, 2H), 1.81-1.85 (m, 1H), 1.56-1.63 (m, 2H), 1.05 (d, *J* = 6.80 Hz, 3H), 0.98 (d, *J* = 6.80 Hz, 3H).

### Example 8

### (S)-8-((2-amino-4-methylpentyl)oxy)-6-isopropylbenzo[c][2,7]naphthyridin-5(6H)-one

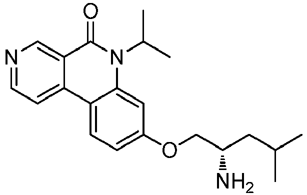

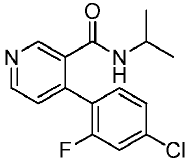

### Part A. 4-(4-chloro-2-fluorophenyl)-N-isopropylnicotinamide

To a solution of 4-(4-chloro-2-fluorophenyl)nicotinic acid (400 mg, 1.590 mmol) (prepared as in Ex. 2, Part A) in DMF (6 mL) cooled to 0 °C was added EDC (457 mg, 2.384 mmol) and HOBT (487 mg, 3.18 mmol) and the mixture was stirred for 5 min. To the resultant solution DIPEA (0.83 mL, 4.77 mmol) followed by propan-2-amine (470 mg, 7.95 mmol) were added and the mixture was stirred at room temperature for 12 h. The reaction mixture was treated with ice and extracted with ethyl acetate (3 x10 mL). The combined organic layers were washed with brine (1x10 mL), dried (Na₂SO₄) and concentrated under reduced pressure to afford 4-(4-chloro-2-fluorophenyl)-N-isopropylnicotinamide (480 mg, 1.64 mmol, 100%) as an off-white solid. LC/MS (ESI) *m*/*e* 293.2 [(M+H)⁺, calcd for C₁₅H₁₅ClFN₂O 293.1]; LC/MS retention time (method C): *t*_{R} = 1.84 min.

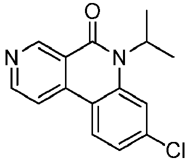

### Part B. 8-chloro-6-isopropylbenzo[c][2,7]naphthyridin-5(6H)-one

To a suspension of sodium hydride (79 mg, 3.28 mmol) in THF (8 mL) at 0 °C was added a solution of 4-(4-chloro-2-fluorophenyl)-N-isopropylnicotinamide (480 mg, 1.640 mmol) in THF (10 mL) dropwise over a period of 10 min. The reaction mixture was stirred at 0 °C for 1 h and then warmed to room temperature and stirred for an additional 1 h. The reaction mixture was then treated with ice and extracted with ethyl acetate (3x5 mL). The combined organic layers were washed with brine (1x5 mL), dried (Na₂SO₄) and concentrated under reduced pressure to afford crude 8-chloro-6-isopropylbenzo[*c*][2,7]naphthyridin-5(6*H*)-one (250 mg, 0.917 mmol, 56% yield). LC/MS (ESI) *m*/*e* 273.0 [(M+H)⁺, calcd for C₁₅H₁₄ClN₂O 273.1]; LC/MS retention time (method C): *t*_{R} = 1.98 min; ¹H NMR (400 MHz, *CDCl₃*) δ ppm 9.67 (s, 1H), 8.70 (d, *J* = 6.0 Hz, 1H), 8.17 - 8.19 (d, *J* = 8.4 Hz, 1H), 7.92 -7.94 (d, *J* = 5.6 Hz, 1H), 7.62 (d, *J* = 1.6 Hz, 1H), 7.26 - 7.31 (dd, *J* = 8.8, 2.0 Hz, 1H), 3.21 - 3.28 (m, 1H), 1.71 (d, *J* = 6.8 Hz, 6H).

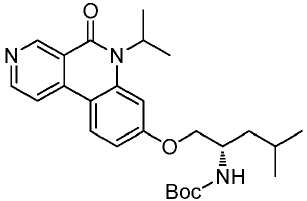

### Part C. (S)-tert-butyl (1-((6-isopropyl-5-oxo-5,6-dihydrobenzo[c][2,7]naphthyridin-8-yl)oxy)-4-methylpentan-2-yl)carbamate

To a solution 8-chloro-6-isopropylbenzo[*c*][2,7]naphthyridin-5(6*H*)-one (250 mg, 0.917 mmol) in toluene (2 mL) at room temperature was added cesium carbonate (448 mg, 1.375 mmol) and di-*tert*-butyl(2',4',6'-triisopropyl-[1,1'-biphenyl]-2-yl)phosphine (234 mg, 0.550 mmol) and the mixture was degassed for 5 min. The mixture was then treated with *N*-Boc-L-leucinol (591 mg, 2.75 mmol) followed by palladium(II)acetate (61.7 mg, 0.275 mmol) and degassed for another 10 min. The reaction mixture was sealed and heated at 80 °C. The reaction mixture was then cooled to room temperature and filtered through diatomaceous earth (Celite®). The bed was washed with ethyl acetate and the combined filtrate was concentrated under reduced pressure to afford crude product which was purified by combi flash (gradient of ethyl acetate and petroleum ether) afford (*S*)-*tert*-butyl (1-((6-isopropyl-5-oxo-5,6-dihydrobenzo[*c*][2,7]naphthyridin-8-yl)oxy)-4-methylpentan-2-yl)carbamate (160 mg, 0.198 mmol, 21% yield) as an off-white solid. LC/MS (ESI) *m*/*e* 454.2 [(M+H)⁺, calcd for C₂₆H₃₆N₃O₄ 454.3]; LC/MS retention time (method C): *t*_{R} = 2.17 min.

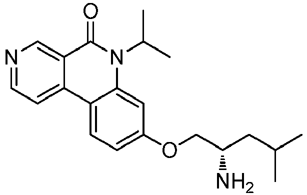

### Part D. (S)-8-((2-amino-4-methylpentyl)oxy)-6-isopropylbenzo[c][2,7]naphthyridin-5(6H)-one

To a solution of ((*S*)-*tert*-butyl (1-((6-isopropyl-5-oxo-5,6-dihydrobenzo[*c*][2,7]naphthyridin-8-yl)oxy)-4-methylpentan-2-yl)carbamate (160mg, 0.353 mmol) in anhydrous dichloromethane (4 mL) was added a 2 M solution of HCl in diethyl ether (0.88 mL, 1.76 mmol) dropwise at 0° C. The reaction mixture was allowed to warm to room temperature and stirred for 2 h. The reaction mixture was then concentrated under reduced pressure to afford crude product which was purified by preparative HPLC (10 mM ammonium acetate in water ; acetonitrile) to afford (*S*)-8-((2-amino-4-methylpentyl)oxy)-6-isopropylbenzo[*c*][2,7]naphthyridin-5(6*H*)-one (45 mg, 0.127 mmol, 36% yield) as an off-white solid. LC/MS (ESI) *m*/*e* 354.2 [(M+H)⁺, calcd for C₂₁H₂₈N₃O₂ 354.2]; LC/MS retention time (method C): *t*_{R} = 1.81 min; HPLC retention time (method A): *t*_{R} = 8.85 min; HPLC retention time (method B): *t*_{R} = 9.50 min. ¹H NMR (400 MHz, *CD₃OD*) δ ppm 9.43 (d, *J* = 0.75 Hz, 1H), 8.75 (d, *J* = 5.77 Hz, 1H), 8.42 (d, *J* = 9.04 Hz, 1H), 8.22 (d, *J* = 5.52 Hz, 1H), 7.30 (d, *J* = 2.26 Hz, 1H), 7.10 (dd, *J* = 8.78, 2.26 Hz, 1H), 5.26 - 5.43 (m, 1H), 4.16 (dd, *J* = 9.16, 3.89 Hz, 1H), 3.98 (dd, *J* = 9.29, 7.03 Hz, 1H), 3.27 - 3.31 (m, 1H), 1.81 - 1.92 (m, 1H), 1.73 (d, *J* = 7.03 Hz, 6H), 1.41 - 1.51 (m, 2H), 1.02 (dd, *J* = 9.54, 6.53 Hz, 6H).

### Example 9

### (S)-6-allyl-8-((2-amino-4-methylpentyl)oxy)benzo[c][2,7]naphthyridin-5(6H)-one

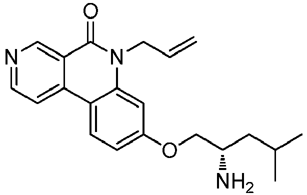

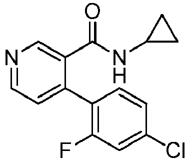

### Part A. 4-(4-chloro-2-fluorophenyl)-N-cyclopropylnicotinamide

To a solution of 4-(4-chloro-2-fluorophenyl)nicotinic acid (500 mg, 1.987 mmol) (prepared as in Ex. 2, Part A) in DMF (6 mL) cooled to 0 °C was added EDC (571 mg, 2.98 mmol) and HOBT (609 mg, 3.97 mmol) and the mixture was stirred for 5 min. To the resultant solution DIPEA (1.04 mL, 5.96 mmol) followed by cyclopropanamine (567 mg, 9.93 mmol) was added and the mixture was stirred at room temperature for 12 h. The reaction mixture was treated with ice and extracted with ethyl acetate (3x10 mL). The combined organic layers were washed with brine (1x10 mL), dried (Na₂SO₄) and concentrated under reduced pressure to afford 4-(4-chloro-2-fluorophenyl)-*N*-cyclopropylnicotinamide (480 mg, 1.65 mmol, 83% yield). LC/MS (ESI) *m*/*e* 291.0 [(M+H)⁺, calcd for C₁₅H₁₃ClFN₂O 291.1]; LC/MS retention time (method C): *t*_{R} = 1.79 min.

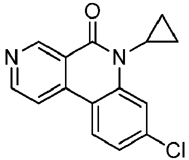

### Part B. 8-chloro-6-cyclopropylbenzo[c][2,7]naphthyridin-5(6H)-one

To a suspension of sodium hydride (72.6 mg, 3.03 mmol) in THF (8 mL) at 0 °C was added a solution of 4-(4-chloro-2-fluorophenyl)-*N*-cyclopropylnicotinamide (440 mg, 1.51 mmol) in THF (10 mL) dropwise over a period of 10 min. The reaction mixture was stirred at 0 °C for 1 h and then warmed to room temperature and stirred for another 1 h. The reaction mixture was then treated with ice and extracted with ethyl acetate (3x5 mL). The combined organic layers were washed with brine (2x5 mL), dried (Na₂SO₄) and concentrated under reduced pressure to afford crude 8-chloro-6-cyclopropylbenzo[*c*][2,7]naphthyridin-5(*6*H)-one (340 mg, 1.26 mmol, 83% yield) as an off-white solid. LC/MS (ESI) *m*/*e* 271.0 [(M+H)⁺, calcd for C₁₅H₁₂ClN₂O 271.1]; LC/MS retention time (method C): *t*_{R} = 1.89 min.

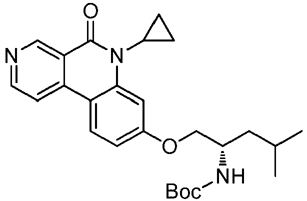

### Part C. (S)-tert-butyl (1-((6-cyclopropyl-5-oxo-5,6-dihydrobenzo[c][2,7]naphthyridin-8-yl)oxy)-4-methylpentan-2-yl)carbamate

To a solution 8-chloro-6-cyclopropylbenzo[*c*][2,7]naphthyridin-5(6*H*)-one (200 mg, 0.739 mmol) in toluene (2 mL) at room temperature was added cesium carbonate (361 mg, 1.108 mmol) and di-*tert*-butyl(2',4',6'-triisopropyl-[1,1'-biphenyl]-2-yl)phosphine (188 mg, 0.443 mmol) and the mixture was degassed with nitrogen for 5 min. The mixture was then treated with *N*-Boc-L-leucinol (477 mg, 2.216 mmol) followed by palladium(II)acetate (49.8 mg, 0.222 mmol) and degassed for another 10 min. The reaction mixture was sealed and heated at 80 °C. After overnight stirring the reaction mixture was cooled to room temperature and filtered through diatomaceous earth (Celite®). The bed was washed with ethyl acetate and the combined filtrate was concentrated under reduced pressure to afford crude product which was purified by combi flash (ethyl acetate and petroleum ether) to afford (*S*)-*tert*-butyl (1-((6-cyclopropyl-5-oxo-5,6-dihydrobenzo[*c*][2,7]naphthyridin-8-yl)oxy)-4-methylpentan-2-yl)carbamate (100 mg, 0.195 mmol, 26% yield) as an off-white solid. LC/MS (ESI) *m*/*e* 452.2 [(M+H)⁺, calcd for C₂₆H₃₄N₃O₄ 452.3]; LC/MS retention time (method C): *t*_{R} = 2.11 min.

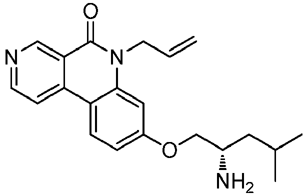

### Part D. (S)-6-allyl-8-((2-amino-4-methylpentyl)oxy)benzo[c][2,7]naphthyridin-5(6H)-one

To a solution of (*S*)-*tert*-butyl (1-((6-cyclopropyl-5-oxo-5,6-dihydrobenzo[*c*][2,7]naphthyridin-8-yl)oxy)-4-methylpentan-2-yl)carbamate (100 mg, 0.221 mmol) in anhydrous dichloromethane (2 mL) was added a 2 M solution of HCl in diethyl ether (0.176 mL, 0.353 mmol) dropwise at 0 °C. The reaction mixture was allowed to warm to room temperature and stirred for 2 h. The reaction mixture was then concentrated under reduced pressure to afford crude product which was purified by preparative HPLC (0.1% TFA in water) to afford (*S*)-6-allyl-8-((2-amino-4-methylpentyl)oxy)benzo[*c*][2,7]naphthyridin-5(6*H*)-one (8 mg, 22.76 mmol, 10% yield) as a yellow solid. LC/MS (ESI) *m*/*e* 352.2 [(M+H)⁺, calcd for C₂₁H₂₆N₃O₂ 352.2]; LC/MS retention time (method C): *t*_{R} = 1.80 min; HPLC retention time (method A): *t*_{R} = 8.06 min; HPLC retention time (method B): *t*_{R} = 9.21 min. ¹H NMR (400 MHz, *CD₃OD*) δ ppm 9.56 (s, 1 H), 8.85 (d, *J* = 6.27 Hz, 1H), 8.55 (d, *J* = 9.04 Hz, 1H), 8.48 (d, *J* = 6.27 Hz, 1H), 7.20 (dd, *J* = 8.91, 2.38 Hz, 1H), 7.14 (d, *J* = 2.26 Hz, 1H), 6.08 (dd, *J* = 17.32, 10.54 Hz, 1H), 5.28 (dd, *J* = 10.54, 1.00 Hz, 1H), 5.18 (dd, *J* = 17.32, 1.25 Hz, 1H), 5.09 - 5.13 (m, 2H), 4.44 (dd, *J* = 10.54, 3.26 Hz, 1H), 4.27 (dd, *J* = 10.42, 6.40 Hz, 1H), 3.77 (dd, *J* = 6.78, 3.51 Hz, 1H), 1.64- 1.88 (m, 3H), 1.07 (dd, *J* = 6.40, 4.89 Hz, 6H).

### Example 10

### (S)-8-((2-amino-4-methylpentyl)oxy)-7,9-dichloro-6-methylbenzo[c][2,7]naphthyridin-5(6H) one

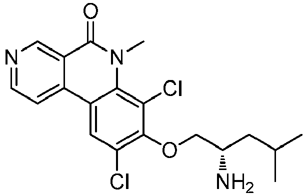

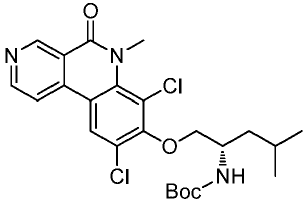

### Part A. (S)-tert-butyl (1-((7,9-dichloro-6-methyl-5-oxo-5,6-dihydrobenzo[c][2,7]naphthyridin-8-yl)oxy)-4-methylpentan-2-yl)carbamate

To a stirred solution of (*S*)-*tert*-butyl (4-methyl-1-((6-methyl-5-oxo-5,6-dihydrobenzo[*c*][2,7]naphthyridin-8-yl)oxy)pentan-2-yl)carbamate (0.120 g, 0.282 mmol) (prepared as in Ex. 2, Part D) in anhydrous acetonitrile (2 mL) under nitrogen was added *N*-chlorosuccinimide (0.045 g, 0.338 mmol) and the solution was heated at 85 °C for 12 h. The reaction mixture was diluted with water (10 mL) and extracted with ethyl acetate (2x20 mL). The combined organic layers were dried over sodium sulfate and evaporated under reduced pressure to afford crude product which was purified by preparative TLC (ethyl acetate in petroleum ether) to afford (*S*)-*tert*-butyl (1-((7,9-dichloro-6-methyl-5-oxo-5,6-dihydrobenzo[*c*][2,7]naphthyridin-8-yl)oxy)-4-methylpentan-2-yl)carbamate (120 mg, 0.243 mmol, 86% yield, with 55% purity by LC/MS) as a yellow solid. LC/MS (ESI) *m*/*e* 494.2 [(M+H)⁺, calcd for C₂₄H₃₀Cl₂N₃O₄ 494.1]; LC/MS retention time (method A): *t*_{R} = 2.24 min.

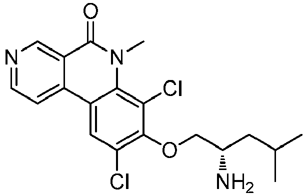

### Part B. (S)-8-((2-amino-4-methylpentyl)oxy)-7,9-dichloro-6-methylbenzo[c][2,7]naphthyridin-5(6H)-one

To a solution of (*S*)-*tert*-butyl (1-((7,9-dichloro-6-methyl-5-oxo-5,6-dihydrobenzo[*c*][2,7]naphthyridin-8-yl)oxy)-4-methylpentan-2-yl)carbamate (0.120 g, 0.243 mmol) in anhydrous methanol (5 mL) was added a 4M solution of HCl in 1,4-1,4-dioxane (2 mL, 8 mmol) dropwise at 0° C. The reaction mixture was allowed to warm to room temperature and stirred for 2 h. The reaction mixture was then concentrated under reduced pressure to afford crude compound which was purified by preparative HPLC (10 mM ammonium acetate in water; acetonitrile) to afford (*S*)-8-((2-amino-4-methylpentyl)oxy)-7,9-dichloro-6-methylbenzo[*c*][2,7]naphthyridin-5(6*H*)-one (28 mg, 0.063 mmol, 26% yield) as a yellow solid. LC/MS (ESI) *m*/*e* 394.2 [(M+H)⁺, calcd for C₁₉H₂₂Cl₂N₃O₂ 394.1]; LC/MS retention time (method A): *t*_{R} = 1.48 min; HPLC retention time (method A): *t*_{R} = 5.21 min; HPLC retention time (method B): *t*_{R} = 5.73 min. ¹H NMR (400 MHz, *CD₃OD*) δ ppm 9.50 (d, *J* = 0.75 Hz, 1H), 8.87 (d, *J* = 5.52 Hz, 1H), 8.58 (s, 1 H), 8.32 (d, *J* = 5.27 Hz, 1H), 4.17 (dd, *J*= 9.03, 4.02 Hz, 1H), 4.05 (dd, *J* = 9.03, 7.28 Hz, 1H), 3.93 (s, 3H), 3.37 - 3.43 (m, 1H), 1.82 - 1.93 (m, 1H), 1.38 - 1.57 (m, 2H), 0.99 - 1.05 (m, 6H).

### Example 11

### (S)-8-((2-amino-4-methylpentyl)oxy)-6-benzylbenzo[c][2,7]naphthyridin-5(6H)-one

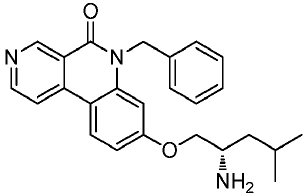

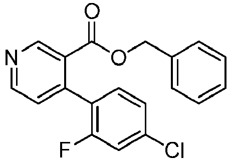

### Part A. N-benzyl-4-(4-chloro-2-fluorophenyl)nicotinamide (S)-8-(2-amino-4-methylpentyloxy)-6-benzylbenzo[c][2,7]naphthyridin-5(6H)-one

To a solution of 4-(4-chloro-2-fluorophenyl)nicotinic acid (600mg, 2.38 mmol) (as prepared in Ex. 2, Part A) in DMF (6 mL) cooled to 0 °C was added EDC (457 mg, 2.38 mmol) and HOBT (365 mg, 2.38 mmol) and the mixture was stirred for 5 min. To the resultant solution was added DIPEA (0.416 mL, 2.384 mmol) followed by benzyl amine (307 mg, 2.86 mmol) and the mixture was stirred at room temperature for 12 h. The reaction mixture was treated with ice and extracted with ethyl acetate (3x10 mL). The combined organic layers were washed with brine (1x10 mL), dried (Na₂SO₄) and concentrated under reduced pressure to afford *N*-benzyl-4-(4-chloro-2-fluorophenyl)nicotinamide (680 mg, 2.00 mmol, 84% yield). LC/MS (ESI) *m*/*e* 341.0 [(M+H)⁺, calcd for C₁₉H₁₅ClFN₂O 341.1]; LC/MS retention time (method A): *t*_{R} = 1.73 min.

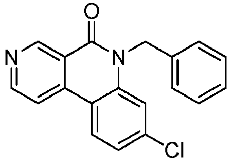

### Part B. 6-benzyl-8-chlorobenzo[c][2,7]naphthyridin-5(6H)-one

To a suspension of sodium hydride (96 mg, 3.99 mmol) in THF (13 mL) at 0 °C was added a solution of *N*-benzyl-4-(4-chloro-2-fluorophenyl)nicotinamide (680 mg, 1.995 mmol) in THF (10 mL) dropwise over a period of 10 min. The reaction mixture was stirred at 0 °C for 1 h and then warmed to room temperature and stirred for another 1 h. The reaction mixture was then treated with ice and extracted with ethyl acetate (3x5 mL). The combined organic layers were washed with brine (1x5 mL), dried (Na₂SO₄) and concentrated under reduced pressure to afford crude 6-benzyl-8-chlorobenzo[*c*][2,7]naphthyridin-5(6*H*)-one (640 mg, 2.00 mmol, 100%). LC/MS (ESI) *m*/*e* 321.0 [(M+H)⁺, calcd for C₁₉H₁₄ClN₂O 321.0]; LC/MS retention time (method A): *t*_{R} = 1.95 min.

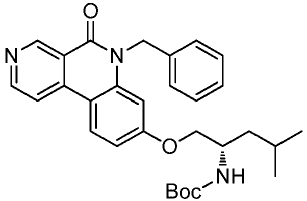

### Part C. (S)-tert-butyl (1-((6-benzyl-5-oxo-5,6-dihydrobenzo[c][2,7]naphthyridin-8-yl)oxy)-4-methylpentan-2-yl)carbamate

To a solution 6-benzyl-8-chlorobenzo[*c*][2,7]naphthyridin-5(6*H*)-one (640mg, 2.00 mmol) in toluene (12 mL) at room temperature, was added cesium carbonate (975 mg, 2.99 mmol) and *N*-Boc L-Leucinol (1287 mg, 5.99 mmol) and the mixture was degassed with nitrogen for 5 min. The mixture was then treated with di-*tert*-butyl(2',4',6'-triisopropyl-[1,1'-biphenyl]-2-yl)phosphine (508 mg, 1.197 mmol) followed by palladium(II)acetate (448 mg, 2.00 mmol) and degassed for another 10 min. The reaction mixture was sealed and heated at 80 °C. After overnight stirring the reaction mixture was cooled to room temperature and filtered through diatomaceous earth (Celite®). The bed was washed with ethyl acetate and the combined filtrate was concentrated under reduced pressure to afford crude product which was purified by combi flash (ethyl acetate/petroleum ether) to afford (*S*)-*tert-*butyl (1-((6-benzyl-5-oxo-5,6-dihydrobenzo[*c*][2,7]naphthyridin-8-yl)oxy)-4-methylpentan-2-yl)carbamate (500mg, 0.738 mmol, 37% yield) as an off-white solid. LC/MS (ESI) *m*/*e* 502.3 [(M+H)⁺, calcd for C₃₀H₃₆N₃O₄ 502.4]; LC/MS retention time (method D): *t*_{R} = 1.26 min.

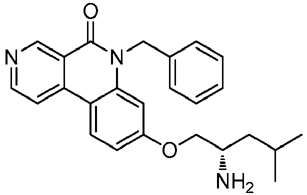

### Part D. (S)-8-((2-amino-4-methylpentyl)oxy)-6-benzylbenzo[c][2,7]naphthyridin-5(6H)-one

To a solution of (*S*)-*tert*-butyl (1-((6-benzyl-5-oxo-5,6-dihydrobenzo[*c*][2,7]naphthyridin-8-yl)oxy)-4-methylpentan-2-yl)carbamate (35mg, 0.070 mmol) in anhydrous dichloromethane (1 mL) was added a 2 M solution of HCl in diethyl ether (0.17 mL, 0.349 mmol) dropwise at 0° C. The reaction mixture was allowed to warm to room temperature and stirred for 2 h. The reaction mixture was then concentrated under reduced pressure to afford crude product which was purified by preparative TLC (ethyl acetate in petroleum ether) to afford (*S*)-8-((2-amino-4-methylpentyl)oxy)-6-benzylbenzo[*c*][2,7]naphthyridin-5(6*H*)-one (10 mg, 0.024 mmol, 35% yield) as a light yellow solid. LC/MS (ESI) *m*/*e* 402.2 [(M+H)⁺, calcd for C₂₅H₂₈N₃O₂ 402.2]; LC/MS retention time (method C): *t*_{R} = 1.77 min; HPLC retention time (method A): *t*_{R} = 5.54 min; HPLC retention time (method B): *t*_{R} = 6.17 min. ¹H NMR (400 MHz *CD₃OD*) δ ppm 9.61 (br. s., 1H), 8.87 (d, *J* = 4.52 Hz, 1H), 8.53 (d, *J* = 9.04 Hz, 1H), 8.47 (d, *J* = 6.02 Hz, 1H), 7.28 - 7.38 (m, 5H), 7.15 (dd, *J* = 8.91, 2.38 Hz, 1H), 7.04 (d, *J* = 2.26 Hz, 1H), 5.72 (d, *J* = 5.77 Hz, 2H), 4.27 (dd, *J* = 10.54, 3.26 Hz, 1H), 4.10 (dd, *J* = 10.42, 6.40 Hz, 1H), 3.69 (dd, *J* = 6.65, 3.14 Hz, 1H), 1.68 - 1.81 (m, 1H), 1.57 - 1.67 (m, 2H), 1.00 - 1.05 (m, 6H).

### Example 12

### (S)-8-((2-amino-4-methylpentyl)oxy)-6-(2-methoxyethyl)benzo[c][2,7]naphthyridin-5(6H)-one

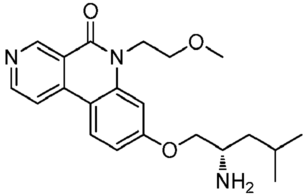

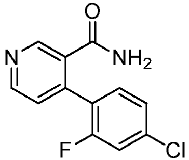

### Part A. 4-(4-chloro-2-fluorophenyl)nicotinamide

To a stirred solution of 4-(4-chloro-2-fluorophenyl)nicotinic acid (1.1 g, 4.37 mmol) (as prepared in Ex. 2, Part A) in anhydrous DMF (10 mL) cooled to 0° C was added HOBT (1.339 g, 8.74 mmol), EDC (1.257 g, 6.56 mmol), DIEA (3.05 mL, 17.49 mmol) followed by ammonium chloride (1.169 g, 21.86 mmol). The reaction mixture was allowed to warm to RT and stirred for 12 h under a nitrogen atmosphere. The reaction mixture was diluted with water (50 mL) and extracted with ethyl acetate (2x100 mL). The combined organic layers were dried over sodium sulfate and concentrated under reduced pressure to afford 4-(4-chloro-2-fluorophenyl)nicotinamide (0.900 g, 3.59 mmol, 82% yield) LC/MS (ESI) *m*/*e* 251 [(M+H)⁺, calcd for C₁₂H₉ClFN₂O 251]; LC/MS retention time (method A): *t*_{R} = 1.34 min.

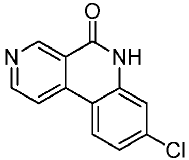

### Part B. 8-chlorobenzo[c][2,7]naphthyridin-5(6H)-one

To a solution of 4-(4-chloro-2-fluorophenyl)nicotinamide (0.9 g, 3.59 mmol) in THF (20 mL) at 0 °C was added sodium hydride (0.258 g, 10.77 mmol) and the reaction mixture was stirred at 0 °C for 1 h and then warmed to room temperature and stirred overnight (14 h). The reaction mixture was then quenched with ice and extracted with ethyl acetate (2x100 mL). The combined organic layers were washed with brine (1x100 mL), dried (Na₂SO₄) and concentrated under reduced pressure to afford crude 8-chlorobenzo[*c*][2,7]naphthyridin-5(6H)-one (0.80 g, 3.47 mmol, 97% yield). LC/MS (ESI) *m*/*e* 231.2 [(M+H)⁺, calcd for C₁₂H₈ClN₂O 231]; LC/MS retention time (method D): *t*_{R} = 0.61 min.

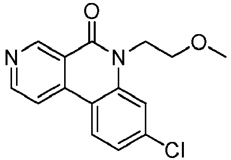

### Part C. 8-chloro-6-(2-methoxyethyl)benzo[c][2,7]naphthyridin-5(6H)-one

To a stirred solution of 8-chlorobenzo[*c*][2,7]naphthyridin-5(6*H*)-one (0.150 g, 0.650 mmol) in anhydrous DMF (2 mL) at 0 °C was added 2-bromoethyl methyl ether (0.090 g, 0.650 mmol) under nitrogen. The reaction mixture was allowed to warm to RT and stirred for 12 h. The reaction mixture was diluted with water (10 mL) and extracted with ethyl acetate (2 x 20 mL). The combined organic layers were dried over sodium sulfate and concentrated under reduced pressure to afford 8-chloro-6-(2-methoxyethyl)benzo[*c*][2,7]naphthyridin-5(6*H*)-one (150mg, 0.520 mmol, 80% yield). LC/MS (ESI) *m*/*e* 289.1 [(M+H)⁺, calcd for C₁₅H₁₃ClN₂O₂ 289.1]; LC/MS retention time (method A): *t*_{R} = 1.65 min.

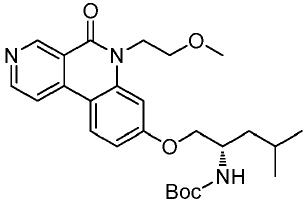

### Part D. (S)-tert-butyl (1-((6-(2-methoxyethyl)-5-oxo-5,6-dihydrobenzo[c][2,7]naphthyridin-8-yl)oxy)-4-methylpentan-2-yl)carbamate

To a solution 8-chloro-6-cyclopropylbenzo[*c*][2,7]naphthyridin-5(6*H*)-one (100 mg, 0.346 mmol) in toluene (5 mL) at room temperature was added cesium carbonate (169 mg, 0.520 mmol) and *N*-Boc-L-leucinol (226 mg, 1.039 mmol) and the mixture was degassed with nitrogen for 5 min. The mixture was then treated with di-*tert*-butyl(2',4',6'-triisopropyl-[1',1'-biphenyl]-2-yl)phosphine (47.8 mg, 0.208 mmol) followed by palladium(II)acetate (23.3 mg, 0.104 mmol) and degassed for another 10 min. The reaction mixture was sealed and heated at 80 °C. After overnight stirring the reaction mixture was cooled to room temperature and filtered through diatomaceous earth (Celite®). The bed was washed with ethyl acetate (15 mL) and the filtrate was concentrated under reduced pressure to afford crude product which was purified by combi flash (gradient of ethyl acetate and petroleum ether) afford (*S*)-*tert*-butyl (1-((6-(2-methoxyethyl)-5-oxo-5,6-dihydrobenzo[*c*][2,7]naphthyridin-8-yl)oxy)-4-methylpentan-2-yl)carbamate (80 mg, 0.170 mmol, 49% yield) as a brown gum. LC/MS (ESI) *m*/*e* 470.3 [(M+H)⁺, calcd C₂₆H₃₆N₃O₅ 470.3]; LC/MS retention time (method A): *t*_{R} = 2.02 min.

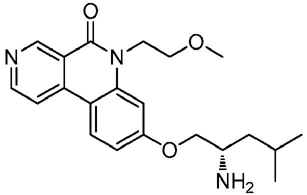

### Part E. (S)-8-((2-amino-4-methylpentyl)oxy)-6-(2 methoxyethyl)benzo[c][2,7]naphthyridin-5(6H)-one

To a solution of (*S*)-*tert*-butyl (1-((6-(2-methoxyethyl)-5-oxo-5,6-dihydrobenzo[*c*][2,7]naphthyridin-8-yl)oxy)-4-methylpentan-2-yl)carbamate (0.080 g, 0.170 mmol) in anhydrous methanol (5 mL) was added a 4M solution of HCl in 1,4-dioxane (0.21 mL, 0.85 mmol) dropwise at 0°C. The reaction mixture was allowed to warm to room temperature and stirred for 2 h. The reaction mixture was concentrated under reduced pressure to afford crude compound which was purified by preparative HPLC (10 mM ammonium acetate in water:acetonitrile) to afford (*S*)-8-((2-amino-4-methylpentyl)oxy)-6-(2-methoxyethyl)benzo[*c*][2,7]naphthyridin-5(6*H*)-one (20 mg, 0.054 mmol, 32% yield) a white solid. LC/MS (ESI) *m*/*e* 370.2 [(M+H)⁺, calcd for C₂₁H₂₈N₃O₃ 370.2]; LC/MS retention time (method A): *t*_{R} = 1.34 min; HPLC retention time (method A): *t*_{R} = 8.36 min; HPLC retention time (method B): *t*_{R} = 8.78 min. ¹H NMR (400 MHz, *CD₃OD*) δ ppm 9.45 (s, 1H), 8.76 (d, *J* = 5.77 Hz, 1H), 8.40 (d, *J* = 9.03 Hz, 1H,), 8.23 (d, *J* = 6.02 Hz, 1H), 7.29 (d, *J* = 2.26 Hz, 1H), 7.11 (d, *J* = 8.78 Hz, 1H), 4.61 (t, *J* = 5.65 Hz, 2H), 4.36 (s, 1H), 4.20 (s, 1H), 3.81 (t, *J* = 5.77 Hz, 2H), 3.63 - 3.71 (m, 1H), 3.37 (s, 3H), 1.86 (m, 1H), 1.67 (qt, *J* = 14.01, 7.09 Hz, 2H), 1.06 (d, *J* = 5.77 Hz, 6H).

### Example 13

### (S)-8-((2-amino-4-methylpentyl)oxy)-6-(cyclopropylmethyl)benzo[c][2,7]naphthyridin-5(6H)-one

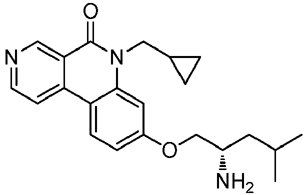

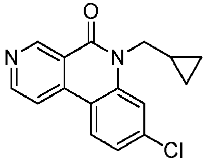

### Part A. 8-chloro-6-(cyclopropylmethyl)benzo[c][2,7]naphthyridin-5(6H)-one

To a stirred solution of 8-chlorobenzo[*c*][2,7]naphthyridin-5(6*H*)-one (100 mg, 0.434 mmol) (as prepared in Ex. 12, Part B) in anhydrous DMF (2 mL) at 0 °C was added cyclopropyl methyl bromide (0.088 g, 0.650 mmol) under nitrogen. The reaction mixture was allowed to warm to RT and stirred for 12 h. The reaction mixture was diluted with water (10 mL) and extracted with ethyl acetate (2x20 mL). The combined organic layers were dried over sodium sulfate and concentrated under reduced pressure to 8-chloro-6-(cyclopropylmethyl)benzo[*c*][2,7]naphthyridin-5(6*H*)-one (100 mg, 0.351 mmol, 81 % yield, 53% pure by LC/MS). LC/MS (ESI) *m*/*e* 285.1 [(M+H)⁺, calcd for C₁₆H₁₄ClN₂O 285.1]; LC/MS retention time (method D): *t*_{R} = 0.91 min.

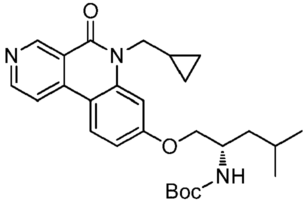

### Part B. (S)-tert-butyl (1-((6-(cyclopropylmethyl)-5-oxo-5,6-dihydrobenzo[c][2,7]naphthyridin-8-yl)oxy)-4-methylpentan-2-yl)carbamate

To a solution 8-chloro-6-(cyclopropylmethyl)benzo[*c*][2,7]naphthyridin-5(6*H*)-one (100 mg, 0.351 mmol) in anhydrous toluene (5 mL) at room temperature was added cesium carbonate (172 mg, 0.527 mmol) and Boc-L-leucinol (229 mg, 1.054 mmol) and the mixture was degassed with nitrogen for 5 min. The mixture was then treated with di-*tert*-butyl(2',4',6'-triisopropyl-[1,1'-biphenyl]-2-yl)phosphine (48.5 mg, 0.211 mmol) followed by palladium(II)acetate (23.65 mg, 0.105 mmol) and degassed for another 10 min. The reaction mixture was sealed and heated at 80 °C. After overnight stirring the reaction mixture was cooled to room temperature and filtered through diatomaceous earth (Celite®). The bed was washed with ethyl acetate (15 mL) and the filtrate was concentrated under reduced pressure to afford crude compound which was purified by preparative HPLC (ethyl acetate in petroleum ether) to afford (*S*)-*tert*-butyl (1-((6-(cyclopropylmethyl)-5-oxo-5,6-dihydrobenzo[*c*][2,7]naphthyridin-8-yl)oxy)-4-methylpentan-2-yl)carbamate (80 mg, 0.172 mmol, 49% yield) as a brown gummy solid. LC/MS (ESI) *m*/*e* 466.3 [(M+H)⁺, calcd C₂₇H₃₆N₃O₄ 466.3]; LC/MS retention time (method A): *t*_{R} = 2.199 min.

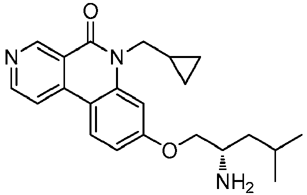

### Part C. (S)-8-((2-amino-4-methylpentyl)oxy)-6 (cyclopropylmethyl)benzo[c][2,7]naphthyridin-5(6H)-one

To a solution of (*S*)-*tert*-butyl (1-((6-(cyclopropylmethyl)-5-oxo-5,6-dihydrobenzo[*c*][2,7]naphthyridin-8-yl)oxy)-4-methylpentan-2-yl)carbamate (0.08 g, 0.172 mmol) in anhydrous methanol (5 mL) was added a 4M solution of HCl in 1,4-dioxane (2 mL, 65.8 mmol) dropwise at 0 °C. The reaction mixture was allowed to warm to room temperature and stirred for 2 h. The reaction mixture was concentrated under reduced pressure to afford crude compound which was purified by preparative HPLC (10 mM ammonium acetate in water:acetonitrile) to afford (*S*)-8-((2-amino-4-methylpentyl)oxy)-6-(cyclopropylmethyl)benzo[*c*][2,7]naphthyridin-5(6*H*)-one (22 mg, 0.060 mmol, 35% yield) as a yellow gum. LC/MS (ESI) *m*/*e* 366.2 [(M+H)⁺, calcd for C₂₂H₂₈N₃O₂ 366.2]; LC/MS retention time (method A): *t*_{R} = 1.47 min; HPLC retention time (method A): *t*_{R} = 9.470 min; HPLC retention time (method B): *t*_{R} = 5.250 min. ¹H NMR (400 MHz, *CD₃OD*) δ ppm 9.47 (d, *J* = 0.50 Hz, 1H), 8.78 (d, *J* = 5.77 Hz, 1H), 8.46 (d, *J* = 9.04 Hz, 1H), 8.26 (d, *J* = 5.52 Hz, 1H), 7.28 (d, *J* = 2.26 Hz, 1H), 7.14 (dd, *J* = 9.03, 2.26 Hz, 1H), 4.39 (d, *J* = 6.78 Hz, 2H), 4.31 (dd, *J* = 9.79, 3.76 Hz, 1H), 4.13 (dd, *J* = 9.79, 6.78 Hz, 1H), 3.53 (dd, *J* = 7.15, 3.64 Hz, 1H), 1.87 (m, 1H), 1.59 (q, *J* = 13.72, 7.03 Hz, 2H), 1.36 - 1.45 (m, 1H), 1.04 (dd, *J* = 7.28, 6.78 Hz, 6H), 0.57 - 0.63 (m, 4H).

### Example 14

### (S)-8-((2-amino-4-methylpentyl)oxy)-9-fluoro-6-methylbenzo[c][2,7]naphthyridin-5(6H)-one

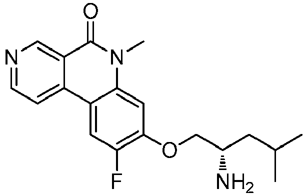

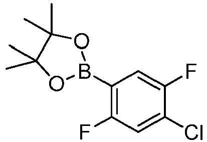

### Part A. 2-(4-chloro-2,5-difluorophenyl)-4,4,5,5-tetramethyl-1,3,2-dioxaborolane

To a solution of 1-bromo-4-chloro-2,5-difluorobenzene (200 mg, 0.879 mmol) in THF (10 mL) cooled to -10 °C was added isopropylmagnesium bromide (1M in THF, 1.055 mL, 1.055 mmol) dropwise and the reaction mixture was stirred at this temperature for 1 h. The reaction mixture was then warmed to 0 °C and stirred for another 1 h. The resultant mixture was again cooled to -10 °C and treated dropwise with a solution of 2-isopropoxy-4,4,5,5-tetramethyl-1,3,2-dioxaborolane (196 mg, 1.055 mmol). The reaction mixture was allowed to warm up to room temperature and treated with a saturated solution of ammonium chloride (3 mL). The layers were separated and aqueous layer was extracted with dichloromethane (2 x 2 mL). The combined organic layer was dried over sodium sulfate, filtered and concentrated under reduced pressure to afford crude compound which was purified by column chromatography on a silica (7:3 - Ethyl acetate:hexane) to afford 2-(4-chloro-2,5-difluorophenyl)-4,4,5,5-tetramethyl-1,3,2-dioxaborolane (150 mg, 0.546 mmol, 62% yield) as an oil. ¹H NMR (400 MHz, *DMSO-d₆*) δ ppm 7.46 - 7.49 (m, 1H), 7.09 - 7.13 (m, 1 H), 1.35 (s, 12H).

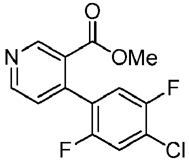

### Part B. methyl 4-(4-chloro-2,5-difluorophenyl)nicotinate

To a stirred solution of methyl 4-chloronicotinate (100 mg, 0.583 mmol) in a solvent mixture of 1,4-dioxane:water (4:1, 20 mL) at room temperature under nitrogen atmosphere was added potassium carbonate (62.6 mg, 0.453 mmol) followed by tetrabutylammonium bromide (73.0 mg, 0.226 mmol). The resultant mixture was degassed with nitrogen for 10 min and treated with *N*-(6-chloro-4-(trimethylstannyl)pyridin-2-yl)acetamide (60.4 mg, 0.181 mmol) followed by bis(triphenylphosphine)palladium(II)chloride (10.60 mg, 0.015 mmol). The mixture was degassed again for 1o min and heated at 80 °C for 8 h. The reaction mixture was then cooled to room temperature and quenched with water (20 mL) and extracted with ethyl acetate (5 mL). The organic layer was separated and washed with water (3x 5 mL), dried (Na₂SO₄) and concentrated under reduced pressure to afford crude methyl 4-(4-chloro-2,5-difluorophenyl)nicotinate (400 mg, 0.592 mmol, 20% yield, 42 % pure by LC/MS). This was taken to the next step without further purification. LC/MS (ESI) *m*/*e* 284.0 [(M+H)⁺, calcd for C₁₃H₉ClF₂NO₂ 284.02]; LC/MS retention time (method A): *t*_{R} = 1.82 min.

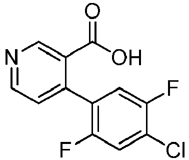

### Part C. 4-(4-chloro-2,5-difluorophenyl)nicotinic acid

To a solution of methyl 4-(4-chloro-2,5-difluorophenyl)nicotinate (400 mg, 1.41 mmol) in water:THF (1:1, 8 mL) was added LiOH (67.5 mg, 2.82 mmol) and the reaction mixture was stirred at room temperature for 4 h. The reaction mixture was quenched with water (5 mL) and washed with ethyl acetate (2x5 mL). The aqueous layer was separated and acidified with 1.5N HCl and concentrated under reduced pressure to afford 4-(4-chloro-2,5-difluorophenyl)nicotinic acid (180 mg, 0.541 mmol, 38% yield) as a solid. LC/MS (ESI) *m*/*e* 270 [(M+H)⁺, calcd for C₁₂H₇ClF₂NO₂ 270]; LC/MS retention time (method B): *t*_{R} = 1.48 min.

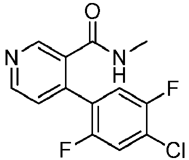

### Part D. 4-(4-chloro-2,5-difluorophenyl)-N-methylnicotinamide

4-(4-chloro-2,5-difluorophenyl)nicotinic acid (170 mg, 0.630 mmol) was taken in thionyl chloride (0.14 mL, 1.89 mmol) and heated at 65 °C for 1 h. The solvent was evaporated under reduced pressure to afford a crude oil which was dissolved in dichloromethane and added to a mixture of methanamine hydrochloride (63.9 mg, 0.946 mmol) and DIEA (0.11 mL, 0.63 mmol) in DCM (8 mL) dropwise at 0 °C. The reaction mixture was then allowed to warm to room temperature and stirred for 7 h. The reaction mixture was diluted with DCM (5 mL) and washed with water (3x 5 mL) followed by brine (1x10 mL). The combined organic extracts were dried over sodium sulfate and concentrated under reduced pressure to provide the crude product which was purified by preparative thin layer chromatography (3:2 Ethyl acetate:hexane) to afford 4-(4-chloro-2,5-difluorophenyl)-*N*-methylnicotinamide (90 mg, 0.264 mmol, 42% yield) as a white solid. LC/MS (ESI) *m*/*e* 283.0 [(M+H)⁺, calcd for C₁₃H₁₀ClF₂N₂O 283.0]; LC/MS retention time (method A): *t*_{R} = 1.46 min.

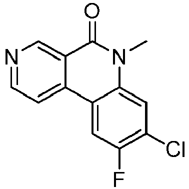

### Part E. 8-chloro-9-fluoro-6-methylbenzo[c][2,7]naphthyridin-5(6H)-one

To a suspension of sodium hydride (56.6 mg, 1.42 mmol) in THF (5 mL) at 0 °C was slowly added 4-(4-chloro-2,5-difluorophenyl)-*N*-methylnicotinamide (100 mg, 0.354 mmol) in THF (5 mL). The resultant mixture was stirred at room temperature for 1 h. The reaction was then quenched by addition of cold water (1 mL) and extracted with ethyl acetate (2x2 mL). The combined organic extracts were dried with sodium sulfate and concentrated under reduced pressure. The residue was purified by column chromatography (1:1 ethyl acetate in petroleum ether) to afford 8-chloro-9-fluoro-6-methylbenzo[*c*][2,7]naphthyridin-5(6*H*)-one (82 mg, 0.297 mmol, 84% yield) as white solid. LC/MS (ESI) *m*/*e* 262.5 [(M+H)⁺, calcd for C₁₃H₉ClFN₂O 263.0]; LC/MS retention time (method D): *t*_{R} = 1.79 min; HPLC retention time (method A): *t*_{R} = 7.35 min; HPLC retention time (method B): *t*_{R} = 7.07 min.

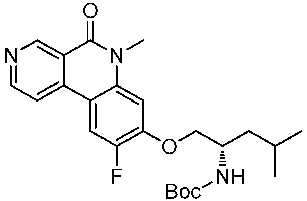

### Part F. (S)-tert-butyl (1-((9-fluoro-6-methyl-5-oxo-5,6-dihydrobenzo[c][2,7]naphthyridin-8-yl)oxy)-4-methylpentan-2-yl)carbamate

To a stirred suspension of 8-chloro-9-fluoro-6-methylbenzo[*c*][2,7]naphthyridin-5(6*H*)-one (40 mg, 0.152 mmol), (*S*)-*tert*-butyl (1-hydroxy-4-methylpentan-2-yl)carbamate (39.7 mg, 0.183 mmol), di*-tert-*butyl(2',4',6'-triisopropyl-[1,1'-biphenyl]-2-yl)phosphine (38.8 mg, 0.091 mmol) and cesium carbonate (74.4 mg, 0.228 mmol) in toluene (4 mL) was added palladium(II)acetate (10.26 mg, 0.046 mmol). Nitrogen gas was bubbled through the mixture for 5 min, and then the reaction mixture was heated to 85 °C for 8 h. The reaction mixture was cooled to room temperature and diluted with ethyl acetate (2 mL) and filtered through diatomaceous earth (Celite®). The filtrate was washed with water (2x2 mL) and brine (2x2 mL), dried over sodium sulfate and concentrated under reduced pressure. The residue was purified via silica gel column chromatography (60% ethyl acetate in hexanes) to afford *(S)-tert-butyl* (1-((9-fluoro-6-methyl-5-oxo-5,6-dihydrobenzo[*c*][2,7]naphthyridin-8-yl)oxy)-4-methylpentan-2-yl)carbamate (28 mg, 0.023 mmol, 15% yield). LC/MS (ESI) *m*/*e* 444.2 [(M+H)⁺, calcd for C₂₄H₃₁lFN₃O₄ 444.2]; LC/MS retention time (method A): *t*_{R} = 2.02 min.

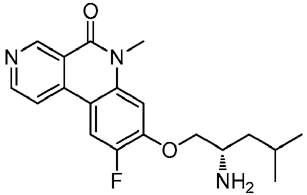

### Part G. (S)-8-((2-amino-4-methylpentyl)oxy)-9-fluoro-6-methylbenzo[c][2,7]naphthyridin-5(6H)-one

To a stirred solution of (*S*)-*tert*-butyl (1-((9-fluoro-6-methyl-5-oxo-5,6-dihydrobenzo[*c*][2,7]naphthyridin-8-yl)oxy)-4-methylpentan-2-yl)carbamate (25 mg, 0.056 mmol) in diethyl ether (4 mL) was added a 2M solution of hydrochloric acid in diethyl ether (42.5 µl, 0.085 mmol) at 0 °C dropwise over 10 min. The ice bath was removed and the reaction mixture was stirred at room temperature for 2 h. The solvent was removed and the crude material was taken up in water (5 mL). The aqueous layer was washed with ethyl acetate (3 mL) and treated with 10 % NaHCO₃ (20 mL). The resultant solution was extracted with ethyl acetate (3x10 mL). The combined organic layers were washed with water (2x10 mL), dried (sodium sulfate), filtered and concentrated under reduced pressure to afford crude material which was purified via preparative TLC (40% Ethyl acetate in Hexane) to afford (*S*)-8-((2-amino-4-methylpentyl)oxy)-9-fluoro-6-methylbenzo[*c*][2,7]naphthyridin-5(6*H*)-one (15 mg, 0.041 mmol, 73% yield) as a white solid. LC/MS (ESI) *m*/*e* 344.2 [(M+H)⁺, calcd for C₁₉H₂₃FN₃O₂ 344.2]; LC/MS retention time (method A): *t*_{R} = 1.35 min; HPLC retention time (method A): *t*_{R} = 8.31 min; HPLC retention time (method B): *t*_{R} = 8.85 min; ¹H NMR (400 MHz, *CD₃OD*) δ ppm 9.66 - 9.71 (m, 1 H), 8.83 - 8.87 (m, 1 H), 7.90 - 7.95 (m, 1 H), 7.79 - 7.83 (m, 1 H), 6.91 - 6.95 (m, 1 H), 4.10 - 4.14 (m, 1 H), 3.89 - 3.95 (m, 1 H), 3.76 - 3.80 (m, 3 H), 3.35 - 3.44 (m, 1 H), 1.78 - 1.86 (m, 1 H), 1.39 (t, *J=* 7.00 Hz, 2 H), 0.96 - 1.03 (m, 6 H).

### Example 15

### 8-(2-amino-5,5,5-trifluoropentyloxy)-6-methylbenzo[c][2,7]naphthyridin-5(6H)-one

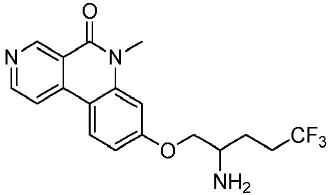

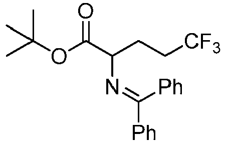

### Part A. Tert-butyl 2-(diphenylmethyleneamino)-5,5,5-trifluoropentanoate

To a stirred solution of *tert*-butyl 2-((diphenylmethylene)amino)acetate (1 g, 3.39 mmol) in THF (20 mL) cooled to -78 °C under nitrogen atmosphere was added a 2M solution of LDA in THF (2.54 mL, 5.08 mmol) dropwise for 30 min. To this mixture was then added 3,3,3-trifluoropropyl trifluoromethanesulfonate (1.083 g, 4.40 mmol). The reaction was gradually warmed to rt and stirred for 4 h. The reaction mixture was quenched by addition of saturated aqueous ammonium chloride at 0 °C. The reaction mixture was then extracted with ethyl acetate (3x10 mL). The combined organic extracts were washed with water (1x10 mL) and brine (1x10 mL), dried over sodium sulfate and then concentrated under reduced pressure. The crude oil was purified by silica gel column chromatography (2% ethyl acetate in hexane) to afford *tert*-butyl 2-((diphenylmethylene)amino)-5,5,5-trifluoropentanoate (800 mg, 2.02 mmol, 60% yield) as a yellow oil. LC/MS (ESI) *m*/*e* 391.9 [(M+H)⁺, calcd for C₂₂H₂₅F₃NO₂, 392.2]; LC/MS retention time (method E): *t*_{R} = 2.49 min.

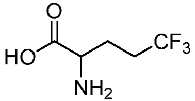

### Part B. 2-amino-5,5,5-trifluoropentanoic acid (hydrochloride salt)

A stirred solution of *tert*-butyl 2-((diphenylmethylene)amino)-5,5,5-trifluoropentanoate (800 mg, 2.023 mmol) in 50% aqueous HCl (0.123 mL, 2.023 mmol) was refluxed at 100 °C for 8 h. The reaction mixture was cooled to rt and concentrated under reduced pressure to afford 2-amino-5,5,5-trifluoropentanoic acid hydrochloride (400 mg, 1.82 mmol, 90% yield, 78% pure by LC/MS) as a white solid. LC/MS (ESI) *m*/*e* 171.7 [(M+H)⁺, calcd for C₅H₇F₃O₂, 172.1]; LC/MS retention time (method E): *t*_{R} = 0.80 min.

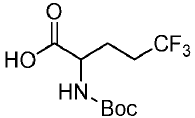

### Part C. 2-(tert-butoxycarbonylamino)-5,5,5-trifluoropentanoic acid

To a stirred solution of 2-amino-5,5,5-trifluoropentanoic acid hydrochloride (400 mg, 1.503 mmol, 78% by LC/MS) in THF (8 mL) and water (8 mL) at rt was added K₂CO₃ (831 mg, 6.01 mmol) and the solution stirred for 10 min. To this mixture was added Boc₂O (656 mg, 3.01 mmol). The reaction mixture was stirred for 8 h at rt then concentrated under reduced pressure. The aqueous layer was washed with ethyl acetate (3x5 mL). The aqueous layer was acidified with saturated citric acid solution (5 mL) and extracted with ethyl acetate (3x8 mL). The combined organic layers were washed with water (3x5 mL) followed by brine solution (1x10 mL), dried over sodium sulfate and concentrated under reduced pressure to afford 2-((tert-butoxycarbonyl)amino)-5,5,5-trifluoropentanoic acid (500 mg, 1.84 mmol, 100% yield) as a colorless oil. The material was taken into the next step without further purification. ¹H NMR (400 MHz, *CDCl₃*) δ 5.04 (s, 1H), 4.38 (s, 1H), 2.15-2.28 (m, 2H), 1.91-1.95 (m, 2H), 1.46 (s, 9H).

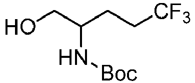

### Part D. Tert-butyl 5,5,5-trifluoro-1-hydroxypentan-2-ylcarbamate

To a stirred solution of 2-((*tert*-butoxycarbonyl)amino)-5,5,5-trifluoropentanoic acid (500 mg, 1.843 mmol) in THF (15 mL) cooled to -10 °C under nitrogen atmosphere was added N-methylmorphline (0.223 mL, 2.028 mmol) followed by isobutyl chloroformate (0.266 mL, 2.028 mmol) dropwise. The solution was then stirred for 30 min then filtered. The filtrate was added to sodium borohydride (147 mg, 3.87 mmol) in water (10 mL), stirred for 5 min and diluted with ethyl acetate (10 mL). The organic layer was separated and washed with brine (2x10 mL), dried (Na₂SO₄) and evaporated under reduced pressure to afford *tert-*butyl (5,5,5-trifluoro-1-hydroxypentan-2-yl)carbamate (400 mg, 1.555 mmol, 84% yield) as a white solid which was taken to the next step without further purification. ¹H NMR (400 MHz, *MeOD*) δ 3.44-3.56 (m, 3H), 2.16-2.26 (m, 2H), 1.83-1.92 (m, 1H), 1.57-1.67 (m, 1H), 1.47 (s, 9H).

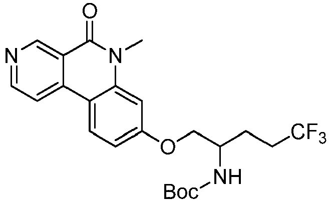

### Part E. Tert-butyl 5,5,5-trifluoro-1-(6-methyl-5-oxo-5,6-dihydrobenzo[c][2,7]naphthyridin-8-yloxy)pentan-2-ylcarbamate

The reaction was carried out as in Ex. 2, Part D to yield product tert-butyl (5,5,5-trifluoro-1-((6-methyl-5-oxo-5,6-dihydrobenzo[*c*][2,7]naphthyridin-8-yl)oxy)pentan-2-yl)carbamate (160 mg, 0.237 mmol, 23% yield) as a white solid. LC/MS (ESI) *m*/*e* 466.31 [(M+H)⁺, calcd for C₂₃H₂₇F₃N₃O₄, 466.19]; LC/MS retention time (method D): *t*_{R} = 1.04 min.

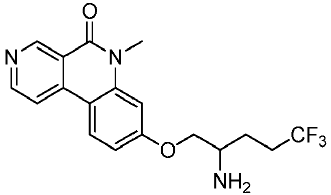

### Part F. 8-(2-amino-5,5,5-trifluoropentyloxy)-6-methylbenzo[c][2,7]naphthyridin-5(6H)-one

To a solution of *tert*-butyl (5,5,5-trifluoro-1-((6-methyl-5-oxo-5,6-dihydrobenzo[*c*][2,7]naphthyridin-8-yl)oxy)pentan-2-yl)carbamate (150 mg, 0.322 mmol) in diethyl ether (10 mL) under nitrogen atmosphere, cooled to 0 °C was added 4M HCl in 1,4-dioxane (0.098 mL, 0.392 mmol) slowly over a period of 5 min. The light yellow reaction mixture was stirred at 0 °C for 5 min then warmed to room temperature and allowed to stir for 12 h. The solvent was removed under reduced pressure. The residual yellow oil was diluted with water (3 mL) and extracted with EtOAc (2x4 mL). The aqueous layer was separated and treated with sat. Na₂CO₃ solution (3 mL). The resultant aqueous layer was extracted with EtOAc (3x5 mL). The combined organic layer s were separated and washed with water (2x5 mL), brine (1x5 mL) and dried over sodium sulfate. The filtrate was evaporated under reduced pressure and purified by SFC (CO₂ and 0.5% DEA in Methanol) to afford 8-((2-amino-5,5,5-trifluoropentyl)oxy)-6-methylbenzo[*c*][2,7]naphthyridin-5(6*H*)-one (102.8 mg, 0.278 mmol, 86% yield) as a white solid. LC/MS (ESI) *m*/*e* 366.2 [(M+H)⁺, calcd for C₁₈H₁₉F₃N₃O₂ 366.13]; LC/MS retention time (method C): *t*_{R} = 1.77 min; HPLC retention time (method A): *t*_{R} = 7.79 min; HPLC retention time (method B): *t*_{R} = 8.20 min. ¹H NMR (400 MHz, *MeOD*) δ 9.48 (d, *J* = 0.80 Hz, 1H), 8.78 (d, *J* = 5.60 Hz, 1H), 8.44 (d, *J* = 8.80 Hz, 1H), 8.27 (d, *J* = 5.60 Hz, 1H), 7.17 (d, *J* = 2.40 Hz, 1H), 7.12 (dd, *J* = 2.40, 8.80 Hz, 1H), 4.17-4.20 (m, 1H), 4.09 (dd, *J* = 6.00, 9.40 Hz, 1H), 3.82 (s, 3H), 3.28-3.28 (m, 1H), 2.33-2.49 (m, 2H), 1.92-1.99 (m, 1H), 1.73-1.80 (m, 1H).

### Example 16

### (S)-8-((2-amino-4-methylpentyl)oxy)-4,6-dimethylbenzo[c][2,7]naphthyridin-5(6H)-one

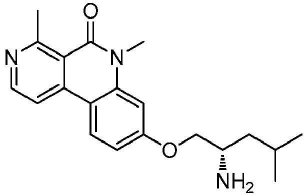

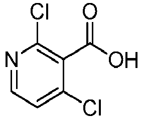

### Part A: 2,4-dichloronicotinic acid

To a solution of 2,4-dichloropyridine (7 g, 47.3 mmol) in THF (70 mL) at - 78°C was added a 2M solution of LDA in THF (28.4 mL, 56.8 mmol) and stirred for 30 min. The reaction mixture was quenched with excess dry ice and stirred for 30 min at RT. After neutralizing with 1.5N HCl, the reaction mixture was diluted with ethyl acetate (100 mL) and washed with brine (2x50 mL) and water (100 mL). The organic layer was separated, dried over Na₂SO₄ and concentrated under reduced pressure to afford 2,4-dichloronicotinic acid (4.5 g, 23.44 mmol, 50% yield) as a brown solid. ¹H NMR (400 MHz, *DMSO-d*₆) δ ppm 8.47 (d, 1H), 7.74 (d, 1H).

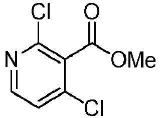

### Part B: Methyl 2,4-dichloronicotinate

To a solution of 2,4-dichloronicotinic acid (500 mg, 2.60 mmol) in acetonitrile (10 mL) cooled to 0 °C was added DBU (0.981 mL, 6.51 mmol) followed by methyl iodide (0.814 mL, 13.0 mmol). The reaction mixture was stirred at RT overnight (14 h). After the reaction completion, the solvent was removed under reduced pressure. The residue was taken up in ethyl acetate (10 mL) and washed with water (1x5 mL), dried over Na₂SO₄, and concentrated under reduced pressure. The residue was purified by silica gel column chromatography (EtOAc-hexane) to afford methyl 2,4-dichloronicotinate (260 mg, 1.262 mmol, 49% yield) as pale yellow oil. LC/MS, (ESI) *m*/*z* 206.1 [(M+H)⁺, calcd for C₇H₆Cl₂NO₂ 205.97]; LC/MS retention time (method D): *t*_{R} = 0.85 min. ¹H NMR (300 MHz, *CD₃OD*) δ ppm 8.42 (d, *J* = 5.4 Hz, 1H), 7.59 (d, *J* = 5.4 Hz, 1H), 3.99 (s, 3H).

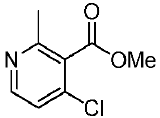

### Part C: Methyl 4-chloro-2-methylnicotinate

A mixture of methyl 2,4-dichloronicotinate (2 g, 9.71 mmol), trimethylboroxine (1.462 g, 11.65 mmol), Cs₂CO₃ (6.33 g, 19.42 mmol) and PdCl₂(dppf) (0.710 g, 0.971 mmol) in 1,4-dioxane (30 mL) and water (2 mL) was purged with nitrogen for 5 min and heated at 70 °C for 16 h. The reaction was concentrated under reduced pressure and purified by silica gel column chromatography (EtOAc-hexane mixture) to afford methyl 4-chloro-2-methylnicotinate (550 mg, 2.94 mmol, 31% yield) as a red oil. ¹H NMR (300 MHz, *CD₃OD*) δ ppm 8.44 (d, *J* = 5.7 Hz, 1H), 7.43 (d, *J* = 5.7 Hz, 1H), 3.98 (s, 3H), 2.54 (s, 3H).

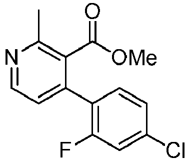

### Part D: Methyl 4-(4-chloro-2-fluorophenyl)-2-methylnicotinate

A mixture of methyl 4-chloro-2-methylnicotinate (4 g, 21.55 mmol), (4-chloro-2-fluorophenyl)boronic acid (4.13 g, 23.71 mmol), tricyclohexylphosphine (1.813 g, 6.47 mmol), Pd(OAc)₂ (0.484 g, 2.155 mmol) and Cs₂CO₃ (14.04 g, 43.1 mmol) in DMA (15 mL) was purged with nitrogen for 5 min and heated at 100 °C overnight (14 h). After cooling, the reaction mixture was filtered through diatomaceous earth (Celite®). DMA was removed under reduced pressure and the residue was diluted with ethyl acetate (10 mL). The organic phase was concentrated under reduced pressure and purified by silica gel column chromatography (EtOAc-hexane) to afford methyl 4-(4-chloro-2-fluorophenyl)-2-methylnicotinate (1 g, 3.58 mmol, 17% yield) as a red oil. LC/MS, (ESI) *m*/*z* 280.1 [(M+H)⁺, calcd for C₁₄H₁₂ClFNO₂ 280.0]; LC/MS retention time (method D): *t*_{R} = 0.80 min.

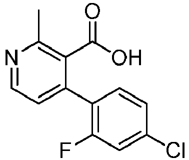

### Part E: 4-(4-chloro-2-fluorophenyl)-2-methylnicotinic acid

To a solution of methyl 4-(4-chloro-2-fluorophenyl)-2-methylnicotinate (40 mg, 0.143 mmol) in MeOH (2 mL) and water (3 mL) was added NaOH (11.44 mg, 0.286 mmol) and the reaction mixture was stirred at RT for 14 h.. The reaction mixture was concentrated under reduced pressure and acidified with 1.5N HCl the extracted with ethyl acetate (5 mL). The organic layer was washed with saturated NaHCO₃ (2x10 mL) and water (10 mL); dried over Na₂SO₄ and concentrated under reduced pressure to afford crude 4-(4-chloro-2-fluorophenyl)-2-methylnicotinic acid (30 mg, 0.113 mmol, 79% yield) as brown solid. This was taken into the next step without further purification. LC/MS, (ESI) *m*/*z* 266.1 [(M+H)⁺, calcd for C₁₃H₁₀ClFNO₂. 266.0]; LC/MS retention time (method D): *t*_{R} = 0.64 min.

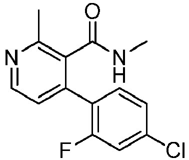

### Part F: 4-(4-chloro-2-fluorophenyl)-N,2-dimethylnicotinamide

To a solution of 4-(4-chloro-2-fluorophenyl)-2-methylnicotinic acid (180 mg, 0.678 mmol) in DCM (5 mL) and DMF (0.2 mL) at 0 °C, was added oxalyl chloride (0.178 mL, 2.033 mmol). The reaction mixture was stirred for 3 h at RT then diluted with DCM (5 mL) and treated with a solution of methylamine hydrochloride (457 mg, 6.78 mmol) in DCM (5 mL) cooled to 0°C. The mixture was stirred for another 1h at RT. The solution was extracted with DCM (20 mL), washed with saturated NaHCO₃ (2x10 mL) and water (20 mL); dried over Na₂SO₄ and concentrated under reduced pressure to afford 4-(4-chloro-2-fluorophenyl)-N,2-dimethylnicotinamide (50 mg, 0.179 mmol, 27% yield) as a brown gum. LC/MS, (ESI) *m*/*z* 279.2 [(M+H)⁺, calcd for C₁₄H₁₃ClFN₂O 279.1]; LC/MS retention time (method D): *t*_{R} = 0.62 min.

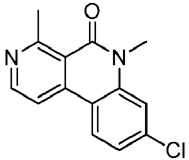

### Part G: 8-chloro-4,6-dimethylbenzo[c][2,7]naphthyridin-5(6H)-one

To a solution of 4-(4-chloro-2-fluorophenyl)-*N*,2-dimethylnicotinamide (40 mg, 0.144 mmol) in THF (3 mL) cooled to 0 °C was added NaH (10.33 mg, 0.431 mmol) and the reaction mixture was stirred for 4 h. After the reaction completion , the reaction mixture was quenched with ice water and the product was extracted with ethyl acetate (20 mL). The organic layer was dried over Na₂SO₄ and concentrated under reduced pressure to afford 8-chloro-4,6-dimethylbenzo[*c*][2,7]naphthyridin-5(6*H*)-one (30 mg, 0.116 mmol, 81% yield) as an off-white solid. LC/MS, (ESI) *m*/*z* 259.2 [(M+H)⁺, calcd for C₁₄H₁₂ClN₂O 259.1]; LC/MS retention time (Method D): t_{R} = 0.65 min.

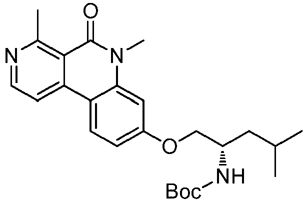

### Part H: (S)-tert-butyl (1-((4,6-dimethyl-5-oxo-5,6-dihydrobenzo[c][2,7]naphthyridin-8-yl)oxy)-4-methylpentan-2-yl)carbamate

A mixture of 8-chloro-4,6-dimethylbenzo[*c*][2,7]naphthyridin-5(6*H*)-one (60 mg, 0.232 mmol), (*S*)-*tert*-butyl(1-hydroxy-4-methylpentan-2-yl)carbamate (101 mg, 0.464 mmol), Cs₂CO₃ (113 mg, 0.348 mmol), Pd(OAc)₂ (15.6 mg, 0.070 mmol) and 2-di-*t*-butylphosphino-2',4',6'-tri-*i*-propyl-1,1'-biphenyl (5.91 mg, 0.014 mmol) in toluene (5 mL) was purged with nitrogen for 5 min and heated at 100 °C overnight (14 h). After cooling, the reaction mixture was filtered through diatomaceous earth (Celite®), concentrated under reduced pressure and dissolved in ethyl acetate (5 mL). The organic layer was washed with brine (2x10 mL) and water (20 mL); dried over Na₂SO₄ and concentrated under reduced pressure to afford (*S*)-*tert*-butyl (1-((4,6-dimethyl-5-oxo-5,6-dihydrobenzo[*c*][2,7]naphthyridin-8-yl)oxy)-4-methylpentan-2-yl)carbamate (55 mg crude) as a gummy solid. The crude product was used in the next step without further purification. LC/MS, (ESI) *m*/*z* 440.4 [(M+H)⁺, calcd for C₂₅H₃₄N₃O₄, 440.2]; LC/MS retention time (method D): t_{R} = 0.89 min.

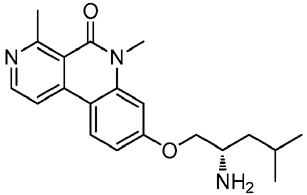

### Part I: (S)-8-((2-amino-4-methylpentyl)oxy)-4,6-dimethylbenzo[c][2,7]naphthyridin-5(6H)-one

To a solution of (*S*)-tert-butyl (1-((4,6-dimethyl-5-oxo-5,6-dihydrobenzo[*c*][2,7]naphthyridin-8-yl)oxy)-4-methylpentan-2-yl)carbamate (30 mg, 0.068 mmol) in MeOH (3 mL) at 0 °C was added HCl (4N in 1,4-dioxane, 3 mL, 12.00 mmol). The solution was warmed to room temperature and stirred for 2 h. The reaction mixture was then concentrated under reduced pressure and the residue dissolved in ethyl acetate (10 mL). The organic layer was washed with saturated NaHCO₃ (2x10 mL) and water (20 mL); dried over Na₂SO₄, and concentrated under reduced pressure. The residue was purified by preparative TLC (DCM-MeOH mixture) to afford (*S*)-8-((2-amino-4-methylpentyl)oxy)-4,6-dimethylbenzo[*c*][2,7]naphthyridin-5(6*H*)-one (8 mg, 0.021 mmol, 31% yield) as a brown solid. LC/MS, (ESI) *m*/*z* 340.2 [(M+H)⁺, calcd for C₂₀H₂₆N₃O₂, 340.2]; LC/MS retention time (method B): t_{R} = 1.27 min. HPLC retention time (method A): *t*_{R} = 8.30 and HPLC retention time (method B): *t*_{R} =8.97 min. ¹H NMR (400 MHz, *CD₃OD*) δ ppm 8.53 (d, *J* = 5.77 Hz, 1H), 8.33 (d, *J* = 9.54 Hz, 1H), 8.08 (d, *J* = 5.77 Hz, 1H), 7.00-7.06 (m, 2H), 4.21 (m, 1H), 4.02 (m, 1H), 3.73 (s, 3H), 3.47-3.52 (m, 1H), 3.06 (s, 3H), 1.82-1.93 (m, 1H), 1.67-1.76 (m, 2H), 1.26-1.56 (m, 6H).

### Example 17

### (S)-4-amino-8-(2-amino-4-methylpentyloxy)-6-methylbenzo[c][2,7]naphthyridin-5(6H)-one

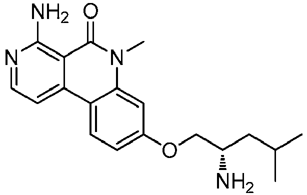

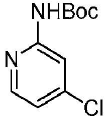

### Part A. tert-butyl (4-chloropyridin-2-yl)carbamate

To a solution of LHMDS (1M in THF, 17.11 mL, 17.11 mmol) in THF (5 mL) at -5 °C was added a solution of 4-chloropyridin-2-amine (1 g, 7.78 mmol) in THF (5 mL) and the mixture was stirred for 5 min. To this mixture was added a solution of Boc₂O (1.898 mL, 8.18 mmol) in THF (5 mL). The mixture was stirred at 0 °C for 2 h and quenched by addition of aqueous NH₄Cl. The pH of the solution was adjusted to 6 by addition of 1.5N HCl and extracted with ethyl acetate (3x15 mL). The combined organic extracts were washed with sodium bicarbonate (15 mL), water (15 mL) and brine (15 mL). The combined organic extracts were dried over sodium sulfate and concentrated under reduced pressure. The residue was purified by silica gel column chromatography (ethyl acetate, petroleum ether gradient) to yield *tert-*butyl (4-chloropyridin-2-yl)carbamate (1.435 g, 6.28 mmol, 81% yield). ¹H NMR. ¹H NMR (400 MHz, *DMSO-d₆*) δ 10.10 (s, 1H), 8.23 (d, *J* = 5.20 Hz, 1H), 7.88 (d, *J* = 2.00 Hz, 1H), 7.15 (dd, *J* = 2.00, 5.20 Hz, 1H), 1.48 (s, 9H).

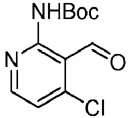

### Part B. tert-Butyl 4-chloro-3-formylpyridin-2-ylcarbamate

To a stirred solution of *tert*-butyl (4-chloropyridin-2-yl)carbamate (1.00 g, 4.37 mmol) in THF (30 mL) cooled to -78 °C was added n-butyllithium (2.55 M in hexane, 4.1 mL, 10.06 mmol) dropwise. After complete addition the solution was stirred at -78 °C for 1 h. DMF (1.591 mL, 20.55 mmol) was added dropwise and the resultant solution stirred at -78 °C for an additional 1 h. The reaction mixture was then quenched by addition of saturated aqueous ammonium chloride solution (20 mL) and extracted with ethyl acetate (2x25 mL). The combined organic extracts were washed with brine (1x20 mL), dried over sodium sulfate and concentrated under reduced pressure. The crude residue was purified via neutral alumina chromatography (ethyl acetate/hexanes) to yield *tert*-butyl (4-chloro-3-formylpyridin-2-yl)carbamate (530 mg, 2.06 mmol, 27% yield) as a yellow solid. ¹H NMR (400 MHz, *CDCl₃*) δ 10.73 (s, 1H), 10.55 (s, 1H), 8.52 (d, *J* = 5.3 Hz, 1H), 7.06 (d, *J* = 5.3 Hz, 1H), 1.56 (s, 9 H); LC/MS (ESI) *m*/*e* 255.2 [(M-H)⁻, calcd for C₁₁H₁₂ClN₂O₃ 255.1]. LC/MS retention time (method A) : *t*_{R} = 1.75 min.

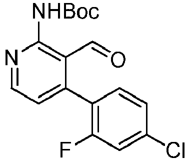

### Part C. tert-Butyl 4-(4-chloro-2-fluorophenyl)-3-formylpyridin-2-ylcarbamate

To a stirred suspension of *tert*-butyl (4-bromo-3-formylpyridin-2-yl)carbamate (100 mg, 0.332 mmol), (4-chloro-2-fluorophenyl)boronic acid (57.9 mg, 0.332 mmol) and cesium carbonate (216 mg, 0.664 mmol) in THF (50 mL) and water (8 mL), was added Pd(PPh₃)₄ (19.19 mg, 0.017 mmol) and the reaction mixture was heated to 85 °C overnight (14 h). The reaction mixture was cooled to room temperature, diluted with water (30 mL) and extracted with ethyl acetate (2x25 mL). The combined organic extracts were washed with brine (1x25 mL), dried over sodium sulfate and concentrated under reduced pressure. The crude residue was purified via silica gel chromatography (ethyl acetate/hexanes) to afford *tert*-butyl 4-(4-chloro-2-fluorophenyl)-3-formylpyridin-2-ylcarbamate (60 mg, 0.17 mmol, 35% yield). LC/MS (ESI) *m*/*e* 351.2 [(M+H)⁺, calcd for C₁₇H₁₇ClFN₂O₃ 351.1]. LC/MS retention time (method A): *t*_{R} = 2.07 min.

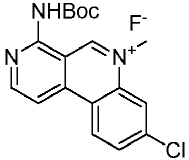

### Part D. 4-((tert-butoxycarbonyl)amino)-8-chloro-6-methylbenzo[c][2,7] naphthyridin-6-ium fluoride

To a stirred solution of *tert*-butyl (4-(4-chloro-2-fluorophenyl)-3-formylpyridin-2-yl)carbamate (700 mg, 1.996 mmol) in ethanol (10 mL) at 0 °C was added methylamine (8M in EtOH) (620 mg, 2.49 mL, 19.96 mmol) and the reaction was stirred at rt for 5 h. After the consumption of starting material, solvent was removed under reduced pressure. The residue was washed with hexane (50 mL) and the solid so obtained was dried under vacuum to yield the fluoride salt of 4-((*tert-*butoxycarbonyl)amino)-8-chloro-6-methylbenzo[*c*][2,7]naphthyridin-6-ium fluoride (900 mg, 1.69 mmol, 84% yield) as a yellow solid. LC/MS (ESI) *m*/*e* 344.2 [(M)⁺, calcd for C₁₈H₁₉ClN₃O₂ 344.1]; LC/MS retention time (method C): *t*_{R} = 1.90 min.

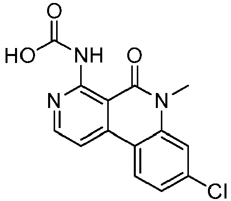

### Part E. 8-chloro-6-methyl-5-oxo-5,6-dihydrobenzo[c][2,7]naphthyridin-4-ylcarbamic acid

To a stirred solution of 4-((*tert*-butoxycarbonyl)amino)-8-chloro-6-methylbenzo[*c*][2,7]naphthyridin-6-ium fluoride (0.35 g, 0.65 mmol) and NaOH (0.162 g, 4.06 mmol) in a mixture of DCM (10 mL) and water (10 mL) was added KMnO₄ (0.642 g, 4.06 mmol) in portions at rt and the reaction was heated to 90 °C for 1 h. The solution was diluted with water (10 mL) and extracted with DCM (2x20 mL). The combined organic layers were washed with water 50 mL, dried over sodium sulfate, and concentrated under reduced pressure to yield (8-chloro-6-methyl-5-oxo-5,6-dihydrobenzo[c][2,7]naphthyridin-4-yl)carbamic acid (305 mg, 0.422 mmol, 65% yield) as a yellow solid, which carried on without further purification. LC/MS (ESI) *m*/*e* 304.1 [(M+H)⁺, calcd for C₁₄H₁₁ClN₃O₃ 304.04]; LC/MS retention time (Method D): *t*_{R} = 1.01 min.

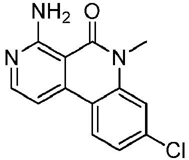

### Part F. 4-amino-8-chloro-6-methylbenzo[c][2,7]naphthyridin-5(6H)-one

A solution of (8-chloro-6-methyl-5-oxo-5,6-dihydrobenzo[*c*][2,7]naphthyridin-4-yl)carbamic acid (300 mg, 0.415 mmol) in conc. HCl (15 mL, 494 mmol) was heated at 90 °C for 2 h. The pH of the reaction mixture was adjusted to 8 with saturated sodium bicarbonate solution and extracted with DCM (2x30 mL). The combined organic layers were washed with brine (30 mL), dried over sodium sulfate, and concentrated under reduced pressure. The residue was purified by prep. HPLC (0.1% TFA in MeOH) to yield 4-amino-8-chloro-6-methylbenzo[*c*][2,7]naphthyridin-5(6*H*)-one (30 mg, 0.110 mmol, 27% yield) as a mono TFA salt. LC/MS (ESI) *m*/*e* 260.0 [(M+H)⁺, calcd for C₁₃H₁₁ClN₃O 260.05]; LC/MS retention time (method B): *t*_{R} = 1.18 min; HPLC retention time (method A): *t*_{R} = 5.24 min; HPLC retention time (method B): *t*_{R} = 6.31 min; 400 MHz, ¹H NMR (*DMSO-d₆*) δ 9.95 (s, 1H), 8.75 (s, 1H), 8.56 (d, *J* = 8.80 Hz, 1H), 8.23 (d, *J* = 6.80 Hz, 1H), 7.82 (dd, *J* = 6.80, 7.20 Hz, 2H), 7.54 (dd, *J* = 2.00, 8.80 Hz, 1H), 3.72 (s, 3H).

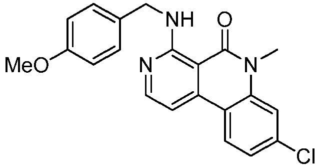

### Part G. 8-chloro-4-(4-methoxybenzylamino)-6-methylbenzo[c][2,7]naphthyridin-5(6H)-one

To a stirred solution of 4-amino-8-chloro-6-methylbenzo[c][2,7]naphthyridin-5(6H)-one (30 mg, 0.059 mmol) in DMF (10 mL) at 0 °C was added NaH (7.09 mg, 0.295 mmol) and the reaction was stirred for 5 min. To this mixture, 1-(chloromethyl)-4-methoxybenzene (0.026 mL, 0.191 mmol) was added and stirred at rt for 16 h. The reaction was quenched by addition of ice and the reaction mixture extracted with ethyl acetate (2x10 mL). The combined organic layers were washed with water (25 mL), dried over sodium sulfate, and concentrated under reduced pressure to yield 8-chloro-4-((4-methoxybenzyl)amino)-6-methylbenzo[*c*][2,7]naphthyridin-5(6*H*)-one (29 mg, 0.044 mmol, 75% yield). LC/MS (ESI) *m*/*e* 380.27 [(M+H)⁺, calcd for C₂₁H₁₉ClN₃O₂ 380.11]; LC/MS retention time (Method D): *t*_{R} = 0.86 min.

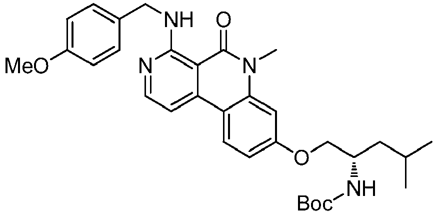

### Part H. (S)-tert-butyl 1-(4-(4-methoxybenzylamino)-6-methyl-5-oxo-5,6-dihydrobenzo[c][2,7]naphthyridin-8-yloxy)-4-methylpentan-2-ylcarbamate

The reaction was carried out as in Ex. 2, Part D to afford crude product which was purified by silica gel column using (ethyl acetate and hexane) to yield (*S*)-*tert-*butyl (1-((4-((4-methoxybenzyl)amino)-6-methyl-5-oxo-5,6-dihydrobenzo[*c*][2,7]naphthyridin-8-yl)oxy)-4-methylpentan-2-yl)carbamate (0.29 g, 0.422 mmol, 61% yield) as a yellow oil. LC/MS (ESI) *m*/*e* 561.5 [(M+H)⁺, calcd for C₃₂H₄₁N₄O₅ 561.3]; LC/MS retention time (Method D): *t*_{R} = 1.03 min.

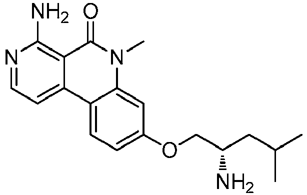

### Part I. (S)-4-amino-8-(2-amino-4-methylpentyloxy)-6-methylbenzo[c][2,7]naphthyridin-5(6H)-one

To a stirred solution of (*S*)-*tert*-butyl (1-((4-((4-methoxybenzyl)amino)-6-methyl-5-oxo-5,6-dihydrobenzo[*c*][2,7]naphthyridin-8-yl)oxy)-4-methylpentan-2-yl)carbamate (100 mg, 0.145 mmol) in DCM (10 mL) at rt was added TFA (4 mL, 51.9 mmol) dropwise and the reaction was heated at 45 °C for 12 h. After the completion of reaction, the volatile organics were evaporated under reduced pressure and the residue obtained was purified by prep. HPLC (0.1% TFA in water/acetonitrile as mobile phase) to yield (*S*)-4-amino-8-((2-amino-4-methylpentyl)oxy)-6-methylbenzo[*c*][2,7]naphthyridin-5(6*H*)-one (16 mg, 0.046 mmol, 32% yield) as a mono TFA salt as an off white solid. LC/MS (ESI) *m*/*e* 341.2 [(M+H)⁺, calcd for C₁₉H₂₅N₄O₂ 341.2]; LC/MS retention time (method B): *t*_{R} = 1.08 min; HPLC retention time (method A): *t*_{R} = 8.02 min; HPLC retention time (method B): *t*_{R} = 8.40 min; ¹H NMR (400 MHz, *MeOD*) δ 8.44 (d, *J* = 8.80 Hz, 1H), 8.00 (d, *J* = 7.20 Hz, 1H), 7.71 (d, *J* = 7.20 Hz, 1H), 7.24 (d, *J* = 2.40 Hz, 1H), 7.21-7.22 (m, 1H), 4.51 (dd, *J* = 3.20, 10.80 Hz, 1H), 4.34 (dd, *J* = 6.40, 10.40 Hz, 1H), 3.84 (s, 3H), 3.78-3.82 (m, 1H), 1.65-1.89 (m, 3H), 1.08 (d, *J* = 4.40 Hz, 3H), 1.06 (d, *J* = 4.40 Hz, 3H).

### Example 18

### (S)-N-(8-(2-amino-4-methylpentyloxy)-6-methyl-5-oxo-5,6 dihydrobenzo[c][2,7]naphthyridin-2-yl)acetamide

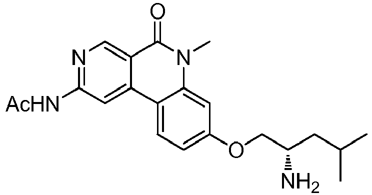

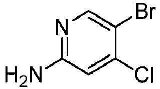

### Part A. 4-chloropyridin-2-amine

To a stirred solution of 4-chloropyridin-2-amine (8 g, 62.2 mmol) in acetonitrile (600 mL) at rt was added *N*-bromosuccinimide (11.08 g, 62.2 mmol) in portions and the reaction was stirred for 14 h. The reaction mixture was concentrated under reduced pressure. The residue was diluted with water (50 mL) and extracted with EtOAc (3x50 mL). The combined organic layers were washed with water (100 mL), brine (100 mL) and dried over sodium sulfate. The organics were concentrated under reduced pressure to afford 5-bromo-4-chloropyridin-2-amine as yellow solid (13 g, 99%) that was used as is without further purification. LC/MS (ESI) *m*/*e* 207.0 [(M+H)⁺, calcd for C₅H₅BrClN₂ 206.9]; LC/MS retention time (method B): *t*_{R} = 0.8 min.

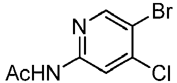

### Part B. N-(5-bromo-4-chloropyridin-2-yl)acetamide

To a stirred solution of 5-bromo-4-chloropyridin-2-amine (11.6 g, 55.9 mmol) in pyridine (100 mL) at 0 °C was added acetyl chloride (3.98 mL, 55.9 mmol) and the reaction was stirred at rt for 3 h. The reaction mixture was quenched with cold water and concentrated under reduced pressure. The residue was diluted with water (50 mL) and extracted with EtOAc (3x50 mL). The combined organic layers were washed with water (100 mL), brine (100 mL) and dried over sodium sulfate. The organics were concentrated under reduced pressure to afford *N*-(5-bromo-4-chloropyridin-2-yl)acetamide (14.6 g, 55.9 mmol, 100% yield) as a white solid that was carried on without further purification. LC/MS (ESI) *m*/*e* 249 [(M+H)⁺, calcd for C₇H₇BrClN₂O 248.9], LC/MS retention time (method B): *t*_{R} = 1.64 min; ¹H NMR (400 MHz, *DMSO-d₆*) δ 10.87 (s, 1H), 8.58 (s, 1H), 8.33 (s, 1H), 2.11 (s, 3H).

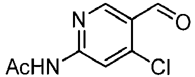

### Part C. N-(4-chloro-5-vinylpyridin-2-yl)acetamide

To a stirred solution of *N*-(5-bromo-4-chloropyridin-2-yl)acetamide (7 g, 28.1 mmol), 2,4,6-trivinyl-1,3,5,2,4,6-trioxatriborinane complex with pyridine (1:1) (8.78 g, 36.5 mmol), sodium carbonate (5.95 g, 56.1 mmol) solution in 7 mL of water and tetrakis(triphenylphosphine)palladium (0.973 g, 0.842 mmol) in a mixture of Toluene (50 mL) and Ethanol (8 mL), nitrogen gas was bubbled for 5 min. The reaction mixture was heated at 85 °C for 14 hours. After the completion, the reaction was diluted with EtOAc (50 mL), filtered through diatomaceous earth (Celite®). The filtrate was diluted with water and the organic layer was separated, washed with brine solution, dried over sodium sulfate. The organics were concentrated under reduced pressure and residue so obtained was purified by comb flash column 120 g using hexane/ethyl acetate. Product eluted at 30% EtOAc in hexane and required fractions were concentrated to yield *N*-(4-chloro-5-vinylpyridin-2-yl)acetamide (5.92 g, 27.7 mmol, 99% yield) as yellow solid. LC/MS (ESI) *m*/*e* 197.2 [(M+H)⁺, calcd for C₉H₁₀ClN₂O 197.04] LC/MS retention time (method A): *t*_{R} = 1.50 min; ¹H NMR (400 MHz, DMSO-d₆) δ 10.79 (s, 1H), 8.64 (s, 1H), 8.18 (d, *J* = 6.40 Hz, 1H), 6.88 (dd, *J* = 11.20, 17.60 Hz, 1H), 5.99 (dd, *J* = 0.80, 17.60 Hz, 1H), 5.47 (dd, *J* = 0.80, 11.40 Hz, 1H), 2.12 (s, 3H).

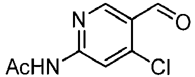

### Part D. N-(4-chloro-5-formylpyridin-2-yl)acetamide

To a stirred solution of N-(4-chloro-5-vinylpyridin-2-yl)acetamide (6 g, 30.5 mmol) and 2,6-lutidine (7.11 mL, 61.0 mmol) in a mixture of 1,4-dioxane (110 mL) and water (25 mL) at 0 °C was added osmium tetroxide (2.5% in 2-methyl-2-propanol, 9.58 mL, 30.5 mmol) followed by the addition of sodium periodate (19.58 g, 92 mmol) and the reaction was stirred for 4 h. The reaction mixture was diluted with water and extracted with EtOAc (2x100 mL). The combined organic layers were washed with brine solution, dried over sodium sulfate, concentrated under reduced pressure and residue so obtained was purified by comb flash column 120 g silica column using hexane/EtOAc as eluant. The desired product was isolated at 30% EtOAc in hexane. The required fractions were concentrated under reduced pressure to yield N-(4-chloro-5-formylpyridin-2-yl)acetamide as a off-white solid (5.8 g, 28.1 mmol, 92% yield). LC/MS (ESI) *m*/*e* 197.0 [(M)⁻, calcd for C₈H₆ClN₂O₂ 197.04] LC/MS retention time (method A): *t*_{R} = 1.21 min; ¹H NMR (400 MHz, *DMSO-d₆*) δ 11.20 (s, 1H), 10.18 (s, 1H), 8.76 (s, 1H), 8.27 (s, 1H), 2.16 (s, 3H).

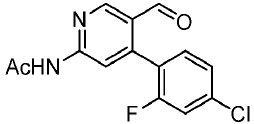

### Part E. N-(4-(4-chloro-2-fluorophenyl)-5-formylpyridin-2-yl)acetamide

To a stirred solution of N-(4-chloro-5-formylpyridin-2-yl)acetamide (3g, 15.11 mmol), (4-chloro-2-fluorophenyl)boronic acid (2.63 g, 15.11 mmol), cesium carbonate (9.84 g, 30.2 mmol) in a mixture of water (8 mL) and THF (25 mL) was added tetrakis(triphenylphosphine)palladium (19.19 mg, 0.017 mmol) and the reaction was heated to 85 °C overnight (14 h). The reaction mixture was diluted with water (25 mL) and extracted with EtOAc (2x25 mL). The combined organic layers were washed with water (25 mL), brine (25 mL), dried over sodium sulfate, and concentrated under reduced pressure. The residue was purified by silica gel chromatography (hexane/EtOAc as eluant), yielding *N*-(4-(4-chloro-2-fluorophenyl)-5-formylpyridin-2-yl)acetamide as an off-white solid (2.8 g, 9.01 mmol, 60% yield). LC/MS (ESI) *m*/*e* 291.0 [(M)⁻, calcd for C₁₄H₉ClFN₂O₂ 291.0], LC/MS retention time (method A): *t*_{R} = 1.69 min; ¹H NMR (400 MHz, *DMSO-d₆*) δ 11.14 (s, 1H), 9.84 (d, *J* = Hz, 1H), 8.88 (s, 1H), 8.13 (s, 1H), 7.61 (dd, *J* = 2.00, 10.00 Hz, 1H), 7.46-7.48 (m, 2H), 2.11 (s, 3H).

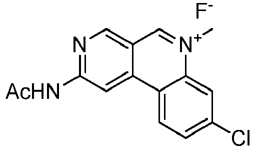

### Part F. 2-Acetamido-8-chloro-6-methylbenzo[c][2,7]naphthyridin-6-ium fluoride

To a stirred solution of *N*-(4-(4-chloro-2-fluorophenyl)-5-formylpyridin-2-yl)acetamide (0.39 g, 1.332 mmol) and 4 Å molecular sieves (50 mg) in dichloromethane (35 mL) at 0 °C was added methyl amine (8M in EtOH, 0.041 g, 0.166 mL,1.332 mmol) dropwise and the reaction was stirred at rt overnight (12 h). The reaction mixture was diluted with DCM (40 mL) and filtered through a bed of diatomaceous earth (Celite®). The filtrate was concentrated under reduced pressure to afford 2-acetamido-8-chloro-6-methylbenzo[c][2,7]naphthyridin-6-ium fluoride (450 mg, 1.24 mmol, 93% yield) as a yellow solid that was used in the next step without purification. LC/MS (ESI) *m*/*e* 286.1 [(M)⁺, calcd for C₁₅H₁₃ClN₃O 286.1]; LC/MS retention time (Method D): *t*_{R} = 0.71 min.

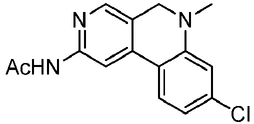

### Part G. N-(8-chloro-6-methyl-5,6-dihydrobenzo[c][2,7]naphthyridin-2-yl)acetamide

To a stirred solution of 2-acetamido-8-chloro-6-methylbenzo[*c*][2,7]naphthyridin-6-ium fluoride (2.1 g, 2.47 mmol) in a mixture of THF (20 mL) and MeOH (5 mL) at 0 °C was added NaBH₄ (0.199 g, 5.27 mmol) in three portions and the reaction was stirred at rt for 45 min. The volatile organics were removed under reduced pressure; saturated ammonium chloride solution (30 mL) was added and the solution extracted with ethyl acetate (2x30 mL). The combined organic layers were washed with brine (25 mL), dried over sodium sulfate, and concentrated under reduced pressure. The residue was purified by silica gel column chromatography (hexane/ ethyl acetate) to afford *N*-(8-chloro-6-methyl-5,6-dihydrobenzo[c][2,7]naphthyridin-2-yl)acetamide (0.71 g, 2.29 mmol, 93 % yield) as a yellow solid. LC/MS (ESI) *m*/*e* 288.2 [(M+H)⁺, calcd for C₁₅H₁₅ClN₃O 288.1]; LC/MS retention time (method C); *t*_{R} = 1.87 min; HPLC retention time (method A): *t*_{R} = 12.76 min; HPLC retention time (method B): *t*_{R} = 13.07 min; ¹H NMR (400 MHz, *DMSO-d₆*) δ 10.46 (s, 1H), 8.36 (s, 1H), 8.16 (d, *J* = 0.40 Hz, 1H), 7.63 (d, *J* = 8.40 Hz, 1H), 6.88 (dd, *J* = 2.00, 8.00 Hz, 1H), 6.82 (d, *J* = 2.00 Hz, 1H), 4.26 (s, 2H), 2.89 (s, 3H), 2.11 (s, 3H).

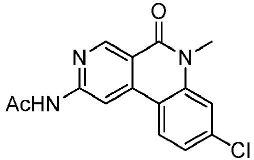

### Part H. N-(8-chloro-6-methyl-5-oxo-5,6-dihydrobenzo[c][2,7]naphthyridin-2-yl)acetamide

To a stirred solution of *N*-(8-chloro-6-methyl-5,6-dihydrobenzo[*c*][2,7]naphthyridin-2-yl)acetamide (0.6 g, 1.199 mmol) in DCM (20 mL) at rt was added barium manganate (1.536 g, 5.99 mmol) in three portions and the reaction was heated at 45 °C for 60 h. After the completion of reaction, the reaction mixture was diluted with DCM (70 mL) and passed through diatomaceous earth (Celite®). The filtrate was concentrated under reduced pressure. The residue was purified by silica gel column chromatography (DCM/ MeOH as an eluant) to yield *N*-(8-chloro-6-methyl-5-oxo-5,6-dihydrobenzo[*c*][2,7]naphthyridin-2-yl)acetamide (0.4 g, 1.071 mmol, 89% yield) as an off white solid. LC/MS (ESI) *m*/*e* 302.1 [(M+H)⁺, calcd for C₁₅H₁₃ClN₃O₂ 302.1]; LC/MS retention time (method C): *t*_{R} = 1.88 min; HPLC retention time (method A); *t*_{R} = 8.82 min; HPLC retention time (method B): *t*_{R} = 7.99 min; ¹H NMR (400 MHz, *DMSO-d₆*) δ 11.01 (s, 1H), 9.24 (s, 1H), 8.92 (s, 1H), 8.26 (d, *J* = 8.80 Hz, 1H), 7.69 (d, *J* = 2.00 Hz, 1H), 7.48 (dd, *J* = 2.00, 8.80 Hz, 1H), 3.68 (s, 3H), 2.20 (s, 3H).

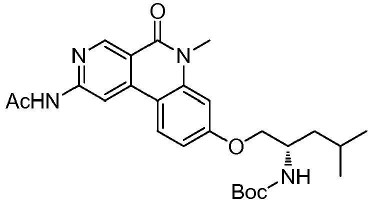

### Part I. (S)-tert-butyl 1-(2-acetamido-6-methyl-5-oxo-5,6-dihydrobenzo[c][2,7]naphthyridin-8-yloxy)-4-methylpentan-2-ylcarbamate

The reaction was carried out as in Ex. 2, Part D to afford crude (*S*)-*tert*-butyl 1-(2-acetamido-6-methyl-5-oxo-5,6-dihydrobenzo[*c*][2,7]naphthyridin-8-yloxy)-4-methylpentan-2-ylcarbamate (160 mg, 0.33 mmol, 20.5% pure by LC/MS) as a brown oil. This was taken to the next step without purification. LC/MS (ESI) *m*/*e* 483.34 [(M+H)⁺, calcd for C₂₆H₃₅N₄O5 483.25]; LC/MS retention time (Method E): *t*_{R} = 1.13 min.

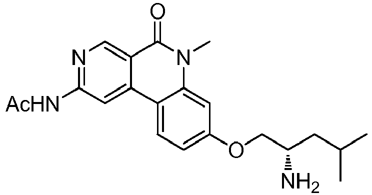

### Part J. (S)-N-(8-(2-amino-4-methylpentyloxy)-6-methyl-5-oxo-5,6-dihydrobenzo[c][2,7]naphthyridin-2-yl)acetamide

To a stirred solution of (*S*)-*tert*-butyl (1-((2-acetamido-6-methyl-5-oxo-5,6-dihydrobenzo[*c*][2,7]naphthyridin-8-yl)oxy)-4-methylpentan-2-yl)carbamate (140 mg, 0.059 mmol) in CH₂Cl₂ (15 mL) at 0 °C was added TFA (1.2 mL, 15.58 mmol) and the reaction mixture was stirred at RT for 40 min. After the completion of reaction, the volatile organics were evaporated under reduced pressure and the residue was purified by preparative HPLC (0.1% TFA in water/Acetonitrile) to yield (*S*)-*N*-(8-((2-amino-4-methylpentyl)oxy)-6-methyl-5-oxo-5,6-dihydrobenzo[*c*][2,7]naphthyridin-2-yl)acetamide (2 mg, 3.93 µmol, 6.6% yield) as a mono TFA salt. LC/MS (ESI) *m*/*e* 383.2 [(M+H)⁺, calcd for C₂₁H₂₇N₄O₃ 383.2]; LC/MS retention time (method C): *t*_{R} = 1.80 min; HPLC retention time (method A): *t*_{R} = 5.14 min; HPLC retention time (method B): *t*_{R} = 5.62 min; ¹H NMR (400 MHz, *CD₃OD*) δ 9.28 (s, 1H), 8.93 (s, 1H), 8.35 (d, *J* = 8.80 Hz, 1H), 7.17 (s, 1H), 7.15 (d, *J* = 2.00 Hz, 1H), 4.46 (d, *J* = 8.80 Hz, 1H), 4.28 (dd, *J* = 6.40, 10.80 Hz, 1H), 3.76-3.79 (m, 4H), 2.29 (s, 3H), 1.65-1.88 (m, 3H), 1.08 (d, *J* = 4.40 Hz, 3H), 1.07 (d, *J* = 4.40 Hz, 3H).

### Example 19

### (R)-8-((2-amino-4-methylpentyl)oxy)-4,6-dimethylbenzo[c][2,7]naphthyridin-5(6H)-one

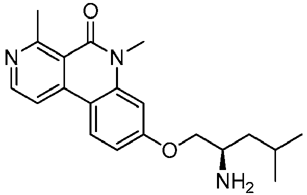

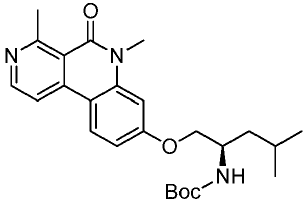

### Part A. (R)-tert-butyl (1-((4,6-dimethyl-5-oxo-5,6-dihydrobenzo[c][2,7]naphthyridin-8-yl)oxy)-4-methylpentan-2-yl)carbamate

The title compound was prepared as described in Example 16 using (*R*)-*tert-*butyl (1-hydroxy-4-methylpentan-2-yl)carbamate in part H to afford (*R*)-*tert*-butyl (1-((4,6-dimethyl-5-oxo-5,6-dihydrobenzo[*c*][2,7]naphthyridin-8-yl)oxy)-4-methylpentan-2-yl)carbamate (620 mg, 0.564 mmol, 49 % yield) as a yellow solid. LC/MS, (ESI) *m*/*z* 440.3 [(M+H)⁺, calcd for C₂₅H₃₄N₃O₄ 440.3].

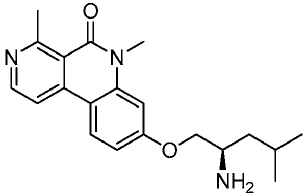

### Part B. (R)-8-((2-amino-4-methylpentyl)oxy)-4,6-dimethylbenzo[c][2,7]naphthyridin-5(6H)-one

An ambient temperature solution of (*R*)-*tert*-butyl (1-((4,6-dimethyl-5-oxo-5,6-dihydrobenzo[*c*][2,7]naphthyridin-8-yl)oxy)-4-methylpentan-2-yl)carbamate (102 mg, 0.232 mmol) in dichloromethane (1 mL) was treated with HCl (1.160 mL, 4.64 mmol) and stirred for 1 h. The resulting mixture was concentrated to an oil and purified by reverse phase high performance liquid chromatography (Phenomenex Luna C18 30x100mm 10 micron; water/methanol/0.1 % TFA gradient elution). Fractions containing product were neutralized with saturated aqueous sodium bicarbonate and concentrated under reduced pressure. The residue was partitioned between ethyl acetate and brine and the layers were separated. The aqueous was extracted twice more with ethyl acetate and the pooled organics were washed once with brine, dried over magnesium sulfate, filtered, and concentrated to afford (*R*)-8-((2-amino-4-methylpentyl)oxy)-4,6-dimethylbenzo[*c*][2,7]naphthyridin-5(6*H*)-one (11.5 mg, 0.033 mmol, 14 % yield) as a milky oil. LC/MS, (ESI) *m*/*z* 340.2 [(M+H)⁺, calcd for C₂₀H₂₆N₃O₂ 340.2]; HPLC retention time (method C): *t*_{R} = 13.02 min; HPLC retention time (method D): *t*_{R} = 13.05 min; ¹H NMR (500MHz, METHANOL-d₄) δ 8.68 - 8.63 (m, 2H), 8.58 (d, *J*=9.0 Hz, 1H), 7.22 (dd, *J*=9.0, 2.3 Hz, 1H), 7.20 (d, *J*=2.3 Hz, 1H), 4.52 (dd, *J*=10.5, 3.3 Hz, 1H), 4.34 (dd, *J*=10.7, 6.6 Hz, 1H), 3.82 (s, 3H), 3.80 - 3.75 (m, 1H), 3.25 (s, 3H), 1.91 - 1.81 (m, 1H), 1.80 - 1.73 (m, 1H), 1.72 - 1.64 (m, 1H), 1.07 (d, *J*=5.8 Hz, 3H), 1.06 (d, *J*=5.8 Hz, 3H).

### Example 20

### (R)-8-((2-amino-4-methylpentyl)oxy)-9-chloro-4,6-dimethylbenzo[c][2,7]naphthyridin-5(6H)-one

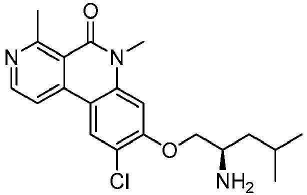

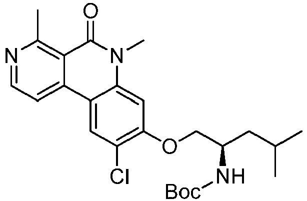

### Part A. (R)-tert-butyl (1-((9-chloro-4,6-dimethyl-5-oxo-5,6-dihydrobenzo[c][2,7]naphthyridin-8-yl)oxy)-4-methylpentan-2-yl)carbamate

A mixture of (*R*)-*tert*-butyl (1-((4,6-dimethyl-5-oxo-5,6-dihydrobenzo[*c*][2,7]naphthyridin-8-yl)oxy)-4-methylpentan-2-yl)carbamate (248 mg, 0.564 mmol) prepared as described in Example 19, Part A, NCS (151 mg, 1.128 mmol), and acetonitrile (5 mL) was heated at 90 °C for 1 h and then stirred at room temperature overnight. The crude material was concentrated under reduced pressure to afford (*R*)-*tert*-butyl (1-((9-chloro-4,6-dimethyl-5-oxo-5,6-dihydrobenzo[*c*][2,7]naphthyridin-8-yl)oxy)-4-methylpentan-2-yl)carbamate (15 mg, 0.032 mmol, 6 % crude yield). The mixture was carried on without further purification. LC/MS, (ESI) *m*/*z* 474.2 [(M+H)⁺, calcd for C₂₅H₃₃ClN₃O₄ 474.2].

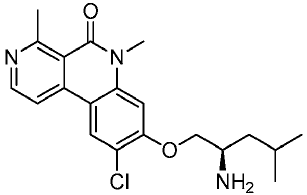

### Part B. (R)-8-((2-amino-4-methylpentyl)oxy)-9-chloro-4,6-dimethylbenzo[c][2,7]naphthyridin-5(6H)-one

An ambient temperature solution of (*R*)-*tert-*butyl (1-((9-chloro-4,6-dimethyl-5-oxo-5,6-dihydrobenzo[*c*][2,7]naphthyridin-8-yl)oxy)-4-methylpentan-2-yl)carbamate (15 mg, 0.032 mmol) in dichloromethane (0.5 mL) was treated with HCl (4M in 1,4-dioxane) (0.396 mL, 1.58 mmol) and stirred for 1 h. The resulting mixture was concentrated and the residue was purified by reverse phase high performance liquid chromatography (Phenomenex Luna C18 30 x 100 mm 10 micron; water/acetonitrile/10 mM ammonium acetate gradient elution). Fractions containing product were concentrated under reduced pressure to afford (*R*)-*tert*-butyl (1-((9-chloro-4,6-dimethyl-5-oxo-5,6-dihydrobenzo[*c*][2,7]naphthyridin-8-yl)oxy)-4-methylpentan-2-yl)carbamate one (9.0 mg, 0.023 mmol, 73 % yield) as a white solid. LC/MS, (ESI) *m*/*z* 373.2 [(M+H)⁺, calcd for C₂₀H₂₅ClN₃O₂ 373.2]; HPLC retention time (method C): *t*_{R} = 14.21 min; HPLC retention time (method D): *t*_{R} = 14.30 min; ¹H NMR (400MHz, CHLOROFORM-d) δ 8.70 (d, *J*=5.8 Hz, 1H), 8.21 (s, 1H), 7.79 (d, *J*=5.8 Hz, 1H), 6.83 (s, 1H), 4.18 (dd, *J*=8.8, 3.8 Hz, 1H), 4.04 - 3.97 (m, 1H), 3.76 (s, 3H), 3.48 (qd, *J*=6.9, 3.5 Hz, 1H), 3.17 (s, 3H), 1.84 (dquin, *J*=13.7, 6.7 Hz, 1H), 1.49 (t, *J*=7.0 Hz, 2H), 1.03 (d, *J*=6.5 Hz, 3H), 1.01 (d, *J*=6.5 Hz, 3H).

### Example 21

### (S)-8-(2-amino-3-cyclopropylpropoxy)-4,6-dimethylbenzo[c][2,7]naphthyridin-5(6H)-one

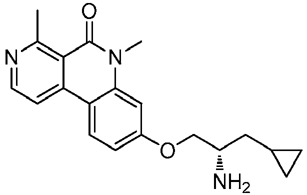

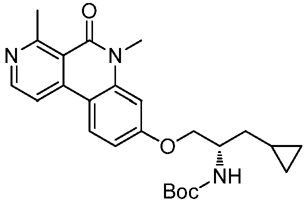

### Part A: (S)-tert-butyl (1-cyclopropyl-3-((4,6-dimethyl-5-oxo-5,6-dihydrobenzo[c][2,7]naphthyridin-8-yl)oxy)propan-2-yl)carbamate

The title compound was prepared as described in Example 16 using (*S*)-*tert-*butyl (1-cyclopropyl-3-hydroxypropan-2-yl)carbamate (0.287 g, 1.334 mmol) in Part H to afford (*S*)-*tert*-butyl (1-cyclopropyl-3-((4,6-dimethyl-5-oxo-5,6-dihydrobenzo[*c*][2,7]naphthyridin-8-yl)oxy)propan-2-yl)carbamate (230 mg, 0.526 mmol, 59 % yield) as a colorless oil. LC/MS, (ESI) *m*/*z* 438.3 [(M+H)⁺, calcd for C₂₅H₃₂N₃O₄ 438.2]; ¹H NMR (400MHz, CHLOROFORM-d) δ 8.61 (d, *J*=5.5 Hz, 1H), 8.09 (d, *J*=8.8 Hz, 1H), 7.77 (d, *J*=5.5 Hz, 1H), 6.96 - 6.79 (m, 2H), 4.92 (br. s., 1H), 4.35 - 3.98 (m, 3H), 3.70 (s, 3H), 3.13 (s, 3H), 1.63 (t, *J*=6.9 Hz, 2H), 1.47 (s, 9H), 0.83 - 0.70 (m, 1H), 0.61 - 0.42 (m, 2H), 0.21 - 0.04 (m, 2H).

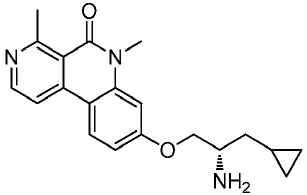

### Part B: (S)-8-(2-amino-3-cyclopropylpropoxy)-4,6-dimethylbenzo[c][2,7]naphthyridin-5(6H)-one

An ambient temperature suspension of (*S*)-*tert*-butyl (1-cyclopropyl-3-((4,6-dimethyl-5-oxo-5,6-dihydrobenzo[*c*][2,7]naphthyridin-8-yl)oxy)propan-2-yl)carbamate (50 mg, 0.114 mmol) (prepared as described in Example 16 using (S)-*tert*-butyl (1-cyclopropyl-3-hydroxypropan-2-yl)carbamate (0.287 g, 1.334 mmol) in Part H) in diethyl ether (1 mL) was treated with HCl (4M in 1,4-dioxane) (0.571 mL, 2.286 mmol) and stirred for 1 h. The resulting mixture was concentrated under reduced pressure to a yellow solid. The crude material was purified by reverse phase high performance liquid chromatography (Phenomenex Luna C18 30 x 100 mm 10 micron; water/methanol/TFA gradient elution). Fractions containing product were concentrated under reduced pressure. The resulting residue was partitioned between saturated aqueous sodium bicarbonate and ethyl acetate. The layers were separated and the aqueous was extracted twice more with ethyl acetate. The pooled organics were washed once with brine, dried over magnesium sulfate, filtered, and concentrated to afford (*S*)-8-(2-amino-3-cyclopropylpropoxy)-4,6-dimethylbenzo[*c*][2,7]naphthyridin-5(6*H*)-one (9.0 mg, 0.025 mmol, 22 % yield for the final step) as a white solid. LC/MS, (ESI) *m*/*z* 338.2 [(M+H)⁺, calcd for C₂₀H₂₄N₃O₂ 338.2]; HPLC retention time (method C): *t*_{R} = 13.05 min; HPLC retention time (method D): *t*_{R} = 13.88 min; ¹H NMR (400MHz, CHLOROFORM-d) δ 8.65 (d, *J*=5.8 Hz, 1H), 8.16 (d, *J*=8.8 Hz, 1H), 7.83 (d, *J*=5.5 Hz, 1H), 6.92 (dd, *J*=8.9, 2.4 Hz, 1H), 6.87 (d, *J*=2.3 Hz, 1H), 4.15 (dd, *J*=8.9, 3.9 Hz, 1H), 3.97 (dd, *J*=8.8, 7.0 Hz, 1H), 3.75 (s, 3H), 3.48 - 3.35 (m, 1H), 3.17 (s, 3H), 1.59 - 1.42 (m, 2H), 0.87 - 0.74 (m, 1H), 0.64 - 0.47 (m, 2H), 0.25 - 0.17 (m, 1H), 0.16 - 0.10 (m, 1H).

### Example 22

### (S)-8-(2-amino-3-cyclobutylpropoxy)-4,6-dimethylbenzo[c][2,7]naphthyridin-5(6H)-one

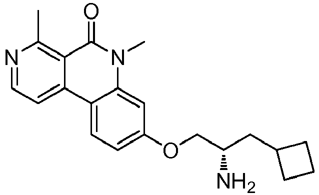

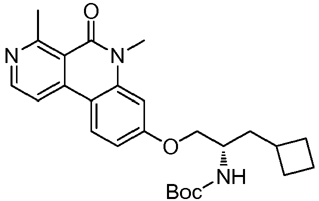

### Part A: (S)-tert-butyl (1-cyclobutyl-3-((4,6-dimethyl-5-oxo-5,6-dihydrobenzo[c][2,7]naphthyridin-8-yl)oxy)propan-2-yl)carbamate

The title compound was prepared as described in Example 16 using (*S*)-*tert-*butyl (1-cyclobutyl-3-hydroxypropan-2-yl)carbamate (0.359 g, 1.566 mmol)in Part H to afford (*S*)-*tert*-butyl (1-cyclobutyl-3-((4,6-dimethyl-5-oxo-5,6-dihydrobenzo[*c*][2,7]naphthyridin-8-yl)oxy)propan-2-yl)carbamate (400 mg, 0.886 mmol, 85 % yield) as a colorless oil. LC/MS, (ESI) *m*/*z* 452.3 [(M+H)⁺, calcd for C₂₆H₃₄N₃O₄ 452.3]; ¹H NMR (400MHz, CHLOROFORM-d) δ 8.62 (d, *J*=5.8 Hz, 1H), 8.11 (d, *J*=8.8 Hz, 1H), 7.79 (d, *J*=5.5 Hz, 1H), 6.94 - 6.79 (m, 2H), 4.76 (d, *J*=7.5 Hz, 1H), 4.07 (d, *J*=3.5 Hz, 2H), 3.93 (br. s., 1H), 3.71 (s, 3H), 3.14 (s, 3H), 2.55 - 2.35 (m, 1H), 2.15 - 2.05 (m, 2H), 1.97 - 1.59 (m, 7H), 1.47 (s, 9H).

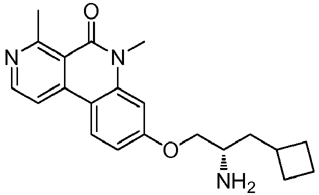

### Part B: (S)-8-(2-amino-3-cyclobutylpropoxy)-4,6-dimethylbenzo[c][2,7]naphthyridin-5(6H)-one

An ambient temperature suspension of (*S*)-*tert*-butyl (1-cyclobutyl-3-((4,6-dimethyl-5-oxo-5,6-dihydrobenzo[*c*][2,7]naphthyridin-8-yl)oxy)propan-2-yl)carbamate (70 mg, 0.155 mmol) in diethyl ether (1 mL) was treated with HCl (4M in 1,4-dioxane) (0.775 mL, 3.10 mmol) and stirred for 1 h. The resulting mixture was concentrated under reduced pressure to a yellow solid. The crude material was purified by reverse phase high performance liquid chromatography (Phenomenex Luna C18 30 x 100 mm 10 micron; water/methanol/TFA gradient elution). Fractions containing product were concentrated under reduced pressure. The resulting residue was partitioned between saturated aqueous sodium bicarbonate and ethyl acetate. The layers were separated and the aqueous was extracted twice more with ethyl acetate. The pooled organics were washed once with brine, dried over magnesium sulfate, filtered, and concentrated under reduced pressure to afford (*S*)-8-(2-amino-3-cyclobutylpropoxy)-4,6-dimethylbenzo[*c*][2,7]naphthyridin-5(6*H*)-one (17 mg, 0.046 mmol, 30 % yield) as a white solid. LC/MS, (ESI) *m*/*z* 352.3 [(M+H)⁺, calcd for C₂₁H₂₀N₃O₂ 352.2]; HPLC retention time (method C): *t*_{R} = 14.15 min; HPLC retention time (method D): *t*_{R} = 14.78 min; ¹H NMR (400MHz, CHLOROFORM-d) δ 8.64 (d, *J*=5.8 Hz, 1H), 8.15 (d, *J*=9.0 Hz, 1H), 7.82 (d, *J*=5.8 Hz, 1H), 6.89 (dd, *J*=8.9, 2.4 Hz, 1H), 6.84 (d, *J*=2.3 Hz, 1H), 4.04 (dd, *J*=8.9, 3.9 Hz, 1H), 3.86 (dd, *J*=8.8, 7.5 Hz, 1H), 3.73 (s, 3H), 3.23 (tdd, *J*=7.5, 5.5, 3.8 Hz, 1H), 3.16 (s, 3H), 2.60 - 2.43 (m, 1H), 2.21 - 2.08 (m, 2H), 2.01 - 1.81 (m, 2H), 1.80 - 1.55 (m, 4H).

### Example 23

### (S)-8-(2-amino-3-cyclopropylpropoxy)-9-chloro-4,6-dimethylbenzo[c][2,7]naphthyridin-5(6H)-one

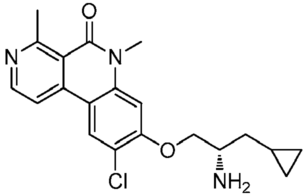

The title compound was prepared as described in Example 20 to afford (*S*)-8-(2-amino-3-cyclopropylpropoxy)-9-chloro-4,6-dimethylbenzo[*c*][2,7]naphthyridin-5(6*H*)-one (12.5 mg, 0.030 mmol, 24 % yield for the final step) as a pale yellow solid. LC/MS, (ESI) *m*/*z* 372.2 [(M+H)⁺, calcd for C₂₀H₂₃ClN₃O₂ 372.2]; HPLC retention time (method C): *t*_{R} = 14.16 min; HPLC retention time (method D): *t*_{R} = 15.03 min; ¹H NMR (400MHz, CHLOROFORM-d) δ 8.66 (d, *J*=5.5 Hz, 1H), 8.17 (s, 1H), 7.74 (d, *J*=5.8 Hz, 1H), 6.81 (s, 1H), 4.22 (dd, *J*=8.7, 3.9 Hz, 1H), 4.05 (dd, *J*=8.5, 7.0 Hz, 1H), 3.73 (s, 3H), 3.49 (qd, *J*=6.8, 4.0 Hz, 1H), 3.20 - 3.12 (m, 3H), 1.63 - 1.46 (m, 2H), 0.89 - 0.75 (m, 1H), 0.67 - 0.45 (m, 2H), 0.25 - 0.18 (m, 1H), 0.17 - 0.10 (m, 1H).

### Example 24

### (S)-8-(2-amino-3-cyclobutylpropoxy)-9-chloro-4,6-dimethylbenzo[c][2,7]naphthyridin-5(6H)-one

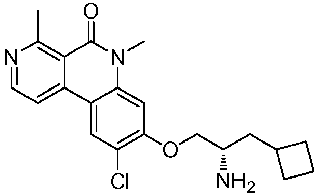

The title compound was prepared as described in Example 20 to afford (*S*)-8-(2-amino-3-cyclobutylpropoxy)-9-chloro-4,6-dimethylbenzo[*c*][2,7]naphthyridin-5(6*H*)-one (10.5 mg, 0.027 mmol, 24 % yield for the final step) as a colorless solid. LC/MS, (ESI) *m*/*z* 386.2 [(M+H)⁺, calcd for C₂₁H₂₅ClN₃O₂ 386.2]; HPLC retention time (method C): *t*_{R} = 14.92 min; HPLC retention time (method D): *t*_{R} = 15.61 min; ¹H NMR (400MHz, CHLOROFORM-d) δ 8.68 (d, *J*=5.5 Hz, 1H), 8.20 (s, 1H), 7.77 (d, *J*=5.8 Hz, 1H), 6.81 (s, 1H), 4.14 (dd, *J*=8.7, 3.9 Hz, 1H), 4.01 - 3.91 (m, 1H), 3.75 (s, 3H), 3.34 (d, *J*=4.5 Hz, 1H), 3.17 (s, 3H), 2.62 - 2.44 (m, 1H), 2.22 - 2.06 (m, 2H), 2.01 - 1.82 (m, 2H), 1.82 - 1.63 (m, 4H).

### Example 25

### (S)-8-(2-amino-3-cyclopropylpropoxy)-4,6,9-trimethylbenzo[c][2,7]naphthyridin-5(6H)-one

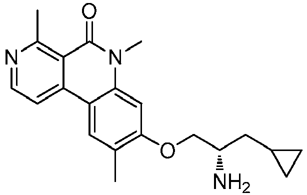

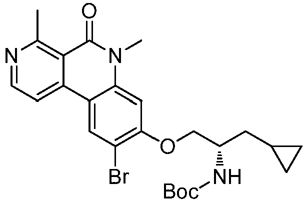

### Part A: (S)-tert-butyl (1-((9-bromo-4,6-dimethyl-5-oxo-5,6-dihydrobenzo[c][2,7]naphthyridin-8-yl)oxy)-3-cyclopropylpropan-2-yl)carbamate

A mixture of (*S*)-*tert*-butyl (1-cyclopropyl-3-((4,6-dimethyl-5-oxo-5,6-dihydrobenzo[*c*][2,7]naphthyridin-8-yl)oxy)propan-2-yl)carbamate (100 mg, 0.229 mmol) prepared as described in Example 21, Part A in acetonitrile (5 mL) was heated to 90 °C for 1 h resulting in a homogeneous orange solution. The solution was treated with saturated aqueous sodium bicarbonate and concentrated under reduced pressure. The residue was partitioned between brine and ethyl acetate and the layers were separated. The aqueous layer was extracted twice more with ethyl acetate and the pooled organics were washed once with brine, dried over magnesium sulfate, filtered, and concentrated to a solid. The solid was purified via silica gel chromatography (1% ammonia in methanol/dichloromethane) to afford (*S*)-*tert*-butyl (1-((9-bromo-4,6-dimethyl-5-oxo-5,6-dihydrobenzo[*c*][2,7]naphthyridin-8-yl)oxy)-3-cyclopropylpropan-2-yl)carbamate (53 mg, 0.103 mmol, 45 % yield) as a colorless oil which solidified upon standing. LC/MS, (ESI) *m*/*z* 516.2, 518.2 Br pattern [(M+H)⁺, calcd for C₂₅H₃₁BrN₃O₄ 516.2].

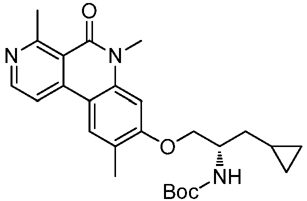

### Part B: (S)-tert-butyl (1-cyclopropyl-3-((4,6,9-trimethyl-5-oxo-5,6-dihydrobenzo[c][2,7]naphthyridin-8-yl)oxy)propan-2-yl)carbamate

An ambient temperature mixture of (*S*)-*tert*-butyl (1-((9-bromo-4,6-dimethyl-5-oxo-5,6-dihydrobenzo[*c*][2,7]naphthyridin-8-yl)oxy)-3-cyclopropylpropan-2-yl)carbamate (51 mg, 0.099 mmol), trimethylboroxine (0.015 mL, 0.109 mmol), PdCl₂(dppf)-CH₂Cl₂Adduct (8.06 mg, 9.88 µmol), cesium carbonate (64.4 mg, 0.198 mmol), 1,4-dioxane (1 mL), and water (0.33 mL) was charged to a pressure rated vial and purged with a stream of nitrogen for 10 minutes. The vial was sealed and stirred under nitrogen at 75 °C for 12 h. The resulting mixture was cooled to room temperature and vacuum filtered. The filtrate was concentrated under reduced pressure and the residue was partitioned between ethyl acetate and brine. The layers were separated and the aqueous layer was extracted twice more with ethyl acetate. The pooled organics were dried over magnesium sulfate, filtered, and concentrated to an oil. The oil was purified via silica gel chromatography (1% 2M ammonia in methanol/dichloromethane) to afford (*S*)-*tert*-butyl (1-cyclopropyl-3-((4,6,9-trimethyl-5-oxo-5,6-dihydrobenzo[*c*][2,7]naphthyridin-8-yl)oxy)propan-2-yl)carbamate (41 mg, 0.091 mmol, 92 % yield) as a colorless oil. LC/MS, (ESI) *m*/*z* 452.3 [(M+H)⁺, calcd for C₂₆H₃₄N₃O₄ 452.3]; ¹H NMR (400MHz, CHLOROFORM-d) δ 8.59 (d, *J*=5.8 Hz, 1H), 7.90 (s, 1H), 7.78 (d, *J*=5.8 Hz, 1H), 6.74 (s, 1H), 4.91 (d, *J*=7.3 Hz, 1H), 4.35 - 4.04 (m, 3H), 3.71 (s, 3H), 3.14 (s, 3H), 2.31 (s, 3H), 1.72 - 1.59 (m, 2H), 1.48 (s, 9H), 0.86 - 0.70 (m, 1H), 0.60 - 0.44 (m, 2H), 0.21 - 0.00 (m, 2H).

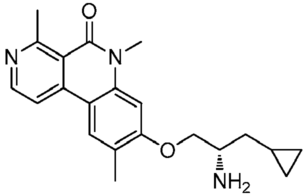

### Part C: (S)-8-(2-amino-3-cyclopropylpropoxy)-4,6,9-trimethylbenzo[c][2,7]naphthyridin-5(6H)-one

An ambient temperature solution of (*S*)-*tert*-butyl (1-cyclopropyl-3-((4,6,9-trimethyl-5-oxo-5,6-dihydrobenzo[*c*][2,7]naphthyridin-8-yl)oxy)propan-2-yl)carbamate (41 mg, 0.091 mmol) in methanol (0.5 mL) was treated with HCl (4M in 1,4-dioxane) (0.454 mL, 1.816 mmol) and stirred for 1 h. The resulting mixture was concentrated under reduced pressure to yellow solid. The crude material was purified by reverse phase high performance liquid chromatography (Phenomenex Luna C18 30x100 mm 10 micron; water/methanol/TFA gradient elution). Product containing fractions were neutralized with saturated aqueous sodium bicarbonate and concentrated under reduced pressure. The resulting residue was partitioned between water and ethyl acetate and the layers were separated. The aqueous was extracted twice more with ethyl acetate and the pooled organics were dried over magnesium sulfate, filtered, and concentrated to afford (*S*)-8-(2-amino-3-cyclopropylpropoxy)-4,6,9-trimethylbenzo[*c*][2,7]naphthyridin-5(6*H*)-one (10.7 mg, 0.028 mmol, 93 % yield) as a colorless solid. LC/MS, (ESI) *m*/*z* 352.2 [(M+H)⁺, calcd for C₂₁H₂₆N₃O₂ 352.2]; HPLC retention time (method C): *t*_{R} = 13.71 min; HPLC retention time (method D): *t*_{R} = 14.29 min; ¹H NMR (400MHz, CHLOROFORM-d) δ 8.63 (d, *J*=5.5 Hz, 1H), 7.98 (s, 1H), 7.84 (d, *J*=5.8 Hz, 1H), 6.74 (s, 1H), 4.15 (dd, *J*=8.8, 4.0 Hz, 1H), 4.00 (dd, *J*=8.7, 6.9 Hz, 1H), 3.76 (s, 3H), 3.50 - 3.37 (m, 1H), 3.17 (s, 3H), 2.37 (s, 3H), 1.61 - 1.44 (m, 2H), 1.02 - 0.74 (m, 1H), 0.68 - 0.45 (m, 2H), 0.30 - 0.06 (m, 2H).

### Example 26

### (S)-8-(2-amino-3-cyclobutylpropoxy)-4,6,9-trimethylbenzo[c][2,7]naphthyridin-5(6H)-one

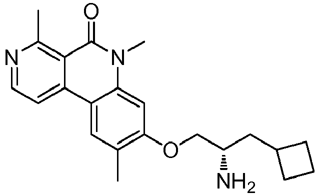

The title compound was prepared as described in Example 25 starting from (*S*)-*tert*-butyl (1-cyclobutyl-3-((4,6-dimethyl-5-oxo-5,6-dihydrobenzo[c][2,7]naphthyridin-8-yl)oxy)propan-2-yl)carbamate, prepared as described in Example 22, Part A to afford (*S*)-8-(2-amino-3-cyclobutylpropoxy)-4,6,9-trimethylbenzo[*c*][2,7]naphthyridin-5(6*H*)-one (18 mg, 0.046 mmol, 26 % yield for the final step) as a hazy film. LC/MS, (ESI) *m*/*z* 366.3 [(M+H)⁺, calcd for C₂₂H₂₈N₃O₂ 366.2]; HPLC retention time (method C): *t*_{R} = 14.65 min; HPLC retention time (method D): *t*_{R} = 15.04 min; ¹H NMR (400MHz, CHLOROFORM-d) δ 8.62 (d, *J*=5.8 Hz, 1H), 7.95 (s, 1H), 7.82 (d, *J*=5.8 Hz, 1H), 6.69 (s, 1H), 4.05 (dd, *J*=8.8, 4.0 Hz, 1H), 3.88 (dd, *J*=8.5, 7.3 Hz, 1H), 3.74 (s, 3H), 3.34 - 3.20 (m, 1H), 3.16 (s, 3H), 2.63 - 2.46 (m, 1H), 2.36 (s, 3H), 2.23 - 2.08 (m, 2H), 2.01 - 1.59 (m, 6H).

### Example 27

### (S)-8-((2-amino-4-methylpentyl)oxy)-9-(difluoromethyl)-4,6-dimethylbenzo[c][2,7]naphthyridin-5(6H)-one

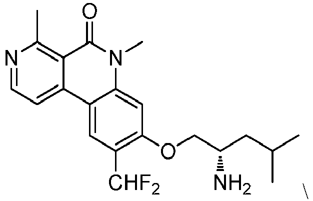

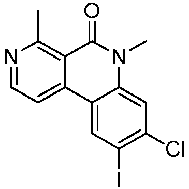

### Part A: 8-chloro-9-iodo-4,6-dimethylbenzo[c][2,7]naphthyridin-5(6H)-one

To a solution of 8-chloro-4,6-dimethylbenzo[*c*][2,7]naphthyridin-5(6*H*)-one (1 g, 3.87 mmol) prepared as described in Example 16, Part G, in acetic acid (30 mL) was added H₂SO₄ (0.021 mL, 0.387 mmol) and periodic acid (0.881 g, 3.87 mmol). The solution was heated at 80°C for 20 min then iodine (0.294 g, 1.160 mmol) was added. The mixture was stirred for another 3 h at 80°C. The solution was cooled to room temperature then concentrated under reduced pressure. EtOAc was added to the residue and the layer was washed with saturated aqueous NaHCO₃ then H₂O. The combined organic layers were dried over Na₂SO₄, filtered and concentrated under reduced pressure to afford 8-chloro-9-iodo-4,6-dimethylbenzo[*c*][2,7]naphthyridin-5(6*H*)-one (1.2 g, 3.12 mmol, 81% crude yield) as an off-white solid. The material was carried on without further purification. LC/MS (ESI) *m*/*e* 385.0 [(M+H)⁺, calcd for C₁₄H₁₁ClIN₂O, 385.0]; LC/MS retention time (method B): *t*_{R} = 1.09 min.

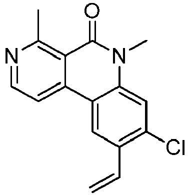

### Part B: 8-chloro-4,6-dimethyl-9-vinylbenzo[c][2,7]naphthyridin-5(6H)-one

A solution of 8-chloro-9-iodo-4,6-dimethylbenzo[*c*][2,7]naphthyridin-5(6*H*)-one (1.2 g, 2.71 mmol), 2,4,6-trivinylcyclotriboroxane pyridine complex (0.784 g, 3.26 mmol), tetrakis(triphenylphosphine)palladium (0.157 g, 0.136 mmol) and Na₂CO₃ (0.575 g, 5.43 mmol) in toluene (30 mL) and water (0.5 mL) was purged with nitrogen and heated at 90°C for 16 h. After cooling to room temperature, the mixture was concentrated under reduced pressure. The residue was taken up in ethyl acetate and filtered through diatomaceous earth (Celite®). The EtOAc layer was washed with brine and water. The combined organic layers were dried over Na₂SO₄, filtered and concentrated under reduced pressure. The crude material was purified by silica gel flash chromatography using EtOAc-hexane as the eluent to afford 8-chloro-4,6-dimethyl-9-vinylbenzo[*c*][2,7]naphthyridin-5(6*H*)-one (0.6 g, 1.43 mmol, 68% yield)) as brown gummy solid. LC/MS (ESI) m/e 285.0 [(M+H)⁺, calcd for C₁₆H₁₄ClN₂O, 285.1]; LC/MS retention time (method B): t_{R} = 2.25 min.

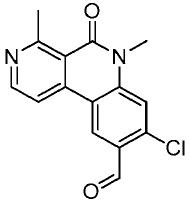

### Part C: 8-chloro-4,6-dimethyl-5-oxo-5,6-dihydrobenzo[c][2,7]naphthyridine-9-carbaldehyde

To a solution of 8-chloro-4,6-dimethyl-9-vinylbenzo[*c*][2,7]naphthyridin-5(6*H*)-one (600 mg, 2.107 mmol), osmium tetroxide (2.5% in 2-methyl-2-propanol) (0.794 mL, 0.063 mmol), and 2,6-dimethylpyridine (0.491 mL, 4.21 mmol) in 1,4-dioxane (30 mL) and water (30 mL) cooled to 0°C was added sodium periodate (1803 mg, 8.43 mmol). The mixture was warmed to room temperature and stirred for 3 h. The mixture was diluted with ethyl acetate and filtered through diatomaceous earth (Celite®). The organic layer was concentrated under reduced pressure to afford a brown solid. The solid was washed with hexane (3x20 mL) and dried under vacuum to afford 8-chloro-4,6-dimethyl-5-oxo-5,6-dihydrobenzo[c][2,7]naphthyridine-9-carbaldehyde 0.6 g, 2.107 mmol, 84% yield) as a gummy brown solid. LC/MS (ESI) *m*/*e* 287.0 [(M+H)⁺, calcd for C₁₅H₁₂ClN₂O₂, 287.1]; LC/MS retention time (method B): *t*_{R} = 2.03 min.

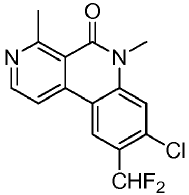

### Part D: 8-chloro-9-(difluoromethyl)-4,6-dimethylbenzo[c][2,7]naphthyridin-5(6H)-one

To a solution of 8-chloro-4,6-dimethyl-5-oxo-5,6-dihydrobenzo[c][2,7] naphthyridine-9-carbaldehyde (600 mg, 1.779 mmol) in DCM (15 mL) was added bis-(2-methoxyethyl)aminosulfur trifluoride (Deoxo-Fluor) (1.968 g, 8.89 mmol). The mixture was then heated at 40°C for 3 h. After cooling to room temperature, the reaction mixture was diluted with DCM and the DCM layer washed with saturated aqueous NaHCO₃ then H₂O, dried over Na₂SO₄, filtered and concentrated under reduced pressure. The residue was purified by silica gel flash chromatography using EtOAc-hexane as the eluent to afford 8-chloro-9-(difluoromethyl)-4,6-dimethylbenzo[*c*][2,7]naphthyridin-5(6*H*)-one (0.7 g, 1.519 mmol, 84% yield) as an off-white solid. LC/MS (ESI) *m*/*e* 309.0 [(M+H)⁺, calcd for C₁₅H₁₂ClF₂N₂O, 309.1]; LC/MS retention time (method B): *t*_{R} = 2.38 min. ¹H NMR (400 MHz, *METHANOL-d*₄) δ ppm 8.68 - 8.74 (m, 2H), 8.32 (d, 1H), 7.78 (s, 1H), 7.00 - 7.33 (m, 1H), 3.79 (s, 3H), 3.13 (s, 3H).

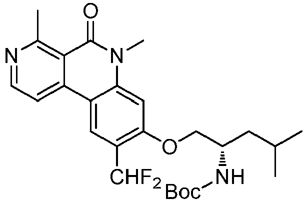

### Part E: (S)-tert-butyl (1-((9-(difluoromethyl)-4,6-dimethyl-5-oxo-5,6-dihydrobenzo[c][2,7]naphthyridin-8-yl)oxy)-4-methylpentan-2-yl)carbamate

A solution of 8-chloro-9-(difluoromethyl)-4,6-dimethylbenzo[*c*][2,7]naphthyridin-5(6*H*)-one (700 mg, 1.519 mmol), (*S*)-*tert*-butyl (1-hydroxy-4-methylpentan-2-yl)carbamate (660 mg, 3.04 mmol), 2-di-t-butylphosphino-2',4',6'-tri-i-propyl-1,1'-biphenyl (387 mg, 0.912 mmol), PdOAc₂ (34.1 mg, 0.152 mmol) and Cs₂CO₃ (990 mg, 3.04 mmol) in toluene (20 mL) was heated at 80°C for 16 h. After cooling to room temperature, the mixture was concentrated under reduced pressure.. The residue was taken up in ethyl acetate and filtered through diatomaceous earth (Celite®). The organic layer was washed with brine and water; dried over Na₂SO₄, filtered and concentrated under reduced pressure. The residue was purified by silica gel flash chromatography using EtOAc-hexane as the eluent to afford (*S*)-*tert*-butyl (1-((9-(difluoromethyl)-4,6-dimethyl-5-oxo-5,6-dihydrobenzo[*c*][2,7]naphthyridin-8-yl)oxy)-4-methylpentan-2-yl)carbamate (0.6 g, 0.58 mmol, 54% yield) as a brown gummy solid. LC/MS (ESI) *m*/*e* 490.2 [(M+H)⁺, calcd for C₂₆H₃₄F₂N₃O₄, 490.2]; LC/MS retention time (method B): *t*_{R} = 2.72 min.

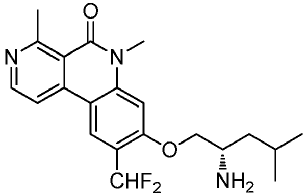

### Part F: (S)-8-((2-amino-4-methylpentyl)oxy)-9-(difluoromethyl)-4,6-dimethylbenzo[c][2,7]naphthyridin-5(6H)-one

To a solution of (*S*)-*tert*-butyl (1-((9-(difluoromethyl)-4,6-dimethyl-5-oxo-5,6-dihydrobenzo[*c*][2,7]naphthyridin-8-yl)oxy)-4-methylpentan-2-yl)carbamate (500 mg, 0.480 mmol) in MeOH (10 mL) at 0°C was added 4N HCl in1,4-dioxane (20 mL, 80 mmol). The solution was warmed to room temperature and stirred for 2 h. The mixture was then was concentrated under reduced pressure. The residue was taken up in EtOAc and the EtOAc layer was washed with saturated aqueous NaHCO₃ then H₂O, dried over Na₂SO₄, filtered and concentrated under reduced pressure. The residue was purified by prep. HPLC to afford (*S*)-8-((2-amino-4-methylpentyl)oxy)-9-(difluoromethyl)-4,6-dimethylbenzo[*c*][2,7]naphthyridin-5(6*H*)-one (60 mg, 0.149 mmol, 31% yield) as pale yellow solid. LC/MS (ESI) *m*/*e* 390.2 [(M+H)⁺, calcd for C₂₁H₂₆F₂N₃O₂, 390.2]; LC/MS retention time (method A): *t*_{R} = 2.09 min; HPLC retention time (method A): *t*_{R} = 10.1 min; HPLC retention time (method B): *t*_{R} = 9.18 min; ¹H NMR (400 MHz, *METHANOL-d*₄) δ ppm 8.73 (s, 1H), 8.66 (s, 2H), 7.07 - 7.41 (m, 2H), 4.66 - 4.53 (m, 1H), 4.52 - 4.49 (m, 1H), 3.78 - 3.89 (m, 4H), 3.22 (s, 3H), 1.74 - 1.88 (m, 2H), 1.63 - 1.71 (m, 1H), 1.04 (m, 6H).

### Example 28

### tert-butyl (2,4-dimethyl-1-((6-methyl-5-oxo-5,6 dihydrobenzo[c][2,7]naphthyridin-8-yl)oxy)pentan-2-yl)carbamate

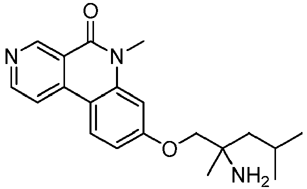

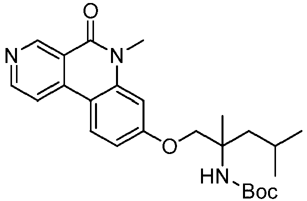

### Part A. tert-butyl (2,4-dimethyl-1-((6-methyl-5-oxo-5,6-dihydrobenzo[c][2,7]naphthyridin-8-yl)oxy)pentan-2-yl)carbamate

8-Chloro-6-methylbenzo[*c*][2,7]naphthyridin-5(*6H*)-one (0.15 g, 0.613 mmol), prepared as described in Example 2, Part C, and *tert*-butyl (1-hydroxy-2,4-dimethylpentan-2-yl)carbamate (0.284 g, 1.226 mmol) were subjected to ether synthesis as described in Example 2, Part D, to afford *tert*-butyl(2,4-dimethyl-1-((6-methyl-5-oxo-5,6-dihydrobenzo[*c*][2,7]naphthyridin-8-yl)oxy)pentan-2-yl)carbamate (0.3 g, 28% yield) as pale yellow solid. LC/MS (ESI) m/e 440.2 [(M+H)⁺, calcd for C₂₅H₃₄N₃O₄ 440.2]; LC/MS retention time (method C): *t*_{R} = 2.67 min.

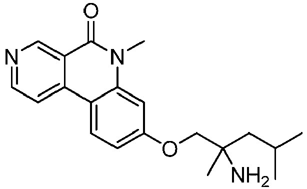

### Part B. 8-((2-amino-2,4-dimethylpentyl)oxy)-6-methylbenzo[c][2,7]naphthyridin-5(6H)-one

*tert*-Butyl (2,4-dimethyl-1-((6-methyl-5-oxo-5,6 dihydrobenzo[*c*][2,7]naphthyridin-8-yl)oxy)pentan-2-yl)carbamate was taken further for deprotection of Boc as described in example 2, part E to afford 8-((2-amino-2,4-dimethylpentyl)oxy)-6-methylbenzo[*c*][2,7]naphthyridin-5(*6H*)-one (30 mg, 12% yield) as off white solid. LC/MS (ESI) *m*/*e* 340.0 [(M+H)⁺, calcd for C₂₀H₂₆N₃O₂, 340.2]; LC/MS retention time (method G): *t*_{R} = 1.88 min. ¹H NMR (400 MHz, *METHANOL-d₄*) δ ppm 9.43 (s, 1H), 8.73 (d, *J*=6.0 Hz, 1H), 8.35 (d, *J*=8.5 Hz, 1H), 8.19 (d, *J*=6.0 Hz, 1H), 7.12 - 7.01 (m, 2H), 4.05 - 3.92 (m, 2H), 3.77 (s, 3H), 1.95 - 1.80 (m, 1H), 1.58 (qd, *J*=14.2, 5.5 Hz, 2H), 1.30 (s, 3H), 1.04 (d, *J*=6.5 Hz, 3H), 1.01 (d, *J*=6.5 Hz, 3H). Chiral HPLC (Method CHIRALPAK AD-H (250x4.6) mm, 5 micron, Mob. Phase CO₂ (65%), Co-solvent 0.3% DEA in MeOH (35%), Back pressure 101), Chiral SFC retention time *t*_{R1} = 5.44 min; Chiral SFC retention time *t*_{R2} = 7.77 min.

### Example 29

### 8-((2-amino-2,4-dimethylpentyl)oxy)-7-fluoro-4,6-dimethylbenzo[c][2,7]naphthyridin-5(6H)-one

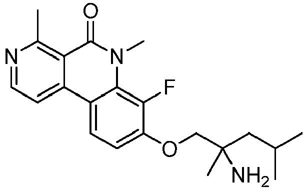

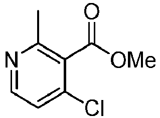

### Part A. Methyl 4-chloro-2-methylnicotinate

To a solution of methyl 2,4-dichloronicotinate (10.0 g, 48.5 mmol) in a solvent mixture of 1,4-dioxane (400 mL) and water (40 mL) was added trimethylboroxine (6.79 mL, 48.5 mmol) followed by PdCl₂(dppf)-CH₂Cl₂ adduct (1.982 g, 2.427 mmol) and cesium carbonate (31.6 g, 97 mmol). The reaction mixture was degassed with argon for 5 minutes. The reaction mixture was heated to reflux for 8 h. The reaction mixture was cooled to room temperature and filtered through diatomaceous earth (Celite®). The bed was washed with ethyl acetate and the combined filtrate was concentrated under reduced pressure to afford the crude product which was purified by silica gel column chromatography (gradient of ethyl acetate and petroleum ether) to afford methyl 4-chloro-2-methylnicotinate (5.20 g, 28 mmol, 58% yield) as a yellow liquid. LC/MS (ESI) *m*/*e* 185.7 [(M+H)⁺, calcd for C₈H₈ClNO₂ 185.6]; LC/MS retention time (method G): *t*_{R} = 1.90 min.

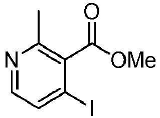

### Part B. Methyl 4-iodo-2-methylnicotinate

To a solution of methyl 4-chloro-2-methylnicotinate (6.00 g, 32.3 mmol) in acetonitrile (80 mL) was added acetyl chloride (3.45 mL, 48.5 mmol) followed by sodium iodide (48.5 g, 323 mmol). The reaction mixture was heated at 80°C for 16hrs. The reaction mixture was evaporated to dryness and the residue was adjusted to pH 6 by adding saturated potassium carbonate solution. The product was extracted with dichloromethane (250 mL). The organic phase was washed with brine (2x100 mL), dried over Na₂SO₄, and concentrated under reduced pressure to afford methyl 4-iodo-2-methylnicotinate (2.00 g, 7.22 mmol, 22% crude yield) as colorless semisolid which was carried on without further purifications. LC/MS (ESI) *m*/*e* 278.0 [(M+H)⁺, calcd for C₈H₉INO₂ 278.0]; LC/MS retention time (method C): *t*_{R} = 2.13 min.

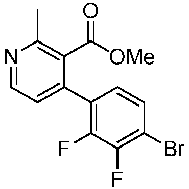

### Part C. Methyl 4-(4-bromo-2,3-difluorophenyl)-2-methylnicotinate

In a microwave vessel, methyl 4-iodo-2-methylnicotinate (1.00 g, 3.61 mmol) was taken in a mixture of 1,4-dioxane (18 mL) and water (2 mL) under inert atmosphere. (4-bromo-2,3-difluorophenyl)boronic acid (1.03 g, 4.33 mmol) and Na₂CO₃ (765 mg, 7.22 mmol) were added to the reaction mixture and degassed for 5 minutes. Pd(Ph₃P)₄ (83 mg, 0.072 mmol) was added to the reaction mixture and heated in microwave at 110°C for 90 min. The reaction mixture was diluted with water (150 mL) and extracted with ethyl acetate (200 mL). The organic phase was washed with brine (2x100 mL), dried over Na₂SO₄, and concentrated under reduced pressure. The residue was purified by silica gel chromatography (gradient of ethyl acetate and petroleum ether) to afford methyl 4-(4-bromo-2,3-difluorophenyl)-2-methylnicotinate (540 mg, 1.58 mmol, 44% yield) as a yellow liquid. LC/MS (ESI) *m*/*e* 344.0 [(M+2H)⁺, calcd for C₁₄H₁₂BrF₂NO₂ 344.1]; LC/MS retention time (method C): *t*_{R} = 2.48 min.

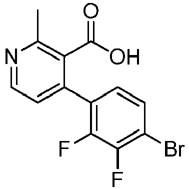

### Part D. 4-(4-bromo-2,3-difluorophenyl)-2-methylnicotinic acid

To a solution of methyl 4-(4-bromo-2,3-difluorophenyl)-2-methylnicotinate (800 mg, 2.34 mmol) in MeOH (10 mL) was added NaOH (935 mg, 23.38 mmol) in Water (10 mL). The reaction mixture was stirred at room temperature for 16hrs. The reaction mixture was evaporated under reduced pressure and the residue obtained was adjusted to pH ∼3 by adding 1.5N HCl solution. The product was extracted with dichloromethane and the layers were separated. The organic phase was dried over Na₂SO₄, and concentrated under reduced pressure to afford 4-(4-bromo-2,3-difluorophenyl)-2-methylnicotinic acid (700 mg, 2.133 mmol, 91 % crude yield) as an off-white solid which was carried on without further pruification. LC/MS (ESI) *m*/*e* 330.0 [(M+2H)⁺, calcd for C₁₃H₁₀BrF₂NO₂ 330.1]; LC/MS retention time (method C): *t*_{R} = 1.34 min.

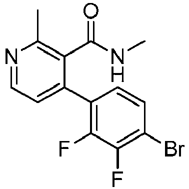

### Part E. 4-(4-bromo-2,3-difluorophenyl)-N,2-dimethylnicotinamide

To a solution of 4-(4-bromo-2,3-difluorophenyl)-2-methylnicotinic acid (700 mg, 2.13 mmol) in dichloromethane (20 mL) at 0 °C was added oxalyl chloride (0.56 mL, 6.40 mmol) drop wise followed by DMF (0.03 mL, 0.43 mmol). The reaction mixture was heated to 45 °C for 4h. The reaction mixture was evaporated to dryness. The residue was taken in dichloromethane (50 mL) at 0 °C and methylamine hydrochloride (1.56 g, 23.08 mmol) was added to it followed by triethylamine (4.83 mL, 34.6 mmol). The reaction mixture was stirred at room temperature for 2h. The reaction mixture was diluted with water (50 mL) and extracted with dichloromethane (100 mL). The organic layer was separated, dried over Na₂SO₄ and concentrated under reduced pressure to afford 4-(4-bromo-2,3-difluorophenyl)-*N*,2-dimethylnicotinamide (600 mg, 1.76 mmol, 76% crude yield) as yellow semi-solid which was carried on without further purification. LC/MS (ESI) *m*/*e* 343.0 [(M+2H)⁺, calcd for C₁₄H₁₃BrF₂N₂O 343.0]; LC/MS retention time (method C): *t*_{R} = 1.61 min.

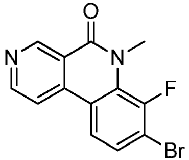

### Part F. 8-bromo-7-fluoro-4,6-dimethylbenzo[c][2,7]naphthyridin-5(6H)-one

To a solution of 4-(4-bromo-2,3-difluorophenyl)-N,2-dimethylnicotinamide (600 mg, 1.76 mmol) in tetrahydrofuran (20 mL) at 0 °C was added NaH (176 mg, 4.40 mmol) and the reaction mixture was brought to room temperature gradually. The reaction mixture was stirred at room temperature for 15h. The reaction mixture was diluted with water (50 mL) and extracted with ethyl acetate (100 mL). The organic layer was separated, dried over Na₂SO₄ and concentrated under reduced pressure to afford 8-bromo-7-fluoro-4,6-dimethylbenzo[*c*][2,7]naphthyridin-5(*6H*)-one (400 mg, 1.24 mmol, 71% yield) as yellow solid. LC/MS (ESI) *m*/*e* 322.8 [(M+2H)⁺, calcd for C₁₄H₁₂BrFN₂O 322.9]; LC/MS retention time (method G): *t*_{R} = 2.21 min.

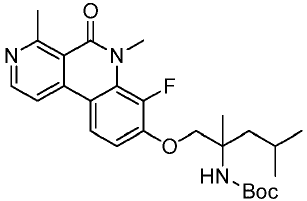

### Part G. tert-butyl (1-((7-fluoro-4,6-dimethyl-5-oxo-5,6-dihydrobenzo[c][2,7]naphthyridin-8-yl)oxy)-2,4-dimethylpentan-2-yl)carbamate

To a stirred solution of 8-bromo-7-fluoro-4,6-dimethylbenzo[*c*][2,7]naphthyridin-5(*6H*)-one (350 mg, 1.090 mmol) and *t*ert-butyl (1-hydroxy-2,4-dimethylpentan-2-yl)carbamate (504 mg, 2.180 mmol) in toluene (10 mL) was added cesium carbonate (355 mg, 1.09 mmol) and 2-Di-t-butylphosphino-2',4',6'-tri-i-propyl-1,1'-biphenyl (28 mg, 0.065 mmol). The reaction mixture was degassed with argon for 5 min and then palladium acetate (7.34 mg, 0.033 mmol) was added to it. The reaction mixture was heated to 100 °C for 18 h. Then, it was filtered through diatomaceous earth (Celite®) and the filtrate was evaporated to afford *tert*-butyl (1-((7-fluoro-4,6-dimethyl-5-oxo-5,6-dihydrobenzo[*c*][2,7]naphthyridin-8-yl)oxy)-2,4-dimethylpentan-2-yl)carbamate which was taken to next step without purification. LC/MS (ESI) *m*/*e* 472.2 [(M+H)⁺, calcd for C₂₆H₃₅FN₃O₄ 472.2]; LC/MS retention time (method C): *t*_{R} = 2.17 min.

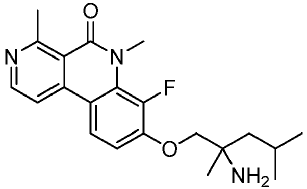

### Part H. 8-((2-amino-2,4-dimethylpentyl)oxy)-7-fluoro-4,6-dimethylbenzo[c][2,7]naphthyridin-5(6H)-one

To a solution of *tert*-butyl (1-((7-fluoro-4,6-dimethyl-5-oxo-5,6 dihydrobenzo[*c*][2,7]naphthyridin-8-yl)oxy)-2,4-dimethylpentan-2-yl)carbamate (200 mg, 0.424 mmol) in MeOH (6 mL) at 0 °C was added 4M HCl in 1,4-dioxane (3 mL, 99.0 mmol). The reaction mixture was stirred at 0 °C for 2h. After 2h, the reaction mixture was concentrated under reduced pressure to afford crude product which was purified by preparative HPLC to afford 8-((2-amino-2,4-dimethylpentyl)oxy)-7-fluoro-4,6-dimethylbenzo[*c*][2,7]naphthyridin-5(*6H*)-one (10 mg, 0.024 mmol, 6% yield) as an off-white solid. LC/MS (ESI) *m*/*e* 372.0 [(M+H)⁺, calcd for C₂₁H₂₇FN₃O₂ 372.2]; LC/MS retention time (method G): *t*_{R} = 1.99 min. HPLC retention time (method A): *t*_{R} = 8.37 min; HPLC retention time (method B): *t*_{R} = 9.72 min. ¹H NMR (400MHz, *METHANOL-d₄*) δ ppm 8.58 (d, *J* = 6 Hz, 1 H), 8.22 (dd, *J* = 9.2 Hz, *J* = 2 Hz, 1 H), 8.14 (d, *J* = 6 Hz, 1 H), 7.23 (m, 1 H), 4.31 (d, *J* = 10 Hz, 1 H), 4.23 (d, *J* = 10.4 Hz, 1 H), 3.88 (d*, J* = 9.2 Hz, 3 H), 3.06 (s, 3 H), 1.90 (m, 2 H), 1.73 (m, 1 H), 1.52 (s, 3 H), 1.05 (m, 6 H).

### Example 30 & Example 31

### 8-(((S)-2-amino-4-methylpentyl)oxy)-9-(1-hydroxyethyl)-4,6 dimethylbenzo[c][2,7]naphthyridin-5(6H)-one

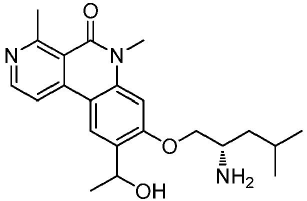

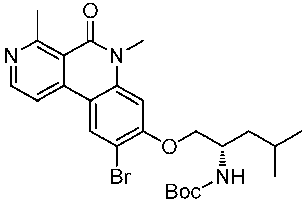

### Part A. (S)-tert-butyl (1-((9-bromo-4,6-dimethyl-5-oxo 5,6-dihydrobenzo[c][2,7]naphthyridin-8-yl)oxy)-4-methylpentan-2-yl)carbamate

*(S)-tert-*Butyl (1-((4,6-dimethyl-5-oxo-5,6-dihydrobenzo[*c*][2,7]naphthyridin-8-yl)oxy)-4-methylpentan-2-yl)carbamate, prepared as described in Example 16, Part H, was subjected to bromination following the condition mentioned in Example 3, Part A to afford *(S)-tert*-butyl (1-((9-bromo-4,6-dimethyl-5-oxo 5,6dihydrobenzo[*c*][2,7]naphthyridin-8-yl)oxy)-4-methylpentan-2-yl)carbamate (1.6 g, 2.62 mmol, 77% yield) as pale yellow solid. LC/MS (ESI) *m*/*e* 518.2 [(M)⁺, calcd for CH₃₃BrN₃O₄ 518.1]; LC/MS retention time (method C): *t*_{R} = 2.46 min.

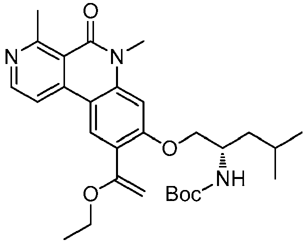

### Part B. (S)-tert-butyl (1-((9-(1-ethoxyvinyl)-4,6 dimethyl-5-oxo-5,6-dihydrobenzo[c][2,7]naphthyridin-8-yl)oxy)-4-methylpentan-2yl)carbamate

To a solution of *(S)-tert-*butyl (1-((9-bromo-4,6-dimethyl-5-oxo-5,6 dihydrobenzo[*c*][2,7]naphthyridin-8-yl)oxy)-4-methylpentan-2-yl)carbamate (500 mg, 0.964 mmol) in 1,4-dioxane (5 mL), tributyl(1-ethoxyvinyl)stannane (697 mg, 1.929 mmol), tris(dibenzylideneacetone)dipalladium (0) (88 mg, 0.096 mmol) and DPPF (53.5 mg, 0.096 mmol) were added. The reaction mixture was allowed to stir at 100 °C for 12h. The reaction mixture was diluted with water and extracted in ethyl acetate. The organic layer was dried over sodium sulfate and concentrated which afforded *(S)-tert-*butyl (1-((9-(1-ethoxyvinyl)-4,6 dimethyl-5-oxo-5,6-dihydrobenzo[*c*][2,7]naphthyridin-8-yl)oxy)-4-methylpentan-2-yl)carbamate (0.3 g, 0.33 mmol, 34% crude yield) as brown solid which was carried on without further purification. LC/MS (ESI) *m*/*e* 510.2 [(M+H)⁺, calcd for C₂₉H₄₀N₃O₅, 510.3]; LC/MS retention time (Method = C): *t*_{R} = 2.60 min.

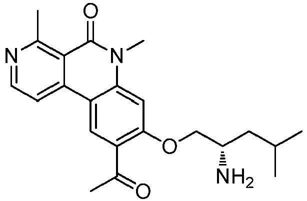

### Part C. (S)-9-acetyl-8-((2-amino-4-methylpentyl)oxy)-4,6-dimethylbenzo[c][2,7]naphthyridin-5(6H)-one

*(S)-tert*-Butyl (1-((9-(1-ethoxyvinyl)-4,6-dimethyl-5-oxo-5,6 dihydrobenzo[*c*][2,7]naphthyridin-8-yl)oxy)-4-methylpentan-2-yl)carbamate (200 mg, 0.392 mmol) was treated with hydrochloric acid in 1,4-dioxane (981 µL, 3.92 mmol) at 0 °C. The reaction mixture was allowed to stir at RT for 2h and then evaporated to dryness. The crude material was neutralized with aqueous 10% NaHCO₃ and extracted with ethyl acetate. The organic layer was dried over Na₂SO₄ and concentrated which afforded *(S)*-9-acetyl-8-((2-amino-4-methylpentyl)oxy)-4,6-dimethylbenzo[*c*][2,7]naphthyridin-5(*6H*)-one (0.11 g, 0.164 mmol, 42% yield) as brown solid. The material was carried on without further purification. LC/MS (ESI) *m*/*e* 382.2 [(M+H)⁺, calcd for C₂₂H₂₈N₃O₃, 382.2]; LC/MS retention time (method E): *t*_{R} = 0.78 min.

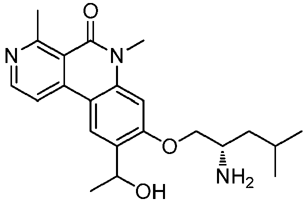

### Part D. 8-(-((S)-2-amino-4-methylpentyl)oxy)-9-(1-hydroxyethyl)-4,6 dimethylbenzo[c][2,7]naphthyridin-5(6H)-one

To a solution of *(S)*-9-acetyl-8-((2-amino-4-methylpentyl)oxy)-4,6 dimethylbenzo[*c*][2,7]naphthyridin-5(*6H*)-one (100 mg, 0.257 mmol) in Ethanol (2 mL) was added NaBH₄ (97 mg, 2.57 mmol) at 0 °C. The reaction mixture was allowed to stir at RT for 16h. Then, it was quenched with NH₄Cl, diluted with water and extracted in ethyl acetate. The combined organic layers were dried over Na₂SO₄ and concentrated. The crude reaction mixture was purified by prep. HPLC which afforded 8-((*(S)*-2-amino-4-methylpentyl)oxy)-9-(1-hydroxyethyl)-4,6-dimethylbenzo[*c*][2,7]naphthyridin-5(6*H*)-one (0.03 g, 27% yield) as pale yellow semi-solid. LC/MS (ESI) *m*/*e* 384.1 [(M+H)⁺, calcd for C₂₂H₃₀N₃O₃, 384.2]; LC/MS retention time (method E): *t*_{R} = 0.73 min.
96533-072 - Chiral separation was done which afforded two diastereomers as off-white solid. Diastereomer-1 was eluted at retention time (HPLC Column : (250X30X5µ) M.Phase A: 0.2% DEA Hexane; M, phase B:Ethanol): *t*_{R} = 21.9 min. 96533-072-11 - ¹H NMR (400 MHz, METHANOL-*d*₄): ppm 1.01 - 1.07 (m, 6H), 1.55 (d, *J*=6.46 Hz, 4H), 1.58 - 1.68 (m, 1H), 1.83 - 1.92 (m, 1H), 3.09 (s, 3H), 3.35 (d, *J*=1.69 Hz, 1H), 3.80 (s, 3H), 4.13 - 4.19 (m, 1H), 4.28 - 4.34 (m, 1H), 5.32 (d, *J*=6.46 Hz, 1H), 7.04 (s, 1H), 8.16 (d, *J*=5.84 Hz, 1H), 8.47 (s, 1H), 8.58 (d, *J*=5.77 Hz, 1H).
Diastereomer-2 was eluted at retention time (HPLC Column : (250X30X5µ) M.Phase A: 0.2% DEA Hexane; M, phase B:Ethanol) *t*_{R} = 24.9 min.
96533-072-12 - ¹H NMR (400 MHz, METHANOL-*d*₄): ppm 1.03 (t, *J*=6.84 Hz, 6H), 1.43 - 1.58 (m, 5H), 1.88 (dt, *J*=13.73, 6.97 Hz, 1H), 3.09 (s, 3H), 3.35 (d, *J*=1.69 Hz, 1H), 3.80 (s, 3H), 4.03 - 4.13 (m, 1H), 4.21 - 4.29 (m, 1H), 5.31 (d, *J*=6.40 Hz, 1H), 7.03 (s, 1H), 8.15 (d, *J*=5.84 Hz, 1H), 8.47 (s, 1H), 8.57 (d, *J*=5.77 Hz, 1H).

### Example 32

### 8-((2-amino-2,4-dimethylpentyl)oxy)-4,6-dimethylbenzo[c][2,7]naphthyri din-5(6H)-one

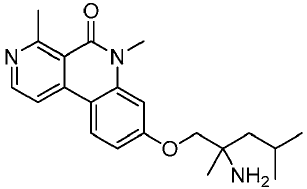

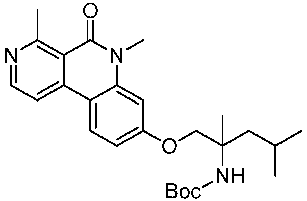

### Part A. tert-butyl(1-((4,6-dimethyl-5-oxo-5,6-dihydrobenzo[c][2,7]naphthyridin-8-yl)oxy)-2,4-dimethylpentan-2-yl)carbamate

8-Chloro-4,6-dimethylbenzo[*c*][2,7]naphthyridin-5(*6H*)-one (0.15 g, 0.580 mmol), prepared as described in Example 16, Part G, and *tert*-butyl (1-hydroxy-2,4-dimethylpentan-2-yl)carbamate (0.161 g, 0.696 mmol) were subjected to the Buchwald coupling as described in Example 16, Part H, to afford *tert*-butyl(1-((4,6-dimethyl-5-oxo-5,6-dihydrobenzo[*c*][2,7]naphthyridin-8-yl)oxy)-2,4-dimethylpentan-2-yl)carbamate (0.22 g, 0.199 mmol, 34% crude yield). Crude product was used for next step without further purification. LC/MS (ESI) *m*/*e* 454.1 [(M+H)⁺, calcd for C₂₆H₃₆N₃O₄, 454.3]; LC/MS retention time (method D): *t*_{R} = 0.93 min.

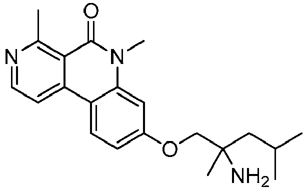

### Part B. 8-((2-amino-2,4-dimethylpentyl)oxy)-4,6-dimethylbenzo[c][2,7]naphthyri din-5(6H)-one

*tert*-Butyl (1-((4,6-dimethyl-5-oxo-5,6-dihydrobenzo[*c*][2,7]naphthyridin-8-yl)oxy)-2,4-dimethylpentan-2-yl)carbamate (0.22 g, 0.199 mmol) was subjected to de-protection of the Boc group using the procedure described in Example 2, Part E to give 8-((2-amino-2,4-dimethylpentyl)oxy)-4,6 dimethylbenzo[*c*][2,7]naphthyridin-5(*6H*)-one (8 mg, 0.022 mmol, 11% yield) as white solid. LC/MS (ESI) *m*/*e* 354.2 [(M+H)⁺, calcd for C₂₁H₂₈N₃O₂, 354.2]; LC/MS retention time (method C): *t*_{R} = 1.57 min. HPLC retention time (method A): *t*_{R} = 8.67 min; HPLC retention time (method B): *t*_{R} = 9.46 min. ¹H NMR (400 MHz, METHANOL-*d*4) ppm δ 8.54 (s, 1H), 8.34-8.36 (bs, 1H), 8.10 (bs, 1H), 7.06 (s, 1H), 4.11-4.17 (m, 2H), 3.73 (s, 3H), 3.06 (s, 3H), 1.70 - 1.98 (m, 2H), 1.68 (bs, 1H), 1.45 (s, 3H),1.02 - 1.08 (m, 6H).

### Example 33

### 8-(((2S,3S)-3-amino-5-methylhexan-2-yl)oxy)-4,6 dimethylbenzo[c][2,7]naphthyridin-5(6H)-one

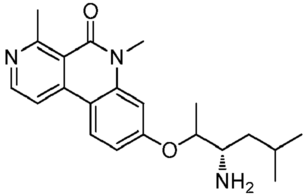

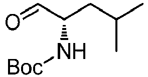

### Part A. (S)-tert-butyl (4-methyl-1-oxopentan-2-yl)carbamate

To a solution of *(S)-tert-*butyl (1-hydroxy-4-methylpentan-2-yl)carbamate (2 g, 9.20 mmol) in DCM (30 mL) was added Dess-Martin Periodinane (5.86 g, 13.81 mmol) at 0 °C. The reaction mixture was allowed to stir at RT for 16h. The reaction mixture was quenched with NaHCO₃, diluted with water and extracted in ethyl acetate. Organic layer was dried over Na₂SO₄ and concentrated which afforded *(S)-tert*-butyl (4-methyl-1-oxopentan-2-yl)carbamate as colorless oil (1.4 g, 6.50 mmol, 71% crude yield). The material was carried on without further purification. ¹H NMR (300 MHz, DMSO-*d6*): δ 0.8-0.96 (m, 6H), 1.31-1.49 (m, 11H), 1.57-1.66 (m, 1H), 3.81-3.89 (m, 1H). 7.27 (d, 1H), 9.43 (s, 1H).

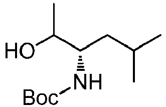

### Part B. tert-butyl ((3S)-2-hydroxy-5-methylhexan-3-yl)carbamate

A solution of *(S)-tert-*butyl (4-methyl-1-oxopentan-2-yl)carbamate (1.3 g, 6.04 mmol) in diethyl ether (50 mL) was cooled to -78 °C and treated with methyl magnesium bromide (1.4 M in diethy ether) (8.63 mL, 12.08 mmol). The slurry was stirred at 0 °C for 1h and then stirred for an additional hour at RT. The reaction mixture was quenched with NH₄Cl. The reaction mixture was extracted with diethyl ether (3 x 100 mL). The combined organic layers were washed with brine, dried over MgSO₄, and concentrated to afford the product, *tert*-butyl ((3*S*)-2-hydroxy-5-methylhexan-3-yl)carbamate (1 g, 4.32 mmol, 72 % crude yield) as colorless oil. The material was carried on without further purification. ¹H NMR (300 MHz, DMSO-*d6*): δ 0.78-0.93 (m, 6H), 0.98 (d, 3H), 1.11-1.41 (m, 2H), 1.42 (s, 9H), 1.45-1.64 (m, 1H), 3.30-3.63 (m, 2H), 4.37 (d, 1H), 6.19 (d, 1H).

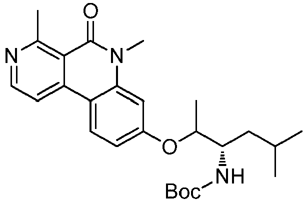

### Part C. tert-butyl ((3S)-2-((4,6-dimethyl-5-oxo-5,6-dihydrobenzo[c][2,7]naphthyridin-8-yl)oxy)-5-methylhexan-3-yl)carbamate

A mixture of 8-chloro-4,6-dimethylbenzo[*c*][2,7]naphthyridin-5(*6H*)-one (0.05 g, 0.193 mol), *tert*-butyl ((3*S*)-2-hydroxy-5-methylhexan-3-yl)carbamate (0.067 g, 0.290 mmol), Cs₂CO₃ (0.315 g, 0.966 mol), 2-di-*t*-butylphosphino-2',4',6'-tri-*i-*propyl-1,1'-biphenyl (8.21 mg, 0.019 mol) and Pd(OAc)₂ (2.60 mg, 0.012 mol) were taken in Toluene (2 mL) and heated overnight at 90 °C. After cooling the reaction mixture was concentrated, then diluted with ethyl acetate and water. Ethyl acetate layer was collected, dried over Na₂SO₄, filtered and concentrated which afforded *tert-*butyl((3*S*)-2-((4,6-dimethyl-5-oxo-5,6-dihydrobenzo[*c*][2,7]naphthyridin-8-yl)oxy)-5-methylhexan-3-yl)carbamate, (70 mg, 0.154 mmol, 80% crude yield) as brown solid. The crude product was used for next step without further purification. LC/MS (ESI) *m*/*e* 454.2 [(M+H)⁺, calcd for C₂₆H₃₆N₃O₄, 454.3]; LC/MS retention time (method D): *t*_{R} = 0.91 min.

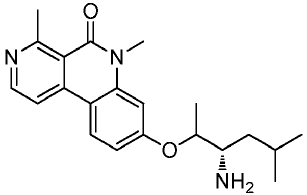

### Part D. 8-(((3S)-3-amino-5-methylhexan-2-yl)oxy)-4,6-dimethylbenzo[c][2,7]naphthyridin-5(6H)-one

*tert*-Butyl ((3*S*)-2-((4,6-dimethyl-5-oxo-5,6-dihydrobenzo[*c*][2,7]naphthyridin-8-yl)oxy)-5-methylhexan-3-yl)carbamate (0.16 g, 0.353 mmol) was subjected to deprotection of the Boc group (procedure described in Example 2, Part E) which afforded the diastereomeric mixture 8-(((3*S*)-3-amino-5-methylhexan-2-yl)oxy)-4,6-dimethylbenzo[*c*][2,7]naphthyridin-5(6*H*)-one (17 mg, 0.017 mmol, 5% yield) as a white solid. LC/MS (ESI) *m*/*e* 354.2 [(M+H)⁺, calcd for C₂₁H₂₈N₃O₂, 354.2]; LC/MS retention time (method C): *t*_{R} = 1.56 min. HPLC retention time (method A): *t*_{R} = 9.40 min; HPLC retention time (method B): *t*_{R} = 9.36 min. ¹H NMR (400MHz, METHANOL-*d*₄) 8.57 (d, *J*=5.5 Hz, 1H), 8.41 (d, *J*=9.0 Hz, 1H), 8.15 (d, *J*=6.0 Hz, 1H), 7.15 - 7.06 (m, 2H), 4.68 (quin, *J*=5.9 Hz, 1H), 3.76 (s, 4H), 3.09 (s, 3H), 1.88 - 1.50 (m, 3H), 1.44 (s, 3H), 1.03 (d, *J*=6.5 Hz, 3H), 0.98 (d, *J*=6.5 Hz, 3H).

### Example 34

### 8-((2-amino-2,4-dimethylpentyl)oxy)-9-methoxy-4,6-dimethylbenzo[c][2,7]naphthyridin-5(6H)-one

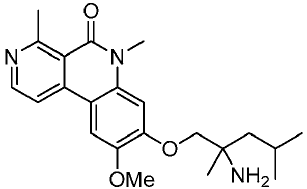

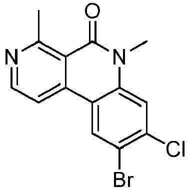

### Part A. 9-bromo-8-chloro-4,6-dimethylbenzo[c][2,7]naphthyridin-5(6H)-one

To a solution of 8-chloro-4,6-dimethylbenzo[*c*][2,7]naphthyridin-5(*6H*)-one (0.2 g, 0.773 mmol) in acetonitrile (4 mL) at -30 °C was added trifluoroacetic acid (0.089 mL, 1.160 mmol) and 1-bromopyrrolidine-2,5-dione (0.151 g, 0.850 mmol). The mixture was heated to 80 °C overnight. The reaction mixture was concentrated, diluted with ethyl acetate and excess aqueous 10% NaHCO₃. The ethyl acetate layer was concentrated. The residue was purified by prep. HPLC to afforded 9-bromo-8-chloro-4,6 dimethylbenzo[*c*][2,7]naphthyridin-5(*6H*)-one (120 mg, 0.351 mmol, 45% yield) as white solid. LC/MS (ESI) *m*/*e* 337.0 [(M+H)⁺, calcd for C₁₄H₁₁BrClN₂O, 337.0]; LC/MS retention time (Method C): *t*_{R} = 2.55 min; ¹H NMR (400 MHz, *METHANOL-d*₄) ppm 3.11 (s, 3H), 3.75 (s, 3H), 7.81 (s, 1H), 8.20-8.26 (m, 1H), 8.68 (d, *J*=5.6 Hz, 1H), 8.79 (s, 1H).

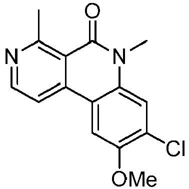

### Part B. 8-chloro-9-methoxy-4,6-dimethylbenzo[c][2,7]naphthyridin-5(6H)-one

A mixture of 9-bromo-8-chloro-4,6-dimethylbenzo[*c*][2,7]naphthyridin-5(*6H*)-one (0.05 g, 0.148 mmol), copper(I)iodide (0.031 g, 0.163 mmol), L-proline (0.020 g, 0.178 mmol), sodium methoxide (0.040 g, 0.741 mmol) and K₂CO₃ (0.041 g,0.296 mmol) were stirred in DMSO (1.5 mL). The mixture was heated to 80 °C for 16h. After cooling, the crude reaction mixture was diluted with methanol, filtered through diatomaceous earth (Celite®) and concentrated under reduced pressure to afford 8-chloro-9-methoxy-4,6-dimethylbenzo[*c*][2,7]naphthyridin-5(*6H*)-one, as brown solid (40 mg). LC/MS (ESI) *m*/*e* 289.0 [(M)⁺, calcd for C₁₅H₁₄ClN₂O₂, 289.1]; LC/MS retention time (method D): *t*_{R} = 0.89 min.

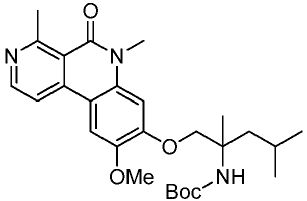

### Part C. tert-butyl(1-((9-methoxy-4,6-dimethyl-5-oxo-5,6-dihydrobenzo[c][2,7]naphthyridin-8-yl)oxy)-2,4-dimethylpentan-2-yl)carbamate

8-Chloro-9-methoxy-4,6-dimethylbenzo[*c*][2,7]naphthyridin-5(*6H*)-one (0.04 g, 0.139 mmol) was subjected to Buchwald coupling (Example 16, Part H) with *tert-*butyl (1-hydroxy-2,4-dimethylpentan-2-yl)carbamate (0.048 g, 0.208 mmol) which afforded *tert*-butyl(1-((9-methoxy-4,6-dimethyl-5-oxo-5,6-dihydrobenzo[*c*][2,7]naphthyridin-8-yl)oxy)-2,4-dimethylpentan-2-yl)carbamate as white solid (15 mg, 0.029 mmol, 21% yield). LC/MS (ESI) *m*/*e* 484.2 [(M+H)⁺, calcd for C₂₇H₃₈N₃O₅, 484.3]; LC/MS retention time (Method C): *t*_{R} = 2.42 min.

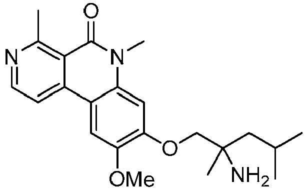

### Part D. 8-((2-amino-2,4-dimethylpentyl)oxy)-9-methoxy-4,6 dimethylbenzo[c][2,7]naphthyridin-5(6H)-one

*tert*-Butyl (1-((9-methoxy-4,6-dimethyl-5-oxo-5,6-dihydrobenzo[*c*][2,7]naphthyridin-8-yl)oxy)-2,4-dimethylpentan-2-yl)carbamate (0.015 g, 0.031 mmol) was subjected to deprotection of the Boc group as per Example 2, Part E to afford 8-((2-amino-2,4-dimethylpentyl)oxy)-9-methoxy-4,6 dimethylbenzo[*c*][2,7]naphthyridin-5(*6H*)-one (8 mg, 0.02 mmol, 63% yield) as a yellow solid. LC/MS (ESI) *m*/*e* 384.2 [(M+H)⁺, calcd for C₂₂H₃₀N₃O₃, 384.5]; LC/MS retention time (Method C), *t*_{R} = 6.57 min. ¹H NMR (400MHz, METHANOL-d₄) 8.63 (d, *J*=6.0 Hz, 1H), 8.51 (br. s., 1H), 8.02 (s, 1H), 7.23 (s, 1H), 4.39 (d, *J*=10.5 Hz, 1H), 4.32 - 4.24 (m, 1H), 4.10 (s, 3H), 3.83 (s, 3H), 3.19 (s, 3H), 1.99 - 1.86 (m, 2H), 1.73 (d, *J*=9.0 Hz, 1H), 1.55 (s, 3H), 1.09 (d, *J*=6.5 Hz, 3H), 1.05 (d, *J*=6.5 Hz, 3H).

### Example 35

### (S)-8-((2-amino-4-methylpentyl)oxy)-9-(2-hydroxypropan-2-yl)-4,6-dimethylbenzo[c][2,7]naphthyridin-5(6H)-one

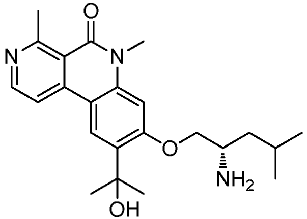

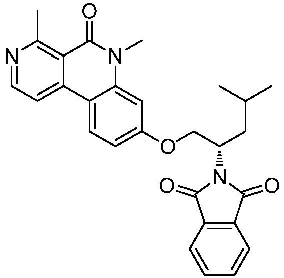

### Part A. (S)-2-(1-((4,6-dimethyl-5-oxo-5,6-dihydrobezo[c][2,7]naphthyridin-8-yl)oxy)-4-methylpentan-2-yl)isoindoline-1,3-dione

8-Chloro-4,6-dimethylbenzo[*c*][2,7]naphthyridin-5(*6H*)-one (1.4 g, 5.41 mmol) and *(S)*-2-(1-hydroxy-4-methylpentan-2-yl)isoindoline-1,3-dione (4.01 g, 16.23 mmol) were subjected to Buchwald coupling as described in Example 16, Part H) to give *(S)*-2-(1-((4,6-dimethyl-5-oxo 5,6-dihydrobenzo[*c*][2,7]naphthyridin-8-yl)oxy)-4-methylpentan-2-yl)isoindoline-1,3-dione as yellow solid (1.4 g, 2.62 mmol, 49% yield). LC/MS (ESI) *m*/*e* 470.2 [(M+H)⁺, calcd for C₂₈H₂₈N₃O₄ 470.2]; LC/MS retention time (method C): *t*_{R} = 2.05 min.

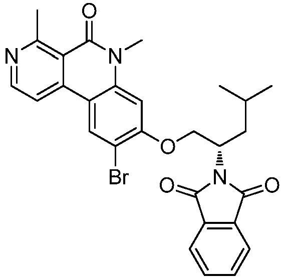

### Part B. (S)-2-(1-((9-bromo-4,6-dimethyl-5-oxo-5,6-dihydrobenzo[c][2,7]naphthyridin-8-yl)oxy)-4-methylpentan-2-yl)isoindoline-1,3-dione

To a solution of *(S)*-2-(1-((4,6-dimethyl-5-oxo-5,6-dihydrobenzo[*c*][2,7]naphthyridin-8-yl)oxy)-4-methylpentan-2-yl)isoindoline-1,3-dione (1g, 1.917 mmol) in acetonitrile (20 mL) was added NBS (0.341 g, 1.917 mmol) and the solution heated to 80 °C overnight to afford *(S)*-2-(1-((9-bromo-4,6-dimethyl-5-oxo-5,6-dihydrobenzo[*c*][2,7]naphthyridin-8-yl)oxy)-4-methylpentan-2-yl)isoindoline-1,3-dione (1.2 g, 1.69 mmol, 88% crude yield) as brown solid. The material was carried forward without further purification. LC/MS (ESI) *m*/*e* 548.0 [(M+H)⁺, calcd for C₂₈H₂₇BrN₃O₄ 548.1; LC/MS retention time (method D): *t*_{R} = 0.76 min.

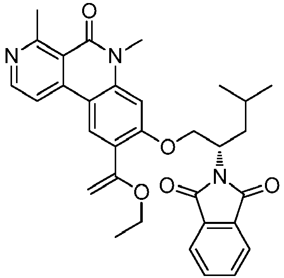

### Part C. (S)-2-(1-((9-(1-ethoxyvinyl)-4,6-dimethyl-5-oxo-5,6-dihydrobenzo[c][2,7]naphthyridin-8-yl)oxy)-4-methylpentan-2-yl)isoindoline-1,3-dione

A solution of *(S)*-2-(1-((9-bromo-4,6-dimethyl-5-oxo-5,6-dihydrobenzo[*c*][2,7]naphthyridin-8-yl)oxy)-4-methylpentan-2-yl)isoindoline-1,3-dione (100 mg, 0.182 mmol), tributyl(1-ethoxyvinyl)stannane (132 mg, 0.365 mmol), DPPF (20.22 mg, 0.036 mmol) and Pd₂(dba)₃ (16.70 mg, 0.018 mmol) in 1,4-dioxane (5 mL) was purged with nitrogen for 5 min then heated at 100 °C overnight. After cooling to ambient temperature, the volatiles were concentrated under reduced pressure. The residue was thaken up in ethyl acetate and filtered through diatomaceous earth (Celite®). The organic filtrate was washed with H₂O, then brine, dried over Na₂SO₄ and concentrated under reduced pressure to afford *(S)-*2-(1-((9-(1-ethoxyvinyl)-4,6-dimethyl-5-oxo-5,6-dihydrobenzo[*c*][2,7]naphthyridin-8-yl)oxy)-4-methylpentan-2-yl)isoindoline-1,3-dione (0.15 g, 0.131 mmol, 72% crude yield) as a black gummy solid. The material was carried forward without further purification. LC/MS (ESI) *m*/*e* 540.3 [(M+H)⁺, calcd for C₃₂H₃₄N₃O₅ 540.2; LC/MS retention time (method G): *t*_{R} = 1.21 min.

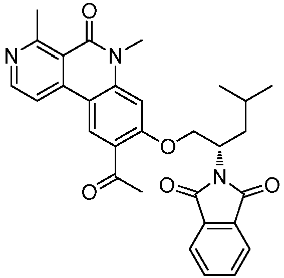

### Part D. (S)-2-(1-((9-acetyl-4,6-dimethyl-5-oxo-5,6-dihydrobenzo[c][2,7]naphthyridin-8-yl)oxy)-4-methylpentan-2-yl)isoindoline-1,3-dione

*(S)*-2-(1-((9-(1-ethoxyvinyl)-4,6-dimethyl-5-oxo-5,6-dihydrobenzo[*c*][2,7]naphthyridin-8-yl)oxy)-4-methylpentan-2-yl)isoindoline-1,3-dione (150 mg, 0.278 mmol) was subjected to acid hydrolysis to afford *(S)*-2-(1-((9-acetyl-4,6-dimethyl-5-oxo-5,6-dihydrobenzo[*c*][2,7]naphthyridin-8-yl)oxy)-4-methylpentan-2-yl)isoindoline-1,3-dione (0.1 g, 0.14 mmol, 51% yield) as brown solid. The crude material was carried forward without further purification. LC/MS (ESI) *m*/*e* 512.1 [(M+H)⁺, calcd for C₃₀H₃₀N₃O₅ 512.2; LC/MS retention time (method D): *t*_{R} = 0.89 min.

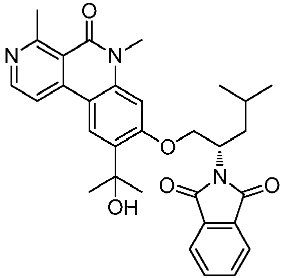

### Part E. (S)-2-(1-((9-(2-hydroxypropan-2-yl)-4,6-dimethyl-5-oxo-5,6-dihydrobenzo[c][2,7]naphthyridin-8-yl)oxy)-4-methylpentan-2-yl)isoindoline-1,3-dione

To a solution of *(S)*-2-(1-((9-acetyl-4,6-dimethyl-5-oxo-5,6-dihydrobenzo[*c*][2,7]naphthyridin-8-yl)oxy)-4-methylpentan-2-yl)isoindoline-1,3-dione (220 mg, 0.430 mmol) in THF (6 mL) was added methyl magnesium bromide (1.4 M in diethyl ether) (0.922 mL, 1.290 mmol) at -10°C and stirred for overnight at RT. The reaction mixture was carefully quenched with water and diluted with EtOAc. The organic layer was separated, dried over sodium sulphate and concentrated under reduced pressure. The residue was purified via silica gel chromatography to afford *(S)*-2-(1-((9-(2-hydroxypropan-2-yl)-4,6-dimethyl-5-oxo-5,6-dihydrobenzo[*c*][2,7]naphthyridin-8-yl)oxy)-4-methylpentan-2-yl)isoindoline-1,3-dione (0.2 g, 0.152 mmol, 35% yield) as a brown gummy solid. LC/MS (ESI) *m*/*e* 528.2 [(M+H)⁺, calcd for C₃₁H₃₄N₃O₅ 528.2; LC/MS retention time (method D): *t*_{R} = 0.89 min.

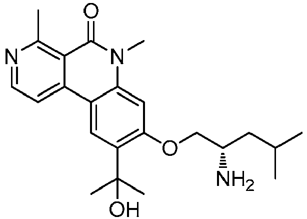

### Part F. (S)-8-((2-amino-4-methylpentyl)oxy)-9-(2-hydroxypropan-2-yl)-4,6-dimethylbenzo[c][2,7]naphthyridin-5(6H)-one

To a solution of (*S*)-2-(1-((9-(2-hydroxypropan-2-yl)-4,6-dimethyl-5-oxo-5,6-dihydrobenzo[*c*][2,7]naphthyridin-8-yl)oxy)-4-methylpentan-2-yl)isoindoline-1,3-dione (200 mg, 0.152 mmol) in ethanol (5 mL), was added hydrazine (4.76 µl, 0.152 mmol) at RT then the mixture was heated to 60 °C for 4h. The reaction mixture was filtered, concentrated and purified by prep. HPLC to afford *(S)*-8-((2-amino-4-methylpentyl)oxy)-9-(2-hydroxypropan-2-yl)-4,6-dimethylbenzo[*c*][2,7]naphthyridin-5(*6H*)-one as white solid (9 mg, 0.021 mmol, 14% yield). LC/MS (ESI) *m*/*e* 398.0 [(M+H)⁺, calcd for C₂₃H₃₂N₃O₃ 398.2]; LC/MS retention time (method G): *t*_{R} = 1.99 min. HPLC retention time (method A): *t*_{R} = 8.63 min; HPLC retention time (method B): *t*_{R} = 9.79 min. ¹H NMR (400 MHz, METHANOL-*d*₄) ppm 0.94 - 1.10 (m, 6H), 1.25 - 1.37 (m, 1H), 1.49 - 1.78(m, 7H), 1.82 - 1.93 (m, 1H), 2.96 - 3.19 (m, 2H), 3.56 - 3.65 (m, 1H), 3.80 (s, 3H), 4.16 - 4.29 (m, 1H), 4.34 - 4.49 (m, 1H), 7.01 - 7.15 (m, 1H), 8.10 - 8.22 (m, 1H), 8.51 - 8.64 (m, 2H).

### Example 36

### (S)-8-((2-amino-4-methylpentyl)oxy)-9-ethyl-4,6-dimethylbenzo[c][2,7]naphthyridin-5(6H)-one

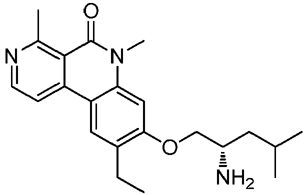

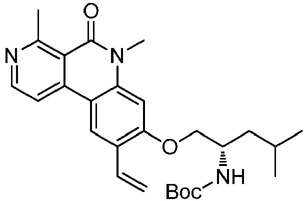

### Part A: (S)-tert-butyl (1-((4,6-dimethyl-5-oxo-9-vinyl-5,6-dihydrobenzo[c][2,7]naphthyridin-8-yl)oxy)-4-methylpentan-2-yl)carbamate

To the solution of *(S)-tert*-butyl (1-((9-bromo-4,6-dimethyl-5-oxo-5,6-dihydrobenzo[*c*][2,7]naphthyridin-8-yl)oxy)-4-methylpentan-2-yl)carbamate (300 mg, 0.579 mmol), prepared as described in Example 3, Part A, in the solvent mixture toluene (10 mL), water (0.5 mL) and ethanol (2 mL) was added sodium carbonate (184 mg, 1.736 mmol), tetrakis(triphenylphosphine)palladium (33.4 mg, 0.029 mmol) and 2,4,6-trivinyl-1,3,5,2,4,6-trioxatriborinane with pyridine (1:1) (167 mg, 0.694 mmol). The reaction mixture was degassed for 30 min. Then the reaction mixture was allowed to stir at 90 °C for 16 h. After cooling to room temperature the mixture was filtered through diatomaceous earth (Celite®) eluting with EtOAc. The filtrate was concentrated under reduced pressure. The residue was taken up in EtOAc and water. The solution was extracted with ethyl acetate (2 x 15 mL). The combined organic layers were washed with water (10 mL), brine (10 mL), dried over sodium sulfate, filtered and concentrated under reduced pressure. The residue was purified by silica gel column chromatography (60% Ethyl acetate in hexane) to afford *(S)-tert-*butyl (1-((4,6-dimethyl-5-oxo-9-vinyl-5,6-dihydrobenzo[c][2,7]naphthyridin-8-yl)oxy)-4-methylpentan-2-yl)carbamate (300 mg, 0.110 mmol, 19% yield) as a pale yellow semi solid. LC/MS, (ESI) *m*/*z* 466.5 [(M+H)⁺, calcd for C₂₇H₃₆N₃O₄ 466.3]; LC/MS retention time (method E): *t*_{R} = 1.18 min.

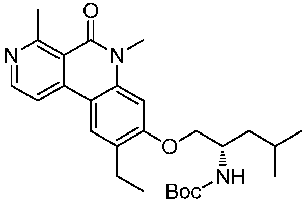

### Part B: (S)-tert-butyl (1-((9-ethyl-4,6-dimethyl-5-oxo-5,6-dihydrobenzo[c][2,7]naphthyridin-8-yl)oxy)-4-methylpentan-2-yl)carbamate

A solution of *(S)-tert*-butyl (1-((4,6-dimethyl-5-oxo-9-vinyl-5,6-dihydrobenzo[*c*][2,7]naphthyridin-8-yl)oxy)-4-methylpentan-2-yl)carbamate (50 mg, 0.107 mmol) in MeOH (3 mL) was degassed with nitrogen for 5 min, then palladium on carbon (1.143 mg, 10.74 µmol) was added. The reaction mixture was allowed to stir under a balloon of hydrogen for 16 h. The reaction mixture was then filtered through diatomaceous earth (Celite®) and the filtrate was concentrated under reduced pressure. The residue was purified by preparative TLC (2% MeOH in MDC) to afford *(S)-tert*-butyl (1-((9-ethyl-4,6-dimethyl-5-oxo-5,6-dihydrobenzo[*c*][2,7]naphthyridin-8-yl)oxy)-4-methylpentan-2-yl)carbamate (31 mg, 0.058 mmol, 54% yield) as pale yellow solid. LC/MS, (ESI) *m*/*z* 468.5 [(M+H)⁺, calcd for C₂₇H₃₈N₃O₄ 468.3]; LC/MS retention time (method D): *t*_{R} = 1.31 min.

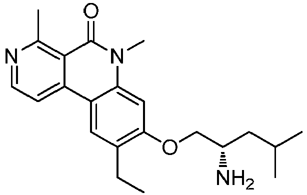

### Part C: (S)-8-((2-amino-4-methylpentyl)oxy)-9-ethyl-4,6-dimethylbenzo[c][2,7]naphthyridin-5(6H)-one

Prepared as described in Example 2, Part E to afford the title compound (4 mg, 10.62 µmoles, 33% yield) as a pale yellow gum. LC/MS, (ESI) *m*/*z* 368.4 [(M+H)⁺, calcd for C₂₂H₃₀N₃O₂ 368.2]; LC/MS retention time (method B): *t*_{R} = 2.18 min. HPLC retention time (method A): *t*_{R} = 8.87 min; HPLC retention time (method B): *t*_{R} = 9.53 min. ¹H NMR (400 MHz, MeOH-*d*₄) δ ppm 8.51 (d, *J*=5.60 Hz, 1H) 8.17 (s, 1H) 8.13 (d, *J*=5.60 Hz,1H) 6.97 (s, 1H) 4.17-4.20(m, 1H) 4.04 - 4.04 (m, 1H) 3.76 (s, 3H) 3.31 - 3.38 (m, 1H) 3.06(s, 3H) 2.80 - 2.82 (m, 2H) 1.81- 1.89 (m, 1H) 1.53-1.58 (m, 1H) 1.45- 1.50 (m, 1H) 1.27 -1.31 (m, 3H), 0.98-1.02 (m, 6H).

### Example 37 (Enantiomer 1) and Example 38 (Enantiomer 2)

### (R)-8-((2-amino-2,4-dimethylpentyl)oxy)-4,6-dimethylbenzo[c][2,7]naphthyri din-5(6H)-one and (S)-8-((2-amino-2,4-dimethylpentyl)oxy)-4,6-dimethylbenzo[c][2,7]naphthyri din-5(6H)-one

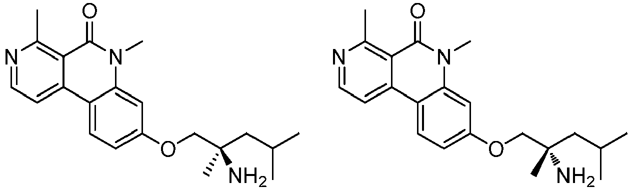

The resolution of 8-((2-amino-2,4-dimethylpentyl)oxy)-4,6 dimethylbenzo[*c*][2,7]naphthyridin-5(*6H*)-one, prepared in Exampl2 32, was carried out by chiral HPLC (Method: Co-solvent: 0.3% DEA in methanol, Column: Chiralpak AD H (250 x 21) mm 5u) resulting into two enantiomers. The absoulte stereochemistry of each enantiomer was not determined.
Enantiomer-1: LC/MS (ESI) *m*/*e* 354.2 [(M+H)⁺, calcd for C₂₁H₂₈N₃O₂, 354.2]; LC/MS retention time (method C): *t*_{R} = 1.57 min. HPLC retention time (method A): *t*_{R} = 8.67 min; HPLC retention time (method B): *t*_{R} = 9.46 min. ¹H NMR (400 MHz, METHANOL-*d*4) ppm δ 8.54 (s, 1H), 8.34-8.36 (bs, 1H), 8.10 (bs, 1H), 7.06 (s, 1H), 4.11-4.17 (m, 2H), 3.73 (s, 3H), 3.06 (s, 3H), 1.70 - 1.98 (m, 2H), 1.68 (bs, 1H), 1.45 (s, 3H),1.02 - 1.08 (m, 6H); Chiral HPLC retention time, *t*_{R} = 9.36 min.
Enantiomer-2: LC/MS (ESI) *m*/*e* 354.2 [(M+H)⁺, calcd for C₂₁H₂₈N₃O₂, 354.2]; LC/MS retention time (method C): *t*_{R} = 1.57 min. HPLC (Method B) retention time (method A): *t*_{R} = 8.67 min; HPLC retention time (method B): *t*_{R} = 9.46 min. ¹H NMR (400 MHz, METHANOL-*d*4) ppm δ 8.54 (s, 1H), 8.34-8.36 (bs, 1H), 8.10 (bs, 1H), 7.06 (s, 1H), 4.11-4.17 (m, 2H), 3.73 (s, 3H), 3.06 (s, 3H), 1.70 - 1.98 (m, 2H), 1.68 (bs, 1H), 1.45 (s, 3H),1.02 - 1.08 (m, 6H); Chiral HPLC (Method B) retention time: *t*_{R} = 10.68 min.

### Example 39

### (S)-8-((2-(dimethylamino)-4-methylpentyl)oxy)-6-methylbenzo[c][2,7]naphthyridin-5(6H)-one

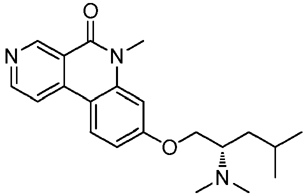

To a stirred solution of (*S*)-8-((2-amino-4-methylpentyl)oxy)-6-methylbenzo[*c*][2,7]naphthyridin-5(6*H*)-one (0.06 g, 0.184 mmol) (synthesis described in Example 2, Part E) in formic acid (0.035 mL, 0.922 mmol) was added formaldehyde (0.025 mL, 0.922 mmol) at ambient temperature. Then reaction mixture was then heated to reflux for 16 h. The reaction mixture was concentrated under reduced pressure. The residue was diluted with 50 mL of water and extracted with 80 mL of dichloromethane. The organic layer wasseparated, dried over sodium sulphate, and concentrated under reduced pressure. The residue was purified by reverse phase HPLC (acetoniltrile/water/10 mM NH₄OAc) to afford *(S)*-8-((2-(dimethylamino)-4-methylpentyl)oxy)-6 methylbenzo[*c*][2,7]naphthyridin-5(*6H*)-one (8 mg, 0.021 mmol, 11% yield) as a white solid. LC/MS (ESI) *m*/*e* 354.0 [(M+H)⁺, calcd for C₂₁H₂₈N₃O₂, 354.5]; LC/MS retention time (method C): *t*_{R} = 1.85 min. HPLC retention time (method A): *t*_{R} = 8.02 min; HPLC retention time (method B): *t*_{R} = 9.19 min¹H NMR (400 MHz, METHANOL-*d*₄) δ ppm 1.01 (d, J = 6.80 Hz, 6H), 1.50-1.61 (m, 2H), 1.73-1.80 (m, 1H), 2.52 (s, 1H), 3.15-3.16 (m, 1H), 3.73 (s, 3H), 4.23-4.31 (m, 2H), 7.04 (d, J = 8.00 Hz, 2H), 8.15 (s, 1H), 8.30 (d, J = 8.00 Hz, 1H), 8.71 (s, 1H), 8.39 (s, 1H).

### Example 40

### (S)-8-((2-(dimethylamino)-4-methylpentyl)oxy)-4,6-dimethylbenzo[c][2,7]naphthyridin-5(6H)-one

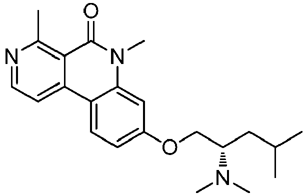

To a slurry of *(S)*-8-((2-amino-4-methylpentyl)oxy)-4,6 dimethylbenzo[*c*][2,7]naphthyridin-5(*6H*)-one (0.1 g, 0.295 mmol) (synthesis described in Example 16, part I) in formic acid (0.011 mL, 0.295 mmol) was added formaldehyde (8.12 µL, 0.295 mmol) at RT. The reaction mixture was heated to 100 °C for 16 h in a sealed tube. After cooling to room temperature, the mixture concentrated under reduced pressure. The residue was then partitioned between water (50 mL) and dichloromethane (80 mL). The organic layer was separated, dried over sodium sulfate and concentrated under reduced pressure. The resiude was purified by reverse phase HPLC (10mM ammonium acetate/AcCN) to afford *(S)*-8-((2-(dimethylamino)-4-methylpentyl)oxy)-4,6-dimethylbenzo[*c*][2,7]naphthyridin-5(*6H*)-one (30 mg, 0.078 mmol, 26% yield) as a white solid. LC/MS (ESI) *m*/*e* 368.0 [(M+H)⁺, calcd for C₂₂H₃₀N₃O₂, 368.2]; LC/MS retention time (Method C): *t*_{R} = 1.86 min. HPLC retention time (method A): *t*_{R} = 7.98 min; HPLC retention time (method B): *t*_{R} = 9.44 min.¹H NMR (400 MHz, METHANOL-*d*₄) ppm δ 1.01 (d, *J* = 6.80 Hz, 6H), 1.61-1.95 (m, 3H), 2.66-2.69 (m, 6H), 3.06 (s, 3H), 3.37-3.43 (m, 1H), 3.74 (s, 3H), 4.37 (d, *J* = 4.80 Hz, 2H), 7.04-7.08 (m, 2H), 8.10 (d, *J* = 5.60 Hz, 1H), 8.36 (d, *J* = 8.80 Hz, 1H), 8.53 (d, *J* = 5.60 Hz, 1H).

### Example 41

### 8-((2-amino-2,4-dimethylpentyl)oxy)-9-chloro-4,6-dimethylbenzo[c][2,7]naphthyridin-5(6H)-one

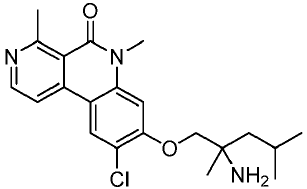

To the stirred solution of 8-((2-amino-2,4-dimethylpentyl)oxy)-4,6 dimethylbenzo[*c*][2,7]naphthyridin-5(*6H*)-one (Prepared in Example 32, Part B) (0.08 g, 0.226 mmol) in acetonitrile (5 mL) was added NCS (0.030 g, 0.226 mmol)) at RT and the reaction mixture was stirred for 2h. The mixture was then evaporated to dryness. The residue was purified by reverse phase HPLC (0.1%TFA in water/AcCN) to afford 8-((2-amino-2,4-dimethylpentyl)oxy)-9-chloro-4,6-dimethylbenzo[*c*][2,7]naphthyridin-5(*6H*)-one (12 mg, 0.030 mmol, 13% yield). LC/MS (ESI) *m*/*e* 388.0 [(M+H)⁺, calcd for C₂₁H₂₇ClN₃O₂, 388.2] as an off-white solid. LC/MS retention time (method C): *t*_{R} = 2.13 min. HPLC retention time (method A): *t*_{R} = 8.97 min; HPLC retention time (method B): *t*_{R} = 10.16 min. ¹H NMR (400MHz, METHANOL-*d₄*) δ ppm 0.99-1.02 (m, 6H), 1.30 (s, 3H), 1.54-1.66 (m, 2H), 1.84-1.90 (m, 1H), 3.05 (s, 1H), 3.73 (s, 1H), 4.04 (q, *J* = 23.20 Hz, 2H), 7.03 (s, 1H), 8.06 (d, *J* = 6.00 Hz, 1H), 8.39 (s, 1H), 8.54 (d, *J* = 6.00 Hz, 1H).

### Example 42

### (S)-8-(2-amino-4-methylpentyloxy)-4-methoxy-6-methylbenzo[c][2,7]naphthyridin-5(6H)-one

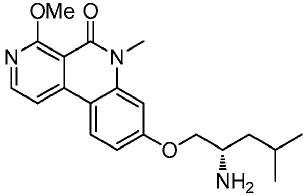

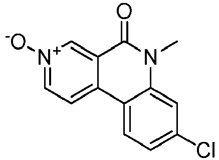

### Part A. 8-chloro-6-methyl-5-oxo-5,6-dihydrobenzo[c][2,7]naphthyridine 3-oxide

To the stirred solution of 8-chloro-6-methylbenzo[*c*][2,7]naphthyridin-5(*6H*)-one (1.0 g, 4.09 mmol), prepared as described in Example 2, Part C, in DCM (20 mL) cooled to 0°C was added *m*-CPBA (1.763 g, 10.22 mmol). The reaction mixture was then warmed to ambient temperature and stirred for 4 h. Reaction mixture was then diluted with dichloromethane (100 mL) and washed with saturated sodium bicarbonate solution (200 mL), brine solution (50 mL). The orgaincs were then dried over sodium sulphate, filtered and concentrate to afford 8-chloro-6-methyl-5-oxo-5,6-dihydrobenzo[*c*][2,7]naphthyridine 3-oxide (1.0 g, 3.84 mmol, 94 % crude yield) as a yellow solid. The material was carried on without further purification. LC/MS (ESI) *m*/*e* 261.0, [(M+H)⁺, calcd for C₁₃H₁₀ClN₂O₂, 261.0]; LC/MS retention time (method C): *t*_{R} = 1.65 min.

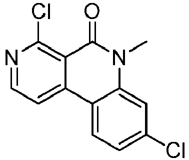

### Part B. 4,8-dichloro-6-methylbenzo[c][2,7]naphthyridin-5(6H)-one

A stirred solution of 8-chloro-6-methyl-5-oxo-5,6-dihydrobenzo[*c*][2,7]naphthyridine 3-oxide (0.5 g, 1.918 mmol) in POCl₃ (3.58 mL, 38.4 mmol) was heated to reflux for 6 h. The reaction mixture was concentrated under reduced pressure. The residue was then partitioned between dichloromethane (100 mL) and saturated sodium bicarbonate solution (200 mL). The organic layer was separated and washed again with brine solution (50 mL), dried over sodium sulphate, and concentrated to afford 4,8-dichloro-6 methylbenzo[*c*][2,7]naphthyridin-5(*6H*)-one (0.3 g, 1.075 mmol, 56% crude yield). The material was carried on without further purification. LC/MS (ESI) *m*/*e* 280.8, [(M+2H)⁺, calcd for C₁₃H₉Cl₂N₂O, 280.8]; LC/MS retention time (method C): *t*_{R} = 2.06 min.

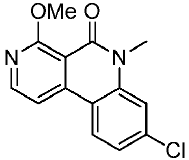

### Part C. 8-chloro-4-methoxy-6-methylbenzo[c][2,7]naphthyridin-5(6H)-one

To a solution of 4,8-dichloro-6-methylbenzo[*c*][2,7]naphthyridin-5(*6H*)-one (1.0 g, 3.58 mmol) in MeOH (1 mL) was added sodium methoxide (0.806 mL, 3.58 mmol). The resultant mixture was heated in a microwave at 80°C for 25 min. The reaction mixture was then diluted with water (20 mL) and extracted with ethyl acetate (30 mL). The organic layer was separated, dried over sodium sulphate and evaporated to dryness to afford 8-chloro-4-methoxy-6-methylbenzo[*c*][2,7]naphthyridin-5(*6H*)-one (0.4 g, 1.391 mmol, 39% crude yield) as a brown solid. The material was carried on without further purification. LC/MS (ESI) *m*/*e* 274.8 [(M+H)⁺, calcd for C₁₄H₁₂ClN₂O₂, 275.0]; LC/MS retention time (method C): *t*_{R} = 2.06 min.

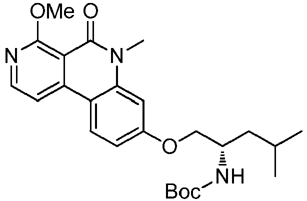

### Part D. (S)-tert-butyl (1-((4-methoxy-6-methyl-5-oxo-5,6-dihydrobenzo[c][2,7]naphthyridin-8-yl)oxy)-4-methylpentan-2-yl)carbamate

Prepared as described in Example 16, Part H to afford the title product (0.1 g, 0.135 mmol, 62% yield) as a semi solid. LC/MS (ESI) m/e 456.0, [(M+H)⁺, calcd for C₂₅H₃₄N₃O₅, 456.2]; LC/MS retention time (method C): t_{R} = 2.26 min.

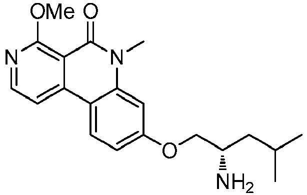

### Part E. (S)-8-((2-amino-4-methylpentyl)oxy)-4-methoxy-6 methylbenzo[c][2,7]naphthyridin-5(6H)-one

Prepared as described in Example 2, Part E to afford the title product (10 mg, 0.027 mmol, 20% yield) as a gum. LC/MS (ESI) *m*/*e* 356.0, [(M+H)⁺, calcd for C₂₀H₂₆N₃O₃, 356.4]; LC/MS retention time (method C): *t*_{R} = 1.83 min. HPLC retention time (method A): *t*_{R} = 8.48 min; HPLC retention time (method B): *t*_{R} = 7.61 min. ¹H NMR (400 MHz, METHANOL-*d₄*) δ ppm 8.24 (d, *J*=6.0 Hz, 1H), 8.13 (d, *J*=9.0 Hz, 1H), 7.62 (d, *J*=6.0 Hz, 1H), 6.99 - 6.91 (m, 1H), 6.90 (d, *J*=2.5 Hz, 1H), 4.11 (dd, *J*=9.5, 4.0 Hz, 1H), 4.06 (s, 3H), 3.92 (dd, *J*=9.3, 7.3 Hz, 1H), 3.65 (s, 3H), 1.93 - 1.81 (m, 1H), 1.45 (td, *J*=7.7, 6.3 Hz, 2H), 1.03 (d, *J*=6.5 Hz, 3H), 1.01 (d, *J*=6.5 Hz, 3H).

### Example 43

### (S)-8-((2-amino-4-methylpentyl)oxy)-9-chloro-6-methylbenzo[c][2,7]naphthyridin-5(6H)-one

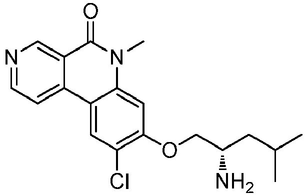

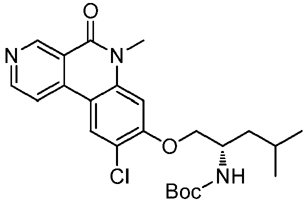

### Part A. (S)-tert-butyl (1-((9-chloro-6-methyl-5-oxo-5,6-dihydrobenzo[c][2,7]naphthyridin-8-yl)oxy)-4-methylpentan-2-yl)carbamate

*(S)-tert*-butyl (4-methyl-1-((6-methyl-5-oxo-5,6-dihydrobenzo[*c*][2,7]naphthyridin-8-yl)oxy)pentan-2-yl)carbamate (0.4 g, 0.714 mmol), prepared as described in Example 19, Part B, was subjected to chlorination using NCS as described in Example 41, to afford *(S)-tert-*butyl (1-((9-chloro-6-methyl-5-oxo-5,6-dihydrobenzo[*c*][2,7]naphthyridin-8-yl)oxy)-4-methylpentan-2-yl)carbamate (0.3 g, 0.437 mmol, 61% yield) as a yellow solid. LC/MS (ESI) *m*/*e* 460.2 [(M+H)⁺, calcd for C₂₄H₃₁ClN₃O₄, 460.2]; LC/MS retention time (Method C) *t*_{R} = 2.17 min.

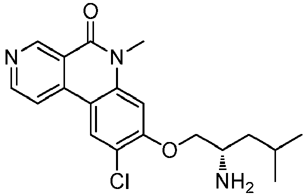

### Part B. (S)-8-((2-amino-4-methylpentyl)oxy)-9-chloro-6 methylbenzo[c][2,7]naphthyridin-5(6H)-one

*(S)-tert*-Butyl (1-((9-chloro-6-methyl-5-oxo-5,6-dihydrobenzo[*c*][2,7]naphthyridin-8-yl)oxy)-4-methylpentan-2-yl)carbamate was subjected to deprotection of Boc, as described in Example 2, Part E, to afford *(S)*-8-((2-amino-4-methylpentyl)oxy)-9-chloro-6 methylbenzo[*c*][2,7]naphthyridin-5(*6H*)-one (0.1 g, 0.27 mmol, 72% yield) as an off-white solid. LC/MS (ESI) *m*/*e* 360.2 [(M+H)⁺, calcd for C₁₉H₂₃ClN₃O₂, 360.1]; HPLC retention time (method A): *t*_{R} = 8.94 min; HPLC retention time (method B): *t*_{R} = 5.4 min. ¹H NMR (400 MHz, METHANOL-*d*4) ppm 9.43 (d, *J*=0.50 Hz, 1H), 8.76 (d, *J*=5.52 Hz, 1H), 8.42 (s, 1H), 8.16 - 8.20 (m, 1H), 7.10 (s, 1H), 4.23 - 4.27 (m, 1H), 4.02 - 4.08 (m, 1H), 3.78 (s, 3H), 3.34 - 3.40 (m, 1H), 1.84 - 1.94 (m, 1H), 1.42 - 1.58 (m, 2H), 1.03 (dd, *J*=8.66, 6.65 Hz, 6H).

### Example 44

### (S)-8-(2-amino-4-methylpentyloxy)-6-(2-hydroxyethyl)benzo[c][2,7]naphthyridin-5(6H)-one

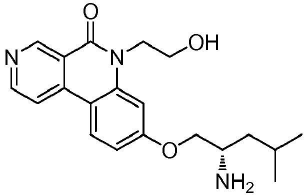

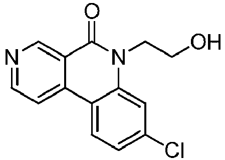

### Part A. 8-chloro-6-(2-hydroxyethyl)benzo[c][2,7]naphthyridin-5(6H)-one

To a stirred solution of 8-chlorobenzo[*c*][2,7]naphthyridin-5(*6H*)-one (80 mg, 0.347 mmol) in anhydrous DMF (5 mL) under a nitrogen atmosphere was added ethyl carbonate (122 mg, 1.387 mmol), K₂CO₃ (96 mg, 0.694 mmol), and 18-crown-6 (18.31 mg, 0.069 mmol). The reaction mixture was heated to 80 °C for 12 h. The reaction mixture was diluted with water (10 mL) nad extracted with ethyl acetate (2x20 mL). The combined ethyl acetate layers were dried over sodium sulphate and concentrated under reduced pressure to afford 8-chloro-6-(2 - hydroxyethyl)benzo[*c*][2,7]naphthyridin-5(*6H*)-one (80 mg, 0.291 mmol, 84 % crude yield) The material was carried on without further purification. LC/MS (ESI) *m*/*e* 275.0 [(M+H)⁺, calcd for C₁₄H₁₂ClN₂O₂, 275.1]; ¹H NMR (400 MHz, *MeOD*) δ 9.55 (s, 1H), 8.87 (d, *J*=5.6 Hz, 1H), 8.50 (d, *J*=8.8 Hz, 1H), 8.34 (d, *J*=5.6 Hz, 1H), 7.88 (d, *J*=1.6 Hz, 1H), 7.44 (dd, *J*=8.4, 2.0 Hz, 1H), 4.57 (t, *J*=6.0 Hz, 2H), 3.96 (t, *J*=6.0 Hz, 2H).

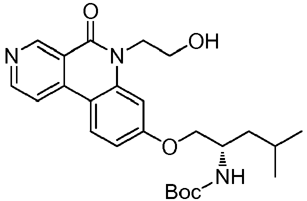

### Part B. (S)-tert-butyl (1-((6-(2-hydroxyethyl)-5-oxo-5,6-dihydrobenzo[c][2,7]naphthyridin-8-yl)oxy)-4-methylpentan-2-yl)carbamate

Prepared as described in Example 16, Part H to afford *(S)-tert*-butyl (1-((6-(2-hydroxyethyl)-5-oxo-5,6-dihydrobenzo[*c*][2,7]naphthyridin-8-yl)oxy)-4-methylpentan-2-yl)carbamate (60 mg, 0.132 mmol, 45% yield) as a white oil, LC/MS (ESI) *m*/*e* 456.2, [(M+H)⁺, calcd for C₂₅H₃₄N₃O₅, 456.2]; LC/MS retention time (method C): *t*_{R} = 1.81 min.

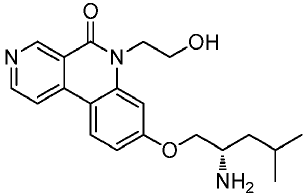

### Part C. (S)-8-((2-amino-4-methylpentyl)oxy)-6-(2-hydroxyethyl)benzo[c][2,7]naphthyridin-5(6H)-one

Prepared as described in Example 2, Part E to afford *(S)*-8-((2-amino-4-methylpentyl)oxy)-6-(2-hydroxyethyl)benzo[*c*][2,7]naphthyridin-5(*6H*)-one (6 mg, 0.015 mmol, 12% yield) as a yellow solid. LC/MS (ESI) *m*/*e* 356.2, [(M+H)⁺, calcd for C20H26N3O3, 356.2]; LC/MS retention time (method C): *t*_{R} = 1.59 min. HPLC retention time (method A): *t*_{R} = 7.66 min; HPLC retention time (method B): *t*_{R} = 7.99 min. ¹H NMR (400 MHz, *MeOD*) δ 9.50 (s, 1H), 8.79 (d, *J*=6.0 Hz, 1H), 8.47 (d, *J*=8.8 Hz, 1H), 8.29 (d, *J*=5.6 Hz, 1H), 7.34 (d, *J*=2.0 Hz, 1H), 7.14 (dd, *J*=8.8, 2.4 Hz, 1H), 4.59 (t, *J*=6.4 Hz, 2H), 4.34 (dd, *J*=10.0, 3.6 Hz, 1H), 4.17 (dd, *J*=10.0, 6.4 Hz, 1H), 3.98 (t, *J*=6.0 Hz, 2H), 3.63-3.53 (m, 1H), 1.92-1.80 (m, 1H), 1.66 - 1.59 (m, 2H), 1.06 - 1.03 (m, 6H).

### Example 45

### (S)-8-(2-amino-4-methylpentyloxy)-6-(2,2,2-trifluoroethyl)benzo[c][2,7]naphthyridin-5(6H)-one

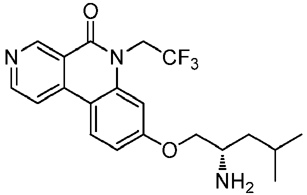

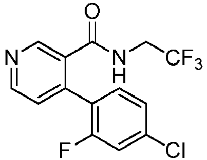

### Part A. 4-(4-chloro-2-fluorophenyl)-N-(2,2,2-trifluoroethyl)nicotinamide

To stirred solution of 4-(4-chloro-2-fluorophenyl)nicotinic acid (300 mg, 1.192 mmol), prepared as described in Example 2, Part A, in anhydrous DMF (2 mL) under nitrogen was added HOBT (365 mg, 2.384 mmol), DIEA (0.927 mL, 4.77 mmol) and EDC (343 mg, 1.788 mmol). The reaction mixture was cooled to 0 °C and treated with 2,2,2-trifluoroethanamine (236 mg, 2.384 mmol) drop wise and warmed to RT and allowed to stir for 12 h. The reaction mixture was diluted with water (20 mL) and extracted with ethyl acetate (2 x 50 mL). The combined organic layers were dried over sodium sulphate and concentrated under reduced pressure to afford 4-(4-chloro-2-fluorophenyl)-*N*-(2,2,2-trifluoroethyl)nicotinamide (200 mg, 0.601 mmol, 50% crude yield) as a yellow solid. LC/MS (ESI) *m*/*e* 333.2, [(M+H)⁺, calcd for C₁₄H₁₀ClF₄N₂O, 333.0]; LC/MS retention time (method A): *t*_{R} = 1.66 min.

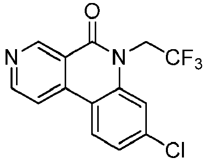

### Part B. 8-chloro-6-(2,2,2-trifluoroethyl)benzo[c][2,7]naphthyridin-5(6H)-one

Prepared as described in Example 2, Part C to afford 8-chloro-6-(2,2,2-trifluoroethyl)benzo[*c*][2,7]naphthyridin-5(*6H*)-one (150 mg, 0.480 mmol, 80 % yield) LC/MS (ESI) *m*/*e* 313.1, [(M+H)⁺, calcd for C₁₄H₉ClF₃N₂O, 313.0]; LC/MS retention time (method D): *t*_{R} = 0.95 min.

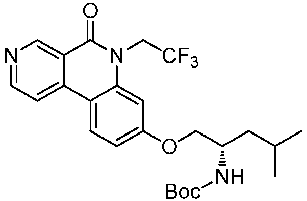

### Part C. (S)-tert-butyl (4-methyl-1-((5-oxo-6-(2,2,2-trifluoroethyl)-5,6-dihydrobenzo[c][2,7]naphthyridin-8-yl)oxy)pentan-2-yl)carbamate

Prepared as described in Example 16, Part H to afford *(S)*-*tert*-butyl (4-methyl-1-((5-oxo-6-(2,2,2-trifluoroethyl)-5,6-dihydrobenzo[*c*][2,7]naphthyridin-8-yl)oxy)pentan-2-yl)carbamate (100 mg, 0.203 mmol, 42% yield) as a brown solid. LC/MS (ESI) *m*/*e* 494.5, [(M+H)⁺, calcd for C₂₅H₃₁F₃N₃O₄ 494.2]; LC/MS retention time (method C): *t*_{R} = 2.17 min.

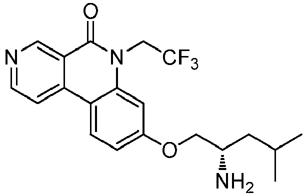

### Part D. (S)-8-((2-amino-4-methylpentyl)oxy)-6-(2,2,2-trifluoroethyl)benzo[c][2,7]naphthyridin-5(6H)-one

Prepared as described in Example 2, Part E to afford *(S)*-8-((2-amino-4-methylpentyl)oxy)-6-(2,2,2-trifluoroethyl)benzo[*c*][2,7]naphthyridin-5(*6H*)-one (25 mg, 0.061 mmol, 30% yield), white solid. LC/MS (ESI) *m*/*e* 394.2, [(M+H)⁺, calcd for C₂₀H₂₃F₃N₃O₂, 394.2]; LC/MS retention time (method C): *t*_{R} = 1.78 min. HPLC retention time (method A): *t*_{R} = 5.26 min; HPLC retention time (method B): *t*_{R} = 5.60 min. ¹H NMR (400 MHz, *MeOD*) δ 9.49 (s, 1H), 8.81 (d, *J*=5.6 Hz, 1H), 8.46 (d, *J*=8.8 Hz, 1H), 8.28 (d, *J*=5.6 Hz, 1H), 7.23 (br s, 1H), 7.15 (dd, *J*=8.8, 2.0 Hz, 1H), 5.32 (dd, *J*=8.8, 6.4 Hz, 2H), 4.21 (dd, *J*=9.2, 3.6 Hz, 1H), 4.03 (dd, *J*=9.2, 7.2 Hz, 1H), 3.40-3.33 (m, 1H), 1.92-1.80 (quin, *J*=6.8, 1H), 1.52 - 1.46 (m, 2H), 1.02 (d, *J*=6.4 Hz, 3H), 1.00 (d, *J*=6.4 Hz, 3H).

### Example 46

### (S)-8-(2-amino-4-methylpentyloxy)-9-ethyl-6-methylbenzo[c][2,7]naphthyridin-5(6H)-one

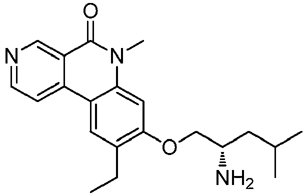

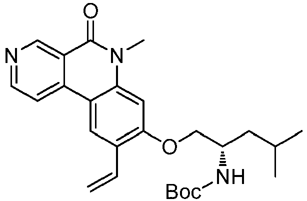

### Part A. (S)-tertbutyl (4-methyl-1-((6-methyl-5-oxo-9-vinyl-5,6 dihydrobenzo[c][2,7]naphthyridin-8-yl)oxy)pentan-2-yl)carbamate

A mixture of *(S)-tert*-butyl (1-((9-bromo-6-methyl-5-oxo-5,6-dihydrobenzo[*c*][2,7]naphthyridin-8-yl)oxy)-4-methylpentan-2-yl)carbamate (0.250 g, 0.496 mmol) (prepared as described in Example 3, Part B) 2,4,6-trivinylcyclotriboroxane pyridine complex (0.239 g, 0.991 mmol), Na₂CO₃ (0.158 g, 1.487 mmol) and Pd(PPh₃)₄ (0.029 g, 0.025 mmol) in toluene (1 mL) water (0.1 mL), and ethanol (0.3 mL) was purged with nitrogen gas and heated at 90 °C for 16 h. After cooling, the reaction mixture was transferred to separatory funnel containing water (10 mL) and extracted with ethyl acetate (3 x 10 mL). The combined organic layers were dried over sodium sulphate and concentrated under reduced pressure. The residue was purified by column chromatography on silica gel (60% ethyl acetate: petroleum ether) to afford a *(S)*-*tert*-butyl(4-methyl-1-((6-methyl-5-oxo-9-vinyl-5,6-dihydrobenzo[*c*][2,7]naphthyridin-8-yl)oxy)pentan-2-yl)carbamate (0.150 g, 0.166 mmol, 34% yield) as a yellow solid. LC/MS (ESI) *m*/*e* 452.4, [(M+H)⁺, calcd for C₂₆H₃₄N₃O₄, 452.2]; LC/MS retention time (method D): *t*_{R} = 0.95 min.

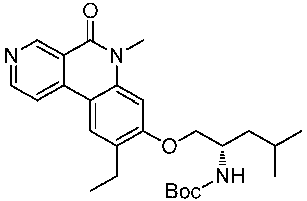

### Part B. (S)-tert-butyl (1-((9-ethyl-6-methyl-5-oxo-5,6-dihydrobenzo[c][2,7]naphthyridin-8-yl)oxy)-4-methylpentan 2-yl)carbamate

A mixture of (*S*)-*tert*-butyl (4-methyl-1-((6-methyl-5-oxo-9-vinyl-5,6-dihydrobenzo[c][2,7]naphthyridin-8-yl)oxy)pentan-2-yl)carbamate (0.15 g, 0.332 mmol) and palladium on carbon (0.075 g, 0.070 mmol) in MeOH (3 mL) and ethyl acetate (3 mL) was stirred at RT under a balloon of hydrogen gas for 24 h. The reaction mixture was filtered through diatomaceous earth (Celite®) and the filtrate was concentrated under reduced pressure. The residue was purified by prep TLC (60 % ethyl acetate and petroleum ether) to afford *(S)-tert-*butyl (1-((9-ethyl-6-methyl-5-oxo-5,6-dihydrobenzo[*c*][2,7]naphthyridin-8-yl)oxy)-4-methylpentan 2-yl)carbamate (0.11 g, 0.133 mmol, 40 % yield) as a yellow semi solid. LC/MS (ESI) *m*/*e* 454.4, [(M+H)⁺, calcd for C₂₆H₃₆N₃O₄, 454.3]; LC/MS retention time (method D): *t*_{R} = 0.96 min.

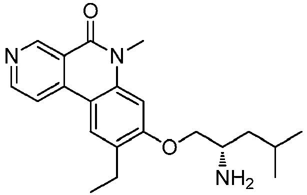

### Part C. (S)-8-((2-amino-4-methylpentyl)oxy)-9-ethyl-6-methylbenzo[c][2,7]naphthyridin-5(6H)-one

Prepared as described in Example 2, Part E to afford the title compound (55 mg, 0.155 mmol, 64% yield) as a yellow solid. LC/MS (ESI) *m*/*e* 354.2, [(M+H)⁺, calcd for C₂₁H₂₈N₃O₂, 354.2]; LC/MS retention time (method C): *t*_{R} = 1.94 min. HPLC retention time (method A): *t*_{R} = 8.76 min; HPLC retention time (method B): *t*_{R} = 5.19 min. ¹H NMR (400 MHz, *MeOD*) δ 9.52 (s, 1H), 8.81 (d, *J*=6.0 Hz, 1H), 8.51 (d, *J*=8.8 Hz, 1H), 8.33 (s, 1H), 7.11 (s, 1H), 4.52 (m, 1H), 4.36 (m, 1H), 3.86 (s, 3H), 3.76-3.84 (m, 1H), 2.86-2.93 (m, 2H), 1.81-1.90 (m, 2H), 1.63 - 1.81 (m, 1H), 1.35 (m, 3H), 1.05 (m, 6H).

### Example 47

### (S)-8-(2-amino-4-methylpentyloxy)-9-isopropyl-6-methylbenzo[c][2,7]naphthyridin-5(6H)-one

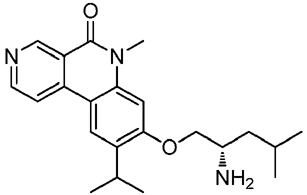

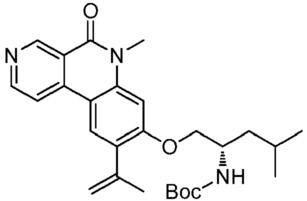

### Part A. (S)-tert-butyl (4-methyl-1-((6-methyl-5-oxo-9-(prop-1-en-2-yl)-5,6-dihydrobenzo[c][2,7]naphthyridin-8-yl)oxy)pentan-2-yl)carbamate

Prepared as described in Example 46, Parts A and B to afford *(S)-tert*-butyl (4-methyl-1-((6-methyl-5-oxo-9-(prop-1-en-2-yl)-5,6-dihydrobenzo[*c*][2,7]naphthyridin-8-yl)oxy)pentan-2-yl)carbamate (100 mg, 0.215 mmol, 90% yield) as a solid. LC/MS (ESI) *m*/*e* 466.4, [(M+H)⁺, calcd for C₂₇H₃₆N₃O₄, 466.3]; LC/MS retention time (method C): *t*_{R} = 2.22 min.

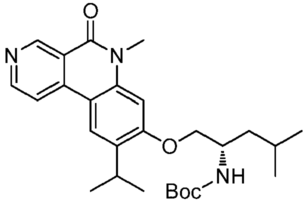

### Part B.(S)-tert-butyl (1-((9-isopropyl-6-methyl-5-oxo-5,6 dihydrobenzo[c][2,7]naphthyridin-8-yl)oxy)-4-methylpentan-2-yl)carbamate

A mixture of *(S)-tert-*butyl (4-methyl-1-((6-methyl-5-oxo-9-(prop-1-en-2-yl)-5,6-dihydrobenzo[*c*][2,7]naphthyridin-8-yl)oxy)pentan-2-yl)carbamate (0.1 g, 0.215 mmol) and palladium on carbon (0.07 g, 0.066 mmol) in MeOH (3 mL) and ethyl acetate (3 mL) was stirred at RT under a balloon of hydrogen gas for 24 h. The reaction mixture was filtered through diatomaceous earth (Celite®) and the filtrate was concentrated under reduced pressure to afford *(S)-tert-*butyl (1-((9-isopropyl-6-methyl-5-oxo-5,6-dihydrobenzo[*c*][2,7]naphthyridin-8-yl)oxy)-4-methylpentan-2-yl)carbamate (0.04 g, 2.464 mmol, 1% yield) as a brown oil, which was carried on without further purification. LC/MS (ESI) *m*/*e* 468.4, [(M+H)⁺, calcd for C₂₇H₃₈N₃O₄, 468.3]; LC/MS retention time (method D): *t*_{R} = 1.00 min.

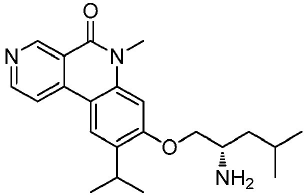

### Part C. (S)-8-(2-amino-4-methylpentyloxy)-9-isopropyl-6-methylbenzo[c][2,7]naphthyridin-5(6H)-one

Prepared as described in Example 2, Part E to afford the title product (12.05 mg, 0.033 mmol, 99% (purity) as a yellow solid. LC/MS (ESI) *m*/*e* 368.2, [(M+H)⁺, calcd for C₂₂H₃₀N₃O₂, 368.2]; LC/MS retention time (method C): *t*_{R} = 1.97 min. HPLC retention time (method A): *t*_{R} = 7.28 min; HPLC retention time (method B): *t*_{R} = 10.15 min.¹H NMR (400 MHz, *MeOD*) δ ppm 9.54 (s, 1H), 8.82 (d, *J*=6.0 Hz, 1H), 8.60 (d, *J*=8.8 Hz, 1H), 8.36 (s, 1H), 7.12 (s, 1H), 4.52 (m, 1H), 4.41 (m, 1H), 3.86 (s, 3H), 3.74-3.84 (m, 1H), 3.61 (m, 1H), 1.80-1.90 (m, 2H), 1.64 - 1.81 (m, 1H), 1.35-1.43 (m, 6H), 1.02-1.11 (m, 6H).

### Example 48

### (S)-8-(2-amino-4-methylpentyloxy)-6-methyl-9-(oxazol-5-yl)benzo[c][2,7]naphthyridin-5(6H)-one

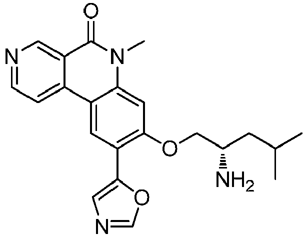

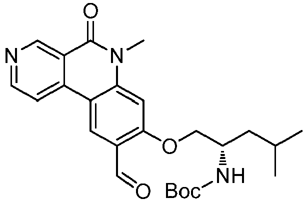

### Part A. (S)-tert-butyl 1-(9-formyl-6-methyl-5-oxo-5,6-dihydrobenzo[c][2,7]naphthyridin-8-yloxy)-4-methylpentan-2-ylcarbamate

A mixture of *(S)-tert-*butyl (4-methyl-1-((6-methyl-5-oxo-9-vinyl-5,6-dihydrobenzo[*c*][2,7]naphthyridin-8-yl)oxy)pentan-2-yl)carbamate (300 mg, 0.664 mmol) (prepared as described in Example 46, Part A) osmium tetroxide (2.5% in 2-methyl-2-propanol) (4.17 µl, 0.013 mmol), and 2,6-dimethylpyridine (0.155 mL, 1.329 mmol) in 1,4-dioxane (5 mL) and water (2 mL), cooled to 0°C, was stirred for 15 min. Sodium metaperiodate (568 mg, 2.66 mmol) was added and the reaction was warmed to room temperature and stirred for 2 h. The reaction mixture was filtered through diatomaceous earth (Celite®), eluting with EtOAc. The EtOAc layer was washed with saturated aqueous NaHCO₃, H₂O, then brine. The organic layer was dried with Na₂SO₄, filtered and concentrated under reduced pressure. The residue was purified by silica gel chromatography (EtOAc/petroleum ether) to afford *(S)-tert-*butyl (1-((9-formyl-6-methyl-5-oxo-5,6-dihydrobenzo[*c*][2,7]naphthyridin-8-yl)oxy)-4-methylpentan-2-yl)carbamate (160 mg, 0.353 mmol, 53 % yield) as a gummy solid. LC/MS (ESI) *m*/*e* 454.1, [(M+H)⁺, calcd for C₂₅H₃₂N₃O₅, 454.2].

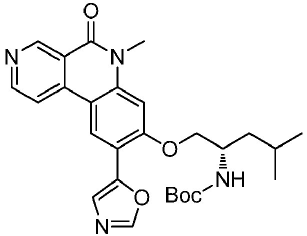

### Part B. (S)-tert-butyl (4-methyl-1-((6-methyl-9-(oxazol-5-yl)-5-oxo-5,6-dihydrobenzo[c][2,7]naphthyridin-8-yl)oxy)pentan-2-yl)carbamate

A mixture of *(S)-tert-*butyl (1-((9-formyl-6-methyl-5-oxo-5,6-dihydrobenzo[*c*][2,7]naphthyridin-8-yl)oxy)-4-methylpentan-2-yl)carbamate (100 mg, 0.220 mmol), K₂CO₃ (33.5 mg, 0.243 mmol) and TOSMIC (47.4 mg, 0.243 mmol) in MeOH (5 mL) was heated at 60 °C for 2 h. After cooling, the MeOH was removed under reduced pressure and residue was taken up in ethyl acetate. The organic layer was washed with H₂O, followed by saturated NaHCO₃, dried over Na₂SO₄ and concentrated under reduced pressure to afford *(S)-tert-*butyl (4-methyl-1-((6-methyl-9-(oxazol-5-yl)-5-oxo-5,6-dihydrobenzo[*c*][2,7]naphthyridin-8-yl)oxy)pentan-2-yl)carbamate (50 mg, 0.054 mmol, 24% crude yield) as a gum. The material was carried on without further purification. LC/MS (ESI) *m*/*e* 493.4, [(M+H)⁺, calcd for C₂₇H₃₃N₄O₅, 493.6]; LC/MS retention time (method E): *t*_{R} = 1.08 min.

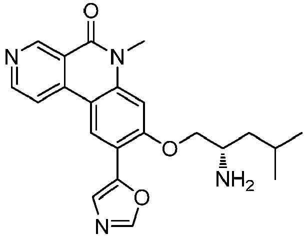

### Part C. (S)-8-(2-amino-4-methylpentyloxy)-6-methyl-9-(oxazol-5-yl)benzo[c][2,7]naphthyridm-5(6H)-one

Prepared as described in Example 2, Part E to afford *(S)*-8-(2-amino-4-methylpentyloxy)-6-methyl-9-(oxazol-5-yl)benzo[*c*][2,7]naphthyridin-5(*6H*)-one (15 mg, 0.038 mmol, 29% yield) as a yellow solid. LC/MS (ESI) *m*/*e* 393.2, [(M+H)⁺, calcd for C₂₂H₂₅N₄O₃, 393.2]; LC/MS retention time (method C): *t*_{R} = 1.70 min. HPLC retention time (method A): *t*_{R} = 8.89 min; HPLC retention time (method B): *t*_{R} = 9.12 min. ¹H NMR (400 MHz, METHANOL-*d*4) δ ppm 1.07 - 1.13 (m, 6 H) 1.75 (d, *J*=6.78 Hz, 1 H) 1.81 - 1.93 (m, 2 H) 3.91 (s, 3 H) 3.94 - 4.00 (m, 1 H) 4.55 (dd, *J*=11.04, 6.53 Hz, 1 H) 4.67 (dd, *J*=10.92, 3.39 Hz, 1 H) 7.30 (s, 1 H) 7.71 (s, 1 H) 8.43 (s, 1 H) 8.55 (d, *J*=5.52 Hz, 1 H) 8.90 (s, 2 H) 9.59 (s, 1 H).

### Example 49

### (S)-8-(2-amino-4-methylpentyloxy)-9-bromo-4-methylbenzo[c][2,7]naphthyridin-5(6H)-one

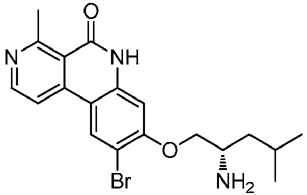

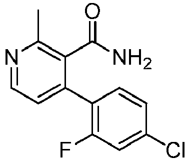

### Part A. 4-(4-chloro-2-fluorophenyl)-2-methylnicotinamide

4-(4-Chloro-2-fluorophenyl)-2-methylnicotinic acid (9 g, 33.9 mmol) (previous described in Example 16, Part E) was taken in DCM (50 mL) and cooled to 0 °C. The solution was treated with oxalyl chloride (14.83 mL, 169 mmol) followed by slow addition of DMF (1 mL). The mixture was heated at 40°C for 3 h. After cooling, the volatiles were removed under reduced pressure. The residue taken up in DCM (25 mL) was cooled to 0°C, and TEA (22.08 mL, 158 mmol) and ammonium chloride (16.94 g, 317 mmol) were added slowly. After stirring at room temperature for 1 h, the reaction mixture was washed with saturated aqueous NaHCO3 (10 mL), water (10 mL), and brine (10 mL). The organic layer was separated and dried with Na₂SO₄ to afford the 4-(4-chloro-2-fluorophenyl)-2-methylnicotinamide (3.5 g, 13.2 mmol, 42% crude yield) as a brown solid. The material was carried forward without further purification. LC/MS (ESI) m/e 264.4 [(M)⁺, calcd for C₁₃H₁₀ClFN₂O 264.0] LC/MS retention time (method C): *t*_{R} = 1.58 min.

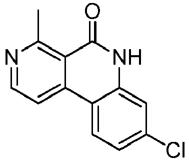

### Part B. 8-chloro-4-methylbenzo[c][2,7]naphthyridin-5(6H)-one

Prepared as described in Example 2, Part C to afford 8-chloro-4-methylbenzo[*c*][2,7]naphthyridin-5(*6H*)-one (800 m g, 3.14 mmol, 24% yield, 96% purity) as a yellow solid. LC/MS (ESI) m/e 245.1 [(M+H)⁺, calcd for C₁₃H₁₀ClN₂O 245.04] LC/MS retention time (method D): *t*_{R} = 0.52 min.

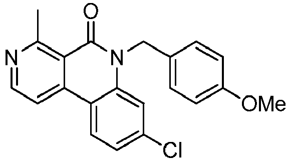

### Part C. 8-chloro-6-(4-methoxybenzyl)-4-methylbenzo[c][2,7]naphthyridin-5(6H)-one

Prepared as described in Example 17, Part G to afford 8-chloro-6-(4-methoxybenzyl)-4-methylbenzo[*c*][2,7]naphthyridin-5(*6H*)-one (710 m g, 1.362 mmol, 56% yield) as a brown gum. LC/MS (ESI) m/e 365.1 [(M+H)⁺, calcd for C₂₁H₁₈ClN₂O₂ 365.1] LC/MS retention time (method D): *t*_{R} = 0.8 min.

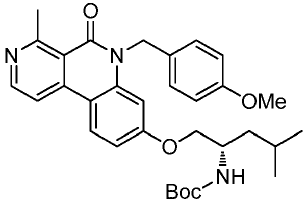

### Part D. (S)-tert-butyl 1-(6-(4-methoxybenzyl)-4-methyl-5-oxo-5,6-dihydrobenzo[c][2,7]naphthyridin-8-yloxy)-4-methylpentan-2-ylcarbamate

Prepared as described in Example 16, Part H to afford *(S)*-*tert*-butyl 1-(6-(4-methoxybenzyl)-4-methyl-5-oxo-5,6-dihydrobenzo[*c*][2,7]naphthyridin-8-yloxy)-4-methylpentan-2-ylcarbamate (130 mg, 0.21 mmol, 31 % yield, 88% purity) as a brown gum. LC/MS (ESI) m/e 546.2 [(M+H)⁺, calcd for C₃₂H₄₀FN₃O₅ 546.3] LC/MS retention time (method D): *t*_{R} = 0.94 min.

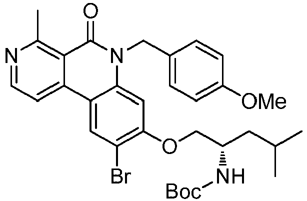

### Part E. (S)-tert-butyl 1-(9-bromo-6-(4-methoxybenzyl)-4-methyl-5-oxo-5,6-dihydrobenzo[c][2,7]naphthyridin-8-yloxy)-4-methylpentan-2-ylcarbamate

Prepared as described in Example 3, Part A to afford (*S)*-*tert*-butyl 1-(9-bromo-6-(4-methoxybenzyl)-4-methyl-5-oxo-5,6-dihydrobenzo[*c*][2,7]naphthyridin-8-yloxy)-4-methylpentan-2-ylcarbamate (140 mg, 0.146 mmol, 61 % yield, 65% purity) as an orange red solid. LC/MS (ESI) m/e 624.2 [(M+H)⁺, calcd for C₃₂H₃₉BrN₃O₅ 624.2] LC/MS retention time (method D): *t*_{R} = 0.98 min.

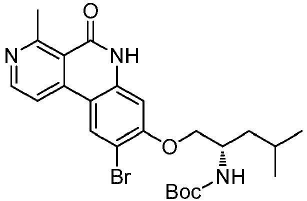

### Part F. (S)-tert-butyl 1-(9-bromo-4-methyl-5-oxo-5,6-dihydrobenzo[c][2,7]naphthyridin-8-yloxy)-4-methylpentan-2-ylcarbamate

PMB deprotection carried out using ceric ammonium nitrate as described in Protective Groups in organic synthesis (Greene, Wuts; 3rd ed., 1999, John Wiley & Sons, Inc.) afford *(S)*-*tert*-butyl 1-(9-bromo-4-methyl-5-oxo-5,6-dihydrobenzo[*c*][2,7]naphthyridin-8-yloxy)-4-methylpentan-2-ylcarbamate (60 mg, 0.12 mmol, 93 % yield) as a red solid. LC/MS (ESI) m/e 504.1 [(M+H)⁺, calcd for C₂₄H₃₁BrN₃O₄ 504.14] LC/MS retention time (method D): *t*_{R} = 0.91 min.

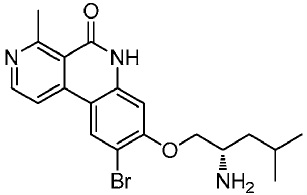

### Part G. (S)-8-(2-amino-4-methylpentyloxy)-9-bromo-4-methylbenzo[c][2,7]naphthyridin-5(6H)-one

Prepared as described in Example 2, Part E to afford *(S)*-8-(2-amino-4-methylpentyloxy)-9-bromo-4-methylbenzo[*c*][2,7]naphthyridin-5(*6H*)-one (12 mg, 0.029 mmol, 35 % yield, 98% purity) as a off-white solid. LC/MS (ESI) m/e 402.0 [(M)⁻, calcd for C₁₉H₂₁BrN₃O₂ 402.1] LC/MS retention time (method C): *t*_{R} = 2.19 min. HPLC retention time (method A): t_{R} = 8.52 min and HPLC retention time (method B): t_{R} = 9.02 min. ¹H NMR (400MHz, METHANOL-*d₄*) δ ppm 8.67 (s, 1H), 8.64 (d, *J*=6.0 Hz, 1H), 8.30 (d, *J*=6.0 Hz, 1H), 6.98 (s, 1H), 4.85 - 4.43 (m, 1H), 4.31 - 4.27 (m, 1H), 3.82 - 3.80 (m, 1H), 3.13 (s, 3H), 1.88 - 1.80 (m, 2H), 1.76 - 1.71 (m, 1H), 1.09 (d, *J*=6.4 Hz, 3H), 1.07 (d, *J*=6.4 Hz, 3H).

### Example 50

### (S)-8-((2-amino-4-methylpentyl)oxy)-4-((4-methoxybenzyl)amino)-6-methylbenzo[c][2,7]naphthyridin-5(6H)-one

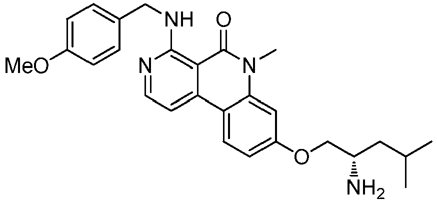

Prepared as described in Example 17, Part H and Example 2, Part E to afford *(S)*-8-((2-amino-4-methylpentyl)oxy)-4-((4-methoxybenzyl)amino)-6-methylbenzo[*c*][2,7]naphthyridin-5(*6H*)-one hydrochloride (19 mg, 0.038 mmol, 26 % yield) as a pale yellow solid. LC/MS (ESI) *m*/*e* 461 [(M+H)⁺, calcd for C₂₇H₃₃N₄O₃, 461.25]; LC/MS retention time (method B): *t*_{R} = 1.42 min. HPLC retention time (method A): *t*_{R} = 11.09 min; HPLC retention time (method B): *t*_{R} = 12.68 min. ¹H NMR (400 MHz, *DMSO-d*₆) δ 10.09 (br. s. 1 H), 8.40 (d, *J*=8.8 Hz, 1H), 8.24 (d, *J*=6.0 Hz, 1H), 8.07 (s, 3H), 7.45 (d, *J*=6.0 Hz, 1H), 7.33 (d, *J*=8.4 Hz, 2H), 7.07 - 7.06 (m, 2H), 6.95-6.91 (m, 2H), 4.67 (d, *J*=5.2 Hz, 1H), 4.38 (dd, *J*=10.4, 3.2 Hz, 2H), 4.21 (dd, *J*=10.8, 6.8 Hz, 1H), 3.75 (s, 3H), 3.68 (s, 3H), 3.62 (br s, 1H), 1.80 (quin, *J*=6.8, 1H), 1.59-1.55 (m, H), 0.95 (d, *J*=6.4 Hz, 3H), 0.94 (d, *J*=6.4 Hz, 3H).

### Example 51

### (S)-8-((2-amino-4-methylpentyl)oxy)-9-(4-fluorophenyl)-6 methylbenzo[c][2,7]naphthyridin-5(6H)-one

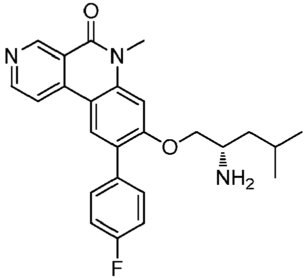

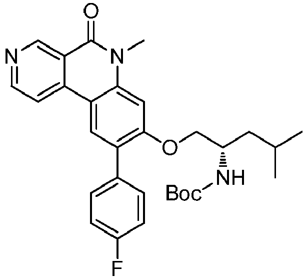

### Part A. (S)-tert-butyl (1-((9-(4-fluorophenyl)-6-methyl-5-oxo-5,6-dihydrobenzo[c][2,7]naphthyridin-8-yl)oxy)-4-methylpentan-2-yl)carbamate

*(S)-tert*-Butyl (1-((9-bromo-6-methyl-5-oxo-5,6-dihydrobenzo[*c*][2,7]naphthyridin-8-yl)oxy)-4-methylpentan-2-yl)carbamate (150 mg, 0.297 mmol) (prepared as described in Example 3, Part A) 4-fluorophenylboronic acid (49.9 mg, 0.357 mmol), Cs₂CO₃ (291 mg, 0.892 mmol) and PdCl₂(dppf)-CH₂Cl₂ adduct (12.14 mg, 0.015 mmol) in 1,4-dioxane (8 mL) and water (0.4 mL) was degassed with nitrogen gas for 5 min then heated at 80 °C overnight. The reaction mixture was cooled to room temperature and concentrated under reduced pressure. The residue was taken up in dichloromethane (10 mL) and water (8 mL). The organic layer was separated, dried with sodium sulfate, filtered and concentrated under reduced pressure to afford *(S)-tert-*butyl (1-((9-(4-fluorophenyl)-6-methyl-5-oxo-5,6-dihydrobenzo[*c*][2,7]naphthyridin-8-yl)oxy)-4-methylpentan-2-yl)carbamate (0.13 g, 0.09 mmol, 31% yield) as a brown gum. The materialwas carried forward without further pruification. LC/MS, (ESI) *m*/*z* 520.4 [(M+H)⁺, calcd for C₃₀H₃₅FN₃O₄ 520.25]; LC/MS retention time (method D): *t*_{R} = 1.08 min.

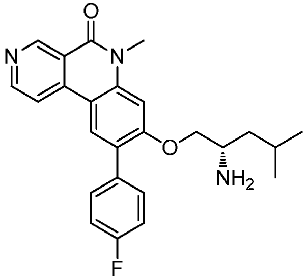

### Part B. (S)-8-((2-amino-4-methylpentyl)oxy)-9-(4-fluorophenyl)-6 methylbenzo[c][2,7]naphthyridin-5(6H)-one

Prepared as described in Example 2, Part E to afford *(S)*-8-((2-amino-4-methylpentyl)oxy)-9-(4-fluorophenyl)-6 methylbenzo[*c*][2,7]naphthyridin-5(*6H*)-one (19 mg, 0.043 mmol, 47% yield) as a pale yellow solid. LC/MS, (ESI) *m*/*z* 420.0 [(M+H)⁺, calcd for C₂₅H₂₇FN₃O₂, 420.3]; LC/MS retention time (method C'): *t*_{R} = 1.61 min.^{1H} NMR (400MHz, METHANOL-*d₄*) δ ppm 9.40 (s, 1H), 8.69 (m, 1H), 8.24 (s, 1H), 8.18 (m, 1H), 7.62 (m, 2H), 7.22 (m, 2H), 7.04 (s, 1H), 4.16 (m, 1H), 3.98 (m, 1H), 3.78 (s, 3H), 3.20 (m, 1H), 1.78 (m, 1H), 1.35 (m, 2H), 0.90-0.98 (m, 6H).

### Example 52

### (S)-4-amino-8-(2-amino-4-methylpentyloxy)-9-fluoro-6-methylbenzo[c][2,7]naphthyridin-5(6H)-one

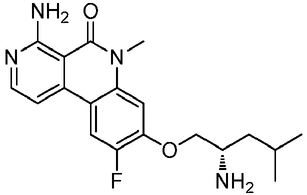

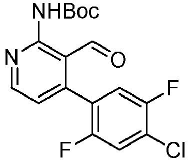

### Part A. tert-butyl 4-(4-chloro-2,5-difluorophenyl)-3-formylpyridin-2-ylcarbamate

Prepared as described in Example 17, Part C by Suzuki coupling between 2-(4-chloro-2,5-difluorophenyl)-4,4,5,5-tetramethyl-1,3,2-dioxaborolane)preparation described in Example 14, Part A) and *tert*-butyl 4-chloro-3-formylpyridin-2-ylcarbamate (Preparation described in Example 17, Part B) to afford *tert*-butyl 4-(4-chloro-2,5-difluorophenyl)-3-formylpyridin-2-ylcarbamate (3.7 g, 8.91 mmol, 76% yield) as a yellow solid. LC/MS (ESI) m/e 367.1 [(M)⁻, calcd for C₁₇H₁₆ClF₂N₂O₃ 367.1] LC/MS retention time (method E): *t*_{R} = 1.07 min.

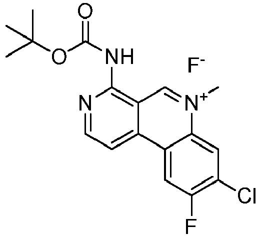

### Part B: 4-(tert-butoxycarbonylamino)-8-chloro-9-fluoro-6-methylbenzo[c][2,7]naphthyridin-6-ium

To a stirred solution of *tert*-butyl (4-(4-chloro-2,5-difluorophenyl)-3-formylpyridin-2-yl)carbamate (2.6 g, 6.26 mmol) in DCM (10 mL) at 0 °C was added dropwise methanamine (2M in MeOH) (75 mL, 6.26 mmol) and the reaction mixture was stirred at rt for 18 hours. The reaction mixture concentrated under reduced pressure. The residue was triturated with EtOAc/ hexane and the solid obtained was collected by vacuum filtration to yield 4-((*tert*-butoxycarbonyl)amino)-8-chloro-9-fluoro-6-methylbenzo[*c*][2,7]naphthyridin-6-ium (2.7 g, 2.399 mmol, 38% yield) as a yellow solid. Sample was taken to the next step without further purification.LC/MS (ESI) m/e 362.2 [(M+H)⁺, calcd for C₁₈H₁₈ClFN₃O₂ 362.1] LC/MS retention time (method E): *t*_{R} = 0.66 min.

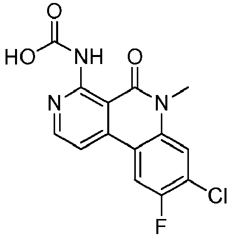

### Part C: 8-chloro-9-fluoro-6-methyl-5-oxo-5,6-dihydrobenzo[c][2,7]naphthyridin-4-ylcarbamic acid

To a stirred solution of 4-((*tert*-butoxycarbonyl)amino)-8-chloro-9-fluoro-6-methylbenzo[*c*][2,7]naphthyridin-6-ium (2.6 g, 2.310 mmol) in a mixture of DCM (10 mL) and water (50 mL) was added NaOH (0.370 g, 9.24 mmol) in three portions, followed by the addition of KMnO₄ (1.461 g, 9.24 mmol) in five protions and the reaction was heated to 90 °C for 5 h. After cooling, the solvent was removed under reduced pressure. The reidue was taken up in EtOAc (50 mL) and MeOH (50 mL) and stirred for 10 min. The reaction mixture was then passed through diatomaceous earth (Celite®), eluting with EtOAc. The filtrate was concentrated to yield (8-chloro-9-fluoro-6-methyl-5-oxo-5,6-dihydrobenzo[*c*][2,7]naphthyridin-4-yl)carbamic acid (2.35 g, 2.082 mmol, 90 % crude yield) as a yellow solid which was taken to the next step without purification. LC/MS (ESI) m/e 322.1 [(M+H)⁺, calcd for C₁₄H₁₀ClFN₃O₃ 322.0] LC/MS retention time (method E): *t*_{R} = 1.05 min.

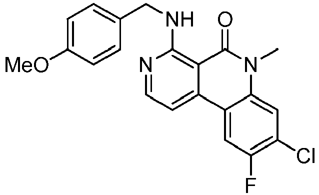

### Part D: 8-chloro-9-fluoro-4-(4-methoxybenzylamino)-6-methylbenzo[c][2,7]naphthyridin-5(6H)-one

Prepared as described in Example 17, Part F to give the 4-amino-8-chloro-9-fluoro-6-methylbenzo[*c*][2,7]naphthyridin-5(*6H*)-one which was then protected with PMB as per procedure described in Example 17, Part G to afford the title product (0.45 g, 0.755 mmol, 17 % yield) as a yellow oil. LC/MS (ESI) m/e 398.2 [(M+H)⁺, calcd for C₂₁H₁₈ClFN₃O₂ 398.1] LC/MS retention time (method E): *t*_{R} = 1.23 min.

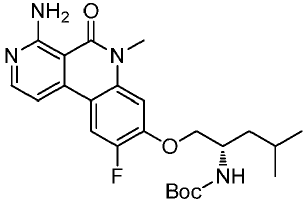

### Part E: (S)-tert-butyl 1-(9-fluoro-4-(4-methoxybenzylamino)-6-methyl-5-oxo-5,6-dihydrobenzo[c][2,7]naphthyridin-8-yloxy)-4-methylpentan-2-ylcarbamate

Prepared as described in Example 16, Part H to afford the title product (910 mg, 0.405 mmol, 54% yield) as a yellow oil. LC/MS (ESI) m/e 579.5 [(M+H)⁺, calcd for C₃₂H₄₀FN₄O₅ 579.3] LC/MS retention time (method E): *t*_{R} = 1.37 min.

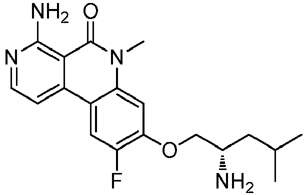

### Part F: (S)-4-amino-8-(2-amino-4-methylpentyloxy)-9-fluoro-6-methylbenzo[c][2,7]naphthyridin-5(6H)-one

Prepared as described in Example 2, Part E to afford the title product (27 mg, 0.045 mmol, 11 % yield) pale yellow solid in the form of TFA salt. LC/MS (ESI) m/e 359.2 [(M+H)⁺, calcd for C₁₉H₂₄FN₄O₂ 359.2] LC/MS retention time (method C): *t*_{R} = 1.53 min. HPLC retention time (method A): t_{R} = 8.01 min and HPLC retention time (method B): *t*_{R} = 9.13 min. ¹H NMR (400MHz, METHANOL-*d₄*) δ ppm 8.33 (d, *J*=12.0 Hz, 1H), 8.02 (d, *J*=7.0 Hz, 1H), 7.67 (d, *J*=7.3 Hz, 1H), 7.34 (d, *J*=7.3 Hz, 1H), 4.60 (dd, *J*=10.7, 3.1 Hz, 1H), 4.46 (dd, *J*=10.8, 6.0 Hz, 1H), 3.87 (s, 3H), 3.86 - 3.78 (m, 1H), 1.95 - 1.64 (m, 3H), 1.08 (d, *J*=4.3 Hz, 3H), 1.07 (d, *J*=4.3 Hz, 3H).

### Example 53

### (S)-8-(2-amino-3-isopropoxypropoxy)-6-methylbenzo[c][2,7]naphthyridin-5(6H)-one

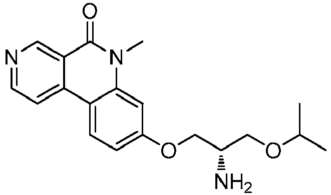

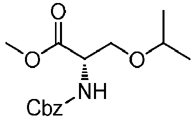

### Part A. (S)-methyl 2-(((benzyloxy)carbonyl)amino)-3-isopropoxypropanoate

Prepared as described in literature Steven, M.S. et.al. Bioorg Med. Chem. Lett., 2009, 19, 981-985.

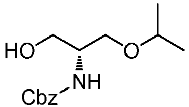

### Part B. (R)-benzyl (1-hydroxy-3-isopropoxypropan-2-yl)carbamate

*(S)*-Methyl 2-(((benzyloxy)carbonyl)amino)-3-isopropoxypropanoate (0.02 g, 0.068 mmol) was taken in 2-propanol (4 mL) and then NaBH₄ (7.69 mg, 0.203 mmol) was added and the mixture stirred for overnight at 50 °C. After cooling to 0 °C, the mixture was quenched with 1N HCl and then extracted with diethyl ether (10 mL). The diethyl ether layer was collected and concentrated under reduced pressure to afford (*R*)-benzyl (1-hydroxy-3 isopropoxypropan-2-yl)carbamate) (14 mg, 0.058 mmol, 86% crude yield) as colorless oil. The product was carried on without further purification. ¹H NMR (400 MHz, *MeOD*) δ 7.31-7.48 (m, 5H), 5.12 (s, 1H), 4.81 (bs, 1H), 3.41-3.91 (m, 6H), 1.12-1.26 (m, 6H).

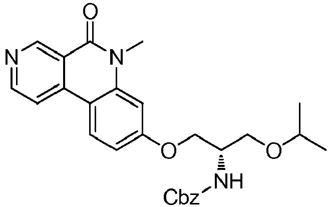

### Part C: (S)-benzyl (1-isopropoxy-3-((6-methyl-5-oxo-5,6-dihydrobenzo[c][2,7]naphthyridin-8-yl)oxy)propan-2-yl)carbamate

Prepared as described in Example 16, Part H to afford the title product *(S)-*benzyl (1-isopropoxy-3-((6-methyl-5-oxo-5,6-dihydrobenzo[*c*][2,7]naphthyridin-8-yl)oxy)propan-2-yl)carbamate (220 mg, 0.130 mmol, 10% yield) as a light yellow solid. LC/MS (ESI) *m*/*e* 476.4, [(M+H)⁺, calcd for C₂₇H₃₀N₃O₅, 476.2]; LC/MS retention time (method E): *t*_{R} = 1.04 min.

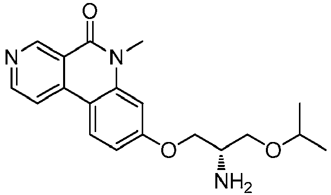

### Part D: (S)-8-(2-amino-3-isopropoxypropoxy)-6-methylbenzo[c][2,7]naphthyridin-5(6H)-one

A solution of (*S*)-benzyl (1-isopropoxy-3-((6-methyl-5-oxo-5,6-dihydrobenzo[*c*][2,7]naphthyridin-8-yl)oxy)propan-2-yl)carbamate (220 mg, 0.130 mmol) in MeOH (12 mL) was degassed with N₂ for 5 min. Pd/C (68.9 mg, 0.065 mmol) was added and the mixture stirred under a balloon of H₂ for 12h. The reaction mixture was filtered through diatomaceous earth (Celite®), eluting with methanol and the filtrate was concentrated under reduced pressure. The rsidue was purified by reverse phase HPLC (10 MM ammonium acetate in water/AcCN) to afford (*S*)-8-(2-amino-3-isopropoxypropoxy)-6-methylbenzo[*c*][2,7]naphthyridin-5(6*H*)-one (90 mg, 0.108 mmol, 83 % yield) as an off-white solid. LC/MS (ESI) *m*/*e* 342.2, [(M+H)⁺, calcd for C₁₉H₂₄N₃O₃, 342.2]; LC/MS retention time (method C): *t*_{R} = 1.50 min. HPLC retention time (method A): *t*_{R} = 7.20 min; HPLC retention time (method B): *t*_{R} = 8.00 min. ¹H NMR (400 MHz, *MeOD*) δ ppm 9.54 (s, 1H), 8.23 (d, *J*=4.4 Hz, 1H), 8.52 (d, *J*=8.8 Hz, 1H), 8.44 (d, *J*=6.0 Hz, 1H), 7.20 (s, 1H), 7.18 (d, *J*=6.0 Hz, 1H), 4.52 (dd, *J*=10.4, 3.6 Hz, 1H), 4.43 (dd, *J*=10.4, 6.8 Hz, 1H), 3.88-3.75 (m, 7H), 1.26 (d, *J*=6.4 Hz, 3H), 1.25 (d, *J*=6.4 Hz, 3H).

### Example 54

### (S)-6-methyl-8-((4-methyl-2-(methylamino)pentyl)oxy)benzo[c][2,7]naphthyridin-5(6H)-one

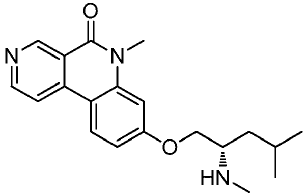

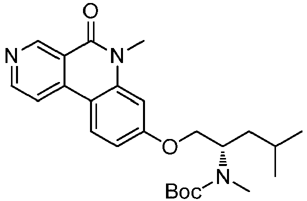

### Part A. (S)-tert-butyl methyl(4-methyl-1-(6-methyl-5-oxo-5,6-dihydrobenzo[c][2,7]naphthyridin-8-yloxy)pentan-2-yl)carbamate

To a solution of *(S)-tert-*butyl (4-methyl-1-((6-methyl-5-oxo-5,6-dihydrobenzo[*c*][2,7]naphthyridin-8-yl)oxy)pentan-2-yl)carbamate (200 mg, 0.470 mmol) (Preparation description in Example 2, Part D) in THF (4 mL) at 0 °C was added NaH (37.6 mg, 0.940 mmol). The reaction was stirred at 0 °C for 30 min then MeI (0.044 mL, 0.705 mmol) was added. The reaction mixture was then stirred at 0 °C for 16 h. The reaction mixture was quenched with ice cold water (50 mL) and extracted with ethyl acetate (3x30 mL). The combined organic layers and washed with brine (1x50 mL), dried over Na₂SO₄, filtered and concentrated under reduced pressure. The crude product was purified by silica gel chromatography using petroleum ether: ethyl acetate mobile phase to afford (*S*)-*tert*-butyl methyl(4-methyl-1-(6-methyl-5-oxo-5,6-dihydrobenzo[*c*][2,7]naphthyridin-8-yloxy)pentan-2-yl)carbamate (180 mg, 0.409 mmol, 87% yield) as a yellow solid. LC/MS (ESI) *m*/*e* 440.4, [(M+H)⁺, calcd for C₂₅H₃₄N₃O₄, 440.3]; LC/MS retention time (method I): *t*_{R} = 2.4 min.

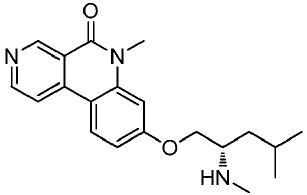

### Part B: (S)-6-methyl-8-((4-methyl-2-(methylamino)pentyl)oxy)benzo[c][2,7]naphthyridin-5(6H)-one

To the solution of *(S)-tert-*butyl methyl(4-methyl-1-((6-methyl-5-oxo-5,6-dihydrobenzo[*c*][2,7]naphthyridin-8-yl)oxy)pentan-2-yl)carbamate (180 mg, 0.078 mmol) in dichloromethane (4 mL) cooled to 0 °C was added HCl in ether (4 mL, 4.00 mmol) slowly over a period of 1 min. The reaction mixture was stirred at 0 °C for 5 min then warmed to room temperature and stirred for 4 h. The volatiles were then removed under reduced pressure. The residue was purified via reverse phase HPLC (0.1% TFA in water:acetonitrile) to afford *(S)*-6-methyl-8-((4-methyl-2-(methylamino)pentyl)oxy)benzo[*c*][2,7]naphthyridin-5(*6H*)-one (15 mg, 0.025 mmol, 32% yield) as a yellow solid. LC/MS (ESI) *m*/*e* 340.2, [(M+H)⁺, calcd for C₂₀H₂₆N₃O₂, 340.2]; LC/MS retention time (method I): *t*_{R} = 1.6 min. HPLC retention time (method A): *t*_{R} = 7.59 min; HPLC retention time (method B): *t*_{R} = 8.08 min. ¹H NMR (400MHz, METHANOL-d₄) δ ppm 9.54 (s, 1H), 8.83 (d, *J*=5.8 Hz, 1H), 8.53 (d, *J*=8.8 Hz, 1H), 8.46 (d, *J*=6.0 Hz, 1H), 7.27 - 7.16 (m, 2H), 4.57 (dd, *J*=11.3, 3.0 Hz, 1H), 4.44 (dd, *J*=11.3, 5.3 Hz, 1H), 3.84 (s, 3H), 3.78 - 3.68 (m, 1H), 2.84 (s, 3H), 1.95 - 1.78 (m, 2H), 1.75 - 1.61 (m, 1H), 1.08 (d, *J*=6.5 Hz, 6H).

### Example 55

### (S)-8-((2-amino-4,4-difluoropentyl)oxy)-6-methylbenzo[c][2,7]naphthyridin-5(6H)-one

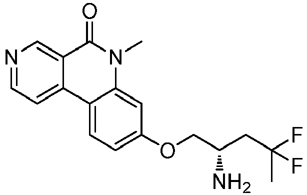

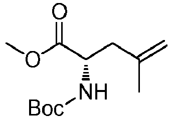

### Part A: (S)-methyl 2-((tert-butoxycarbonyl)amino)-4-methylpent-4-enoate

To a solution of *(S)*-2-((*tert*-butoxycarbonyl)amino)-4-methylpent-4-enoic acid (550 mg, 2.399 mmol) in tetrahydrofuran (11 mL) at 0 °C, was added TMS-diazomethane (4.80 mL, 4.80 mmol) dropwise over 5 min. The reaction mixture was then stirred at 0 °C for 15 min. After gradually warmed to room temperature, the mixture was stirred for 16h. The reaction mixture was evaporated to dryness to afford *(S)*-methyl 2-((*tert*-butoxycarbonyl)amino)-4-methylpent-4-enoate (580 mg, 2.386 mmol, 99% crude yield) as a dark yellow oil which was taken to the next step without purification. ¹H NMR (400 MHz, *CDCl₃*): δ ppm 4.91 (s, 1H), 4.85 (s, 1H), 4.75 (s, 1H), 4.38 (q, *J* = 16.00 Hz, 1H), 3.73 (s, 3H), 2.33-2.53 (m, 2H), 1.71 (s, 3H), 1.43 (s, 9H).

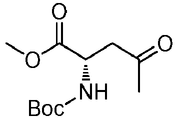

### Part B: (S)-methyl 2-((tert-butoxycarbonyl)amino)-4-oxopentanoate

To a solution of *(S)*-methyl 2-((*tert*-butoxycarbonyl)amino)-4-methylpent-4-enoate (500 mg, 2.055 mmol) in a solvent mixture of 1,4-dioxane (10 mL) and water (2.5 mL) at 0 °C, was added 2,6-lutidine (0.479 mL, 4.11 mmol) and osmium tetroxide (2.5% in 2-methyl-2-propanol) (0.516 mL, 0.041 mmol), followed by sodium metaperiodate (1.758 g, 8.22 mmol). The reaction mixture was stirred at 0 °C for 15 min and warmed to room temperature and stirred for an additional 3h. The mixture was diluted with ethyl acetate (150 mL) and washed with 10% aqueous NaHCO₃. The organic layer was separated, dried over sodium sulphate and concentrated under reduced pressure to afford *(S)*-methyl 2-((*tert-*butoxycarbonyl)amino)-4-oxopentanoate (500 mg, 2.040 mmol, 100% crude yield) as a yellow liquid. The material was caried on without further purification. ¹H NMR (400 MHz, CDCl₃): δ ppm 5.48 (s, 1H), 4.51 (t, *J* = 8.00 Hz, 1H), 3.75 (s, 3H), 3.23 (d, *J* = 4.00 Hz, 1H), 3.17 (d, *J* = 4.00 Hz, 1H), 2.18 (s, 3H), 1.46 (s, 9H).

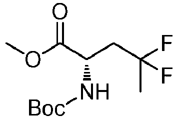

### Part C: (S)-methyl 2-((tert-butoxycarbonyl)amino)-4,4-difluoropentanoate

To *(S)*-methyl 2-((*tert*-butoxycarbonyl)amino)-4-oxopentanoate (300 mg, 1.223 mmol) at 0 °C, was added DAST (M in) (323 µL, 2.446 mmol) dropwise. The reaction mixture was stirred at room temperature for 84h. The reaction mixture was cooled to 0 °C and then quenched with aqueous 10% NaHCO₃ solution. The resulting suspension was extracted with ethyl acetate (3 x 25 mL). The organic layer was washed with brine (15 mL), dried over Na₂SO4, filtered, and concentrated under reduced pressure. The crude product was purified by silica gel chromatography to afford *(S)*-methyl 2-((*tert*-butoxycarbonyl)amino)-4,4-difluoropentanoate (70 mg, 0.26 mmol, 19 % yield) as a yellow oil. ¹H NMR (400 MHz, CDCl₃): δ ppm 5.15 (s, 1H), 4.50 (s, 1H), 3.75 (s, 3H), 3.23 (t, *J* = 8.00 Hz, 1H), 2.30-2.45 (m, 2H), 1.60 (s, 3H), 1.44 (s, 9H).

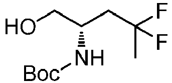

### Part D: (S)-tert-butyl (4,4-difluoro-1-hydroxypentan-2-yl)carbamate

To a solution of *(S)*-methyl 2-((*tert*-butoxycarbonyl)amino)-4,4-difluoropentanoate (70 mg, 0.262 mmol) in tetrahydrofuran (4 mL) at -10 °C, was added Lithium aluminum hydride, 2M in THF (0.262 mL, 0.524 mmol). The reaction mixture was stirred at -10 °C for 2h, then quenched with aqueous ammonium chloride (5 mL) and extracted with ethyl acetate (2 x 10 mL). The combined organic layers were washed with brine (15 mL), dried over Na₂SO₄, filtered, and concentrated under reduced pressure to afford *(S)-tert-*butyl (4,4-difluoro-1-hydroxypentan-2-yl)carbamate (38 mg, 0.159 mmol, 61 % crude yield) as colorless oil. The materialwas carried forward without further purification. ¹H NMR (400 MHz, CDCl₃): δ ppm 3.89 (d, *J* = 4.00 Hz, 2H), 2.10-2.14 (m, 1H), 1.61-1.66 (m, 2H), 1.51 (s, 3H), 1.44 (s, 9H).

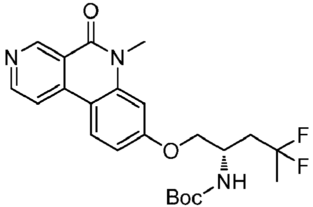

### Part E: (S)-tert-butyl (4,4-difluoro-1-((6-methyl-5-oxo-5,6-dihydrobenzo[c][2,7]naphthyridin-8-yl)oxy)pentan-2-yl)carbamate

In a sealed tube containing 8-chloro-6-methylbenzo[*c*][2,7]naphthyridin-5(*6H*)-one (30 mg, 0.123 mmol) and *(S)-tert-*butyl (4,4-difluoro-1-hydroxypentan-2-yl)carbamate (35.2 mg, 0.147mmol) was added toluene (3 mL). Di-*tert*-butyl(2',4',6'-triisopropyl-[1,1'-biphenyl]-2-yl)phosphine (31.2 mg, 0.074 mmol) and cesium carbonate (59.9 mg, 0.184 mmol) were added to the reaction mixture followed by palladium (II)acetate (8.26 mg, 0.037 mmol). The reaction mixture was then heated at 85 °C for 16 h. The reaction mixture was cooled to room temperature, diluted with ethyl acetate and filtered through diatomaceous earth (Celite®), eluting with ethyl acetate. The filtrate was evaporated under reduced pressure and the crude product was purified by silica gel chromatography to afford *(S)-tert*-butyl (4,4-difluoro-1-((6-methyl-5-oxo-5,6-dihydrobenzo[*c*][2,7]naphthyridin-8-yl)oxy)pentan-2-yl)carbamate (12 mg, 0.027 mmol, 22% yield) as yellow solid. LC/MS (ESI) *m*/*e* 448.3 [(M+H)⁺, calcd for C₂₃H₂₈F₂N₃O₄ 448.2]; LC/MS retention time (method E): *t*_{R} = 0.96 min.

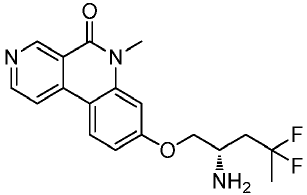

### Part F: (S)-8-((2-amino-4,4-difluoropentyl)oxy)-6-methylbenzo[c][2,7]naphthyridin-5(6H)-one

To a solution of *(S)-tert-*butyl (4,4-difluoro-1-((6-methyl-5-oxo-5,6-dihydrobenzo[*c*][2,7]naphthyridin-8-yl)oxy)pentan-2-yl)carbamate (15 mg, 0.024 mmol) in dichloromethane (2 mL) at 0 °C was added 4M HCl in diethyl ether (2 mL, 2.00 mmol) dropwise over a period of 5 min. The reaction mixture was stirred at 0 °C for 5 min and warmed to room temperature and stirred for 3h. The volatiles were removed under reduced pressure. The residue was dissolved in water (10 mL) and washed with ethyl acetate (2 x 5 mL). The aqueous layer was lyophilized to afford *(S)*-8-((2-amino-4,4-difluoropentyl)oxy)-6-methylbenzo[*c*][2,7]naphthyridin-5(*6H*)-one, 2HCl (6 mg, 0.018 mmol, 72 % yield, 93% purity) as a brown sticky solid. LC/MS (ESI) *m*/*e* 348.2 [(M+H)⁺, calcd for C₁₈H₂₀F₂N₃O₂ 348.1]; LC/MS retention time (method E): *t*_{R} = 0.56 min. HPLC retention time (method A): *t*_{R} = 6.60 min; HPLC retention time (method B): *t*_{R} = 6.53 min; ¹H NMR (400MHz, METHANOL-*d₄*) δ ppm 9.60 (br. s., 1H), 8.88 (br. s., 1H), 8.73 (br. s., 1H), 8.60 (br. s., 1H), 7.23 (br. s., 2H), 4.63 - 4.36 (m, 2H), 4.13 - 4.06 (m, 1H), 3.84 (br. s., 3H), 2.75 - 2.40 (m, 2H), 1.78 (t, *J*=18.8 Hz, 3H).

### Example 56

### 8-((2-amino-5,5,5-trifluoropentyl)oxy)-6-methyl-5-oxo-5,6 dihydrobenzo[c][2,7]naphthyridine-9-carbonitrile

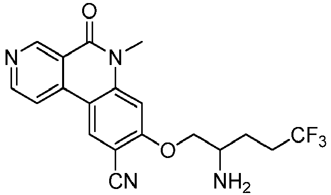

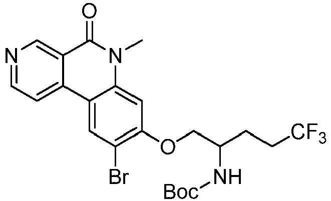

### Part A: tert-butyl (1-((9-bromo-6-methyl-5-oxo-5,6-dihydrobenzo[c][2,7]naphthyridin-8-yl)oxy)-5,5,5 trifluoropentan-2-yl)carbamate

Prepared as described in Example 15, Part E and Example 3, Part A to afford the title product *tert*-butyl (1-((9-bromo-6-methyl-5-oxo-5,6-dihydrobenzo[*c*][2,7]naphthyridin-8-yl)oxy)-5,5,5 trifluoropentan-2-yl)carbamate (0.720 g, 0.807mmol, 60% yield) as a yellow solid. LC/MS (ESI) *m*/*e* 546.5, [(M+2H)⁺, calcd for C₂₃H₂₇BrF₃N₃O₄, 546.1]; LC/MS retention time (method D): *t*_{R} = 0.90 min.

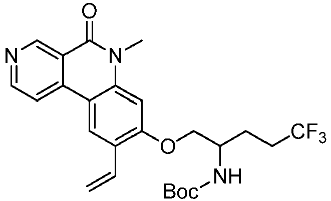

### Part B: a tert-butyl (5,5,5-trifluoro-1-((6-methyl-5-oxo-9-vinyl-5,6-dihydrobenzo[c][2,7]naphthyridin-8-yl)oxy)pentan-2-yl)carbamate

Prepared as described in Example 46, Part B to afford the title product *tert-*butyl (5,5,5-trifluoro-1-((6-methyl-5-oxo-9-vinyl-5,6-dihydrobenzo[*c*][2,7]naphthyridin-8 yl)oxy)pentan-2-yl)carbamate (0.6 g, 0.855 mmol, 75 % yield) as a yellow solid.
LC/MS (ESI) *m*/*e* 492.6, [(M+H)⁺, calcd for C₂₅H₂₉F₃N₃O₄, 492.2]; LC/MS retention time (method D): *t*_{R} = 0.90 min.

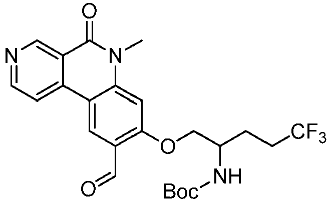

### Part C: tert-butyl (5,5,5-trifluoro-1-((9-formyl-6-methyl-5-oxo-5,6-dihydrobenzo[c][2,7]naphthyridin-8-yl)oxy)pentan-2-yl)carbamate

Prepared as described in Example 55, Part B to afford the title product *tert-*butyl (5,5,5-trifluoro-1-((9-formyl-6-methyl-5-oxo-5,6-dihydrobenzo[*c*][2,7]naphthyridin-8-yl)oxy)pentan-2-yl)carbamate (0.081 g, 0.079 mmol, 62% yield) as a brown solid. LC/MS (ESI) *m*/*e* 494.5 (M+H)⁺, calcd for C₂₄H₂₇F₃N₃O₅, 494.2]; LC/MS retention time (method D): *t*_{R} = 0.85 min.

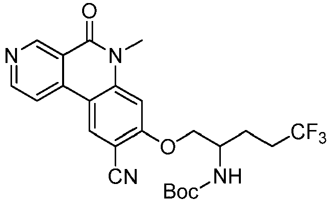

### Part D: tert-butyl (1-((9-cyano-6-methyl-5-oxo-5,6-dihydrobenzo[c][2,7]naphthyridin-8 yl)oxy)-5,5,5-trifluoropentan-2-yl)carbamate

Iodine (0.047 g, 0.184 mmol) was added to a stirred solution of *tert*-butyl (5,5,5-trifluoro-1-((9-formyl-6-methyl-5-oxo-5,6-dihydrobenzo[*c*][2,7]naphthyridin-8-yl)oxy)pentan-2-yl)carbamate (0.165g, 0.167 mmol) in aqueous ammonia (4 mL, 48.1 mmol) and tetrahydrofuran (6 mL) at room temperature. The dark solution became light gray after stirring for 3 h, an indication that the reaction was complete. The reaction mixture was charged with aqueous Na2S2O3 (25 mL of 5% solution) and extracted with ethyl acetate (3×30 mL). The combined the organic layers were dried over sodium sulphate and concentrated under reduced pressure to afford *tert-*butyl (1-((9-cyano-6-methyl-5-oxo-5,6 dihydrobenzo[c][2,7]naphthyridin-8-yl)oxy)-5,5,5-trifluoropentan-2-yl)carbamate (0.14 g, 0.143 mmol, 85 % crude yield) as a pale yellow solid. The material was carried forward without further purification. LC/MS (ESI) *m*/*e* 491.5 (M+H)⁺, calcd for C₂₄H₂₆F₃N₄O₄, 491.2]; LC/MS retention time (method D): *t*_{R} = 0.87 min.

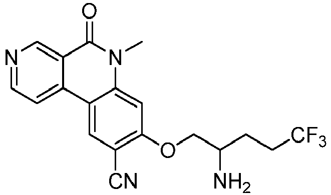

### Part E: 8-((2-amino-5,5,5-trifluoropentyl)oxy)-6-methyl-5-oxo-5,6 dihydrobenzo[c][2,7]naphthyridine-9-carbonitrile

Prepared as described in Example 2, Part E to afford the title product 8-((2-amino-5,5,5- trifluoropentyl)oxy)-6-methyl-5-oxo-5,6-dihydrobenzo[*c*][2,7]naphthyridine-9-carbonitrile (0.027 g, 0.066 mmol, 34% yield) as a white solid. LC/MS (ESI) *m*/*e* 391.2 [(M+H)⁺, calcd for C₁₉H₁₈F₃N₄O₂, 391.1] LC/MS retention time (method C): *t*_{R} = 1.61 min. HPLC retention time (method A): *t*_{R} = 8.09 min; HPLC retention time (method B): *t*_{R} = 10.36 min. ¹H NMR (400MHz, METHANOL-d₄) δ ppm 9.50 (s, 1H), 8.87 - 8.78 (m, 2H), 8.31 (d, *J*=5.8 Hz, 1H), 7.20 (s, 1H), 4.42 - 4.30 (m, 1H), 4.29 - 4.20 (m, 1H), 3.84 (s, 3H), 3.40 - 3.38 (m, 1H), 2.58 - 2.31 (m, 2H), 2.09 - 1.94 (m, 1H), 1.90 - 1.72 (m, 1H).

### Example 57

### 6-methyl-8-(piperidin-2-ylmethoxy)benzo[c][2,7]naphthyridin-5(6H)-one

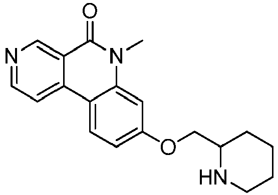

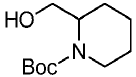

### Part A: tert-butyl 2-(hydroxymethyl)piperidine-1-carboxylate

To a stirred solution of piperidin-2-ylmethanol (1.5 g, 13.02 mmol) in dichloromethane (25 mL) was added DIPEA (6.82 mL, 39.1 mmol). After stirring for 5 min Boc₂O (3.63 mL, 15.63 mmol) was added and the mixture was stirred at RT overnight. The mixture was quenched with water and diluted with DCM (50 mL). The organic layer was separated, dried over Na₂SO₄ and concentrated to give *tert-*butyl 2-(hydroxymethyl)piperidine-1-carboxylate (2 g, 9.29 mmol, 71% crude yield) as a colorless oil, which was taken to the next step without further purification. ¹H NMR (400 MHz, *CDCl₃*) *ppm* δ 4.27-4.28 (m, 1H), 3.80-3.82 (m, 1H), 3.75-3.77 (m, 1H), 3.56-3.58 (m, 1H), 2.63 (t, J = 7.20 Hz, 1H), 1.55-1.55 (m, 5H), 1.43 (s, 9H).

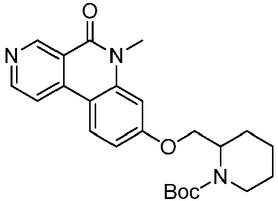

### Part B: tert-butyl 2-(((6-methyl-5-oxo-5,6-dihydrobenzo[c][2,7]naphthyridin-8-yl)oxy)methyl)piperidine-1-carboxylate

Prepared as described in Example 16, Part H to afford the title product (380 mg, 0.332 mmol, 41 % yield), yellow oil. LC/MS (ESI) *m*/*e* 424.2, [(M+H)⁺, calcd for C₂₄H₃₀N₃O₄, 424.2]; LC/MS retention time (method I): *t*_{R} = 2.29 min.

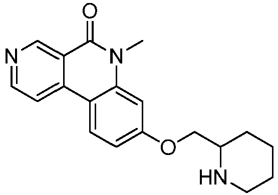

### Part B: 6-methyl-8-(piperidin-2-ylmethoxy)benzo[c][2,7]naphthyridin-5(6H)-one

Prepared as described in Example 2, Part E to afford the title compound (30 mg,0.092 mmol, 53 % yield) as a pale yellow mono TFA salt. LC/MS (ESI) *m*/*e* 324.2 [(M+H)⁺, calcd for C₉H₂₂N₃O₂ 324.2]; LC/MS retention time (method F): *t*_{R} = 1.23 min. HPLC retention time (method A): *t*_{R} = 5.95 min; HPLC retention time (method B): *t*_{R} = 6.87 min. ¹H NMR (400MHz, METHANOL-d₄) δ ppm 9.52 (s, 1H), 8.82 (d, *J*=6.0 Hz, 1H), 8.51 (d, *J*=9.3 Hz, 1H), 8.42 (d, *J*=5.8 Hz, 1H), 7.25 - 7.15 (m, 2H), 4.49 (dd, *J*=10.5, 3.5 Hz, 1H), 4.30 (dd, *J*=10.8, 7.3 Hz, 1H), 3.83 (s, 3H), 3.70 (td, *J*=7.5, 3.6 Hz, 1H), 3.56 - 3.45 (m, 1H), 3.21 - 3.07 (m, 1H), 2.19 - 1.95 (m, 3H), 1.88 - 1.64 (m, 3H).

### Example 58 and Example 59

### (S)-tert-butyl (2,4-dimethyl-1-((6-methyl-5-oxo-5,6 dihydrobenzo[c][2,7]naphthyridin-8-yl)oxy)pentan-2-yl)carbamate and (R)-tert-butyl (2,4-dimethyl-1-((6-methyl-5-oxo-5,6 dihydrobenzo[c][2,7]naphthyridin-8-yl)oxy)pentan-2-yl)carbamate

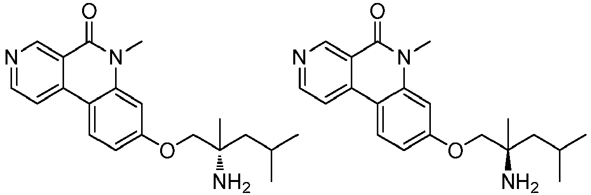

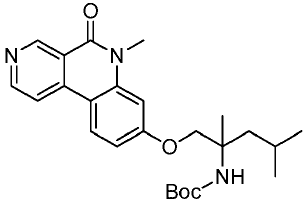

### Part A: tert-butyl (2,4-dimethyl-1-((6-methyl-5-oxo-5,6-dihydrobenzo[c][2,7]naphthyridin-8-yl)oxy)pentan-2-yl)carbamate

Synthesis of 8-chloro-6-methylbenzo[*c*][2,7]naphthyridin-5(*6H*)-one was described in example 2, Part C. 8-chloro-6-methylbenzo[*c*][2,7]naphthyridin-5(*6H*)-one (0.15 g, 0.613 mmol) and *tert*-butyl (1-hydroxy-2,4-dimethylpentan-2-yl)carbamate (0.284 g, 1.226 mmol) were subjected to ether synthesis as described in Example 16, Part H, to afford product, *tert*-butyl(2,4-dimethyl-1-((6-methyl-5-oxo-5,6-dihydrobenzo[*c*][2,7]naphthyridin-8-yl)oxy)pentan-2-yl)carbamate (0.3 g, 28% yield) as pale yellow solid. LC/MS (ESI) *m*/*e* 440.2 [(M+H)⁺, calcd for C₂₅H₃₄N₃O₄, 440.2]; LC/MS retention time (method C): *t*_{R} = 2.67 min.

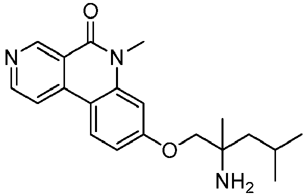

### Part B: 8-((2-amino-2,4-dimethylpentyl)oxy)-6-methylbenzo[c][2,7]naphthyridin-5(6H)-one

*tert*-Butyl (2,4-dimethyl-1-((6-methyl-5-oxo-5,6 dihydrobenzo[*c*][2,7]naphthyridin-8-yl)oxy)pentan-2-yl)carbamate was subjected to deprotection of the Boc group as described in Example 2, Part E to afford 8-((2-amino-2,4-dimethylpentyl)oxy)-6-methylbenzo[*c*][2,7]naphthyridin-5(*6H*)-one (30 mg, 12% yield) as an off-white solid. Resolution of 8-((2-amino-2,4-dimethylpentyl)oxy)-6-methylbenzo[*c*][2,7]naphthyridin-5(*6H*)-one by chiral HPLC (Co-solvent: 0.3% DEA in methanol, Column: Chiralpak AD H (250 x 21) mm 5u) resulted into two enantiomers.
Enantiomer-1: LC/MS (ESI) *m*/*e* 340.0 [(M+H)⁺, calcd for C₂₀H₂₆N₃O₂, 340.2]; LC/MS retention time (Method C): *t*_{R} = 1.88 min. HPLC retention time (method B): *t*_{R} = 9.29 min; HPLC retention time (method A): *t*_{R} = 8.19 min. ¹H NMR (400 MHz, METHANOL-d₄) 9.43 (s, 1H), 8.73 (d, *J*=6.0 Hz, 1H), 8.35 (d, *J*=8.5 Hz, 1H), 8.19 (d, *J*=6.0 Hz, 1H), 7.12 - 7.01 (m, 2H), 4.05 - 3.92 (m, 2H), 3.77 (s, 3H), 1.95 - 1.80 (m, 1H), 1.58 (qd, *J*=14.2, 5.5 Hz, 2H), 1.30 (s, 3H), 1.04 (d, *J*=6.5 Hz, 3H), 1.01 (d, *J*=6.5 Hz, 3H); HPLC retention time, *t*_{R} = 5.44 min.
Enantiomer-2: LC/MS (ESI) *m*/*e* 340.0 [(M+H)⁺, calcd for C₂₀H₂₆N₃O₂, 340.2]; LC/MS retention time (Method C): *t*_{R} = 1.88 min. HPLC retention time (method B): *t*_{R} = 9.29 min; HPLC retention time (method A): *t*_{R} = 8.19 min. ¹H NMR (400 MHz, METHANOL-d₄) 9.43 (s, 1H), 8.73 (d, *J*=6.0 Hz, 1H), 8.35 (d, *J*=8.5 Hz, 1H), 8.19 (d, *J*=6.0 Hz, 1H), 7.12 - 7.01 (m, 2H), 4.05 - 3.92 (m, 2H), 3.77 (s, 3H), 1.95 - 1.80 (m, 1H), 1.58 (qd, *J*=14.2, 5.5 Hz, 2H), 1.30 (s, 3H), 1.04 (d, *J*=6.5 Hz, 3H), 1.01 (d, *J*=6.5 Hz, 3H); HPLC retention time: *t*_{R} = 7.77 min.

### Example 60

### (S)-8-(2-amino-4-methylpentyloxy)-4-(difluoromethyl)-6-methylbenzo[c][2,7]naphthyridin-5(6H)-one

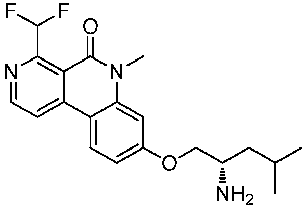

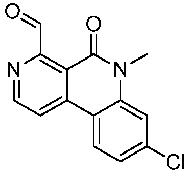

### Part A: 8-chloro-6-methyl-5-oxo-5,6-dihydrobenzo[c][2,7]naphthyridine-4-carbaldehyde

To a solution of 8-chloro-4,6-dimethylbenzo[*c*][2,7]naphthyridin-5(*6H*)-one (100 mg, 0.387 mmol), prepared as described in Example 16, Part G, in AcOH (4 mL) was added selenium dioxide (51.5 mg, 0.464 mmol). The reaction mixture was allowed to stir at 70 °C for 3h. Volatiles were removed under reduced pressure and the residue so obtained was dissolved in dichloromethane (2 mL). The mixture was filtered through diatomaceous earth (Celite®) and the filtrate was concentrated under reduced pressure to afford the title product (0.1 g, 0.202 mmol, 52% crude yield) as pale yellow solid. The material was carried on without further purification. LC/MS, (ESI) *m*/*z* 273.0 [(M+H)⁺, calcd for C₁₄H₁₀ClN₂O₂, 273.0]; LC/MS retention time (method H): *t*_{R} = 1.89 min.

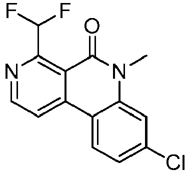

### Part B: 8-chloro-4-(difluoromethyl)-6-methylbenzo[c][2,7]naphthyridin-5(6H)-one

To the solution of 8-chloro-6-methyl-5-oxo-5,6-dihydrobenzo[*c*][2,7]naphthyridine-4-carbaldehyde (200 mg, 0.733 mmol) in DCM (1 mL) was added bis-(2-methoxyethyl)aminosulfur trifluoride (811 mg, 3.67 mmol). The tube was sealed and the reaction mixture was allowed to stir at 50° C overnight. The reaction mixture was basified with saturated aqueous NaHCO3 (15 mL) and extracted with ethyl acetate (3x10 mL). The combined organic layers were dried over Na₂SO₄ and concentrated under reduced pressure to afford 8-chloro-4-(difluoromethyl)-6-methylbenzo[*c*][2,7]naphthyridin-5(*6H*)-one (43 mg, 0.092 mmol, 13% crude yield) as a white solid. The material was carried on without further purification. LC/MS, (ESI) *m*/*z* 295.0 [(M+H)⁺, calcd for C₁₄H₁₀ClF₂N₂O, 295.0]; LC/MS retention time (Method H): *t*_{R} = 1.96 min.

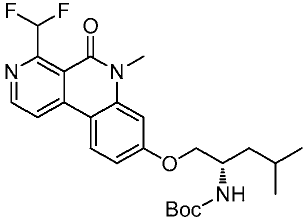

### Part C: (S)-tert-butyl (1-((4-(difluoromethyl)-6-methyl-5-oxo-5,6-dihydrobenzo[c][2,7]naphthyridin-8-yl)oxy)-4-methylpentan-2-yl)carbamate

Preparation as described in Example 16, Part H to afford the title compound (15 mg, 0.016 mmol, 19% yield). LC/MS, (ESI) *m*/*z* 476.3 [(M+H)⁺, calcd for C₂₅H₃₂F₂N₃O₄ 476.2]; LC/MS retention time (method E): *t*_{R} = 1.14 min.

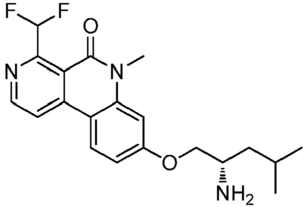

### Part D: (S)-8-((2-amino-4-methylpentyl)oxy)-4-(difluoromethyl)-6-methylbenzo[c][2,7]naphthyridin-5(6H)-one

Preparation as described in Example 2, Part E to afford title product (1.5 mg, 3.95 µmol, 9% yield) as an off-white solid. LC/MS, (ESI) *m*/*z* 376.2 [(M+H)⁺, calcd for C₂₀H₂₄F₂N₃O₂, 376.2]; LC/MS retention time (method C): *t*_{R} = 1.63 min. HPLC retention time (method A): *t*_{R} = 5.97 min; HPLC retention time (method B): *t*_{R} = 7.12 min. ¹H NMR (400 MHz, METHANOL-*d*₄) δ ppm 8.81 (d, *J*=5.52 Hz, 1H) 8.42 - 8.48 (m, 2H) 7.97 - 8.26 (m, 1H) 7.09 - 7.15 (m, 2H) 4.22 (dd, *J*=9.54, 4.02 Hz, 1H) 4.04 (dd, *J*=9.29, 7.03 Hz, 1H) 3.79 (s, 3H) 3.39 (dt, *J*=3.33, 1.73 Hz, 1H) 1.81 - 1.92 (m, 1H) 1.43 - 1.57 (m, 2H) 1.02 (dd, *J*=8.78, 6.53 Hz, 6H). ¹⁹F NMR (400 MHz, METHANOL-*d*₄) δ ppm 120.45.

### Example 61

### (S)-8-((2-amino-4-methylpentyl)oxy)-7-chloro-4,6-dimethylbenzo[c][2,7]naphthyridin-5(6H)-one

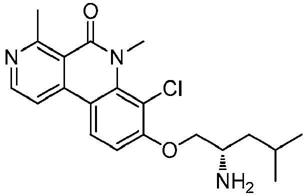

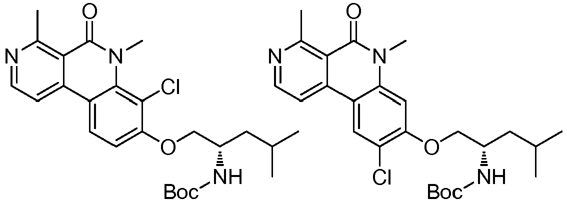

### Part A: (S)-tert-butyl (1-((7 or 9-chloro-4,6-dimethyl-5-oxo-5,6-dihydrobenzo[c][2,7]naphthyridin-8-yl)oxy)-4-methylpentan-2-yl)carbamate

To a solution of *(S)-tert*-butyl (1-((4,6-dimethyl-5-oxo-5,6-dihydrobenzo[*c*][2,7]naphthyridin-8-yl)oxy)-4-methylpentan-2-yl)carbamate (50 mg, 0.114 mmol), prepared as described in Example 16, Part H, in acetonitrile (2 mL) was added 1-chloropyrrolidine-2,5-dione (15.19 mg, 0.114 mmol). The reaction mixture was allowed to stir at 55 °C for 16 h overnight. The reaction mixture was basified with saturate aqueous sodium bicarbonate (2 mL) and water (10 mL). The solution was extracted with methylene dichloride (3 x 10 mL). The combined organic layers were washed with brine (10 mL), dried with sodium sulfate, filtered and concentrated under reduced pressure to afford a mixture of (*S*)-*tert-*butyl (1-((7-chloro-4,6-dimethyl-5-oxo-5,6-dihydrobenzo[*c*][2,7]naphthyridin-8-yl)oxy)-4-methylpentan-2-yl)carbamate and (*S*)-*tert-*butyl (1-((9-chloro-4,6-dimethyl-5-oxo-5,6-dihydrobenzo[*c*][2,7]naphthyridin-8-yl)oxy)-4-methylpentan-2-yl)carbamate (60 mg, 0.037 mmol, 32% combined crude yield) as a pale yellow solid. The material was carried on without further purification. LC/MS (ESI) *m*/*e* 474.4, [(M+H)⁺, calcd for C₂₅H₃₃ClN₃O₄, 474.2]; LC/MS retention time (method E): *t*_{R} = 1.24 min.

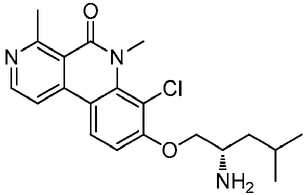

### Part B: (S)-8-((2-amino-4-methylpentyl)oxy)-7-chloro-4,6-dimethylbenzo[c][2,7]naphthyridin-5(6H)-one

To a mixture of (*S*)-*tert-*butyl (1-((7-chloro-4,6-dimethyl-5-oxo-5,6-dihydrobenzo[*c*][2,7]naphthyridin-8-yl)oxy)-4-methylpentan-2-yl)carbamate and (*S*)-*tert*-butyl (1-((9-chloro-4,6-dimethyl-5-oxo-5,6-dihydrobenzo[*c*][2,7]naphthyridin-8-yl)oxy)-4-methylpentan-2-yl)carbamate (30 mg, 0.063 mmol) was added HCl in 1,4-dioxane (241 µL, 0.964 mmol) at 0° C. The reaction mixture was allowed to stir for 2 h, then was concentrated under reduced pressure. The residue was purified by reverse phase HPLC (Symmetry C18 column (19 x 250 mm) 7.0 micron; mobile phase A: 0.1 % TFA in water; mobile phase B: acetonitrile; flow rate: 14.0 mL). The HPLC fractions were concentrated, basified with NaHCO₃ and extracted with DCM (2x). The combined organic layers were concentrated *in vacuo* to afford the purified mixture of 7-Cl and 9-Cl final products as an off-white solid. This was subjected to second purification (to resolve regioisomers) by normal phase HPLC (chiralpak ODH, (4.6 X 250 mm) 5.0 micron; mobile phase A: n-hexane; mobile phase B: ethanol); The HPLC fractions were concentrated *in vacuo* to afford the 7-chloro-regioisomer: *(S)*-8-((2-amino-4-methylpentyl)oxy)-7-chloro-4,6-dimethylbenzo[*c*][2,7]naphthyridin-5(*6H*)-one (3 mg, 7.2 umol, 11% yield) as a pale yellow solid. LC/MS, (ESI) *m*/*z* 373.8 [(M+H)⁺, calcd for C₂₀H₂₅ClN₃O₂, 374.2]; LC/MS retention time (method C): *t*_{R} = 1.64 min. HPLC retention time (method A): *t*_{R} = 8.44 min; ¹H NMR (400 MHz, DMSO-*d*₆) δ ppm 8.65 (d, *J*=5.65 Hz, 1 H) 8.44 (d, *J*=9.22 Hz, 1 H) 8.20 (d, *J*=5.58 Hz, 1 H) 7.22 (d, *J*=9.04 Hz, 1 H) 4.05 - 4.10 (m, 1 H) 3.98 (dd, *J*=9.25, 6.43 Hz, 1 H) 3.73 (s, 3 H) 3.13 - 3.18 (m, 1 H) 2.99 (s, 3 H) 1.81 - 1.89 (m, 1 H) 1.28 - 1.42 (m, 2 H) 0.91 (dd, *J*=13.80, 6.59 Hz, 2 H).

### Example 62

### (S)-8-((2-amino-4-methylpentyl)oxy)-9-chloro-4,6-dimethylbenzo[c][2,7]naphthyridin-5(6H)-one

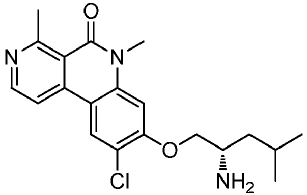

The mixture of 7-Cl and 9-Cl products prepared in Example 61, Part B was purified (to resolve regioisomers) as shown above by normal phase HPLC (chiralpak ODH, (4.6 X 250 mm) 5.0 micron; mobile phase A: n-hexane; mobile phase B: esthanol); The HPLC fractions were concentrated *in vacuo* to afford the 9-chloro-regioisomer: *(S)*-8-((2-amino-4-methylpentyl)oxy)-9-chloro-4,6-dimethylbenzo[*c*][2,7]naphthyridin-5(*6H*)-one (5 mg, 0.013 mmol, 21% yield) as an off white solid. LC/MS, (ESI) *m*/*z* 373.8 [(M+H)⁺, calcd for C₂₀H₂₅ClN₃O₂, 374.2]; LC/MS retention time (method C): *t*_{R} = 1.61 min. HPLC retention time (method A): *t*_{R} = 8.37 min; HPLC retention time (method B): *t*_{R} = 9.14 min. ¹H NMR (400 MHz, DMSO-*d*₆) δ ppm 8.63 (d, *J*=5.65 Hz, 1 H) 8.60 (s, 1 H) 8.26 (d, *J*=5.71 Hz, 1 H) 7.12 (s, 1 H) 4.11 - 4.16 (m, 1 H) 4.00 - 4.06 (m, 1 H) 3.70 (s, 3 H) 3.13-3.16 (m, 1H) 3.00 (s, 3 H) 1.82 - 1.90 (m, 1 H) 1.34 - 1.42 (m, 1 H) 1.23 - 1.32 (m, 1 H) 0.90-0.95 (m, 6 H).

### Example 63

### (S)-8-(2-amino-4-methylpentyloxy)-4,6,9-trimethylbenzo[c][2,7]naphthyridin-5(6H)-one

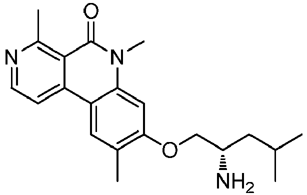

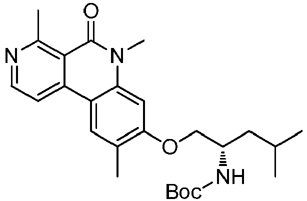

### Part A: (S)-tert-butyl (4-methyl-1-((4,6,9-trimethyl-5-oxo-5,6-dihydrobenzo[c][2,7]naphthyridin-8-yl)oxy)pentan-2-yl)carbamate

Prepared as described in Example 3, Part A and Example 4, Part A to afford *(S)-tert*-butyl (4-methyl-1-((4,6,9-trimethyl-5-oxo-5,6-dihydrobenzo[*c*][2,7]naphthyridin-8-yl)oxy)pentan-2-yl)carbamate (30 mg, 0.038 mmol, 40% yield) as a pale yellow solid. LC/MS, (ESI) *m*/*z* 453.9 [(M+H)⁺, calcd for C₂₆H₃₆N₃O₄ 454.3]; LC/MS retention time (method C): *t*_{R} = 2.16 min.

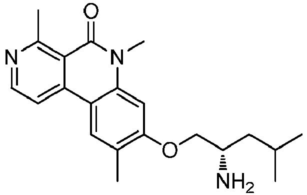

### Part B: (S)-8-((2-amino-4-methylpentyl)oxy)-4,6,9-trimethylbenzo[c][2,7]naphthyridin-5(6H)-one

Prepared as described in Example 2, Part E to afford *(S)*-8-((2-amino-4-methylpentyl)oxy)-4,6,9-trimethylbenzo[*c*][2,7]naphthyridin-5(*6H*)-one (15 mg, 0.040 mmol, 62% yield) as an off-white solid. LC/MS, (ESI) *m*/*z* 353.9 [(M+H)⁺, calcd for C₂₁H₂₈N₃O₂ 354.2]; LC/MS retention time (method C): *t*_{R} = 1.64 min. HPLC retention time (method A): *t*_{R} = 8.21 min. ¹H NMR (400 MHz, DMSO-*d*₆) δ ppm 8.59 (d, *J*=5.77 Hz, 1H) 8.27 (s, 1H) 8.15 (d, *J*=5.60 Hz, 1H) 6.92 (s, 1H) 3.90 - 4.08 (m, 2H) 3.68 (s, 3H) 3.12 - 3.20 (m, 1H) 2.99 (s, 3H) 2.24(s, 3H) 1.85 (td, *J*=13.68, 6.53 Hz, 3H) 1.22 - 1.43 (m, 3H) 0.86 - 0.95 (m, 6H).

### Example 64

### (S)-8-(2-amino-4-methylpentyloxy)-4,6-dimethyl-5-oxo-5,6-dihydrobenzo[c][2,7]naphthyridine-9-carbonitrile

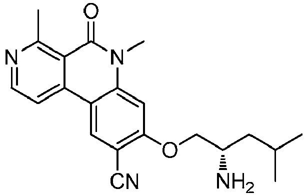

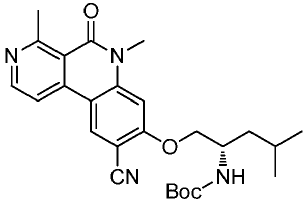

### Part A. (S)-tert-butyl 1-(9-cyano-4,6-dimethyl-5-oxo-5,6-dihydrobenzo[c][2,7]naphthyridin-8-yloxy)-4-methylpentan-2-ylcarbamate

A suspension of *(S)-tert*-butyl (1-((9-bromo-4,6-dimethyl-5-oxo-5,6-dihydrobenzo[*c*][2,7]naphthyridin-8-yl)oxy)-4-methylpentan-2-yl)carbamate (2.4 g, 4.63 mmol), prepared as described in Example 3, Part A, Pd₂(dba)₃ (0.212 g, 0.231 mmol), DPPF (0.257 g, 0.463 mmol) and zinc(II) cyanide (0.544 g, 4.63 mmol) in DMF (20 mL) and water (1 mL) was degassed with nitrogen and heated to 130 °C overnight. After cooling to room temperature, the volatiles were concentrated under reduced pressure. The residue was reconstituted in ethyl acetate and filtered through diatomaceous earth (Celite®). The organic layer was washed with H₂O, followed by brine, dried over Na₂SO₄ and concentrated under reduced pressure. The crude product was purified by silica gel column (petroleum ether and ethyl acetate) to afford *(S)-tert-*butyl 1-(9-cyano-4,6-dimethyl-5-oxo-5,6-dihydrobenzo[*c*][2,7]naphthyridin-8-yloxy)-4-methylpentan-2-ylcarbamate (1.9 g, 4.09 mmol, 88% yield) as a yellow solid. LC/MS (ESI) *m*/*e* 465.2 [(M+H)⁺, calcd for C₂₆H₃₃N₄O₄, 465.2]; LC/MS retention time (method C): *t*_{R} = 2.74 min.

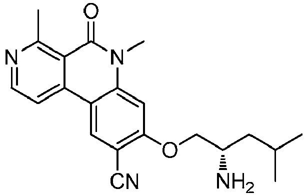

### Part B. (S)-8-(2-amino-4-methylpentyloxy)-4,6-dimethyl-5-oxo-5,6-dihydrobenzo[c][2,7]naphthyridine-9-carbonitrile

Prepared as described in Example 2, Part E to afford *(S)*-8-(2-amino-4-methylpentyloxy)-4,6-dimethyl-5-oxo-5,6-dihydrobenzo[*c*][2,7]naphthyridine-9-carbonitrile (1.6 g, 3.89 mmol, 95 % yield) as a light yellow solid. LC/MS (ESI) *m*/*e* 365.2 [(M+H)⁺, calcd for C₂₁H₂₅N₄O₂, 365.2]; LC/MS retention time (method C): *t*_{R} = 2.13 min. HPLC retention time (method A): *t*_{R} = 8.70 min; HPLC retention time (method B): *t*_{R} = 9.57 min. ¹H NMR (400 MHz, *MeOD*) δ ppm 9.07 (s, 1 H), 8.75 (s, 2 H), 7.28 (s, 1 H), 4.69 (m, 1 H), 4.55 (m, 1 H), 3.87 (m, 4 H), 3.27 (s, 3 H), 1.87 (m, 2 H), 1.74 (m, 1 H), 1.08 (m, 6 H).

### Example 65

### (S)-4,6-dimethyl-8-(4-methyl-2-(methylamino)pentyloxy)-5-oxo-5,6-dihydrobenzo[c][2,7]naphthyridine-9-carbonitrile

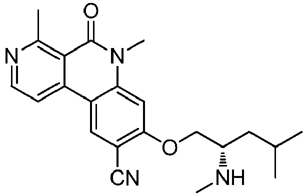

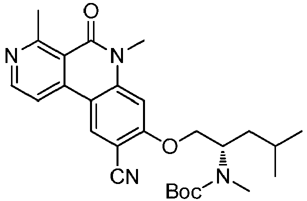

### Part A. (S)-tert-butyl (1-((9-cyano-4,6-dimethyl-5-oxo-5,6-dihydrobenzo[c][2,7]naphthyridin-8-yl)oxy)-4-methylpentan-2-yl)(methyl)carbamate

*(S)-tert*-Butyl (1-((9-cyano-4,6-dimethyl-5-oxo-5,6-dihydrobenzo[c] [2,7]naphthyridin-8-yl)oxy)-4-methylpentan-2-yl)carbamate (60 mg, 0.129 mmol), prepared as described in Example 64, Part B, was taken in DMF (3 mL) and cooled to 0°C. The reaction mixture was treated with NaH (10.33 mg, 0.258 mmol) followed by MeI (0.016 mL, 0.258 mmol). After stirring at 0°C for 10 min, the reaction mixture was warmed to room temperature and stirred overnight. The mixture was then quenched with ice-cold water and extracted with ethyl acetate (2x3 mL). The combined organic layers were dried with sodium sulfate and concentrated under reduced pressure to afford *(S)-tert*-butyl (1-((9-cyano-4,6-dimethyl-5-oxo-5,6-dihydrobenzo[*c*][2,7]naphthyridin-8-yl)oxy)-4-methylpentan-2-yl)(methyl)carbamate (70 mg, 0.127 mmol, 99% crude yield) as an off-white solid. The material was carried forward without further purification. LC/MS (ESI) *m*/*e* 479.2 [(M+H)⁺, calcd for C₂₇H₃₅N₄O₄ 479.3]; LC/MS retention time (method C): *t*_{R} = 2.16 min.

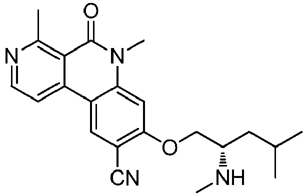

### Part B. (S)-4,6-dimethyl-8-((4-methyl-2-(methylamino)pentyl)oxy)-5-oxo-5,6-dihydrobenzo[c][2,7]naphthyridine-9-carbonitrile

Prepared as described in Example 2, Part E to afford *(S)*-4,6-dimethyl-8-((4-methyl-2-(methylamino)pentyl)oxy)-5-oxo-5,6-dihydrobenzo[c][2,7]naphthyridine-9-carbonitrile (25 mg, 0.058 mmol, 46% yield) as an off-white solid. LC/MS (ESI) *m*/*e* 379.0, [(M+H)⁺, calcd for C₂₂H₂₇N₄O₂ 379.2]; LC/MS retention time (method C): *t*_{R} = 1.87 min. HPLC retention time (method A): *t*_{R} = 8.59 min; HPLC retention time (method B): *t*_{R} = 9.88 min. ¹H-NMR (400 MHz, MeOD): δ ppm 1.03 (d, *J* = 22.80 Hz, 6H), 1.56-1.62 (m, 1H), 1.67-1.72 (m, 1H), 1.84-1.87 (m, 1H), 2.67 (s, 3H), 3.10 (s, 3H), 3.28-3.30 (m, 1H), 3.81 (s, 3H), 4.35 (s, 1H), 4.49 (s, 1H), 7.18 (s, 1H), 8.20 (d, *J* = 5.60 Hz, 1H), 8.82 (s, 1H).

### Example 66

### (S)-8-((2-amino-4-methylpentyl)oxy)-2,6-dimethylbenzo[c][2,7]naphthyridin-5(6H)-one

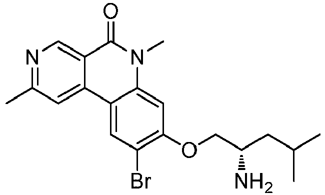

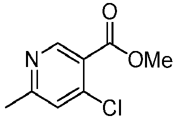

### Part A: Methyl 4-chloro-6-methylnicotinate

To a solution of methyl 4,6-dichloronicotinate (3.00 g, 14.56 mmol) and trimethylboroxine (1.097 g, 8.74 mmol) in 1,4-dioxane (70 mL) and water (7 mL) was added cesium carbonate (14.23 g, 43.7 mmol). The mixture was degassed with argon over a period of 5 minutes. PdCl₂(dppf)-CH₂Cl₂ adduct (1.189 g, 1.456 mmol) was added and the reaction mixture was heated to 110 °C for 16 h. The reaction mixture was cooled to room temperature, diluted with water (100 mL) and extracted with ethyl acetate (200 mL). The organic phase was washed with brine (2x100 mL), dried over Na₂SO₄, and concentrated under reduced pressure. The residue obtained was purified by silica gel chromatography using ethyl acetate-petroleum ether mixture to afford methyl 4-chloro-6-methylnicotinate (700 mg, 2.87 mmol, 20% yield) as a yellow liquid. LC/MS (ESI) *m*/*e* 186.0 [(M+H)⁺, calcd for C₈H₉ClNO₂ 185.6]; LC/MS retention time (Method I) *t*_{R} = 1.79 min.

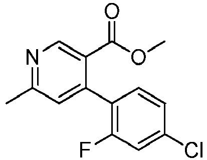

### Part B: Methyl 4-(4-chloro-2-fluorophenyl)-6-methylnicotinate

To a solution of methyl 4-chloro-6-methylnicotinate (660 mg, 3.56 mmol) and (4-chloro-2-fluorophenyl)boronic acid (620 mg, 3.56 mmol) in 1,4-dioxane (20 mL) was added cesium carbonate (3.48 g, 10.67 mmol). The reaction mixture was degassed with argon over a period of 5 min. Pd(Ph₃P)₄ (205 mg, 0.178 mmol) was added to the reaction mixture and heated to 80 °C for 16h. The mixture was cooled to room temperature, diluted with water, and extracted with ethyl acetate (100 mL). The organic phase was washed with brine (2x50 mL), dried over Na₂SO₄, and concentrated under reduced pressure. The residue obtained was purified by silica gel chromatography to afford methyl 4-(4-chloro-2-fluorophenyl)-6-methylnicotinate (500 mg, 1.53 mmol, 43% yield) as a yellow liquid. LC/MS (ESI) *m*/*e* 280.0 [(M+H)⁺, calcd for C₁₄H₁₂ClFNO₂ 279.6]; LC/MS retention time (method C): *t*_{R} = 1.87 min.

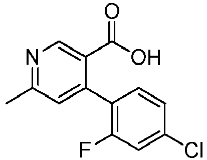

### Part C: 4-(4-chloro-2-fluorophenyl)-6-methylnicotinic acid

To a solution of methyl 4-(4-chloro-2-fluorophenyl)-6-methylnicotinate (700 mg, 2.50 mmol) in MeOH (5 mL) was added NaOH (200 mg, 7.51 mmol) in water (5 mL). The reaction mixture was stirred at room temperature for 2h. The solvent was evaporated under reduced pressure and the residue obtained was adjusted to pH ∼3 by adding 1.5N HCl. The meaction mxture was extracted with dichloromethane. The organic phase was dried over Na₂SO₄, and concentrated under reduced pressure to afford 4-(4-chloro-2-fluorophenyl)-6-methylnicotinic acid (500 mg, 1.730 mmol, 92 % crude yield) as white solid. The material was carried forward without further purification. LC/MS (ESI) *m*/*e* 266.0 [(M+H)⁺, calcd for C₁₃H₁₀ClFNO₂ 266.03]; LC/MS (Method I) *t*_{R} = 1.51 min.

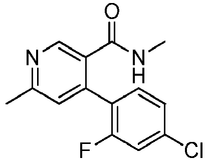

### Part D: 4-(4-chloro-2-fluorophenyl)-N,6-dimethylnicotinamide

To a solution of 4-(4-chloro-2-fluorophenyl)-6-methylnicotinic acid (500 mg, 1.88 mmol) in dichloromethane (10 mL) at 0 °C was added oxalyl chloride (0.988 mL, 11.29 mmol) drop wise followed by DMF (0.2 mL). The reaction mixture was stirred at room temperature for 4h. The reaction mixture was evaporated to dryness. The residue was taken in up dichloromethane (50 mL) and cooled to 0°C. Methylamine hydrochloride (1.27 g, 18.82 mmol) was added to followed by triethylamine (2.62 mL, 18.82 mmol) and the reaction mixture was stirred at room temperature for 2h. The reaction mixture was diluted with water (50 mL) and extracted with dichloromethane (100 mL). The organic layer was separated, dried over Na₂SO₄ and concentrated under reduced pressure to afford 4-(4-chloro-2-fluorophenyl)-*N*,6-dimethylnicotinamide (520 mg, 1.43 mmol, 76% crude yield) as yellow solid. The material was carried forward without further purification. LC/MS (ESI) *m*/*e* 279.0 [(M+H)⁺, calcd for C₁₄H₁₃ClFN₂O 279.1]; LC/MS retention time ((Method I) *t*_{R} = 1.82 min.

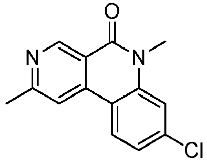

### Part E: 8-chloro-2,6-dimethylbenzo[c][2,7]naphthyridin-5(6H)-one

To a solution of 4-(4-chloro-2-fluorophenyl)-*N*,6-dimethylnicotinamide (520 mg, 1.86 mmol) in tetrahydrofuran (20 mL) at 0 °C was added NaH (224 mg, 5.60 mmol). The reaction mixture was brought to room temperature gradually and stirred at room temperature for 15h. The reaction mixture was diluted with water (50 mL) and extracted with ethyl acetate (100 mL). The organic layer was separated, dried over Na₂SO₄ and concentrated under reduced pressure to afford 8-chloro-2,6 dimethylbenzo[*c*][2,7]naphthyridin-5(*6H*)-one (480 mg, 1.51 mmol, 81% yield) as a brick-red solid. LC/MS (ESI) *m*/*e* 259.0 [(M+H)⁺, calcd for C₁₄H₁₂ClN₂O 259.05]; LC/MS retention time (method C): *t*_{R} = 1.91 min. ¹H NMR (400 MHz, METHANOL-*d*₄) δ ppm 9.41 (s, 1 H), 8.48 (d, *J*=8.59 Hz, 1 H), 8.23 (s, 1 H), 7.69 (d, *J*=1.98 Hz, 1 H), 7.44 (dd, *J*=8.64, 1.94 Hz, 1 H), 3.78 (s, 3 H), 2.75 (s, 3 H).

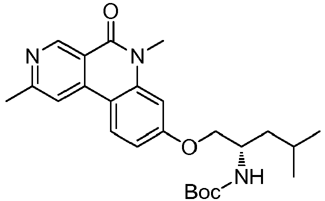

### Part F: (S)-tert-butyl (1-((2,6-dimethyl-5-oxo-5,6-dihydrobenzo[c][2,7]naphthyridin-8-yl)oxy)-4-methylpentan-2-yl)carbamate

To the stirred solution of 8-chloro-2,6-dimethylbenzo[*c*][2,7]naphthyridin-5(*6H*)-one (100 mg, 0.387 mmol) and *tert*-butyl (1-hydroxy-2,4-dimethylpentan-2-yl)carbamate (168 mg, 0.773 mmol) in toluene (5 mL) was added cesium carbonate (189 mg, 0.580 mmol) and 2-di-*t*-butylphosphino-2',4',6'-tri-*i*-propyl-1,1'-biphenyl (9.85 mg, 0.023 mmol). The reaction mixture was degassed with argon for 5 minutes and palladium(II) acetate (26 mg, 0.116 mmol) was added. The reaction mixture was heated to 100 °C for 18h. The reaction mixture was filtered through diatomaceous earth (Celite®) and the filtrate was concentrated under reduced pressure. The residue was purified by silica gel chromatography using ethyl acetate-petroleum ether to afford *(S)-tert-butyl* (1-((2,6-dimethyl-5-oxo-5,6 dihydrobenzo[c][2,7]naphthyridin-8-yl)oxy)-4-methylpentan-2-yl)carbamate (100 mg, 0.171 mmol, 44% yield) as a dark yellow semi-solid. LC/MS (ESI) *m*/*e* 440.6 [(M+H+)⁺, calcd for C₂₅H₃₄N₃O₄ 440.2]; LC/MS retention time (method D): *t*_{R} = 0.89 min.

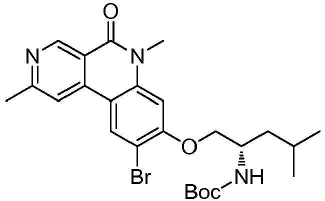

### Part G: (S)-tert-butyl (1-((9-bromo-2,6-dimethyl-5-oxo-5,6-dihydrobenzo[c][2,7]naphthyridin-8-yl)oxy)-4-methylpentan-2-yl)carbamate

To the stirred solution of *(S)-tert-butyl* (1-((2,6-dimethyl-5-oxo-5,6 dihydrobenzo[*c*][2,7]naphthyridin-8-yl)oxy)-4-methylpentan-2-yl)carbamate (40 mg, 0.069 mmol) in acetonitrile (3 mL) at room temperature was added NBS (12 mg, 0.069 mmol). The reaction mixture was heated to reflux for 2h. The reaction mixture was diluted with water (50 mL) and extracted with dichloromethane (100 mL). The organic layer was separated, dried over Na₂SO₄ and concentrated under reduced pressure to afford *(S)-tert*-butyl (1-((9-bromo-2,6-dimethyl-5-oxo-5,6-dihydrobenzo[*c*][2,7]naphthyridin-8-yl)oxy)-4-methylpentan-2-yl)carbamate (30 mg, 0.027 mmol, 40% crude yield) as a yellow oil. The material was carried forward without further purification. LC/MS (ESI) *m*/*e* 518.2 [(M+H)⁺, calcd for C₂₅H₃₃BrN₃O₄ 518.2]; LC/MS retention time (method E): *t*_{R} = 1.25 min.

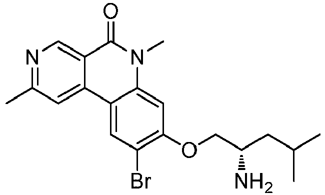

### Part H: (S)-8-((2-amino-4-methylpentyl)oxy)-2,6-dimethylbenzo[c][2,7]naphthyridin-5(6H)-one

To a solution of *(S)-tert*-butyl (1-((9-bromo-2,6-dimethyl-5-oxo-5,6-dihydrobenzo[*c*][2,7]naphthyridin-8-yl)oxy)-4-methylpentan-2-yl)carbamate (30 mg, 0.058 mmol) in MeOH (3 mL) at 0 °C was added 4M HCl in 1,4-dioxane (0.362 mL, 1.447 mmol). The reaction mixture was stirred at 0 °C for 2h. The reaction mixture was concentrated under reduced pressure to afford crude product which was purified by preparative HPLC to afford *(S)*-8-((2-amino-4-methylpentyl)oxy)-2,6-dimethylbenzo[*c*][2,7]naphthyridin-5(*6H*)-one (6 mg, 0.014 mmol, 24% yield) as an off-white solid. LC/MS (ESI) *m*/*e* 418.0 [(M+H)⁺, calcd for C₂₀H₂₅BrN₃O₂ 418.1]; LC/MS retention time (method H): *t*_{R} = 1.70 min; HPLC retention time (method A): *t*_{R} = 6.33 min; HPLC retention time (method B): *t*_{R} = 7.19 min. ¹H NMR (400 MHz, METHANOL-*d*₄) δ ppm 9.31 (s, 1 H), 9.28 - 9.33 (m, 1 H), 8.58 (s, 1 H), 8.08 (s, 1 H), 7.09 (s, 1 H), 4.42 (dd, *J*=9.79, 3.26 Hz, 1 H), 4.25 (dd, *J*=9.91, 6.15 Hz, 1 H), 3.78 (s, 3 H), 3.63 (br. s., 1 H), 2.73 (s, 3 H), 2.00 (br. s., 4 H), 1.85 - 1.92 (m, 1 H), 1.71 - 1.80 (m, 1 H), 1.57 - 1.66 (m, 1 H), 1.06 (dd, *J*=6.40, 4.64 Hz, 6 H).

### Example 67

### (S)-8-((2-amino-4-methylpentyl)oxy)-2,6dimethylbenzo[c][2,7]naphthyridin-5(6H)-one

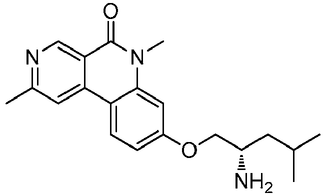

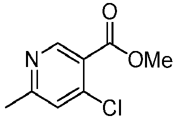

### Part A: Methyl 4-chloro-6-methylnicotinate

To a solution of methyl 4,6-dichloronicotinate (3.00 g, 14.56 mmol) and trimethylboroxine (1.097 g, 8.74 mmol) in 1,4-dioxane (70 mL) and water (7 mL), was added cesium carbonate (14.23 g, 43.7 mmol). The mixture was degassed with argon over a period of 5 min. PdCl₂(dppf)-CH₂Cl₂ adduct (1.189 g, 1.456 mmol) was added to the reaction mixture and heated to 110 °C for 16h. The reaction mixture was cooled to room temperature, diluted with water (100 mL) and extracted with ethyl acetate (200 mL). The organic phase was washed with brine (2 x 100 mL), dried over Na₂SO₄, and concentrated under reduced pressure. The residue obtained was purified by silica gel chromatography using ethyl acetate-petroleum ether mixture to afford methyl 4-chloro-6-methylnicotinate (700 mg, 2.87 mmol, 20% yield) as a yellow liquid. LC/MS (ESI) *m*/*e* 186.0 [(M+H)⁺, calcd for C₈H₉ClNO₂ 186.0]; LC/MS retention time (Method I): *t*_{R} = 1.79 min.

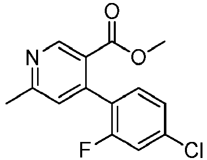

### Part B: Methyl 4-(4-chloro-2-fluorophenyl)-6-methylnicotinate

To a solution of methyl 4-chloro-6-methylnicotinate (660 mg, 3.56 mmol) and (4-chloro-2-fluorophenyl)boronic acid (620 mg, 3.56 mmol) in 1,4-dioxane (20 mL) was added cesium carbonate (3.48 g, 10.67 mmol). The reaction mixture was degassed with argon over a period of 5 min. Pd(Ph₃P)₄ (205 mg, 0.178 mmol) was added to the reaction mixture and heated to 80 °C for 16h. The mixture was cooled to room temperature, diluted with water, and extracted with ethyl acetate (100 mL). The organic phase was washed with brine (2 x 50 mL), dried over Na₂SO₄, and concentrated under reduced pressure. The residue obtained was purified by silica gel chromatography to afford Methyl 4-(4-chloro-2-fluorophenyl)-6-methylnicotinate (500 mg, 1.53 mmol, 43% yield) as a yellow liquid. LC/MS (ESI) *m*/*e* 280.0 [(M+H)⁺, calcd for C₁₄H₁₂ClFNO₂ 280.0]; LC/MS retention time (Method C): *t*_{R} = 1.87 min.

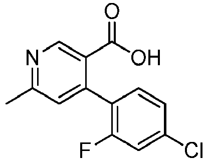

### Part C: 4-(4-chloro-2-fluorophenyl)-6-methylnicotinic acid

To a solution of Methyl 4-(4-chloro-2-fluorophenyl)-6-methylnicotinate (700 mg, 2.50 mmol) in MeOH (5 mL) was added NaOH (200 mg, 7.51 mmol) in water (5 mL). The reaction mixture was stirred at room temperature for 2h. Then it was evaporated under reduced pressure and the residue obtained was adjusted to pH ∼3 by adding 1.5N HCl solution. The product was extracted with dichloromethane. The organic phase was dried over Na₂SO₄, and concentrated under reduced pressure to afford 4-(4-chloro-2-fluorophenyl)-6-methylnicotinic acid (500 mg, 1.730 mmol, 69% crude yield) as white solid. The material was carried forward without further purification. LC/MS (ESI) *m*/*e* 266.0 [(M+H)⁺, calcd for C₁₃H₁₀ClFNO₂ 266.0]; LC/MS retention time (Method I): *t*_{R} = 1.51 min.

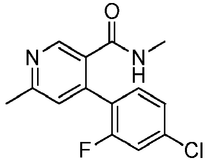

### Part D: 4-(4-chloro-2-fluorophenyl)-N,6-dimethylnicotinamide

To a solution of 4-(4-chloro-2-fluorophenyl)-6-methylnicotinic acid (500 mg, 1.88 mmol) in dichloromethane (10 mL) at 0 °C was added oxalyl chloride (0.988 mL, 11.29 mmol) drop wise followed by DMF (0.2 mL). The reaction mixture was stirred at room temperature for 4h. The reaction mixture was evaporated to dryness. The residue was taken in dichloromethane (50 mL) and cooled to 0 °C. Methylamine hydrochloride (1.27 g, 18.82 mmol) and triethylamine (2.62 mL, 18.82 mmol) were added. The reaction mixture was stirred at room temperature for 2h. The reaction mixture was diluted with water (50 mL) and extracted with dichloromethane (100 mL). The organic layer was separated, dried over Na₂SO₄ and concentrated under reduced pressure to afford 4-(4-chloro-2-fluorophenyl)-*N*,6-dimethylnicotinamide (520 mg, 1.43 mmol, 76% crude yield) as yellow solid. The material was carried forward without further purification. LC/MS (ESI) *m*/*e* 279.0 [(M+H)⁺, calcd for C₁₄H₁₃ClFN₂O 279.1]; LC/MS retention time (Method I): *t*_{R} = 1.82 min.

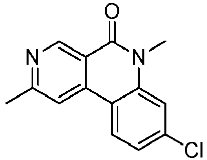

### Part E: 8-chloro-2,6-dimethylbenzo[c][2,7]naphthyridin-5(6H)-one

To a solution of 4-(4-chloro-2-fluorophenyl)-*N*,6-dimethylnicotinamide (520 mg, 1.86 mmol) in tetrahydrofuran (20 mL) at 0 °C was added NaH (224 mg, 5.60 mmol). The reaction mixture was brought to room temperature gradually and stirred at room temperature for 15h. The reaction mixture was diluted with water (50 mL) and extracted with ethyl acetate (100 mL). The organic layer was separated, dried over Na₂SO₄ and concentrated under reduced pressure to afford product, 8-chloro-2,6 dimethylbenzo[*c*][2,7]naphthyridin-5(*6H*)-one (480 mg, 1.51 mmol, 81% crude yield) as a red solid. The material was carried forward without further purification. LC/MS (ESI) *m*/*e* 259.0 [(M+H)⁺, calcd for C₁₄H₁₂ClN₂O 259.0]; LC/MS retention time (Method C): *t*_{R} = 1.74 min. ¹H NMR (300 MHz, METHANOL-*d*₄) δ ppm 9.41 (s, 1 H), 8.48 (d, *J*=8.59 Hz, 1 H), 8.23 (s, 1 H), 7.69 (d, *J*=1.98 Hz, 1 H), 7.44 (dd, *J*=8.64, 1.94 Hz, 1 H), 3.78 (s, 3 H), 2.75 (s, 3 H).

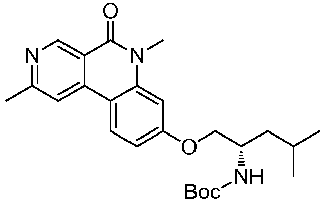

### Part F: (S)-tert-butyl (1-((2,6-dimethyl-5-oxo-5,6-dihydrobenzo[c][2,7]naphthyridin-8-yl)oxy)-4-methylpentan-2-yl)carbamate

To the stirred solution of 8-chloro-2,6-dimethylbenzo[*c*][2,7]naphthyridin-5(*6H*)-one (100 mg, 0.387 mmol) and *tert*-butyl (1-hydroxy-2,4-dimethylpentan-2-yl)carbamate (168 mg, 0.773 mmol) in toluene (5 mL) was added cesium carbonate (189 mg, 0.580 mmol) and 2-di-*t*-butylphosphino-2',4',6'-tri-*i*-propyl-1,1'-biphenyl (9.85 mg, 0.023 mmol). The reaction mixture was degassed with argon for 5 min and palladium(II) acetate (26 mg, 0.116 mmol) was added to it. The reaction mixture was heated to 100 °C for 18h. The reaction mixture was filtered through diatomaceous earth (Celite®) and the filtrate was evaporated. The residue obtained was purified by silica gel chromatography using ethyl acetate-petroleum ether to afford product, *(S)-tert*-butyl (1-((2,6-dimethyl-5-oxo-5,6 dihydrobenzo[*c*][2,7]naphthyridin-8-yl)oxy)-4-methylpentan-2-yl)carbamate (100 mg, 0.171 mmol, 44% yield) as dark yellow semi-solid. LC/MS (ESI) *m*/*e* 440.7 [(M+H)⁺, calcd for C₂₅H₃₄N₃O₄ 440.2]; LC/MS retention time (method D): *t*_{R} = 0.89 min.

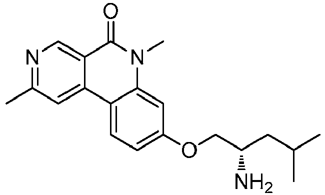

### Part G. (S)-8-((2-amino-4-methylpentyl)oxy)-2,6 dimethylbenzo[c][2,7]naphthyridin-5(6H)-one

*(S)-tert*-Butyl (1-((2,6-dimethyl-5-oxo-5,6-dihydrobenzo[*c*][2,7]naphthyridin-8-yl)oxy)-4-methylpentan-2-yl)carbamate (0.05 g, 0.114 mmol) was subjected to deprotection of the Boc group as described in Example 2, Part E, to give *(S)*-8-((2-amino-4-methylpentyl)oxy)-2,6 dimethylbenzo[*c*][2,7]naphthyridin-5(*6H*)-one (13 mg, 0.037 mmol, 33%) as colorless semi-solid. LC/MS (ESI) *m*/*e* 340.2 [(M+H)⁺, calcd for C₂₀H₂₆N₃O₂ 340.2]; LC/MS retention time (Method I): *t*_{R} = 1.62 min. HPLC retention time (method A): *t*_{R} = 8.17 min; HPLC retention time (method B): *t*_{R} = 7.50 min. ¹H NMR (400 MHz, METHANOL-*d*₄) ppm 9.33 (s, 1H), 8.38 (d, *J*=8.78 Hz, 1H), 8.34 - 8.41 (m, 1H), 8.10 (s, 1H), 8.06 - 8.12 (m, 1H), 7.04 - 7.13 (m, 2H), 4.17 (dd, *J*=9.29, 4.02 Hz, 1H), 3.99 (dd, *J*=9.29, 7.28 Hz, 1H), 3.77 (s, 3H), 3.66 (d, *J*=2.76 Hz, 1H), 2.72 (s, 3H), 1.80 - 1.92 (m, 1H), 1.39 - 1.54 (m, 2H), 1.01 (dd, *J*=9.29, 6.53 Hz, 7H).

### Example 68

### (S)-8-((2-amino-4-methylpentyl)oxy)-9-chloro-2,6-dimethylbenzo[c][2,7]naphthyridin-5(6H)-one

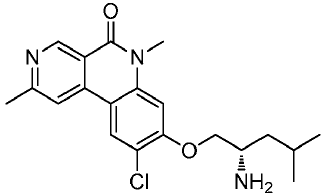

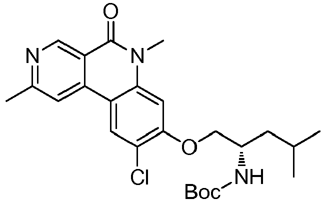

### Part A. (S)-tert-butyl (1-((9-chloro-2,6-dimethyl-5-oxo-5,6-dihydrobenzo[c][2,7]naphthyridin-8-yl)oxy)-4-methylpentan-2-yl)carbamate

*(S)-tert*-butyl (1-((2,6-dimethyl-5-oxo-5,6-dihydrobenzo[*c*][2,7]naphthyridin-8-yl)oxy)-4-methylpentan-2-yl)carbamate (0.05 g, 0.114 mmol), prepared as described in Example 67, Part F, was subjected to chlorination using NCS (0.018 g, 0.137 mmol), using the procedure described in Example 20, Part A, to give *(S)-tert-butyl* (1-((9-chloro-2,6-dimethyl-5-oxo-5,6-dihydrobenzo[*c*][2,7]naphthyridin-8-yl)oxy)-4-methylpentan-2-yl)carbamate (50 mg, 0.052 mmol, 45% yield) as colorless oil. LC/MS (ESI) *m*/*e* 474.7 [(M+H)⁺, calcd for C₂₅H₃₂ClN₃O₄ 474.2]; LC/MS retention time (method H): *t*_{R} = 0.94 min.

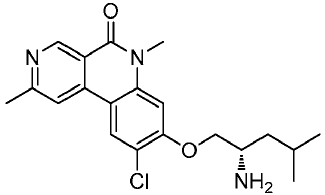

### Part B. (S)-8-((2-amino-4-methylpentyl)oxy)-9-chloro-2,6-dimethylbenzo[c][2,7]naphthyridin-5(6H)-one

*(S)-tert*-Butyl (1-((9-chloro-2,6-dimethyl-5-oxo-5,6 dihydrobenzo[*c*][2,7]naphthyridin-8-yl)oxy)-4-methylpentan-2-yl)carbamate (0.04 g, 0.041 mmol) was subjected to deprotection of the Boc group using the procedure described in Example 2, Part E, to afford *(S)*-8-((2-amino-4-methylpentyl)oxy)-9-chloro-2,6-dimethylbenzo[*c*][2,7]naphthyridin-5(*6H*)-one (0.006 g, 0.015 mmol, 37% yield) as an off-white solid . LC/MS (ESI) *m*/*e* 373.8 [(M+H)⁺, calcd for C₂₀H₂₅ClN₃O₂ 374.2]; LC/MS retention time (Method C): *t*_{R} = 1.55 min; HPLC retention time (Method A): *t*_{R} = 10.18 min; HPLC retention time (Method B): *t*_{R} = 15.48 min. ¹H NMR (400 MHz, METHANOL-*d*₄) ppm 9.37 (s, 1H), 8.46 - 8.53 (m, 1H), 8.14 (s, 1H), 7.19 (s,1H), 4.23 - 4.31 (m, 1H), 4.03 - 4.11 (m, 1H), 3.78 - 3.88 (m, 3H) 2.73 (s, 1H), 2.70 - 2.77 (m, 3H), 1.81 -1.90 (m, 1H), 1.44 - 1.58 (m, 2H), 0.96 - 1.06 (m, 6H).

### Example 69

### (S)-8-(2-amino-3-methoxypropoxy)-6-methylbenzo[c][2,7]naphthyridin-5(6H)-one

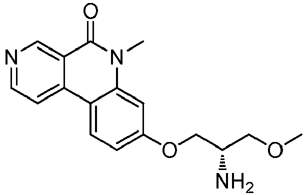

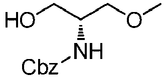

### Part A: (R)-2-amino-3-methoxypropan-1-ol

Preparation as described in the literature Kim, H. J. et.al. Bioorg Med. Chem. Lett., 2011, 21, 3809 - 3812

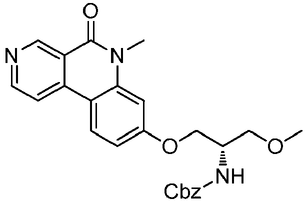

### Part B: (S)-benzyl (1-methoxy-3-((6-methyl-5-oxo-5,6-dihydrobenzo[c][2,7]naphthyridin-8-yl)oxy)propan-2-yl)carbamate

Preparation as described in Example 16, Part H to afford the title product (0.12 g, 0.145 mmol, 24% yield) yellow gum. LC/MS, (ESI) *m*/*z* 448.6 [(M+H)⁺, calcd for C₂₅H₂₆N₃O₅, 448.5]; LC/MS retention time (method D): *t*_{R} = 0.74 min.

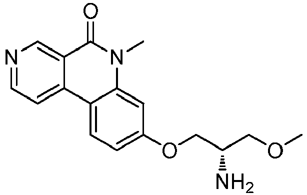

### Part C: (S)-8-(2-amino-3-methoxypropoxy)-6-methylbenzo[c][2,7]naphthyridin-5(6H)-one

Prepared as described in Example 2, Part E to afford the title product (14 mg, 0.042 mmol, 70% yield) as a brown gum. LC/MS, (ESI) *m*/*z* 314.2 [(M+H)⁺, calcd for C₁₇H₂₀N₃O₃, 314.1]; LC/MS retention time (method C): *t*_{R} = 1.38 min. HPLC retention time (method A): *t*_{R} = 5.29 min; HPLC retention time (method B): *t*_{R} = 5.31 min. ¹H NMR (400 MHz, METHANOL-*d*₄) δ ppm 9.47 (s, 1 H) 8.77 (d, *J*=5.77 Hz, 1 H) 8.42 (d, *J*=8.78 Hz, 1 H) 8.25 (d, *J*=5.77 Hz, 1 H) 7.04 - 7.17 (m, 2 H) 4.26 (dd, *J*=9.29, 4.77 Hz, 1 H) 4.10 - 4.19 (m, 1 H) 3.81 (s, 3 H) 3.52 - 3.65 (m, 2 H) 3.44 (s, 3 H) 3.38 (dt, *J*=3.33, 1.73 Hz, 1 H).

### Example 70

### (S)-8-(2-amino-4-methylpentyloxy)-9-fluoro-4,6-dimethylbenzo[c][2,7]naphthyridin-5(6H)-one

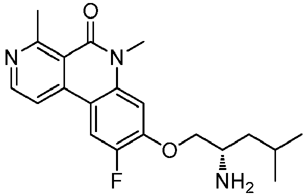

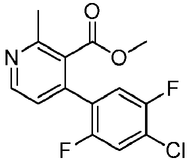

### Part A. Methyl 4-(4-chloro-2,5-difluorophenyl)-2-methylnicotinate

Prepared as described in Example 16, Part D by carrying out a Suzuki coupling between methyl 4-chloro-2-methylnicotinate (prepared as described in Example 14, Part A) and 2-(4-chloro-2,5-difluorophenyl)-4,4,5,5-tetramethyl-1,3,2-dioxaborolane (prepared as described in Example 16, Part A-Part C) to afford methyl 4-(4-chloro-2,5-difluorophenyl)-2-methylnicotinate (3 g, 4.13 mmol, 24% yield) as a brown oil. ¹H NMR (400 MHz, *CDCl*₃) δ ppm 8.60 (d, *J* = 5.2 Hz, 1 H), 7.36 (m, 1 H), 7.20 (m, 2 H), 3.74 (s, 3 H), 2.49 (s, 3 H).

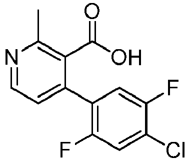

### Part B. 4-(4-chloro-2,5-difluorophenyl)-2-methylnicotinic acid

Prepared as described in Example 16, Part E by carrying out hydrolysis of Methyl 4-(4-chloro-2,5-difluorophenyl)-2-methylnicotinate to afford 4-(4-chloro-2,5-difluorophenyl)-2-methylnicotinic acid (1.7 g, 4.02 mmol, 97 % yield) as a white solid. ¹H NMR (400 MHz, *DMSO*-*d*₆) δ ppm 13.38 (br. s., 1H), 8.58 (d, *J=* 5.2 Hz, 1H), 7.74 (m, 1H), 7.53 (m, 1H), 7.41 (d, *J* = 4.8 Hz, 1H), 2.43 (s, 3H).

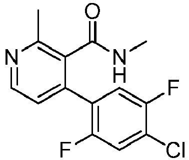

### Part C. 4-(4-chloro-2,5-difluorophenyl)-N,2-dimethylnicotinamide

To the stirred solution of 4-(4-chloro-2,5-difluorophenyl)-2-methylnicotinic acid (1.7 g, 4.02 mmol) in DMF (0.5 mL) was added DIEA (2.81 mL, 16.06 mmol), HOBT (1.230 g, 8.03 mmol), EDC (1.155 g, 6.02 mmol) at 0 °C followed by addition of methylamine hydrochloride (0.488 g, 7.23 mmol). After stirring for 10 min, the ice bath was removed and the reaction mixture was allowed to stir overnight. The reaction mixture was concnetrated under reduced pressure. The residue was diluted with ethyl acetate (10 mL) and washed with water (10 mL) followed by brine (10 mL). The organic layer was separated and dried over sodium sulphate, filtered, and concentrated under reduced pressure to afford 4-(4-chloro-2,5-difluorophenyl)-*N*,2-dimethylnicotinamide (1 g, 2.123 mmol, 53% crude yield) as a brown solid which was taken to the next step without purification. LC/MS (ESI) *m*/*e* 297.3 [(M+H)⁺, calcd for C₁₄H₁₂ClF₂N₂O 297.1]; LC/MS retention time (method B): *t*_{R} = 0.76 min.

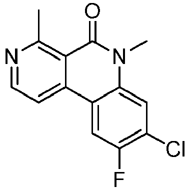

### Part D. 8-chloro-9-fluoro-4,6-dimethylbenzo[c][2,7]naphthyridin-5(6H)-one

Prepared as described in Example 16, Part G from 4-(4-chloro-2,5-difluorophenyl)-2-methylnicotinic acid to afford 8-chloro-9-fluoro-4,6 dimethylbenzo[*c*][2,7]naphthyridin-5(*6H*)-one (530 mg, 1.532 mmol, 72% yield) as a white solid. LC/MS (ESI) *m*/*e* 277.2 [(M+H)⁺, calcd for C₁₄H₁₁ClFN₂O 277.04]; LC/MS retention time (method C): *t*_{R} = 1.83 min.

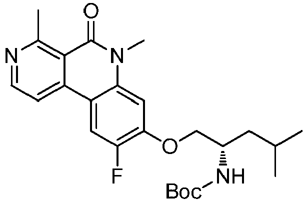

### Part E. (S)-tert-butyl 1-(9-fluoro-4,6-dimethyl-5-oxo-5,6-dihydrobenzo[c][2,7]naphthyridin-8-yloxy)-4-methylpentan-2-ylcarbamate

Prepared as described in Example 16, Part H from 8-chloro-9-fluoro-4,6-dimethylbenzo[*c*][2,7]naphthyridin-5(*6H*)-one to afford *(S)-tert-*butyl 1-(9-fluoro-4,6-dimethyl-5-oxo-5,6-dihydrobenzo[*c*][2,7]naphthyridin-8-yloxy)-4-methylpentan-2-ylcarbamate (350 mg, 0.367 mmol, 24 %) as an oil. LC/MS (ESI) *m*/*e* 458.6 [(M+H)⁺, calcd for C₂₅H₃₃FN₃O₄ 458.23]; LC/MS retention time (method B): *t*_{R} = 1.24 min.

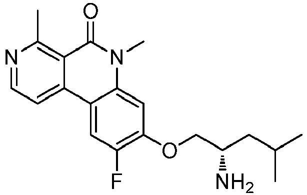

### Part F. (S)-8-(2-amino-4-methylpentyloxy)-9-fluoro-4,6-dimethylbenzo[c][2,7]naphthyridin-5(6H)-one

Prepared as described in Example 16, Part I from *(S)-tert-*butyl 1-(9-fluoro-4,6-dimethyl-5-oxo-5,6-dihydrobenzo[*c*][2,7]naphthyridin-8-yloxy)-4-methylpentan-2-ylcarbamate to afford *(S)*-8-(2-amino-4-methylpentyloxy)-9-fluoro-4,6-dimethylbenzo[*c*][2,7]naphthyridin-5(*6H*)-one (57.6 mg, 0.095 mmol, 91 %) as an yellow oil. LC/MS (ESI) *m*/*e* 358.2 [(M+H)⁺, calcd for C₂₀H₂₅FN₃O₂ 358.2]; LC/MS retention time (method C): *t*_{R} = 1.53 min; HPLC retention time (method B): *t*_{R} = 8.16 min. ¹H NMR (400MHz, METHANOL-*d*₄) δ ppm 8.58 (d, *J*=5.8 Hz, 1H), 8.23 (d, *J*=12.0 Hz, 1H), 8.09 (d, *J*=5.0 Hz, 1H), 7.19 (d, *J*=6.8 Hz, 1H), 4.26 (dd, *J*=9.5, 4.0 Hz, 1H), 4.08 (t, *J*=8.2 Hz, 1H), 3.79 (s, 3H), 3.41 - 3.38 (m, 1H), 3.10 (s, 3H), 1.94 - 1.82 (m, 1H), 1.58 - 1.39 (m, 2H), 1.04 (d, *J*=6.5 Hz, 3H), 1.01 (d, *J*=6.5 Hz, 3H).

### Example 71

### (S)-8-(2-amino-4-methylpentyloxy)-9-(hydroxymethyl)-4,6-dimethylbenzo[c][2,7]naphthyridin-5(6H)-one

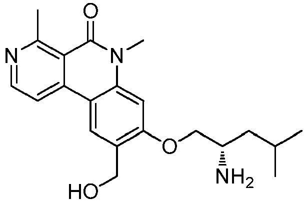

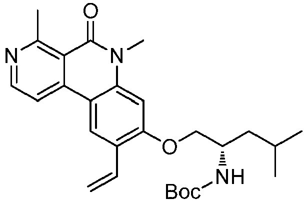

### Part A. (S)-tert-butyl 1-(4,6-dimethyl-5-oxo-9-vinyl-5,6-dihydrobenzo[c][2,7]naphthyridin-8-yloxy)-4-methylpentan-2-ylcarbamate

Prepared as described in Example 64, Part A and Example 46, Part B from *(S)-tert-butyl* 1-(9-bromo-4,6-dimethyl-5-oxo-5,6-dihydrobenzo[*c*][2,7]naphthyridin-8-yloxy)-4-methylpentan-2-ylcarbamate to afford the title product (1.2 g, 1.985 mmol, 64% yield). LC/MS (ESI) *m*/*e* 466.2 [(M+H)⁺, calcd for C₂₇H₃₆N₃O₄ 466.3]; LC/MS retention time (method B): *t*_{R} = 1.79 min.

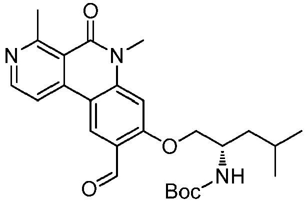

### Part B. (S)-tert-butyl 1-(9-formyl-4,6-dimethyl-5-oxo-5,6-dihydrobenzo[c][2,7]naphthyridin-8-yloxy)-4-methylpentan-2-ylcarbamate

Prepared as described in Example 55, Part B from *(S)-tert-*butyl 1-(4,6-dimethyl-5-oxo-9-vinyl-5,6-dihydrobenzo[*c*][2,7]naphthyridin-8-yloxy)-4-methylpentan-2-ylcarbamate to afford the title product (1.2 g, 0.980 mmol, 49% yield) as a yellow gum. LC/MS (ESI) *m*/*e* 468.2 [(M+H)⁺, calcd for C₂₆H₃₄N₃O₅ 468.2]; LC/MS retention time (method B): *t*_{R} = 1.66 min.

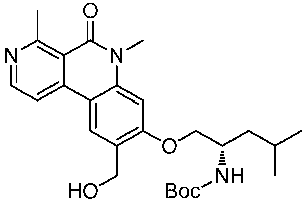

### Part C. (S)-tert-butyl 1-(9-(hydroxymethyl)-4,6-dimethyl-5-oxo-5,6-dihydrobenzo[c][2,7]naphthyridin-8-yloxy)-4-methylpentan-2-ylcarbamate

Prepared as described in Example 31, Part E to afford the title product (0.100 g, 0.160 mmol, 87 % yield) as a brown gum. LC/MS (ESI) *m*/*e* 470.2 [(M+H)⁺, calcd for C₂₆H₃₆N₃O₅ 470.2]; LC/MS retention time (method B): *t*_{R} = 1.48 min.

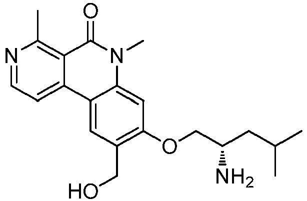

### Part D. (S)-8-(2-amino-4-methylpentyloxy)-9-(hydroxymethyl)-4,6-dimethylbenzo[c][2,7]naphthyridin-5(6H)-one

Prepared as described in Example 2, Part E to afford the title product (28 mg, 0.073 mmol, 34% yield) as an off-white solid. LC/MS (ESI) *m*/*e* 370.2 [(M+H)⁺, calcd for C₂₁H₂₈N₃O₃ 370.2]; LC/MS retention time (method B): *t*_{R} = 1.03 min. HPLC retention time (method A): *t*_{R} = 7.46 min; HPLC retention time (method B): *t*_{R} = 8.13 min; ¹H NMR (400MHz, METHANOL-*d*₄) δ ppm 8.55 (d, *J*=5.8 Hz, 1H), 8.37 (s, 1H), 8.12 (d, *J*=5.8 Hz, 1H), 7.01 (s, 1H), 4.80 (s, 2H), 4.28 (dd, *J*=9.5, 3.5 Hz, 1H), 4.08 (dd, *J*=9.5, 7.0 Hz, 1H), 3.78 (s, 3H), 3.48 - 3.40 (m, 1H), 3.08 (s, 3H), 1.92 - 1.80 (m, 1H), 1.64 - 1.41 (m, 2H), 1.05 (d, *J*=6.5 Hz, 3H), 1.03 (d, *J*=6.8 Hz, 3H).

### Example 72

### (S)-8-(2-amino-4-methylpentyloxy)-4,6-dimethyl-9-(oxazol-5-yl)benzo[c][2,7]naphthyridin-5(6H)-one

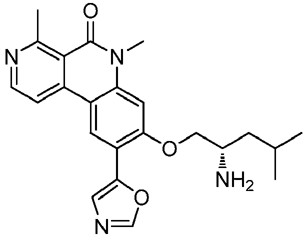

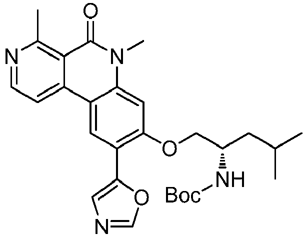

### Part A. (S)-tert-butyl 1-(4,6-dimethyl-9-(oxazol-5-yl)-5-oxo-5,6-dihydrobenzo[c][2,7]naphthyridin-8-yloxy)-4-methylpentan-2-ylcarbamate

Prepared as described in an Example 71, Part D and Example 48, Part B from *(S)*-*tert*-butyl 1-(9-formyl-4,6-dimethyl-5-oxo-5,6-dihydrobenzo[*c*][2,7]naphthyridin-8-yloxy)-4-methylpentan-2-ylcarbamate to afford the title product (150 mg, 0.068 mmol, 56% yield) as a yellow gum. LC/MS (ESI) *m*/*e* 507.2 [(M+H)⁺, calcd for C₂₈H₃₅N₄O₅ 507.2] LC/MS retention time (method B): *t*_{R} = 1.64 min.

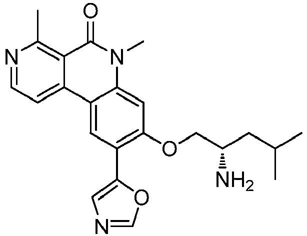

### Part B. (S)-8-(2-amino-4-methylpentyloxy)-4,6-dimethyl-9-(oxazol-5-yl)benzo[c][2,7]naphthyridin-5(6H)-one

Prepared as described in Example 2, Part E from *(S)-tert-*butyl 1-(9-formyl-4,6-dimethyl-5-oxo-5,6-dihydrobenzo[*c*][2,7]naphthyridin-8-yloxy)-4-methylpentan-2-ylcarbamate to afford the title product (20 mg, 0.045 mmol, 67% yield) as a yellow solid. LC/MS (ESI) *m*/*e* 407.2 [(M+H)⁺, calcd for C₂₃H₂₇N₄O₃ 407.2] LC/MS retention time (method B): *t*_{R} = 1.64 min. HPLC retention time (method A): *t*_{R} = 7.46 min; HPLC retention time (method A): *t*_{R} = 8.89 min HPLC retention time (method B): *t*_{R} = 8.80 min ¹H NMR (400MHz, METHANOL-*d*₄) δ ppm 8.66 (s, 1H), 8.57 (d, *J*=5.8 Hz, 1H), 8.34 (s, 1H), 8.15 (d, *J*=5.8 Hz, 1H), 7.60 (s, 1H), 7.08 (s, 1H), 4.38 - 4.26 (m, 1H), 4.24 - 4.17 (m, 1H), 3.76 (s, 3H), 3.48 (dd, *J*=3.3, 1.8 Hz, 1H), 3.05 (s, 3H), 1.93 - 1.82 (m, 1H), 1.65 - 1.44 (m, 2H), 1.04 (d, *J*=2.5 Hz, 3H), 1.02 (d, *J*=2.5 Hz, 3H).

### Example 73

### 8-(2-amino-5,5,5-trifluoropentyloxy)-4,6-dimethyl-5-oxo-5,6-dihydrobenzo[c][2,7]naphthyridine-9-carbonitrile

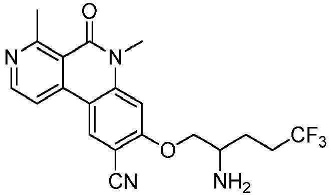

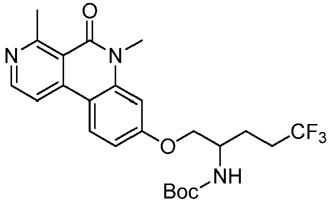

### Part A. tert-butyl 1-(4,6-dimethyl-5-oxo-5,6-dihydrobenzo[c][2,7]naphthyridin-8-yloxy)-5,5,5-trifluoropentan-2-ylcarbamate

Prepared as described in Example 16, Part H to afford *tert*-butyl 1-(4,6-dimethyl-5-oxo-5,6-dihydrobenzo[*c*][2,7]naphthyridin-8-yloxy)-5,5,5-trifluoropentan-2-ylcarbamate (300 mg, 0.416 mmol, 54 % yield) as a white solid. ¹H LC/MS (ESI) *m*/*e* 480.2 [(M+H)⁺, calcd for C₂₄H₂₉F₃N₃O₄ 480.2] LC/MS retention time (method H): *t*_{R} = 2.22 min.

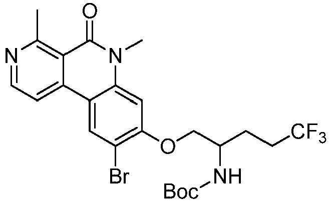

### Part B. tert-butyl 1-(9-bromo-4,6-dimethyl-5-oxo-5,6-dihydrobenzo[c][2,7]naphthyridin-8-yloxy)-5,5,5-trifluoropentan-2-ylcarbamate

Prepared as described in Example 3, Part A to afford the title product (50 mg, 0.06 mmol, 58 % yield) as a pale yellow solid. ¹H LC/MS (ESI) *m*/*e* 559.0 [(M+2H)⁺, calcd for C₂₄H₂₉BrF₃N₃O₄ 559.1] LC/MS retention time (method C): *t*_{R} = 2.24 min.

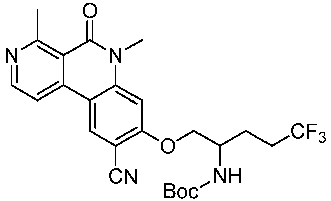

### Part C. tert-butyl 1-(9-cyano-4,6-dimethyl-5-oxo-5,6-dihydrobenzo[c][2,7]naphthyridin-8-yloxy)-5,5,5-trifluoropentan-2-ylcarbamate

Prepared as described in Example 64, Part B to afford the title product (40 mg, 0.079 mmol, 11 % yield) as a pale yellow solid. LC/MS (ESI) *m*/*e* 505.4 [(M+H)⁺, calcd for C₂₅H₂₈F₃N₄O₄ 505.2] LC/MS retention time (method E): *t*_{R} = 1.1 min.

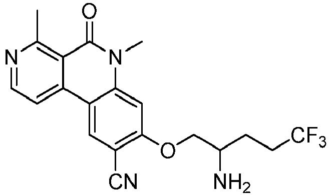

### Part D. 8-(2-amino-5,5,5-trifluoropentyloxy)-4,6-dimethyl-5-oxo-5,6-dihydrobenzo[c][2,7]naphthyridine-9-carbonitrile

Prepared as described in Example 2, Part E to afford the title product (50 mg, 0.036 mmol, 45 % yield) as an off white solid. LC/MS (ESI) *m*/*e* 405.4 [(M+H)⁺, calcd for C₂₀H₂₀F₃N₄O₂ 405.1]; LC/MS retention time (method E): *t*_{R} = 0.71 min. HPLC retention time (method A): *t*_{R} = 8.35 min; HPLC retention time (method B): *t*_{R} = 9.38 min. ¹H NMR (400MHz, DMSO-d₆) δ ppm 8.98 (s, 1H), 8.67 (d, *J*=5.6 Hz, 1H), 8.30 (d, *J*=5.6 Hz, 1H), 7.12 (s, 1H), 4.19 (d, *J*=6.0 Hz, 2H), 3.71 (s, 3H), 3.16 - 3.14 (m, 1H), 3.00 (s, 3H), 2.45 - 2.30 (m, 2H), 1.89 - 1.80 (m, 1H), 1.58 - 1.55 (m, 1H).

### Example 74

### (S)-8-(2-amino-4-methylpentyloxy)-4,6-dimethyl-9-(trifluoromethyl)benzo[c][2,7]naphthyridin-5(6H)-one

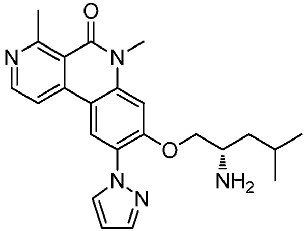

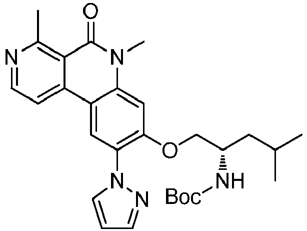

### Part A. (S)-tert-butyl 1-(4,6-dimethyl-5-oxo-9-(1H-pyrazol-1-yl)-5,6-dihydrobenzo[c][2,7]naphthyridin-8-yloxy)-4-methylpentan-2-ylcarbamate

To the solution of *(S)-tert*-butyl (1-((9-bromo-4,6-dimethyl-5-oxo-5,6-dihydrobenzo[*c*][2,7]naphthyridin-8-yl)oxy)-4-methylpentan-2-yl)carbamate (50 mg, 0.096 mmol) and 1*H*-pyrazole (7.88 mg, 0.116 mmol) in 1,4-dioxane (2 mL) and DMF (0.5 mL), was added copper(I) iodide (18.37 mg, 0.096 mmol), *N*1,*N*2-dimethylethane-1,2-diamine (17.00 mg, 0.193 mmol) and potassium phosphate (61.4 mg, 0.289 mmol). The reaction mixture was degassed with nitrogen for 5 min and heated to 170°C for 1 h in a microwave. The reaction mixture was cooled to room temperature, filtered through diatomaceous earth (Celite®) and the filtrate concentrated under reduced pressure. The residue was purified by preparative TLC (eluent 2% MeOH :DCM) to afford *(S)-tert-*butyl 1-(4,6-dimethyl-5-oxo-9-(1H-pyrazol-1-yl)-5,6-dihydrobenzo[*c*][2,7]naphthyridin-8-yloxy)-4-methylpentan-2-ylcarbamate (40 mg, 0.028 mmol, 29% yield) as a brown solid. LC/MS (ESI) m/e 406.5 [(M+H)⁺, calcd for C₂₃H₂₈N₅O₂ 406.2] LC/MS retention time (method E): *t*_{R} = 0.71 min.

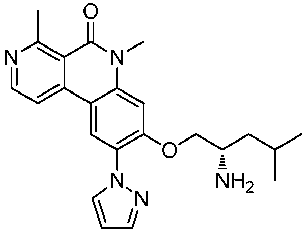

### Part B. (S)-8-(2-amino-4-methylpentyloxy)-4,6-dimethyl-9-(trifluoromethyl)benzo[c][2,7]naphthyridin-5(6H)-one

Prepared as described in Example 2, Part E from *(S)-tert-*butyl 1-(4,6-dimethyl-5-oxo-9-(1*H*-pyrazol-1-yl)-5,6-dihydrobenzo[*c*][2,7]naphthyridin-8-yloxy)-4-methylpentan-2-ylcarbamate to afford *(S)*-8-(2-amino-4-methylpentyloxy)-4,6-dimethyl-9-(trifluoromethyl)benzo[*c*][2,7]naphthyridin-5(*6H*)-one (2 mg, 0.005 mmol, 24% yield) as a green yellow semisolid. LC/MS (ESI) m/e 406.2 [(M+H)⁺, calcd for C₂₃H₂₈N₅O₂ 406.2]; HPLC retention time (method A): *t*_{R} = 8.37 min HPLC retention time (method B): *t*_{R} = 9.54 min; ¹H NMR of racemic compound (400MHz, DMSO-*d*₆) δ ppm 8.98 (s, 1H), 8.67 (d, *J*=5.6 Hz, 1H), 8.30 (d, *J*=5.6 Hz, 1H), 7.12 (s, 1H), 4.19 (d, *J*=6.0 Hz, 2H), 3.71 (s, 3H), 3.16 - 3.14 (m, 1H), 3.00 (s, 3H), 2.45 - 2.30 (m, 2H), 1.89 - 1.80 (m, 1H), 1.58 - 1.55 (m, 1H).

### Example 75

### (S)-8-(2-amino-4-methylpentyloxy)-4,6-dimethyl-9-(trifluoromethyl)benzo[c][2,7]naphthyridin-5(6H)-one

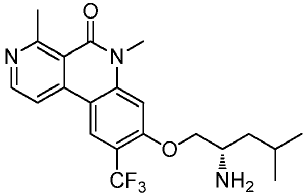

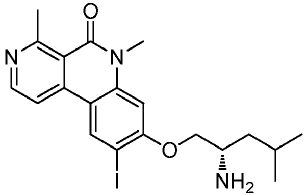

### Part A. (S)-2-(1-(4,6-dimethyl-5-oxo-5,6-dihydrobenzo[c][2,7]naphthyridin-8-yloxy)-4-methylpentan-2-yl)isoindoline-1,3-dione

*(S)-tert*-butyl (1-((4,6-dimethyl-5-oxo-5,6-dihydrobenzo[*c*][2,7]naphthyridin-8-yl)oxy)-4-methylpentan-2-yl)carbamate (300 mg, 0.683 mmol) was taken up in AcOH (15 mL) and treated with periodic acid (156 mg, 0.683 mmol) and H₂SO₄ (0.018 mL, 0.341 mmol). The reaction mixture was heated to 80°C for 20 min after which time iodine (104 mg, 0.410 mmol) was added and heating was continued for an additional 3 h. After cooling, the volatiles were evaporated. The residue was taken up in ethyl acetate (25 mL) and organic layer was washed with saturated aqueous NaHCO₃ (10 mL), then brine (10 mL), dried with Na₂SO₄, filtered and concentrated under reduced pressure to afford *(S)*-2-(1-(4,6-dimethyl-5-oxo-5,6-dihydrobenzo[*c*][2,7]naphthyridin-8-yloxy)-4-methylpentan-2-yl)isoindoline-1,3-dione (300 mg, 0.61 mmol, 90% crude yield) as a brown solid. This was taken to the next step without further purification. LC/MS (ESI) m/e 466.1 [(M+H)⁺, calcd for C₂₀H₂₅IN₃O₂ 466.1]; LC/MS retention time (method F): *t*_{R} = 0.58 min.

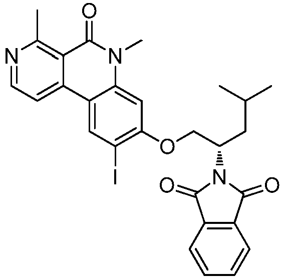

### Part B. (S)-2-(1-(9-iodo-4,6-dimethyl-5-oxo-5,6-dihydrobenzo[c][2,7]naphthyridin-8-yloxy)-4-methylpentan-2-yl)isoindoline-1,3-dione

To a solution of *(S)*-8-((2-amino-4-methylpentyl)oxy)-9-iodo-4,6-dimethylbenzo[*c*][2,7]naphthyridin-5(*6H*)-one (130 mg, 0.140 mmol) in 1,4-dioxane (3 mL) was added *N*-ethyl-*N*-isopropylpropan-2-amine (54.2 mg, 0.419 mmol) and isobenzofuran-1,3-dione (24.83 mg, 0.168 mmol). The reaction mixture was refluxed for 16 h. After cooling, the reaction mixture was diluted with EtOAc (10 mL). The organics were washed with water, saturated aqueous NaHCO₃. The combined aqueous layers were extracted with ethyl acetate (3 x 10 mL). The combined organic layers were dried over sodium sulfate and concentrated under reduced pressure. The residue was purified by preparative TLC plate using 2 % Methanol: DCM as a eluent system to afford *(S)*-2-(1-((9-iodo-4,6-dimethyl-5-oxo-5,6-dihydrobenzo[*c*][2,7]naphthyridin-8-yl)oxy)-4-methylpentan-2-yl)isoindoline-1,3-dione (85 mg, 0.084 mmol, 60% yield) as a pale yellow solid. LC/MS (ESI) m/e 596.5 [(M+H)⁺, calcd for C₂₈H₂₇IN₃O₄ 596.1] LC/MS retention time (method E): *t*_{R} = 1.3 min.

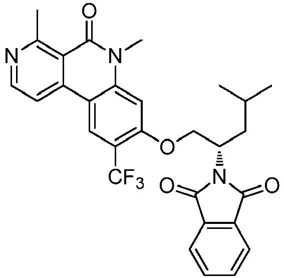

### Part C. (S)-2-(1-(4,6-dimethyl-5-oxo-9-(trifluoromethyl)-5,6-dihydrobenzo[c][2,7]naphthyridin-8-yloxy)-4-methylpentan-2-yl)isoindoline-1,3-dione

Dried potassium fluoride (19.51 mg, 0.336 mmol) and copper(I) iodide (64.0 mg, 0.336 mmol) were mixed and heated to 200°C under gentle shaking at reduced pressure (1 Torr) until a light yellow-greenish color appeared. After cooling, anhydrous *N*-methyl-2-pyrrolidinone (1 mL) was added, followed by *(S)*-2-(1-((9-iodo-4,6-dimethyl-5-oxo-5,6-dihydrobenzo[*c*][2,7]naphthyridin-8-yl)oxy)-4-methylpentan-2-yl)isoindoline-1,3-dione (100 mg, 0.168 mmol) and trimethyl(trifluoromethyl)silane (0.075 mL, 0.504 mmol). The brown solution thus obtained was heated at 90°C for 16 h. After cooling, aqueous ammonia (10 mL) was added to the reaction mixture. The reaction mixture was and extracted with ethyl acetate (3x10 mL). The combined organic layers were wsahed with brine (1x10 mL), dried over sodium sulfate and concentrated under reduced pressure. The residue was purified by preparative TLC (50 % ethyl acetate: petroleum ether as eluent system) to afford *(S)*-2-(1-(4,6-dimethyl-5-oxo-9-(trifluoromethyl)-5,6-dihydrobenzo[*c*][2,7]naphthyridin-8-yloxy)-4-methylpentan-2-yl)isoindoline-1,3-dione (80 mg, 0.033 mmol, 20% yield, 22% purity) as a pale yellow solid. LC/MS (ESI) m/e 538.6 [(M+H)⁺, calcd for C₂₉H₂₇F₃N₃O₄ 538.2] LC/MS retention time (method E): *t*_{R} = 1.27 min.

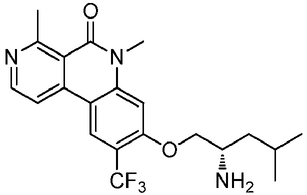

### Part D. (S)-8-(2-amino-4-methylpentyloxy)-4,6-dimethyl-9-(trifluoromethyl)benzo[c][2,7]naphthyridin-5(6H)-one

To a solution of *(S)*-2-(1-((4,6-dimethyl-5-oxo-9-(trifluoromethyl)-5,6-dihydrobenzo[*c*][2,7]naphthyridin-8-yl)oxy)-4-methylpentan-2-yl)isoindoline-1,3-dione (50 mg, 0.019 mmol) in EtOH (4 mL) was added hydrazine hydrate (6.52 mg, 0.130 mmol). The reaction mixture was allowed to stir at 45 °C for 2 h. After cooling, the reaction mixture was concentrated under reduced pressure. The residue was purified by reverse phase HPLC (Symmetry C-18 column (19.0 x 250 mm) 7.0 micron; mobile phase A:Ammonium acetate in water; mobile phase B: acetontirile; flow rate: 16.0 mL) to afford the *(S)*-8-((2-amino-4-methylpentyl)oxy)-4,6-dimethyl-9 (trifluoromethyl)benzo[*c*][2,7]naphthyridin-5(*6H*)-one (5 mg, 0.012 mmol, 64% yield) as an off-white solid. LC/MS (ESI) m/e 408.2 [(M+H)⁺, calcd for C₂₁H₂₅F₃N₃O₂ 408.2] LC/MS retention time (method B): *t*_{R} = 1.25 min. HPLC retention time (method A): *t*_{R} = 10.10 min HPLC retention time (method B): *t*_{R} = 10.69 min; ¹H NMR (400MHz, METHANOL-*d*₄) δ ppm 8.66 (s, 1H), 8.63 (d, *J=5.6* Hz, 1H), 8.22 (d, *J*=5.6 Hz, 1H), 7.19 (s, 1H), 4.42 - 4.38 (m, 1H), 4.28 - 4.20 (m, 1H), 3.83 (s, 3H), 3.58 - 3.49 (m, 1H), 3.11 (s, 3H), 1.90 - 1.82 (m, 1H), 1.68 - 1.62 (m, 1H), 1.58 - 1.53 (m, 1H), 1.10 -1.00 (m, 6H).

### Example 76

### (S)-8-(2-amino-4-methylpentyloxy)-4,6-dimethyl-9-(methylsulfonyl)benzo[c][2,7]naphthyridin-5(6H)-one

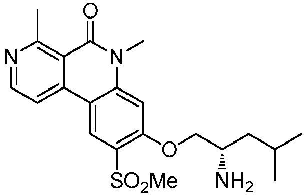

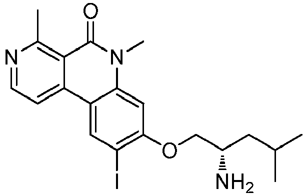

### Part A. (S)-2-(1-(4,6-dimethyl-5-oxo-5,6-dihydrobenzo[c][2,7]naphthyridin-8-yloxy)-4-methylpentan-2-yl)isoindoline-1,3-dione

*(S)-tert*-Butyl (1-((4,6-dimethyl-5-oxo-5,6-dihydrobenzo[*c*][2,7]naphthyridin-8-yl)oxy)-4-methylpentan-2-yl)carbamate (300 mg, 0.683 mmol) was taken up in AcOH (15 mL) and treated with periodic acid (156 mg, 0.683 mmol), and H₂SO₄ (0.018 mL, 0.341 mmol). The reaction mixture was heated to 80°C and maintained for 20 min after which iodine (104 mg, 0.410 mmol) was added and heating was continued at 80°C for an additional 3 h. After cooling, the volatiles were exaporated and the residue was taken up in with ethyl acetate (25 mL). The organic layer was washed with saturated aqueous NaHCO₃ (10 mL), then brine (10 mL), dried over Na₂SO₄, filtered and concentrated under reduced pressure to afford *(S)-*2-(1-(4,6-dimethyl-5-oxo-5,6-dihydrobenzo[*c*][2,7]naphthyridin-8-yloxy)-4-methylpentan-2-yl)isoindoline-1,3-dione (300mg, 0.61 mmol, 90% crude yield) as a brown solid. The material was taken to the next step without further purification. LC/MS (ESI) m/e 466.1 [(M+H)⁺, calcd for C₂₀H₂₅IN₃O₂ 466.1] LC/MS retention time (method D): *t*_{R} = 0.58 min.

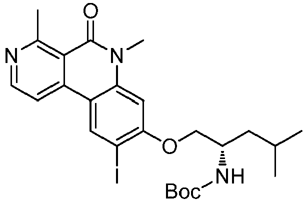

### Part B. (S)-tert-butyl 1-(9-iodo-4,6-dimethyl-5-oxo-5,6-dihydrobenzo[c][2,7]naphthyridin-8-yloxy)-4-methylpentan-2-ylcarbamate

Prepared as described in Example 15, Part C to afford *(S)-tert-*butyl 1-(9-iodo-4,6-dimethyl-5-oxo-5,6-dihydrobenzo[*c*][2,7]naphthyridin-8-yloxy)-4-methylpentan-2-ylcarbamate (300 mg, 0.531 mmol, 82% yield) as a red-orange solid. LC/MS (ESI) m/e 566.5 [(M+H)⁺, calcd for C₂₅H₃₃IN₃O₄ 566.2]; LC/MS retention time (method E): *t*_{R} = 1.27 min.

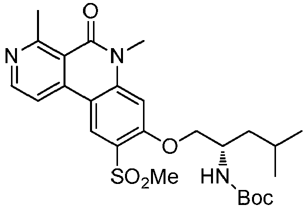

### Part C. (S)-tert-butyl 1-(4,6-dimethyl-9-(methylsulfonyl)-5-oxo-5,6-dihydrobenzo[c][2,7]naphthyridin-8-yloxy)-4-methylpentan-2-ylcarbamate

*(S)-tert*-butyl (1-((9-iodo-4,6-dimethyl-5-oxo-5,6-dihydrobenzo[*c*][2,7]naphthyridin-8-yl)oxy)-4-methylpentan-2-yl)carbamate (100 mg, 0.177 mmol), L-proline (20.36 mg, 0.177 mmol), methanesulfinic acid, sodium salt (181 mg, 1.769 mmol),NaOH (11.32 mg, 0.283 mmol) and copper(I) iodide (33.7 mg, 0.177 mmol) were taken up in DMSO (5 mL) and purged with nitrogen gas for 5 min. The resultant mixture was sealed in a microwave tube and heated at 100 °C in a microwave for 2.5h. The reaction mixture was cooled to room temperature, treated with ice-cold water (5 mL) and extracted with ethyl acetate (3x5 mL). The combined organic layers were dried over sodium sulfate, filtered and concentrated under reduced pressure to afford *(S)-tert-*butyl 1-(4,6-dimethyl-9-(methylsulfonyl)-5-oxo-5,6-dihydrobenzo[*c*][2,7]naphthyridin-8-yloxy)-4-methylpentan-2-ylcarbamate (150 mg, 0.07 mmol, 39 % crude yield, 24 % purity) as a black gummy solid. The material was taken to the next step with out purification. LC/MS (ESI) m/e 518.2 [(M+H)⁺, calcd for C₂₆H₃₆N₃O₆S 518.2] LC/MS retention time (method B): *t*_{R} = 1.51 min.

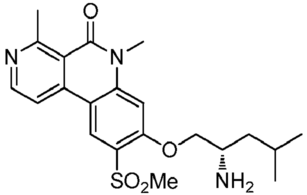

### Part D. (S)-8-(2-amino-4-methylpentyloxy)-4,6-dimethyl-9-(methylsulfonyl)benzo[c][2,7]naphthyridin-5(6H)-one

Prepared as in Example 2, Part E to afford *(S)*-8-(2-amino-4-methylpentyloxy)-4,6-dimethyl-9-(methylsulfonyl)benzo[*c*][2,7]naphthyridin-5(6*H*)-one (4 mg, 0.009 mmol, 13% yield) as an off-white solid. LC/MS (ESI) m/e 466.1 [(M+H)⁺, calcd for C₂₀H₂₅IN₃O₂ 466.1]; LC/MS retention time (method D): *t*_{R} = 0.58 min. HPLC retention time (method): *t*_{R} = min and HPLC retention time (method): *t*_{R} =min. ¹H NMR (400 MHz, *CD_{3O}D*) δ ppm - 8.85 (s 1 H), 8.65 (d, *J=* 6 Hz, 1 H), 8.25 (d, *J* = 6 Hz, 1 H), 7.25 (s, 1 H), 4.65 (m, 1 H), 4.32 (m, 1 H), 3.85 (s, 4 H), 3.1 (s, 3 H), 1.93 (s, 3 H), 1.92 (m, 1 H), 1.65 (m, 2 H), 1.01 (m, 6 H).

### Example 77

### (S)-8-(2-amino-4-methylpentyloxy)-4-methylbenzo[c][2,7]naphthyridin-5(6H)-one

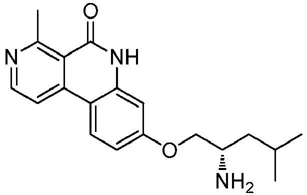

To a solution of *(S)-tert*-butyl (1-((6-(4-methoxybenzyl)-4-methyl-5-oxo-5,6-dihydrobenzo[*c*][2,7]naphthyridin-8-yl)oxy)-4-methylpentan-2-yl)carbamate (500 mg, 0.192 mmol) (prepared as described in Example 49), in acetonitrile (5 mL) and water (5 mL) was added ceric ammonium nitrate (527 mg, 0.962 mmol). The reaction mixture was stirred for 5 h at room temperature. The reaction mixture was diluted with ethyl acetate (5 mL) and water (5 mL). The organic layer was separated, dried with sodium sulfate and concentrated under reduced pressure. The residue was purified by silica gel chromatography (1:1 ethyl acetate : hexane) to afford *(S)-*8-(2-amino-4-methylpentyloxy)-4-methylbenzo[*c*][2,7]naphthyridin-5(*6H*)-one (100 mg, 0.154 mmol, 80% yield) as an off-white solid. LC/MS (ESI) m/e 326.2 [(M+H)⁺, calcd for C₁₉H₂₄N₃O₂ 326.2] LC/MS retention time (method C): *t*_{R} = 1.56 min. HPLC retention time (method A): *t*_{R} = 6.98 min; HPLC retention time (method B): *t*_{R} = 6.96 min. ¹H NMR (400 MHz, *CD₃OD*) δ ppm 6.32 (m, 1 H), 8.44 (m, 2 H), 7.13 (m, 1 H), 6.94 (d, J = 2.4 Hz, 1 H), 4.4 (m, 1 H), 4.3 (m, 1 H), 3.75 (m, 1 H), 3.2 (s, 3 H), 1.75 (m, 3 H), 1.10 (m, 6 H).

### Example 78

### (R)-8-(2-amino-4-methylpentyloxy)-4,6-dimethyl-5-oxo-5,6-dihydrobenzo[c][2,7]naphthyridine-9-carbonitrile

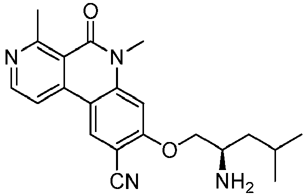

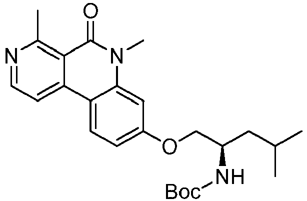

### Part A. (R)-tert-butyl 1-(4,6-dimethyl-5-oxo-5,6-dihydrobenzo[c][2,7]naphthyridin-8-yloxy)-4-methylpentan-2-ylcarbamate

Buchwald coupling performed as described in Example 16, Part H to afford title compound (900 mg, 1.58 mmol, 54% yield, 77% purity) as an off-white solid. LC/MS (ESI) m/e 440.3 [(M+H)⁺, calcd for C₂₅H₃₄N₃O₄ 440.2] LC/MS retention time (method D): *t*_{R} = 0.89 min.

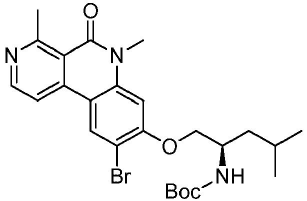

### Part B. (R)-tert-butyl 1-(9-bromo-4,6-dimethyl-5-oxo-5,6-dihydrobenzo[c][2,7]naphthyridin-8-yloxy)-4-methylpentan-2-ylcarbamate

Prepared as described in Example 3, Part A to afford title compound (750 mg, 0.94 mmol, 79% yield, 65% purity) as an orange red solid. LC/MS (ESI) m/e 518.2 [(M+H)⁺, calcd for C₂₅H₃₃BrN₃O₄ 518.2] LC/MS retention time (method D): *t*_{R} = 0.92 min.

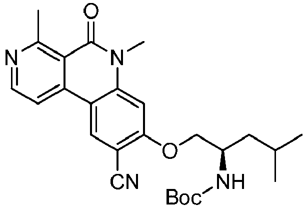

### Part C. (R)-tert-butyl 1-(9-cyano-4,6-dimethyl-5-oxo-5,6-dihydrobenzo[c][2,7]naphthyridin-8-yloxy)-4-methylpentan-2-ylcarbamate

Prepared as described in Example 64, Part B to afford title compound (300 mg, 0.18 mmol, 47% yield, 28% purity) as a black gum. LC/MS (ESI) m/e 465.2 [(M+H)⁺, calcd for C₂₆H₃₃N₄O₄ 465.2] LC/MS retention time (method C): *t*_{R} = 2.74 min.

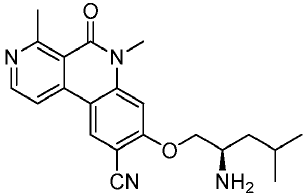

### Part D. (R)-8-(2-amino-4-methylpentyloxy)-4,6-dimethyl-5-oxo-5,6-dihydrobenzo[c][2,7]naphthyridine-9-carbonitrile

Prepared as described in Example 2, Part E to afford title compound (20 mg, 0.051 mmol, 59% yield, 93% purity) as a yellow solid. LC/MS (ESI) m/e 365.2 [(M+H)⁺, calcd for C₂₁H₂₅N₄O₂ 365.2] LC/MS retention time (method C): *t*_{R} = 2.10 min. HPLC retention time (method A): *t*_{R} = 8.35 min and HPLC retention time (method B): *t*_{R} = 9.17 min. ¹H NMR (400MHz, METHANOL-*d*₄) δ ppm 8.98 (s, 1H), 8.71 (d, *J*=6.3 Hz, 1H), 8.53 (d, *J*=6.5 Hz, 1H), 7.23 (s, 1H), 4.65 (dd, *J*=10.7, 3.1 Hz, 1H), 4.50 (dd, *J*=10.8, 6.0 Hz, 1H), 3.85 (br. s., 1H), 3.84 (s, 3H), 3.20 (s, 3H), 1.92 - 1.80 (m, 2H), 1.78 - 1.66 (m, 1H), 1.09 (d, *J*=1.8 Hz, 3H), 1.07 (d, *J*=1.5 Hz, 3H).

### Example 79

### (S)-8-((2-amino-4-methylpentyl)oxy)-9-chloro-6-(2-methoxyethyl)-4-methylbenzo[c][2,7]naphthyridin-5(6H)-one

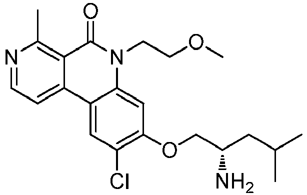

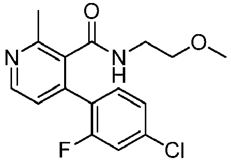

### Part A: 4-(4-chloro-2-fluorophenyl)-N-(2-methoxyethyl)-2-methylnicotinamide

A solution of 4-(4-chloro-2-fluorophenyl)-2-methylnicotinic acid (2g, 5.19 mmol), prepared as described in Example 16, Part E, in thionyl chloride (5 mL, 68.5 mmol) was heated at 70 °C for 2 h. After cooling, the volatiles were removed under reduced pressure. The residue was dissolved in 4 mL of anhydrous DCM and added to a pre-cooled solution of 2-methoxyethanamine (0.390 g, 5.19 mmol) and triethyl amine (3.62 mL, 26.0 mmol) in DCM (8 mL) at 0 °C. The resulting reaction mixture was stirred at rt for 4 h. Water (30 mL) was then added and the solution was extracted with DCM (2 x 20 mL). The combined organic layers were washed with brine (20 mL), dried over sodium sulphate, and concentrated under erduced pressure. The residue was purified by silica gel chromatography using a gradient of EtOAc and hexane as eluant to yield 4-(4-chloro-2-fluorophenyl)-*N*-(2-methoxyethyl)-2-methylnicotinamide (1.3 g, 2.88 mmol, 56% yield) as a yellow oil. LC/MS (ESI) m/e 323.1 [(M+H)⁺, calcd for C₁₆H₁₇ClFN₂O₂ 323.1]; LC/MS retention time (method D): *t*_{R} = 0.61 min.

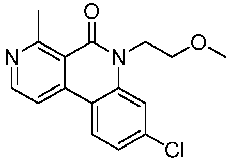

### Part B: 8-chloro-6-(2-methoxyethyl)-4-methylbenzo[c][2,7]naphthyridin-5(6H)-one

4-(4-Chloro-2-fluorophenyl)-*N*-(2-methoxyethyl)-2-methylnicotinamide was subjected to a cyclization reaction as described in Example 2, Part C to yield 8-chloro-6-(2-methoxyethyl)-4-methylbenzo[*c*][2,7]naphthyridin-5(*6H*)-one (1.12 g, 2.55 mmol, 89 % yield) as a white solid. LC/MS (ESI) m/e 303.2 [(M+H)⁺, calcd for C₁₆H₁₆ClN₂O₂ 303.1]; LC/MS retention time (method B): *t*_{R} = 1.38 min.

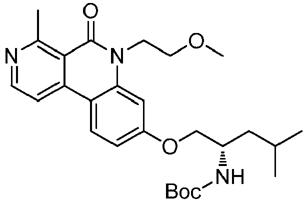

### Part C: (S)-tert-butyl (1-((6-(2-methoxyethyl)-4-methyl-5-oxo-5,6-dihydrobenzo[c][2,7]naphthyridin-8-yl)oxy)-4-methylpentan-2-yl)carbamate

8-Chloro-6-(2-methoxyethyl)-4-methylbenzo[*c*][2,7]naphthyridin-5(*6H*)-one was subjected to ether synthesis as described Example 16, Part H to afford *(S)-tert-*butyl (1-((6-(2-methoxyethyl)-4-methyl-5-oxo-5,6-dihydrobenzo[*c*][2,7]naphthyridin-8-yl)oxy)-4-methylpentan-2-yl)carbamate (511 mg, 0.723 mmol, 53% yield) as a white solid. LC/MS (ESI) m/e 484.6 [(M+H)⁺, calcd for C₂₇H₃₈N₃O₅ 484.3];LC/MS retention time (method E): *t*_{R} = 1.17 min.

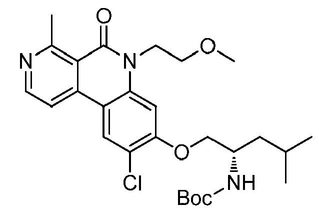

### Part D: (S)-tert-butyl (1-((9-chloro-6-(2-methoxyethyl)-4-methyl-5-oxo-5,6-dihydrobenzo[c][2,7]naphthyridin-8-yl)oxy)-4-methylpentan-2-yl)carbamate

*(S)-tert*-Butyl (1-((6-(2-methoxyethyl)-4-methyl-5-oxo-5,6-dihydrobenzo[*c*][2,7]naphthyridin-8-yl)oxy)-4-methylpentan-2-yl)carbamate was subjected to chlorination as described in Example 41, Part A to afford *(S)-tert*-butyl (1-((9-chloro-6-(2-methoxyethyl)-4-methyl-5-oxo-5,6 dihydrobenzo[*c*][2,7]naphthyridin-8-yl)oxy)-4-methylpentan-2-yl)carbamate (260 mg, 0.227 mmol, 80 % crude yield) as a brown solid. LC/MS (ESI) m/e 518.2 [(M+H)⁺, calcd for C₂₇H₃₇ClN₃O₅ 518.2]; LC/MS retention time (method D): *t*_{R} = 0.91 min; HPLC retention time (method B): *t*_{R} = 1.84 min.

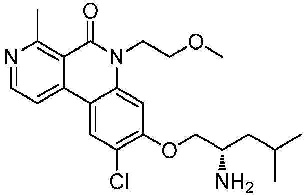

### Part E: (S)-8-((2-amino-4-methylpentyl)oxy)-9-chloro-6-(2-methoxyethyl)-4-methylbenzo[c][2,7]naphthyridin-5(6H)-one

*(S)-tert*-Butyl (1-((9-chloro-6-(2-methoxyethyl)-4-methyl-5-oxo-5,6-dihydrobenzo[*c*][2,7]naphthyridin-8-yl)oxy)-4-methylpentan-2-yl)carbamate was subjected Boc-deprotection as described in Example 2, Part E to afford (S)-8-((2-amino-4-methylpentyl)oxy)-9-chloro-6-(2-methoxyethyl)-4-methylbenzo[*c*][2,7]naphthyridin-5(*6H*)-one, 2 HCl (11.23 mg, 0.021 mmol, 48% yield) as a yellow solid. LC/MS (ESI) m/e 418.2 [(M+H)⁺, calcd for C₂₂H₂₉ClN₃O₃ 418.2]; HPLC retention time (method A): *t*_{R} = 7.91 min; HPLC retention time (Method B): *t*_{R} = 9.29 min; ¹H NMR (400MHz, METHANOL-*d*₄) δ ppm 8.79 - 8.66 (m, 3H), 7.51 (s, 1H), 4.70 (t, *J*=4.9 Hz, 2H), 4.63 - 4.54 (m, 1H), 4.45 (dd, *J*=10.4, 5.1 Hz, 1H), 3.89 - 3.86 (m, 3H), 3.38 (s, 3H), 3.28 (s, 3H), 1.91 - 1.81 (m, 2H), 1.80 - 1.69 (m, 1H), 1.11 - 1.08 (m, 3H), 1.07 (br. s., 3H).

### Example 80

### (S)-8-((2-amino-4-methylpentyl)oxy)-6-(2-methoxyethyl)-4-methylbenzo[c][2,7]naphthyridin-5(6H)-one

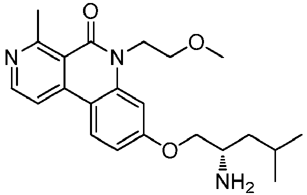

Boc-deprotection of *(S)-tert*-butyl (1-((6-(2-methoxyethyl)-4-methyl-5-oxo-5,6-dihydrobenzo[*c*][2,7]naphthyridin-8-yl)oxy)-4-methylpentan-2-yl)carbamate, (prepared as described in Example 79, Part C) was carried out as described in Example 2, Part E to afford *(S)*-8-((2-amino-4-methylpentyl)oxy)-6-(2-methoxyethyl)-4 methylbenzo[*c*][2,7]naphthyridin-5(*6H*)-one (31.3 mg, 0.081 mmol, 77% yield) as a white solid. LC/MS (ESI) m/e 384.2 [(M+H)⁺, calcd for C₂₂H₃₀N₃O₃ 384.2]; LC/MS retention time (method B): *t*_{R} = 1.06 min; HPLC retention time (method A): *t*_{R} = 8.11 min; HPLC retention time (method B): *t*_{R} = 9.29 min; ¹H NMR (400MHz, METHANOL-*d*₄) δ ppm 8.55 (d, *J*=5.8 Hz, 1H), 8.36 (d, *J*=9.0 Hz, 1H), 8.12 (d, *J*=5.8 Hz, 1H), 7.22 (d, *J*=2.3 Hz, 1H), 7.04 (dd, *J*=9.0, 2.5 Hz, 1H), 4.58 (t, *J*=5.8 Hz, 2H), 4.18 (dd, *J*=9.4, 3.9 Hz, 1H), 4.00 (dd, *J*=9.3, 7.0 Hz, 1H), 3.81 (t, *J*=5.8 Hz, 2H), 3.39 (s, 3H), 3.38 - 3.36 (m, 1H), 3.08 (s, 3H), 1.91 - 1.79 (m, 1H), 1.57 - 1.40 (m, 2H), 1.04 (d, *J*=6.5 Hz, 3H), 1.01 (d, *J*=6.8 Hz, 3H).

### Example 81

### (S)-8-(2-amino-4-methylpentyloxy)-7-fluoro-4,6-dimethylbenzo[c][2,7]naphthyridin-5(6H)-one

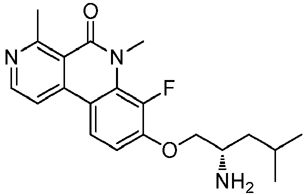

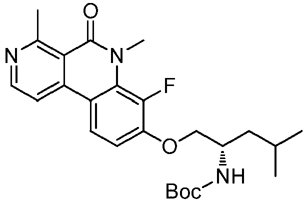

### Part A: (S)-tert-butyl 1-(7-fluoro-4,6-dimethyl-5-oxo-5,6-dihydrobenzo[c][2,7]naphthyridin-8-yloxy)-4-methylpentan-2-ylcarbamate

Prepared as described in Example 16, Part H from 8-bromo-7-fluoro-4,6-dimethylbenzo[*c*][2,7]naphthyridin-5(*6H*)-one, (prepared as described in Example 29, Part F).to afford *(S)-tert-*butyl 1-(7-fluoro-4,6-dimethyl-5-oxo-5,6-dihydrobenzo[*c*][2,7]naphthyridin-8-yloxy)-4-methylpentan-2-ylcarbamate (0.013 g, 0.028 mmol, 37% yield) as a white solid. NMR (400MHz, *DMSO*-*d*₆) δ ppm 8.64 (d, *J*=7.2 Hz, 1H), 8.36 (m, 1H), 8.21 (m, 1H), 7.23 (m, 1H), 6.84 (d, *J*=11.2 Hz,1H), 6.28 (s, 1H), 4.06 (m, 2 H), 3.92 (m, 1 H), 3.78 (m, 3 H), 2.99 (s, 1 H), 1.65 (m, 1H), 1.38 (m, 11H), 0.89 (m, 6 H).

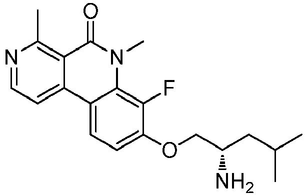

### Part B: (S)-8-(2-amino-4-methylpentyloxy)-7-fluoro-4,6-dimethylbenzo[c][2,7]naphthyridin-5(6H)-one

Prepared as described in Example 2, Part E to afford the title product (8.5 mg, 0.024 mmol, 98 % yield as a brown solid. LC/MS (ESI) m/e 358.2 [(M+H)⁺, calcd for C₂₀H₂₅FN₃O₂ 358.2] LC/MS retention time (method B): *t*_{R} = 3.58 min. HPLC retention time (method A): *t*_{R} = 7.68 min and HPLC retention time (method B): *t*_{R} = 8.02 min. ¹H NMR (400MHz, DMSO-d₆) δ ppm 8.69 (d, *J*=5.8 Hz, 1H), 8.36 (d, *J*=7.5 Hz, 1H), 8.27 (d, *J*=5.8 Hz, 1H), 8.07 (br. s., 2H), 7.35 - 7.24 (m, 1H), 4.38 (dd, *J*=10.7, 3.4 Hz, 1H), 4.26 (dd, *J*=10.8, 6.0 Hz, 1H), 3.81 (d, *J*=9.3 Hz, 3H), 3.67 - 3.60 (m, 1H), 3.02 (s, 3H), 1.87 - 1.72 (m, 1H), 1.59 (td, *J*=7.1, 3.9 Hz, 2H), 0.96 (d, *J*=2.8 Hz, 3H), 0.94 (d, *J*=2.8 Hz, 3H).

### Example 82

### (S)-8-(2-amino-4-methylpentyloxy)-7-fluoro-4-methylbenzo[c][2,7]naphthyridin-5(6H)-one

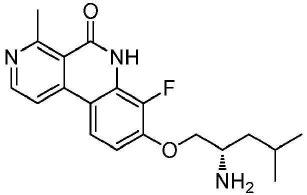

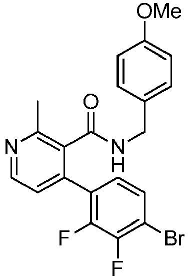

### Part A. 4-(4-bromo-2,3-difluorophenyl)-N-(4-methoxybenzyl)-2-methylnicotinamide

Prepared as described in Example 29, Part E by reaction of biaryl acid (described in Example 29, Part D) with PMB-amine to afford 4-(4-bromo-2,3-difluorophenyl)-*N*-(4-methoxybenzyl)-2-methylnicotinamide (0.16 g, 0.314 mmol, 92%) as an off-white solid. LC/MS (ESI) m/e 447.0 [(M+H)⁺, calcd for C₂₁H₁₈BrF₂N₂O₂ 447.1]; LC/MS retention time (method E): *t*_{R} = 0.94 min.

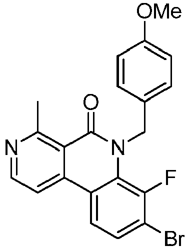

### Part B: 8-bromo-7-fluoro-6-(4-methoxybenzyl)-4-methylbenzo[c][2,7]naphthyridin-5(6H)-one

Prepared as described in Example 29, Part F to afford 8-bromo-7-fluoro-6-(4-methoxybenzyl)-4-methylbenzo[*c*][2,7]naphthyridin-5(*6H*)-one (0.12 g, 0.269 mmol, 90%) as a off-white solid. LC/MS (ESI) m/e 429.1 [(M+2H)⁺, calcd for C₂₁H₁₈BrFN₂O₂ 429.0]; LC/MS retention time (method E): *t*_{R} = 1.15 min.

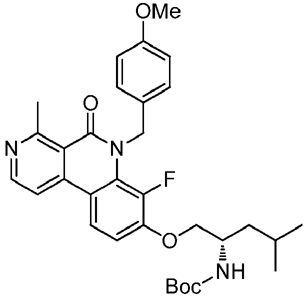

### Part C. tert-butyl 1-(7-fluoro-6-(4-methoxybenzyl)-4-methyl-5-oxo-5,6-dihydrobenzo[c][2,7]naphthyridin-8-yloxy)-2,4-dimethylpentan-2-ylcarbamate

Prepared as described in Example 16, Part H to afford *tert*-butyl 1-(7-fluoro-6-(4-methoxybenzyl)-4-methyl-5-oxo-5,6-dihydrobenzo[*c*][2,7]naphthyridin-8-yloxy)-2,4-dimethylpentan-2-ylcarbamate (46 mg, 0.081 mmol, 13% yield) as an off-white solid. LC/MS (ESI) m/e 564.3 [(M+H)⁺, calcd for C₃₂H₃₉FN₃O₅ 564.3]; LC/MS retention time (Method H): *t*_{R} = 2.35 min.

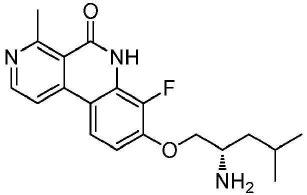

### Part D. (S)-8-(2-amino-4-methylpentyloxy)-7-fluoro-4-methylbenzo[c][2,7]naphthyridin-5(6H)-one

Prepared as described in Example 17, Part I to afford *(S)*-8-(2-amino-4-methylpentyloxy)-7-fluoro-4-methylbenzo[*c*][2,7]naphthyridin-5(*6H*)-one (8 mg, 0.023 mmol, 72% yield) as an off-white solid. LC/MS (ESI) m/e 342.2 [(M)⁻, calcd for C₉H₂₁FN₃O₂ 342.2]; LC/MS retention time (method C): *t*_{R} = 1.99 min; HPLC retention time (method B): *t*_{R} = 6.91 min; ¹H NMR (400MHz, METHANOL-*d*₄) δ ppm 8.69 (d, *J*=6.3 Hz, 1H), 8.44 (d, *J*=6.3 Hz, 1H), 8.28 (dd, *J*=9.3, 2.0 Hz, 1H), 7.26 (dd, *J*=9.0, 7.8 Hz, 1H), 4.51 (dd, *J*=10.7, 3.1 Hz, 1H), 4.36 (dd, *J*=10.7, 6.1 Hz, 1H), 3.85 - 3.72 (m, 1H), 3.19 (s, 3H), 1.92 - 1.63 (m, 3H), 1.07 (d, *J*=3.8 Hz, 3H), 1.06 (d, *J*=3.8 Hz, 3H).

### Example 83

### (S)-8-(2-amino-4-methylpentyloxy)-4,6-dimethyl-9-(thiazol-5-yl)benzo[c][2,7]naphthyridin-5(6H)-one

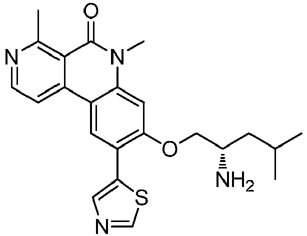

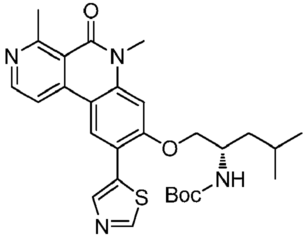

### Part A. (S)-tert-butyl 1-(4,6-dimethyl-5-oxo-9-(thiazol-5-yl)-5,6-dihydrobenzo[c][2,7]naphthyridin-8-yloxy)-4-methylpentan-2-ylcarbamate

5-Bromothiazole (0.050 g, 0.305 mmol) and hexamethyltin (0.158 mL, 0.762 mmol) were taken up in 1,4-dioxane (1 mL) and purged with nitrogen for 10 min. Tetrakis (triphenylphosphine)palladium (0.035 g, 0.030 mmol) was added and the reaction mixture was stirred for 2 h at rt. (*S*)-*tert*-butyl (1-((9-bromo-4,6-dimethyl-5-oxo-5,6 dihydrobenzo[*c*][2,7]naphthyridin-8-yl)oxy)-4-methylpentan-2-yl)carbamate (0.158 g, 0.305 mmol) and lithium chloride (0.013 g, 0.305 mmol) were added and the mixture was purged with N₂ for 10min. The reaction mixture was then heated for 16 h at 90 °C. After cooling, the reaction mixture was filtered through diatomaceous earth (Celite®) and concentrated under reduced pressure. The residue was purified by preparative HPLC to afford *(S)*-*tert*-butyl 1-(4,6-dimethyl-5-oxo-9-(thiazol-5-yl)-5,6-dihydrobenzo[*c*][2,7]naphthyridin-8-yloxy)-4-methylpentan-2-ylcarbamate (40 mg, 0.053 mmol, 18% yield) as a yellow solid. LC/MS (ESI) m/e 23.6 [(M+H)⁺, calcd for C₂₈H₃₅N₄O₄S 523.2]; LC/MS retention time (method E): *t*_{R} = 1.09 min.

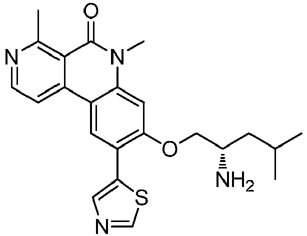

### Part B. (S)-8-(2-amino-4-methylpentyloxy)-4,6-dimethyl-9-(thiazol-5-yl)benzo[c][2,7]naphthyridin-5(6H)-one

Prepared as described in Example 2, Part E to afford *(S)*-8-(2-amino-4-methylpentyloxy)-4,6-dimethyl-9-(thiazol-5-yl)benzo[*c*][2,7]naphthyridin-5(*6H*)-one (16 mg, 0.037 mmol, 80 % yield) as an off-white solid. LC/MS (ESI) m/e 423.2 [(M+H)⁺, calcd for C₂₃H₂₇N₄O₂S 423.2]; LC/MS retention time (method C): *t*_{R} = 2.04 min; HPLC retention time (method A): t_{R} = 9.1 min; retention time (method B): t_{R} = 9.72 min; ¹H NMR (400MHz, METHANOL-*d₄*) δ ppm 9.10 (s, 1H), 8.73 (s, 1H), 8.61 (d, *J*=5.8 Hz, 1H), 8.49 (s, 1H), 8.30 (d, *J*=5.8 Hz, 1H), 7.16 (s, 1H), 4.38 (dd, *J*=9.5*,* 4.0 Hz, 1H), 4.25 (dd, *J*=9.5, 6.5 Hz, 1H), 3.82 (s, 3H), 3.59 - 3.51 (m, 1H), 3.10 (s, 3H), 1.91 - 1.82 (m, 1H), 1.69 - 1.46 (m, 2H), 1.04 (d, *J*=5.5 Hz, 3H), 1.03 (d, *J*=5.8 Hz, 3H).

### Example 84

### (S)-methyl 8-((2-amino-4-methylpentyl)oxy)-4,6-dimethyl-5-oxo-5,6-dihydrobenzo[c][2,7]naphthyridine-9-carboxylate

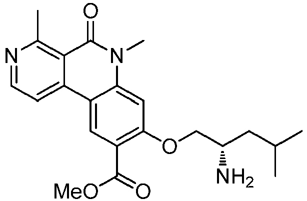

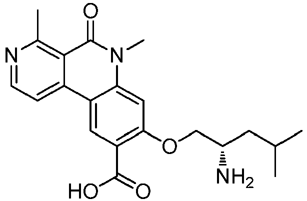

### Part A: (S)-8-((2-amino-4-methylpentyl)oxy)-4,6-dimethyl-5-oxo-5,6-dihydrobenzo[c][2,7]naphthyridine-9-carboxylic acid

To a flask containing *(S)*-8-((2-amino-4-methylpentyl)oxy)-4,6-dimethyl-5-oxo-5,6-dihydrobenzo[*c*][2,7]naphthyridine-9-carbonitrile (60 mg, 0.165 mmol), (prepared as described in Example 64) was added concentrated HCl (5 mL). The resultant mixture was heated to 80 °C for 12 h. After cooling, the volatiles were removed under reduced pressure to afford *(S)-*8-((2-amino-4-methylpentyl)oxy)-4,6-dimethyl-5-oxo-5,6-dihydrobenzo[*c*][2,7]naphthyridine-9-carboxylic acid (40 mg, 0.104 mmol, 63% cude yield) as a yellow solid. The material was carried on without further purification. LC/MS (ESI) *m*/*e* 384.1 [(M+H)⁺, calcd for C₂₁H₂₆N₃O₄ 384.2]; LC/MS retention time (method D): *t*_{R} = 0.71 min.

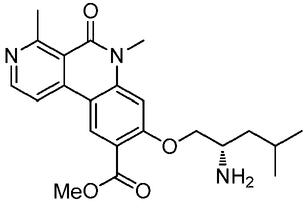

### Part B: (S)-methyl 8-((2-amino-4-methylpentyl)oxy)-4,6-dimethyl-5-oxo-5,6-dihydrobenzo[c][2,7]naphthyridine-9-carboxylate

To a flask containing *(S)*-8-((2-amino-4-methylpentyl)oxy)-4,6-dimethyl-5-oxo-5,6-dihydrobenzo[*c*][2,7]naphthyridine-9-carboxylic acid (40 mg, 0.104 mmol) in MeOH (3 mL) at 0 °C was added SOCl₂ (0.076 mL, 1.043 mmol) dropwise. The resultant solution was heated to 70 °C for 12 h. After cooling, the volatiles were removed under reduced pressure. The residue was purified by prep HPLC (using 0.1% TFA ACN : water) to afford *(S)*-methyl 8-((2-amino-4-methylpentyl)oxy)-4,6-dimethyl-5-oxo-5,6-dihydrobenzo[*c*][2,7]naphthyridine-9-carboxylate, 2 TFA (1.65 mg, 2.427 µmol, 2% yield) as pale brown solid. LC/MS (ESI) *m*/*e* 398.2 [(M+H)⁺, calcd for C₂₂H₂₈N₃O₄ 398.2]; LC/MS retention time (method C): *t*_{R} = 1.58 min; HPLC retention time (method A): *t*_{R} = 9.14 min; HPLC retention time (method B): *t*_{R} = 9.58 min; ¹H NMR (400MHz, METHANOL-d₄) δ ppm 9.01 (s, 1H), 8.68 (d, *J*=6.0 Hz, 1H), 8.40 (d, *J*=6.0 Hz, 1H), 7.23 (s, 1H), 4.69 (dd, *J*=10.0, 3.0 Hz, 1H), 4.36 (dd, *J*=10.5, 7.0 Hz, 1H), 4.02 (s, 3H), 3.88 -3.81 (m, 4H), 3.16 (s, 3H), 1.93 - 1.65 (m, 3H), 1.09 (d, *J*=4.0 Hz, 3H), 1.07 (d, *J*=4.0 Hz, 3H).

### Example 85

### (S)-8-((2-amino-4-methylpentyl)oxy)-1-fluoro-6-methylbenzo[c][2,7]naphthyridin-5(6H)-one

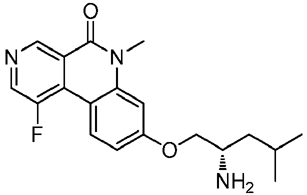

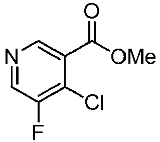

### Part A: methyl 4-chloro-5-fluoronicotinate

To a solution of 4-chloro-5-fluoronicotinic acid (1.7 g, 9.68 mmol) in acetonitrile (18 mL) cooled to 0 °C was added DBU (3.65 mL, 24.21 mmol) dropwise. The reaction mixture was stirred for 30 min. To this mixture iodomethane (3.03 mL, 48.4 mmol) was added dropwise and stirred at rt for 12 h. The volatiles were removed under reduced pressure. The residue was purified via silica gel column chromatography (hexane and ethyl acetate) to afford methyl 4-chloro-5-fluoronicotinate (1.2 g, 6.33 mmol, 65% yield) as a yellow solid. LC/MS (ESI) m/e 190.0 [(M+H)⁺, calcd for C₇H₆ClFNO₂ 189.9]; LC/MS retention time (method D): *t*_{R} = 0.77 min.

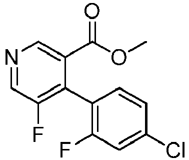

### Part B: Methyl 4-(4-chloro-2-fluorophenyl)-5-fluoronicotinate

To a solution of methyl 4-chloro-5-fluoronicotinate (1.2 g, 6.33 mmol) in 1,4-dioxane (12 mL) and water (0.5 mL) was added (4-chloro-2-fluorophenyl)boronic acid (1.214 g, 6.96 mmol). The mixture was then purgend with nitrogen gas for 5 min. Potassium phosphate, dibasic (2.205 g, 12.66 mmol) and PdCl₂(dppf) (0.371 g, 0.506 mmol) were added and again the mixture was purged with N₂ for 5 min.The reaction ixture was heated to 80 °C for 12 h. After cooling, water was added to and the mixture was extracted with ethyl acetate (3x25 mL). The combined organic layers were washed with brine, dried over sodium sulfate and concentrated under reduced pressure. The residue was purified by silica gel chromatography using hexane and ethyl acetate to afford methyl 4-(4-chloro-2-fluorophenyl)-5-fluoronicotinate (900 mg, 3.17 mmol, 50% yield) as a yellow solid. LC/MS (ESI) m/e 284.0 [(M+H)⁺, calcd for C₁₃H₉ClF₂NO₂ 284.02]; LC/MS retention time (method D): *t*_{R} = 1.01 min.

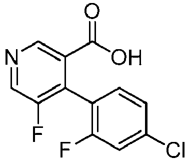

### Part C: 4-(4-chloro-2-fluorophenyl)-5-fluoronicotinic acid

To the solution of methyl 4-(4-chloro-2-fluorophenyl)-5-fluoronicotinate (600 mg, 2.115 mmol) in tetrahydrofuran (2.5 mL), MeOH (2.5 mL) and water (2.5 mL) was added LiOH (50.7 mg, 2.115 mmol) and the mixture was stirred at rt for 12 h. The volatiles were removed and the residue was diluted with water. The pH was adjusted to 4 with 1.5N HCl. The product was extracted with ethyl acetate (2x5 mL). The combined organic layers were dried over sodium sulfate and concentrated under reduced pressure to afford 4-(4-chloro-2-fluorophenyl)-5-fluoronicotinic acid (530 mg, 1.966 mmol, 93 % crude yield) the pale brown solid. The material was carried on without further purification. LC/MS (ESI) m/e 269.9 [(M+H)⁺, calcd for C₁₂H₇ClF₂NO₂ 270.0]; LC/MS retention time (method D): *t*_{R} = 0.88 min.

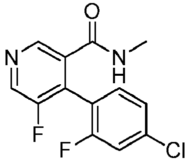

### Part D: 4-(4-chloro-2-fluorophenyl)-5-fluoro-N-methylnicotinamide

To the solution of 4-(4-chloro-2-fluorophenyl)-5-fluoronicotinic acid (530mg, 1.966 mmol) in DMF (5 mL) cooled at 0 °C, was added HOBT (602 mg, 3.93 mmol) and EDC (565 mg, 2.95 mmol), then the mixture was stirred for 5 min. Methylamine hydrochloride (531 mg, 7.86 mmol) and DIEA (1.030 mL, 5.90 mmol) were added and the resultant mixture was stirred at RT for 12 h. Ice-cold water was added to the reaction and the solid obtained was collected by vacuum filtration. The solid so obtained was washed with water (50 mL) and air dried to afford 4-(4-chloro-2-fluorophenyl)-5-fluoro-*N*-methylnicotinamide (350 mg, 1.238 mmol, 63 % yield) as an orange oil. LC/MS (ESI) m/e 282.9 [(M+H)⁺, calcd for C₁₃H₁₀ClF₂N₂O 283.0]; LC/MS retention time (method D): *t*_{R} = 0.83 min.

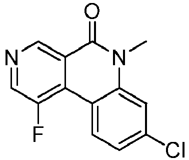

### Part E: 8-chloro-1-fluoro-6-methylbenzo[c][2,7]naphthyridin-5(6H)-one

4-(4-Chloro-2-fluorophenyl)-5-fluoro-*N*-methylnicotinamide was subjected to cyclization as described in Example 2 and Part C to afford 8-chloro-1-fluoro-6-methylbenzo[*c*][2,7]naphthyridin-5(*6H*)-one (85 mg, 0.320 mmol, 60% yield) as a white solid. LC/MS (ESI) m/e 263.0 [(M+H)⁺, calcd for C₁₃H₉ClFN₂O 263.0]; LC/MS retention time (method C): *t*_{R} = 1.77 min.

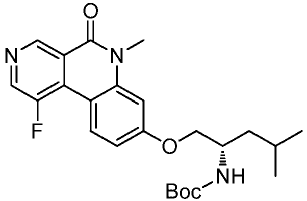

### Part F: (S)-tert-butyl (1-((1-fluoro-6-methyl-5-oxo-5,6-dihydrobenzo[c][2,7]naphthyridin-8-yl)oxy)-4-methylpentan-2-yl)carbamate

8-Chloro-1-fluoro-6-methylbenzo[*c*][2,7]naphthyridin-5(*6H*)-one was subjected to ether synthesis as described in Example 16, Part H to afford *(S)-tert-*butyl (1-((1-fluoro-6-methyl-5-oxo-5,6-dihydrobenzo[*c*][2,7]naphthyridin-8-yl)oxy)-4-methylpentan-2-yl)carbamate (60 mg, 0.135 mmol, 71% yield) as an off white solid. LC/MS (ESI) m/e 444.2 [(M+H)⁺, calcd for C₂₄H₃₁FN₃O₄ 445.2]; LC/MS retention time (method D): *t*_{R} = 1.11 min.

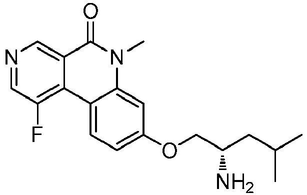

### Part G: (S)-8-((2-amino-4-methylpentyl)oxy)-1-fluoro-6-methylbenzo[c][2,7]naphthyridin-5(6H)-one

*(S)-tert-*Butyl (1-((1-fluoro-6-methyl-5-oxo-5,6-dihydrobenzo[*c*][2,7]naphthyridin-8-yl)oxy)-4-methylpentan-2-yl)carbamate was subjected to Boc-deprotection as described in Example 2, Part E. The crude product was purified by preparative HPLC using 0.1%TFA ACN:water to afford *(S)*-8-((2-amino-4-methylpentyl)oxy)-1-fluoro-6-methylbenzo[c][2,7]naphthyridin-5(*6H*)-one, TFA (42.68 mg, 0.091 mmol, 95 % yield) as a yellow solid. LC/MS (ESI) m/e 344.2 [(M+H)⁺, calcd for C₁₉H₂₃FN₃O₂ 344.2]; LC/MS retention time (method C): *t*_{R} = 2.07 min; HPLC retention time (method A): t_{R} = 5.52 min; HPLC retention time (method B): t_{R} = 6.63 min; ¹H NMR (400MHz, METHANOL-*d₄*) δ ppm 9.38 (s, 1H), 8.77 (d, *J*=5.0 Hz, 1H), 8.67 (dd, *J*=9.0, 3.0 Hz, 1H), 7.21 (d, *J*=2.0 Hz, 1H), 7.16 (dq, *J*=9.1, 1.3 Hz, 1H), 4.48 (dd, *J*=10.5, 3.0 Hz, 1H), 4.29 (dd, *J*=10.5, 6.0 Hz, 1H), 3.82 (s, 3H), 3.80 - 3.73 (m, 1H), 1.94 - 1.82 (m, 1H), 1.82 - 1.63 (m, 2H), 1.08 (d, *J*=4.0 Hz, 3H), 1.07 (d, *J*=4.0 Hz, 3H).

### Example 86

### (S)-8-((2-amino-4-methylpentyl)oxy)-4,6-dimethyl-9-(pyridin-2-yl)benzo[c][2,7]naphthyridin-5(6H)-one

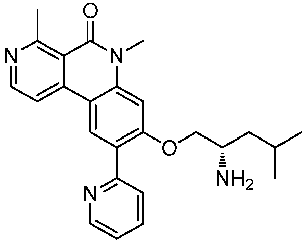

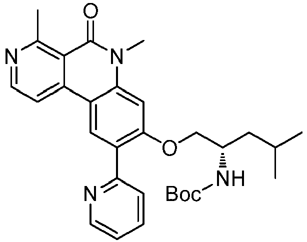

### Part A. (S)-tert-butyl (1-((4,6-dimethyl-5-oxo-9-(pyridin-2-yl)-5,6-dihydrobenzo[c][2,7]naphthyridin-8-yl)oxy)-4-methylpentan-2-yl)carbamate

To a solution of *(S)-tert-*butyl (1-((9-bromo-4,6-dimethyl-5-oxo-5,6-dihydrobenzo[*c*][2,7]naphthyridin-8-yl)oxy)-4-methylpentan-2-yl)carbamate (100 mg, 0.193 mmol) in 1,4-dioxane (2 mL) was added 2-(tributylstannyl)pyridine (85 mg, 0.231 mmol) and Pd(PPh₃)₄ (11.14 mg, 9.64 µmol). The reaction mixture was degassed for 30 min and heated to 120 °C for 1 h in a microwave. The reaction mixture was cooled and diluted with water (10 mL) and extracted with ethyl acetate (3 x 10 mL). The combined organic layers were dried over sodium sulfate and concentrated under reduced pressure. The crude product was purified by silica gel chromatography (3% methanol in chloroform) to afford *(S)-tert-*butyl (1-((4,6-dimethyl-5-oxo-9-(pyridin-2-yl)-5,6-dihydrobenzo[*c*][2,7]naphthyridin-8-yl)oxy)-4-methylpentan-2-yl)carbamate (50 mg, 0.120 mmol, 62% yield) as a pale yellow solid which was carried on without further purification LC/MS (ESI) m/e 517.7 [(M+H)⁺, calcd for C₃₀H₃₇N₄O₄ 517.3]; LC/MS retention time (method E): *t*_{R} = 1.19 min.

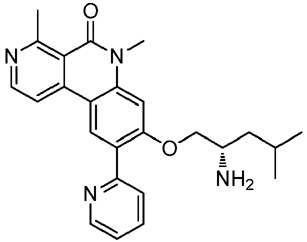

### Part B. (S)-8-((2-amino-4-methylpentyl)oxy)-4,6-dimethyl-9-(pyridin-2-yl)benzo[c][2,7]naphthyridin-5(6H)-one

To a solution of *(S)-tert*-butyl (1-((4,6-dimethyl-5-oxo-9-(pyridin-2-yl)-5,6-dihydrobenzo[*c*][2,7]naphthyridin-8-yl)oxy)-4-methylpentan-2-yl)carbamate (35 mg, 0.068 mmol) in Methanol (4 mL) was added HCl in 1,4-dioxane (0.169 mL, 0.677 mmol) at 0 °C. Then the reaction mixture was stirred at RT for 2 h. The volatiles were concentrated under reduced pressure. The residue so obtained was basified with saturated aqueous NaHCO₃, diluted with water and extracted with DCM (3 x 5 mL). The combined organic layers were dried over Na₂SO₄ and concentrated in *vacuo.* The crude product was purified by reverse phase prep HPLC (Sunfire C18 column (19.0 x 150 mm) 10.0 micron; mobile phase A: 10 mM ammonium acetate in water; mobile phase B: acetonitrile; flow rate: 16.0 mL) to afford *(S)*-8-((2-amino-4-methylpentyl)oxy)-4,6-dimethyl-9-(pyridin-2-yl)benzo[*c*][2,7]naphthyridin-5(*6H*)-one (10 mg, 0.024 mmol, 35% yield) as an off-white solid. LC/MS (ESI) m/e 417.6 [(M+H)⁺, calcd for C₂₅H₂₉N₄O₂ 417.2]; LC/MS retention time (method E): *t*_{R} = 0.84 min; HPLC retention time (method A): t_{R} = 7.61 min; HPLC retention time (method B): t_{R} = 8.29 min; ¹H NMR (400MHz, METHANOL-*d*₄). ¹H NMR (400MHz, METHANOL-*d₄*) δ ppm 8.70 (d, *J*=4.3 Hz, 1H), 8.64 (s, 1H), 8.58 (d, *J*=5.5 Hz, 1H), 8.21 (d, *J*=5.8 Hz, 1H), 8.04 - 7.94 (m, 1H), 7.93 - 7.86 (m, 1H), 7.46 (ddd, *J*=7.3, 5.0, 1.3 Hz, 1H), 7.18 (s, 1H), 4.32 (dd, *J*=9.5, 4.3 Hz, 1H), 4.13 (dd, *J*=9.4, 6.7 Hz, 1H), 3.84 (s, 3H), 3.36 - 3.34 (m, 1H), 3.11 (s, 3H), 1.86 - 1.70 (m, 1H), 1.53 - 1.32 (m, 2H), 0.97 (d, *J*=6.8 Hz, 3H), 0.93 (d, *J*=6.5 Hz, 3H).

### Example 87 and Example 88

### (S)-8-((2-amino-5,5,5-trifluoro-4-methylpentyl)oxy)-4,6-dimethylbenzo[c][2,7]naphthyridin-5(6H)-one; diastereomers 1 and 2

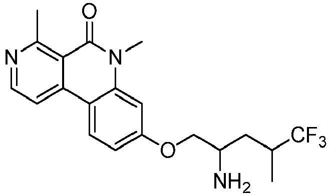

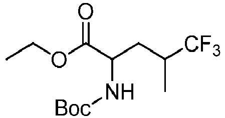

### Part A. ethyl 2-amino-5,5,5-trifluoro-4-methylpentanoate hydrochloride

To 2-amino-5,5,5-trifluoro-4-methylpentanoic acid (50 mg, 0.270 mmol) in a flask was added hydrochloric acid 2N in ethanol (1350 µl, 5.40 mmol). The solution was refluxed at 80 °C for 5 h. After cooling, the ethanol was removed under reduced pressure. The residue was triturated with diethyl ether (2x). The solid so obtained (60 mg, 0.240 mmol) was taken up in dichloromethane (5 mL). To this suspension cooled to 0°C was added triethylamine (0.167 mL, 1.202 mmol) and di-*tert*-butyl carbonate (0.054 mL, 0.312 mmol). The reaction mixture was allowed to warm to room temperature and stirred for 12 h. The reaction mixture was diluted with water (10 mL) and extracted with ethyl acetate (3 x 10 mL). The combined organic layers were dried over sodium sulfate and concentrated under reduced pressure to afford ethyl 2-((*tert*-butoxycarbonyl)amino)-5,5,5-trifluoro-4-methylpentanoate (55 mg, 0.176 mmol, 73% crude yield) as a white solid. The material was carried on without further purification. GCMS (ESI) m/e 212 [(M)⁻, calcd for C₈H₁₃F₃NO₂ 212] GC/MS (Method: Agilent GCMS Module-7890 (GC) 5975C(MSD) HP-5MS, 30m x 0.25mm ID x 0.25u Film thickness 0.9 mL/min at constant flow of Helium) retention time *t*_{R} = 6.38 min.

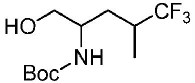

### Part B. Tert-butyl (5,5,5-trifluoro-1-hydroxy-4-methylpentan-2-yl)carbamate

To the solution of ethyl 2-((*tert*-butoxycarbonyl)amino)-5,5,5-trifluoro-4-methylpentanoate (500 mg, 1.596 mmol) in ethanol (2 mL) at 0 °C was added NaBH₄ (604 mg, 15.96 mmol). The reaction mixture was allowed to stir at RT for 16 h. The reaction mixture was quenched with aqueous ammonia. The solutiom was filter through a glass funnel and the resulting filtrate was concentrated to afford the *tert-*butyl (5,5,5-trifluoro-1-hydroxy-4-methylpentan-2-yl)carbamate (400 mg, 1.475 mmol, 92 % crude yield) as a colorless gummy semisolid material. The material was carried on without further purification. LC/MS (ESI) m/e 172.1 [(M+H)⁺, calcd for C₆H₁₃F₃NO 172.1] LC/MS retention time (Method A): *t*_{R} = 2.1 min.

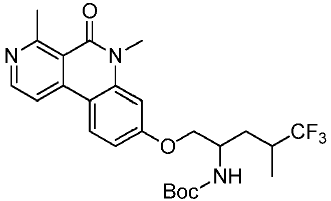

### Part C: Tert-butyl (1-((4,6-dimethyl-5-oxo-5,6-dihydrobenzo[c][2,7]naphthyridin-8-yl)oxy)-5,5,5-trifluoro-4-methylpentan-2-yl)carbamate

8-Chloro-4,6-dimethylbenzo[*c*][2,7]naphthyridin-5(*6H*)-one and *tert*-butyl (5,5,5-trifluoro-1-hydroxy-4-methylpentan-2-yl)carbamate was subjected to ether synthesis as described in Example 16, Part H. The compound was purified by reverse phase HPLC (Sunfire C18 (250 x 30 mm, 10µm) column; mobile phase A: 10 mM ammonium acetate in water; mobile phase B: acetonitrile; flow rate: 25 mL) to afford the product as a off-white solid. The diasteromeric mixture so obtained was subjected to diastereomeric separation by normal phase chiral prep HPLC column: IC (250 x 30 mm, 5µm); mobile phase A: 0.2 % DEA : hexane; mobile phase B: Ethanol; flow rate: 16 mL / min) to afford *tert*-butyl (1-((4,6-dimethyl-5-oxo-5,6-dihydrobenzo[*c*][2,7]naphthyridin-8-yl)oxy)-5,5,5-trifluoro-4-methylpentan-2-yl)carbamate as two diastereomeric mixtures (1 and 2). The absolute sterochemistry of the diasteromers was not determined.
**Diastereomeric mixture 1:** (30 mg, 0.061 mmol, 10% yield) as an off-white solid. LC/MS of Diastereomeric mixture (ESI) m/e 494.3 [(M+H)⁺, calcd for C₂₅H₃₁F₃N₃O₄ 494.2]; LC/MS retention time (Method C) *t*_{R} = 2.17 min.
**Diastereomeric mixture 2:** (80 mg, 0.162 mmol, 26% yield) as an off-white solid. LC/MS of Diastereomeric mixture (ESI) m/e 494.2 [(M+H)⁺, calcd for C₂₅H₃₁F₃N₃O₄ 494.2]; LC/MS retention time (Method C) *t*_{R} = 2.16 min.

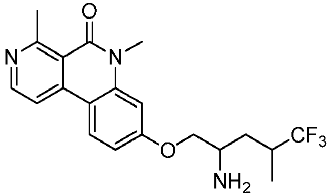

### Part D: 8-((2-amino-5,5,5-trifluoro-4-methylpentyl)oxy)-4,6-dimethylbenzo[c][2,7]naphthyridin-5(6H)-one: (Diastereomer 2)

Diastereomeric mixture 1 (30 mg, 0.061 mmol) was subjected to Boc deprotection as described in Example 2, Part E to afford 8-((2-amino-5,5,5-trifluoro-4-methylpentyl)oxy)-4,6-dimethylbenzo[*c*][2,7]naphthyridin-5(*6H*)-one (72.3 mg, 0.172 mmol, 99% yield) as an off white solid. LC/MS (ESI) m/e 394.6 [(M+H)⁺, calcd for C₂₀H₂₃F₃N₃O₂ 394.2]; HPLC retention time (method A): t_{R} = 8.62 min; HPLC retention time (method B): t_{R} = 8.77 min; ¹H NMR (400MHz, *DMSO-d₆*) δ ppm 8.62 (d, *J*=5.5 Hz, 1H), 8.44 (d, *J*=8.8 Hz, 1H), 8.19 (d, *J*=5.8 Hz, 1H), 7.08 - 6.92 (m, 2H), 4.11 - 3.90 (m, 2H), 3.67 (s, 3H), 3.01 (s, 3H), 3.17 - 3.10 (m, 1H), 1.96 - 1.82 (m, 1H), 1.66 - 1.48 (m, 2H), 1.10 (d, *J*=6.8 Hz, 3H).
Boc-protected Diastereomeric mixture 2 (80 mg, 0.162 mmol) was subjected to Boc deprotection as described in Example 2, Part E to afford 8-((2-amino-5,5,5-trifluoro-4-methylpentyl)oxy)-4,6-dimethylbenzo[*c*][2,7]naphthyridin-5(*6H*)-one (10 mg, 0.025 mmol, 50 % yield) as an off white solid. LC/MS (ESI) m/e 394.6 [(M+H)⁺, calcd for C₂₀H₂₃F₃N₃O₂ 394.2]; HPLC retention time (method A): t_{R} = 8.75 min; HPLC retention time (method B): t_{R} = 8.8 min; ¹H NMR (400MHz, CHLOROFORM-d) δ ppm 8.64 (d, *J*=5.5 Hz, 1H), 8.16 (d, *J*=8.8 Hz, 1H), 7.83 (d, *J*=5.8 Hz, 1H), 6.89 (dd, *J*=8.8, 2.3 Hz, 1H), 6.85 (d, *J*=2.3 Hz, 1H), 4.05 (dd, *J*=9.0, 4.0 Hz, 1H), 3.89 (dd, *J*=8.8, 6.5 Hz, 1H), 3.73 (s, 3H), 3.40 - 3.34 (m, 1H), 3.16 (s, 3H), 2.60 - 2.42 (m, 1H), 1.98 (dt, *J*=14.3, 6.1 Hz, 1H), 1.50 - 1.40 (m, 1H), 1.23 (d, *J*=7.0 Hz, 3H).

### Example 89

### (S)-8-((2-amino-4-methylpentyl)oxy)-9-methoxy-4,6-dimethylbenzo[c][2,7]naphthyridin-5(6H)-one

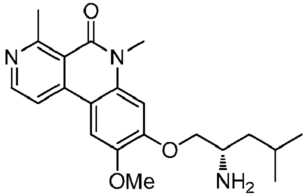

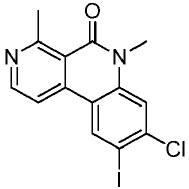

### Part A. 8-chloro-9-iodo-4,6-dimethylbenzo[c][2,7]naphthyridin-5(6H)-one

To a stirred solution of 8-chloro-4,6-dimethylbenzo[*c*][2,7]naphthyridin-5(*6H*)-one (1.0 g, 3.87 mmol) in acetic Acid (25 mL) was added H₂SO₄ (0.206 mL, 3.87 mmol) followed by periodic acid (0.881 g, 3.87 mmol). The mixture was then heated to 80 °C for 20 min. I₂ (0.294 g, 1.160 mmol) was then added at 80 °C and stirred for 120 min. After cooling to ambient temperature, the acetic acid was removed under reduced pressure. The residue was neutralized with 70 mL of saturated aqueous sodium bicarbonate solution. The solid so obtained was collected by vacuum filtration and air dried to afford 8-chloro-9-iodo-4,6-dimethylbenzo[*c*][2,7]naphthyridin-5(*6H*)-one (1.3 g, 3.21 mmol, 83 % yield) as a brown solid. LC/MS (ESI) m/e 385.1 [(M+H)⁺, calcd for C₁₄H₁₁ClIN₂O 384.9]; LC/MS retention time (method E): *t*_{R} = 1.09 min; ¹H NMR (400 MHz, *DMSO-d₆*) δ ppm 8.96 (s, 1 H) 8.69 (d, J=5.58 Hz, 1 H) 8.36 (d, *J*=5.52 Hz, 1 H) 7.76 (s, 1 H) 3.63 (s, 3 H) 3.00 (s, 3 H).

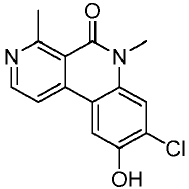

### Part B. 8-chloro-9-hydroxy-4,6-dimethylbenzo[c][2,7]naphthyridin-5(6H)-one

To the stirred solution of 8-chloro-9-iodo-4,6-dimethylbenzo[*c*][2,7]naphthyridin-5(*6H*)-one (1.0 g, 2.60 mmol) in DMSO (5 mL) and Water (5 mL) in an inert atmosphere were added copper(I) iodide (0.050 g, 0.260 mmol) followed by 1,10-phenanthroline (0.047 g, 0.260 mmol) and KOH (2.188 g, 39.0 mmol). The reaction mixture was heated to 100 °C for 16 h and monitored by LC/MS. Upon completion, the reaction mixture was cooled and pH adjusted to between 3 to 4 with 1 N HCl. The reaction mixture was diluted with 50 mL of water and extracted with 80 mL of dichloromethane. The organic layer was separated, washed with brine, dried over Na₂SO₄ and concentrated under reduced pressure to afford 8-chloro-9-hydroxy-4,6-dimethylbenzo[*c*][2,7]naphthyridin-5(*6H*)-one (0.6 g, 2.053 mmol, 79% crude yield) as a green solid. The material was carried on without further purification. LC/MS (ESI) m/e 273.0 [(M)⁻, calcd for C₁₄H₁₀ClN₂O₂ 273.05]; LC/MS retention time (method C): *t*_{R} = 2.06 min.

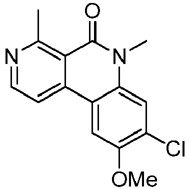

### Part C. 8-chloro-9-methoxy-4,6-dimethylbenzo[c][2,7]naphthyridin-5(6H)-one

To the stirred solution of 8-chloro-9-hydroxy-4,6-dimethylbenzo[*c*][2,7]naphthyridin-5(*6H*)-one (0.3 g, 1.092 mmol) in DMF (10 mL) cooled to 0 °C, was added NaH (0.052 g, 2.184 mmol) followed by methyl iodide (0.205 mL, 3.28 mmol). The resultant mixture was allowed to stir at ambient temperature for 10 h. The reaction mixture was then diluted with water (50 mL) and extracted with ethyl acetate (2x80 mL). The combined organic layers were separated, washed with brine, dried over Na₂SO₄ and concentrated under reduced pressure. The crude product was purified by silica gel column chromatography using 70% ethyl acetate in petroleum ether to afford 8-chloro-9-methoxy-4,6-dimethylbenzo[*c*][2,7]naphthyridin-5(*6H*)-one (0.12 g, 0.416 mmol, 38% yield) as a semi-solid. LC/MS (ESI) m/e 288.4 [(M)⁺, calcd for C₁₅H₁₃ClN₂O₂ 288.1]; LC/MS retention time (Method A): *t*_{R} = 1.9 min.

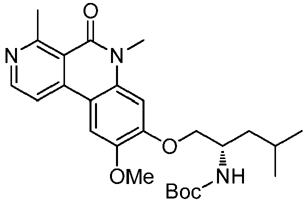

### Part D. (S)-tert-butyl (1-((9-methoxy-4,6-dimethyl-5-oxo-5,6-dihydrobenzo[c][2,7]naphthyridin-8-yl)oxy)-4-methylpentan-2-yl)carbamate

8-Chloro-9-methoxy-4,6-dimethylbenzo[*c*][2,7]naphthyridin-5(6*H*)-one was subjected to ether synthesis as described in Example 16, Part H to afford *(S)-tert-*butyl (1-((9-methoxy-4,6-dimethyl-5-oxo-5,6-dihydrobenzo[*c*][2,7]naphthyridin-8-yl)oxy)-4-methylpentan-2-yl)carbamate (90 mg, 0.073 mmol, 18% yield) as a semi solid. LC/MS (ESI) m/e 470.3 [(M+H)⁺, calcd for C₂₆H₃₆N₃O₅ 470.5]; LC/MS retention time (method D) *t*_{R} = 0.87 min.

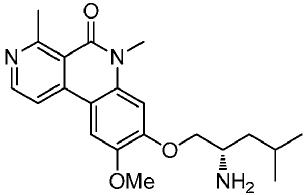

### Part E. (S)-8-((2-amino-4-methylpentyl)oxy)-9-methoxy-4,6-dimethylbenzo[c][2,7]naphthyridin-5(6H)-one

*(S)-tert*-Butyl (1-((9-methoxy-4,6-dimethyl-5-oxo-5,6-dihydrobenzo[*c*][2,7]naphthyridin-8-yl)oxy)-4-methylpentan-2-yl)carbamate was subjected to Boc-deprotection as described in Example 2, Part E to afford *(S)-*8-((2-amino-4-methylpentyl)oxy)-9-methoxy-4,6-dimethylbenzo[*c*][2,7]naphthyridin-5(*6H*)-one (6 mg, 0.015 mmol, 8% yield) as a yellow solid. LC/MS (ESI) m/e 370.2[(M+H)⁺, calcd for C₂₁H₂₈N₃O₃ 370.2]; LC/MS retention time (method C): *t*_{R} = 2.03min. HPLC retention time (method A): t_{R} = 7.31 min and HPLC retention time (method B): t_{R} = 8.32 min; ¹H NMR (400MHz, METHANOL-*d₄)* δ ppm 8.63 (d, *J*=6.3 Hz, 1H), 8.50 (d, *J*=6.3 Hz, 1H), 8.01 (s, 1H), 7.22 (s, 1H), 4.49 (dd, *J*=10.7, 3.1 Hz, 1H), 4.35 (dd, *J*=10.9, 6.4 Hz, 1H), 4.09 (s, 3H), 3.83 (s, 3H), 3.81 - 3.75 (m, 1H), 3.19 (s, 3H), 1.92 - 1.74 (m, 2H), 1.73 - 1.61 (m, 1H), 1.07 (d, *J*=4.0 Hz, 3H), 1.06 (d, *J*=4.0 Hz, 3H).

### Example 90

### (S)-8-((2-amino-4-methylpentyl)oxy)-9-(difluoromethoxy)-4,6-dimethylbenzo[c][2,7]naphthyridin-5(6H)-one

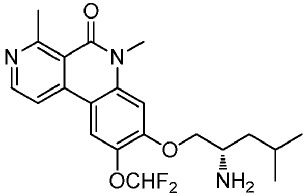

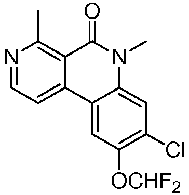

### Part A: 8-chloro-9-(difluoromethoxy)-4,6-dimethylbenzo[c][2,7]naphthyridin-5(6H)-one

To the stirred solution of 8-chloro-9-hydroxy-4,6-dimethylbenzo[*c*][2,7]naphthyridin-5(*6H*)-one (0.5 g, 1.456 mmol), prepared as described in Example 89, Part B in acetonitrile (5 mL) was added K₂CO₃ (0.604 g, 4.37 mmol) followed by sodium 2-chloro-2,2-difluoroacetate (0.444 g, 2.91 mmol). The mixture was heated to 80°C for 18 h. After cooling, the reaction mixture was diluted with water (50 mL) and extracted with ethyl acetate (2x80 mL). The organic layer was washed with brine solution, dried over Na₂SO₄, filtered and concentrated under reduced pressure to afford 8-chloro-9-(difluoromethoxy)-4,6-dimethylbenzo[*c*][2,7]naphthyridin-5(*6H*)-one (50 mg, 0.154 mmol, 11% crude yield) as a semi solid. The material was carried on without further purification. LC/MS (ESI) m/e 325.0 [(M+H)⁺, calcd for C₁₅H₁₂ClF₂N₂O₂ 325.04]; LC/MS retention time (method C): *t*_{R} = 2.39 min.

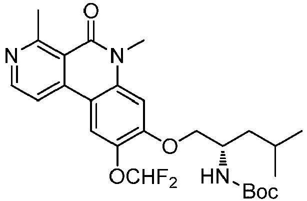

### Part B: (S)-tert-butyl (1-((9-(difluoromethoxy)-4,6-dimethyl-5-oxo-5,6-dihydrobenzo[c][2,7]naphthyridin-8-yl)oxy)-4-methylpentan-2-yl)carbamate

8-Chloro-9-(difluoromethoxy)-4,6-dimethylbenzo[*c*][2,7]naphthyridin-5(*6H*)-one was subjected to ether synthesis as described in Example 16, Part H to afford *(S)-*tert-butyl (1-((9-(difluoromethoxy)-4,6-dimethyl-5-oxo-5,6-dihydrobenzo[*c*][2,7]naphthyridin-8-yl)oxy)-4-methylpentan-2-yl)carbamate (0.2 g, 0.033 mmol, 27% yield) as a colorless oil. LC/MS (ESI) m/e 506.3 [(M+H)⁺, calcd for C₂₆H₃₄F₂N₃O₅ 506.2]; LC/MS retention time (method E): *t*_{R} = 1.21 min.

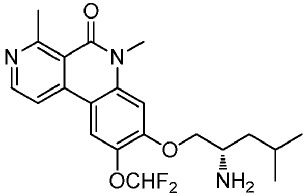

### Part C: (S)-8-((2-amino-4-methylpentyl)oxy)-9-(difluoromethoxy)-4,6-dimethylbenzo[c][2,7]naphthyridin-5(6H)-one

*(S)-tert-*Butyl (1-((9-(difluoromethoxy)-4,6-dimethyl-5-oxo-5,6-dihydrobenzo[*c*][2,7]naphthyridin-8-yl)oxy)-4-methylpentan-2-yl)carbamate was subjected to Boc deprotection as described in Example 2, Part E to afford *(S)*-8-((2-amino-4-methylpentyl)oxy)-9-(difluoromethoxy)-4,6-dimethylbenzo[*c*][2,7]naphthyridin-5(*6H*)-one (2 mg, 4.24 umol, 13% yield) as a green gum. LC/MS (ESI) m/e 406.2 [(M+H)⁺, calcd for C₂₁H₂₆F₂N₃O₃ 406.2]; LC/MS retention time (method C): *t*_{R} = 1.75 min. HPLC retention time (method B): t_{R} = 10.11 min; ¹H NMR (400MHz, METHANOL-*d*₄) δ ppm 8.3 (s, 1 H), 8.2 (m, 1 H), 7.25 (s, 1 H), 6.9 (t, 1 H), 4.55 (m, 1 H), 4.45 (m, 1 H), 3.8 (m, 4 H), 3.05-3.2 (m, 3 H), 1.8-1.95 (m, 3 H), 1.05-1.1 (m, 6 H).

### Example 91 and Example 92

### (S)-8-(2-amino-4,4,4-trifluorobutoxy)-4,6-dimethylbenzo[c][2,7]naphthyridin-5(6H)-one and (R)-8-(2-amino-4,4,4-trifluorobutoxy)-4,6-dimethylbenzo[c][2,7]naphthyridin-5(6H)-one

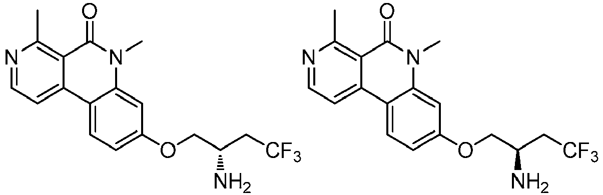

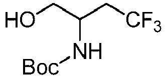

### Part A. Prepared as per literature reference: Ling, F. et.al., J.Org. Chem., 2003, 68, 7544 - 7547.

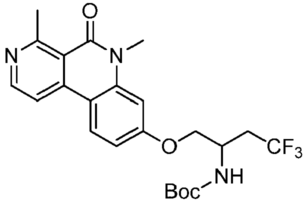

### Part B. tert-butyl (1-((4,6-dimethyl-5-oxo-5,6-dihydrobenzo[c][2,7]naphthyridin-8-yl)oxy)-4,4,4-trifluorobutan-2-yl)carbamate

8-Chloro-4,6-dimethylbenzo[*c*][2,7]naphthyridin-5(*6H*)-one and *tert*-butyl (4,4,4-trifluoro-1-hydroxybutan-2-yl)carbamate were used for ether synthesis as described in Example 16, Part H to afford *tert*-butyl (1-((4,6-dimethyl-5-oxo-5,6-dihydrobenzo[*c*][2,7]naphthyridin-8-yl)oxy)-4,4,4-trifluorobutan-2-yl)carbamate (0.22 g, 0.473 mmol, 49% yield) as a semi-solid. LC/MS (ESI) m/e 466.2 [(M+H)⁺, calcd for C₂₃H₂₇F₃N₃O₄ 466.2]; LC/MS retention time (method D): *t*_{R} = 0.82 min.

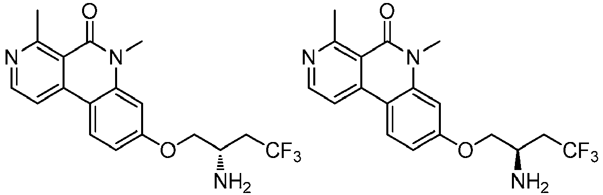

### Part C. 8-(2-amino-4,4,4-trifluorobutoxy)-4,6-dimethylbenzo[c][2,7]naphthyridin-5(6H)-one

Prepared as described in Example 2, Part E to afford racemic 8-(2-amino-4,4,4-trifluorobutoxy)-4,6-dimethylbenzo[*c*][2,7]naphthyridin-5(*6H*)-one (40 mg, 0.066 mmol, 26% yield, 98% purity) as an off-white solid. The racemic product was resolved by Chiral preparative SFC purification Conditions: (CHIRALPAK IA (250x21 mm) 5 micron Mob. Phase: 60% CO₂, Co-solvent: 40% (0.5% DEA in Methanol), Flow rate: 60 g per min, Back pressure: 100 bar) to afford two enantiomers. The absolute stereochemistry of the enantiomers was not determined.
**Enantiomer 1:** (7 mg, 0.014 mmol, 6% yield, 98% purity) as an off-white solid. LC/MS (ESI) m/e 366.1 [(M+H)⁺, calcd for C₁₈H₁₉F₃N₃O₂ 366.1]; LC/MS retention time (Method A): *t*_{R} = 1.81 min; ¹H NMR (400MHz, METHANOL-*d₄*) δ ppm 8.57 (d, *J*=5.5 Hz, 1H), 8.41 (d, *J*=9.0 Hz, 1H), 8.16 (d, *J*=6.0 Hz, 1H), 7.12 - 7.05 (m, 2H), 4.30 - 4.08 (m, 2H), 3.77 (s, 3H), 3.62 - 3.58 (m, 1H), 3.10 (s, 3H), 2.74 - 2.58 (m, 1H), 2.44 (m, 1H). Chiral HPLC retention time (method B): *t*_{R} = 8.08 min; Chiral SFC Method (CHIRALPAK IA (250x4.6 mm) 5 micron Mob. Phase: 65% CO₂, Flow rate: 2.6 g per min, Co-solvent: 35% (0.3% DEA in Methanol), Flow rate: 1.4 g per min, Back pressure: 100 bar) *t*_{R} = 3.53 min.
**Enantiomer 2:** (7 mg, 0.014 mmol, 6% yield, 98% purity) as an off-white solid. LC/MS (ESI) m/e 366.1 [(M+H)⁺, calcd for C₁₈H₁₉F₃N₃O₂ 366.1]; LC/MS retention time (Method A): *t*_{R} = 1.81 min; ¹H NMR (400MHz, METHANOL-*d₄*) δ ppm 8.57 (d, *J*=5.5 Hz, 1H), 8.41 (d, *J*=9.0 Hz, 1H), 8.16 (d, *J*=6.0 Hz, 1H), 7.12 - 7.05 (m, 2H), 4.30 - 4.08 (m, 2H), 3.77 (s, 3H), 3.62 - 3.58 (m, 1H), 3.10 (s, 3H), 2.74 - 2.58 (m, 1H), 2.44 (m, 1H). Chiral HPLC retention time (method B): *t*_{R} = 7.93 min. Chiral SFC Method (CHIRALPAK IA (250x4.6 mm) 5 micron Mob. Phase: 65% CO₂, Flow rate: 2.6 g per min, Co-solvent: 35% (0.3% DEA in Methanol), Flow rate: 1.4 g per min, Back pressure: 100 bar) *t*_{R} = 4.62 min.

### Example 93

### 8-(2-amino-4,4,4-trifluorobutoxy)-6-methylbenzo[c][2,7]naphthyridin-5(6H)-one

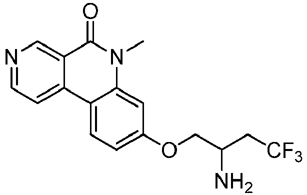

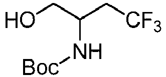

### Part A. Prepared as per literature reference: Ling, F. et.al., J.Org. Chem., 2003, 68, 7544 - 7547.

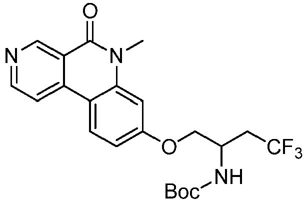

### Part B. tert-butyl 4,4,4-trifluoro-1-(6-methyl-5-oxo-5,6-dihydrobenzo[c][2,7]naphthyridin-8-yloxy)butan-2-ylcarbamate

Prepared as described in Example 16, Part H to afford *tert*-butyl 4,4,4-trifluoro-1-(6-methyl-5-oxo-5,6-dihydrobenzo[*c*][2,7]naphthyridin-8-yloxy)butan-2-ylcarbamate (200 mg, 0.221 mmol, 45% yield) as an off white solid. LC/MS (ESI) m/e 452.2 [(M+H)⁺, calcd for C₂₂H₂₅F₃N₃O₄ 452.2]; LC/MS retention time (Method C): *t*_{R} = 2.01min.

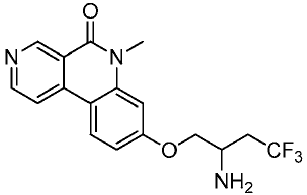

### Part C. 8-(2-amino-4,4,4-trifluorobutoxy)-6-methylbenzo[c][2,7]naphthyridin-5(6H)-one

Prepared as described in Example 2, Part E to afford 8-(2-amino-4,4,4-trifluorobutoxy)-6-methylbenzo[*c*][2,7]naphthyridin-5(*6H*)-one (35 mg, 0.098 mmol, 44% yield) as an off white solid. LC/MS (ESI) m/e 352.0 [(M+H)⁺, calcd for C₁₇H₁₇F₃N₃O₂ 352.1]; LC/MS retention time (Method C): *t*_{R} = 1.92 min; HPLC retention time (method A): t_{R} = 7.07 min; HPLC retention time (method B): t_{R} = 7.44 min; ¹H NMR (400MHz, METHANOL-d₄) δ 9.46 (s, 1H), 8.76 (d, *J*=6.0 Hz, 1H), 8.40 (d, *J*=8.8 Hz, 1H), 8.23 (d, *J*=5.6 Hz, 1H), 7.14 (d, *J*=2.4 Hz, 1H), 7.10 (dd, *J*=8.8, 2.4 Hz, 1H), 4.22 - 4.13 (m, 2H), 3.79 (s, 3H), 3.67 - 3.57 (m, 1H), 2.71 - 2.63 (m, 1H), 2.49 - 2.40 (m, 1H).

### Example 94

### (S)-8-((2-amino-4-methylpentyl)oxy)-4,6-dimethyl-5-oxo-5,6-dihydrobenzo[c][2,7]naphthyridine-9-carboxamide

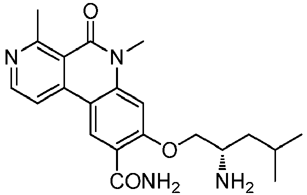

In a 25 mL round-bottomed flask, *(S)*-8-((2-amino-4-methylpentyl)oxy)-4,6-dimethyl-5-oxo-5,6-dihydrobenzo[*c*][2,7]naphthyridine-9-carbonitrile (0.1 g, 0.274 mmol), (prepared as described in Example 64) and Cs₂CO₃ (0.358 g, 1.098 mmol) were taken up in DMF (2 mL) and water (0.6 mL). The mixture was irradiated in a microwave at 120 °C for 2 h. The reaction mixture was concentrated under reduced pressure, and diluted with ethyl acetate and water. The combined organics were concentrated under reduced pressure. The resiude was purified by Prep. HPLC (Column: Xbridge Phenyl (150 x 4.6 mm) 3.5 micron SC/749 using buffer: 0.05% TFA in water) to afford *(S)*-8-((2-amino-4-methylpentyl)oxy)-4,6-dimethyl-5-oxo-5,6-dihydrobenzo[*c*][2,7]naphthyridine-9-carboxamide (5 mg, 0.012 mmol, 5% yield) as an off-white solid. LC/MS (ESI) m/e 383.2 [(M+H)⁺, calcd for C₂₁H₂₇N₄O₃ 383.2]; LC/MS retention time (method C): *t*_{R} = 1.74 min; HPLC retention time (method A): t_{R} = 10.51 min; HPLC retention time (method B): t_{R} = 10.58 min; ¹H NMR (400 MHz, methanol-*d₄*): δ ppm 8.64 (s, 1H), 8.46 (d, 1H), 8.12 (d, 1H), 6.67 (s, 1H), 3.72-3.79 (m, 1H), 3.69 (s, 3H), 3.65-3.69 (m, 2H), 3.04 (s, 3H), 1.84-1.88 (m, 1H), 1.62-1.68 (m, 1H), 1.49-1.57 (m, 1H), 1.31 (d, 3H), 1.29 (d, 3H).

### Example 95

### (S)-4,6-dimethyl-8-(4-methyl-2-(methylamino)pentyloxy)benzo[c][2,7]naphthyridin-5(6H)-one

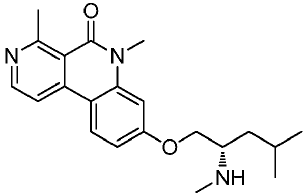

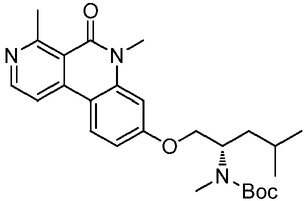

### Part A: (S)-tert-butyl 1-(4,6-dimethyl-5-oxo-5,6-dihydrobenzo[c][2,7]naphthyridin-8-yloxy)-4-methylpentan-2-yl(methyl)carbamate

*(S)-tert-*Butyl (1-((4,6-dimethyl-5-oxo-5,6-dihydrobenzo[*c*][2,7]naphthyridin-8-yl)oxy)-4-methylpentan-2-yl)carbamate (0.150 g, 0.341 mmol), prepared as described in Example 16, Part H, was taken up in DMF (1 mL) and cooled to 0 °C. NaH (0.027 g, 0.683 mmol) was added and the reaction mixture was stirred for 45 min at °0 C. Iodomethane (0.107 mL, 1.706 mmol) was then added and reaction mixture was warmed to room temperature and stirred for 15 min. The mixture was quenched with ice and partitioned between ethyl acetate (4 mL) and water (2 mL). The organic layer was separated, washed with brine (2 mL), dried over sodium sulfate, filtered and concentrated under reduced pressure to afford crude which was purified by preparative HPLC to afford *(S)-tert-butyl* (1-((4,6-dimethyl-5-oxo-5,6-dihydrobenzo[*c*][2,7]naphthyridin-8-yl)oxy)-4-methylpentan-2-yl)(methyl)carbamate (60 mg, 0.125 mmol, 37% yield) as a light yellow solid. LC/MS (ESI) m/e 454.4 [(M+H)⁺, calcd for C₂₆H₃₆N₃O₂ 454.3]; LC/MS retention time (method E): *t*_{R} = 1.21 min.

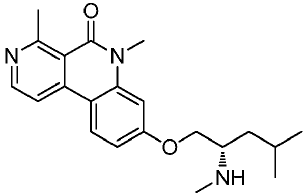

### Part B: (S)-4,6-dimethyl-8-(4-methyl-2-(methylamino)pentyloxy)benzo[c][2,7]naphthyridin-5(6H)-one

Prepared as described in Example 2, Part E to afford title compound (30 mg, 0.070 mmol, 64% yield) as a pale yellow solid. LC/MS (ESI) m/e 354.0 [(M+H)⁺, calcd for C₂₁H₂₈N₃O₂ 354.2] LC/MS retention time (method C): *t*_{R} = 1.81 min. HPLC retention time (method A): t_{R} = 8.22 min and HPLC retention time (method B): t_{R} = 9.21 min. ¹H NMR (400MHz, METHANOL-*d₄*) δ ppm 8.74 (d, *J*=7.0 Hz, 1H), 8.69 - 8.65 (m, 1H), 8.62 (d, *J*=9.0 Hz, 1H), 7.30 - 7.22 (m, 2H), 4.62 (dd, *J*=11.0, 3.0 Hz, 1H), 4.49 (dd, *J*=11.5, 5.0 Hz, 1H), 3.85 (s, 3H), 3.74 (dd, *J*=8.3, 3.8 Hz, 1H), 3.28 (s, 3H), 2.84 (s, 3H), 1.99 - 1.78 (m, 2H), 1.76 - 1.63 (m, 1H), 1.07 (d, *J*=6.0 Hz, 6H).

### Example 96

### (S)-8-((2-amino-4-methylpentyl)oxy)-N,N,4,6-tetramethyl-5-oxo-5,6-dihydrobenzo[c][2,7]naphthyridine-9-carboxamide

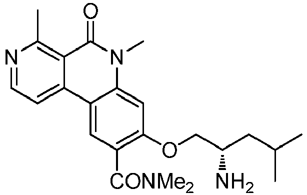

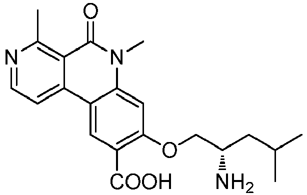

### Part A: (S)-8-((2-amino-4-methylpentyl)oxy)-4,6-dimethyl-5-oxo-5,6-dihydrobenzo[c][2,7]naphthyridine-9-carboxylic acid

To a solution of *(S)*-8-((2-amino-4-methylpentyl)oxy)-4,6-dimethyl-5-oxo-5,6-dihydrobenzo[*c*][2,7]naphthyridine-9-carbonitrile (450 mg, 1.235 mmol), prepared as described in Example 64, in ethanol (5 mL) and water (5 mL) was added NaOH (494 mg, 12.35 mmol). The mixture was heated to 80 °C for 12 h. The volatiles were evaporated and the residue was diluted with water The pH was adjusted to 3 using 1.5N HCl. The solution was extracted with ethyl acetate (2 x 5 mL). The combined organic layers were dried over sodium sulfate, filtered and concentrated under reduced pressure to afford *(S)*-8-((2-amino-4-methylpentyl)oxy)-4,6-dimethyl-5-oxo-5,6-dihydrobenzo[*c*][2,7]naphthyridine-9-carboxylic acid (90 mg, 0.235 mmol, 19% crude yield) as a yellow solid. The material was carried forward without further purification. LC/MS (ESI) m/e 384.1 [(M+H)⁺, calcd for C₂₁H₂₆N₃O₄ 384.2]; LC/MS retention time (method D): *t*_{R} = 0.72 min.

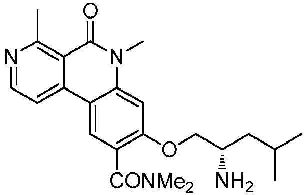

### Part B: (S)-8-((2-amino-4-methylpentyl)oxy)-N,N,4,6-tetramethyl-5-oxo-5,6-dihydrobenzo [c] [2,7]naphthyridine-9-carboxamide

The solution of *(S)*-8-((2-amino-4-methylpentyl)oxy)-4,6-dimethyl-5-oxo-5,6-dihydrobenzo[*c*][2,7]naphthyridine-9-carboxylic acid (90 mg, 0.070 mmol) in DMF (0.9 mL) was cooled to 0 °C. HOBT (21.57 mg, 0.141 mmol) and EDC (20.25 mg, 0.106 mmol) were added and stirred for 5 min. To this dimethylamine (0.282 mL, 0.563 mmol) was added followed by DIPEA (0.037 mL, 0.211 mmol) and stirred for 30 min. The reaction was then warmed to rt and stirred for 12 h. The reaction was quenched by addition of ice, the solid crashed out was filtered. The solid was washed with excess of water and dried completely to afford the orange oil upon warming to rt. The crude product was purified by prep HPLC (0.1% TFA in ACN : Water) to afford *(S)*-8-((2-amino-4-methylpentyl)oxy)-N,N,4,6-tetramethyl-5-oxo-5,6-dihydrobenzo[*c*][2,7]naphthyridine-9-carboxamide, 2 TFA (3.36 mg, 4.95 µmol, 7 % yield) as a light green solid. LC/MS (ESI) m/e 411.2 [(M+H)⁺, calcd for C₂₃H₃₁N₄O₃ 411.2] LC/MS retention time (method C): *t*_{R} = 2.22 min. HPLC retention time (method A): t_{R} = 10.98 min and HPLC retention time (method B): t_{R} = 11.63 ¹H NMR (400MHz, METHANOL-d₄) δ ppm 8.44 (d, *J*=6.0 Hz, 1H), 8.31 (d, *J*=6.0 Hz, 1H), 8.25 (s, 1H), 6.76 (s, 1H), 3.76 (s, 3H), 3.69 (dd, *J*=4.8, 3.3 Hz, 2H), 3.34 (s, 3H), 3.20 - 3.08 (m, 7H), 1.84 - 1.78 (m, 1H), 1.66 - 1.51 (m, 2H), 1.05 (d, *J*=7.0 Hz, 3H), 0.99 (d, *J*=6.5 Hz, 3H).

### Example 97

### 8-(2-amino-2,4-dimethylpentyloxy)-1-fluoro-6-methylbenzo[c][2,7]naphthyridin-5(6H)-one

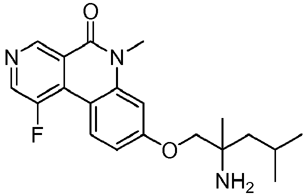

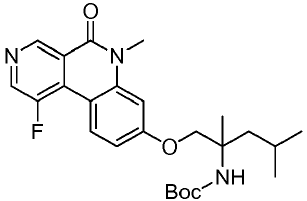

### Part A: tert-butyl 1-(1-fluoro-6-methyl-5-oxo-5,6-dihydrobenzo[c][2,7]naphthyridin-8-yloxy)-2,4-dimethylpentan-2-ylcarbamate

Prepared as described in Example 16, Part H from 8-chloro-1-fluoro-6-methylbenzo[*c*][2,7]naphthyridin-5(*6H*)-one, prepared as described in Example 85, Part E, to afford *(S)*-8-((2-amino-4-methylpentyl)oxy)-7-fluoro-4,6-dimethylbenzo[*c*][2,7]naphthyridin-5(*6H*)-one (35 mg, 0.076 mmol, 50 % yield). LC/MS (ESI) m/e 458.2 [(M+H)⁺, calcd for C₂₅H₃₃FN₃O₄ 458.2]; LC/MS retention time (method C): *t*_{R} = 2.15 min.

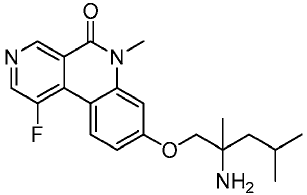

### Part B: 8-(2-amino-2,4-dimethylpentyloxy)-1-fluoro-6-methylbenzo[c][2,7]naphthyridin-5(6H)-one

Prepared as described in Example 2, Part E to afford 8-(2-amino-2,4-dimethylpentyloxy)-1-fluoro-6-methylbenzo[*c*][2,7]naphthyridin-5(*6H*)-one (16.5 mg, 0.035mmol, 45 % yield as a white solid. LC/MS (ESI) m/e 358.2[(M+H)⁺, calcd for C₂₀H₂₅FN₃O₂ 358.2]; LC/MS retention time (method C): *t*_{R} = 2.06 min; HPLC retention time (method A): t_{R} = 11.79 min; HPLC retention time (method B): t_{R} = 6.66 min; ¹H NMR (400MHz, METHANOL-d₄) δ ppm 9.41 (s, 1H), 8.79 (d, *J*=4.8 Hz, 1H), 8.71 (dd, *J*=9.2, 2.9 Hz, 1H), 7.24 (d, *J*=2.3 Hz, 1H), 7.22 - 7.15 (m, 1H), 4.36 (d, *J*=10.3 Hz, 1H), 4.24 (d, *J*=10.3 Hz, 1H), 3.84 (s, 3H), 2.02 - 1.83 (m, 2H), 1.80 - 1.70 (m, 1H), 1.55 (s, 3H), 1.11 (d, *J*=6.5 Hz, 3H), 1.06 (d, *J*=6.3 Hz, 3H).

### Example 98

### (S)-8-((2-amino-4-methylpentyl)oxy)-1-methoxy-6-methylbenzo[c][2,7]naphthyridin-5(6H)-one

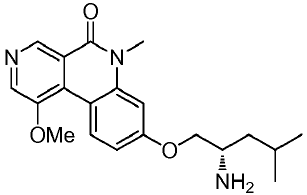

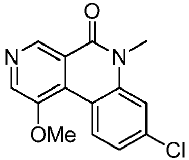

### Part A: 8-chloro-1-methoxy-6-methylbenzo[c][2,7]naphthyridin-5(6H)-one

To the solution of 8-chloro-1-fluoro-6-methylbenzo[*c*][2,7]naphthyridin-5(*6H*)-one (40 mg, 0.152 mmol), prepared as described in Example 85, Part E, in MeOH (0.2 mL) in a microwave vial, 18-crown-6 (4.03 mg, 0.015 mmol) and sodium methoxide (32.9 mg, 0.152 mmol) in methanol were added and heated in a microwave oven at 100 °C for 30 min. Upon completion, the reaction mixture was diluted with water and extracted with ethyl acetate (2 x 5mL). The combined organic layers were washed with brine solution, dried over sodium sulfate, filtered and concentrated under reduced pressure to afford 8-chloro-1-methoxy-6-methylbenzo[*c*][2,7]naphthyridin-5(*6H*)-one (40 mg, 0.146 mmol, 96 % crude yield) as a white solid. The material was carried forward without further purification. LC/MS (ESI) m/e 275.0 [(M+H)⁺, calcd for C₁₄H₁₂ClN₂O₂ 275.05]; LC/MS retention time (method D): *t*_{R} = 0.72 min.

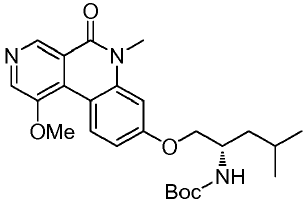

### Part B: (S)-tert-butyl (1-((1-methoxy-6-methyl-5-oxo-5,6-dihydrobenzo[c][2,7]naphthyridin-8-yl)oxy)-4-methylpentan-2-yl)carbamate

To the solution of 8-chloro-1-methoxy-6-methylbenzo[*c*][2,7]naphthyridin-5(*6H*)-one (40 mg, 0.146 mmol) in toluene (0.5 mL) was added cesium carbonate (71.2 mg, 0.218 mmol) and di-*tert*-butyl(2',4',6'-triisopropyl-[1,1'-biphenyl]-2-yl)phosphine (6.18 mg, 0.015 mmol). The solution was purged with N2 for 5 min. To this palladium (II) acetate (1.635 mg, 7.28 µmοl) was added and the solution purged with N2 for 10 min. The resultant mixture was heated at 90 °C for 21 h. After cooling, the reaction mixture was filtered through diatomaceous earth (Celite®), eluting with EtOAc. The filtrate was concentrated under reduced pressure. The crude product was purified by silica gel chromatography using hexanes and ethyl acetate to afford *(S)-tert-butyl* (1-((1-methoxy-6-methyl-5-oxo-5,6-dihydrobenzo[c][2,7]naphthyridin-8-yl)oxy)-4-methylpentan-2-yl)carbamate (40 mg, 0.041 mmol, 28 % yield). LC/MS (ESI) m/e 456.2 [(M+H)⁺, calcd for C₂₅H₃₄N₃O₅ 456.2]; LC/MS retention time (method D): *t*_{R} = 0.9 min.

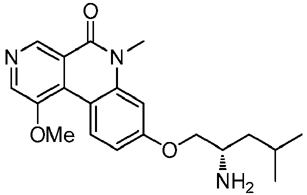

### Part C: (S)-8-((2-amino-4-methylpentyl)oxy)-1-methoxy-6-methylbenzo[c][2,7]naphthyridin-5(6H)-one

To the solution of *(S)-tert-butyl* (1-((1-methoxy-6-methyl-5-oxo-5,6-dihydrobenzo[*c*][2,7]naphthyridin-8-yl)oxy)-4-methylpentan-2-yl)carbamate (40 mg, 0.041 mmol) in DCM (0.9 mL) cooled to 0 °C, TFA (0.2 mL, 2.60 mmol) was added dropwise and stirred for 5 min. The reaction mixture was warmed to rt and stirred for 3 h. The volatiles were removed under reduced pressure. The crude product was purified by prep HPLC using (0.1% TFA in ACN:water) to afford *(S)*-8-((2-amino-4-methylpentyl)oxy)-1-methoxy-6-methylbenzo[*c*][2,7]naphthyridin-5(*6H*)-one, 2 TFA (17.11mg, 0.028 mmol, 69 % yield) as white solid. LC/MS (ESI) m/e 356.0 [(M+H)⁺, calcd for C₂₀H₂₆N₃O₃ 356.2]; LC/MS retention time (method C): *t*_{R} = 1.82 min; HPLC retention time (method A): t_{R} = 9.44 min; HPLC retention time (method B): t_{R} = 9.78 min; ¹H NMR (400MHz, METHANOL-*d₄*) δ ppm 9.17 (s, 1H), 9.14 (d, *J*=9.0 Hz, 1H), 8.54 (s, 1H), 7.09 (d, *J*=2.5 Hz, 1H), 7.02 (dd, *J*=9.0, 2.5 Hz, 1H), 4.21 (s, 3H), 4.19 - 4.15 (m, 1H), 4.04 - 3.95 (m, 2H), 3.78 (s, 3H), 1.97 - 1.80 (m, 1H), 1.51 - 1.42 (m, 2H), 1.04 (d, *J*=6.5 Hz, 3H), 1.02 (d, *J*=6.5 Hz, 3H).

### Example 99

### (S)-8-(2-amino-4-methylpentyloxy)-7-methoxy-4-methylbenzo[c][2,7]naphthyridin-5(6H)-one

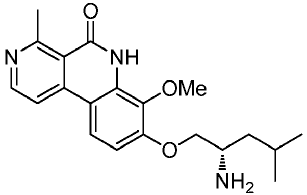

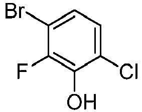

### Part A. 3-bromo-6-chloro-2-fluorophenol

To a stirred solution of 1-bromo-4-chloro-2-fluorobenzene (5 g, 23.87 mmol) in tetrahydrofuran (40 mL) cooled to -78 °C was added LDA (14.92 mL, 29.8 mmol) dropwise. The reaction mixture was stirred at this temperature for 30 min. then allowed to warm to -20 °C and stirred for 30 min. The reaction was then cooled to - 78 °C and trimethyl borate (3.47 mL, 31.0 mmol) dissolved in THF (5 mL) was added dropwise. The reaction mixture was warmed to -20°C and stirred for 1 h. The reaction mixture was then cooled to -78 °C and peracetic acid (16 mL, 84 mmol) as slowly added dropwise. The mixture was allowed to warm to rt and stirred for 12 h. The reaction mixture was again cooled to 0 °C and quenched with 5% ammonium chloride The solution was extracted with ethyl acetate (2x50 mL). The combined organic layers were washed with brine (50 mL), dried over sodium sulfate, filtered and concentrated under reduced pressure to afford 3-bromo-6-chloro-2-fluorophenol (4.99 g, 18.25 mmol, 76 % crude yield) as ayellow oil. The material was carried forward without further purification.LC/MS (ESI) m/e 225.1 [(M+H)⁺, calcd for C₆H₄BrClFO 224.9]; LC/MS retention time (method E): *t*_{R} = 0.87 min.

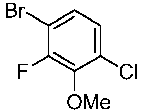

### Part B. 1-bromo-4-chloro-2-fluoro-3-methoxybenzene

To a stirred solution of 3-bromo-6-chloro-2-fluorophenol (4.2 g, 18.63 mmol) in acetonitrile (35 mL) was added potassium carbonate (5.15 g, 37.3 mmol) followed by methyl iodide (2.330 mL, 37.3 mmol) dropwise at rt. The reaction mixture was heated to 85 °C for 3 h. After cooling, the volatiles were concentrated under reduced pressure and the residue was diluted with water (50 mL) and extracted with ethyl acetate (2x80 mL). The combined organic layers were washed with brine (100 mL), dried over sodium sulfate, filtered and concentrated under reduced pressure to afford 1-bromo-4-chloro-2-fluoro-3-methoxybenzene (4 g, 16.7 mmol, 90% crude yield) as a brown solid. The material was carried forward without further purification. ¹H NMR (400MHz, *CDCl₃*) δ ppm 7.2 (m, 1 H), 7.04 (m, 1 H), 3.98 (s, 3 H).

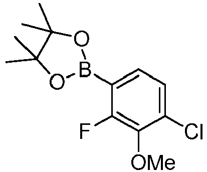

### Part C. 2-(4-chloro-2-fluoro-3-methoxyphenyl)-4,4,5,5-tetramethyl-1,3,2-dioxaborolane

To a stirred solution of 1-bromo-4-chloro-2-fluoro-3-methoxybenzene (3 g, 12.53 mmol) in tetrahydrofuran (20 mL) cooled to -10 °C was added isopropylmagnesium bromide (5.18 mL, 15.03 mmol) dropwise and the reaction mixture was stirred at -10 °C for 1 h. The reaction mixture was then warmed to 0 °C and stirred for 1 h. The reaction was then cooled to -10 °C and 2-isopropoxy-4,4,5,5-tetramethyl-1,3,2-dioxaborolane (2.56 mL, 12.53 mmol) was slowly added dropwise. The reaction mixture was allowed to warm to room temperature and stirred for 16 h. The reaction mixture was quenched with 5% aqueous sodium hydroxide and extracted with ethyl acetate (2x25 mL). The combined organic layers were washed with brine solution, dried over sodium sulfate, filtered and concentrated under reduced pressure to afford 2-(4-chloro-2-fluoro-3-methoxyphenyl)-4,4,5,5-tetramethyl-1,3,2-dioxaborolane (2.3 g, 8.03 mmol, 64% crude yield) as a brown oil. The material was carried forward without further purification. ¹H NMR (400MHz, *CDCl₃*) δ ppm 7.35 (m, 1 H), 7.15 (m, 1 H), 3.95 (s, 3 H), 1.33 (s, 6 H), 1.23 (s, 6 H).

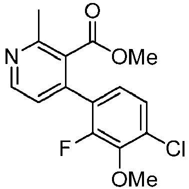

### Part D. methyl 4-(4-chloro-2-fluoro-3-methoxyphenyl)-2-methylnicotinate

A mixture of methyl 4-chloro-2-methylnicotinate (1.5 g, 8.08 mmol), 2-(4-chloro-2-fluoro-3-methoxyphenyl)-4,4,5,5-tetramethyl-1,3,2-dioxaborolane (2.316 g, 8.08 mmol), phosphoric acid, potassium salt (3.43 g, 16.16 mmol) and PdCl₂(dppf)-CH₂Cl₂ adduct (0.660 g, 0.808 mmol) in 1,4-dioxane (25 mL) and water (5 mL) was purged with nitrogen for 5 min. The reaction was then heated to 100 °C for 18 h. After cooling, the reaction was diluted with water (50 mL) and extracted with ethyl acetate (2x75 mL). The combined organic layers were washed with brine solution, dried over sodium sulfate, filtered and concentrated. The residue was purified by silica gel chromatography using 50% ethyl acetate in hexanes to yield methyl 4-(4-chloro-2-fluoro-3-methoxyphenyl)-2-methylnicotinate (1.2 g, 2.52 mmol, 31% yield) as pale brown oil. LC/MS (ESI) m/e 309.9 [(M+H)⁺, calcd for C₁₅H₁₄ClFNO₃ 310.1]; LC/MS retention time (method D): *t*_{R} = 0.84 min.

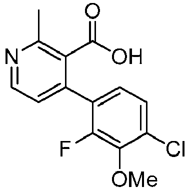

### Part E. 4-(4-chloro-2-fluoro-3-methoxyphenyl)-2-methylnicotinic acid

Prepared as described in Example 16, Part E by hydrolysis of the methyl 4-(4-chloro-2-fluoro-3-methoxyphenyl)-2-methylnicotinate to afford 4-(4-chloro-2-fluoro-3-methoxyphenyl)-2-methylnicotinic acid (780 mg, 2.137 mmol, 93 % yield) as a brown oil. LC/MS (ESI) m/e 295.9 [(M+H)⁺, calcd for C₁₄H₁₂ClFNO₃ 296.04]; LC/MS retention time (method D): *t*_{R} = 0.61 min.

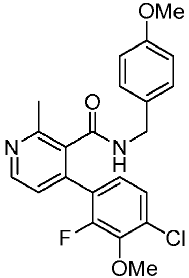

### Part F. 4-(4-chloro-2-fluoro-3-methoxyphenyl)-N-(4-methoxybenzyl)-2-methylnicotinamide

To a stirred solution of (4-methoxyphenyl)methanamine (0.437 g, 3.18 mmol) in CH₂Cl₂ (25 mL) was added DIEA (0.556 mL, 3.18 mmol) and the reaction mixture was stirred at 0 °C for 15 min. 4-(4-chloro-2-fluoro-3-methoxyphenyl)-2-methylnicotinoyl chloride (1 g, 3.18 mmol) (prepared by treatment of carboxylic acid with thionyl chloride) dissolved in 10 mL of DCM was then added to the reaction mixture and the reaction was stirred at rt for 10 h. The reaction was quenched by addition of water (50 mL) and the solution was extracted with DCM (2x50 mL). The combined organic layers were washed with brine solution 50 mL, dried over sodium sulfate, filtered and concentrated under reduced pressure. The residue was purified by silica gel chromatography using ethyl acetate in hexanes as eluant to yield 4-(4-chloro-2-fluoro-3-methoxyphenyl)-N-(4-methoxybenzyl)-2-methylnicotinamide (800 mg, 1.109 mmol, 35% yield) as a brown oil. LC/MS (ESI) m/e 415.1 [(M+H)⁺, calcd for C₂₂H₂₁ClFN₂O₃ 415.1]; LC/MS retention time (method D): *t*_{R} = 0.78 min.

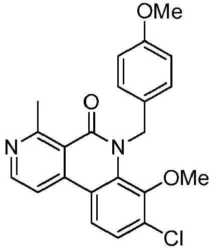

### Part G. 8-chloro-7-methoxy-6-(4-methoxybenzyl)-4-methylbenzo[c][2,7]naphthyridin-5(6H)-one

Prepared as described in Example 2, Part C from 4-(4-chloro-2-fluoro-3-methoxyphenyl)-N-(4-methoxybenzyl)-2-methylnicotinamide to afford 8-chloro-7-methoxy-6-(4-methoxybenzyl)-4-methylbenzo[*c*][2,7]naphthyridin-5(*6H*)-one (110 mg, 0.245 mmol, 34% yield) as yellow solid. LC/MS (ESI) m/e 395.1 [(M+H)⁺, calcd for C₂₂H₂₀ClN₂O₃ 395.1] LC/MS retention time (method D): *t*_{R} = 0.81 min.

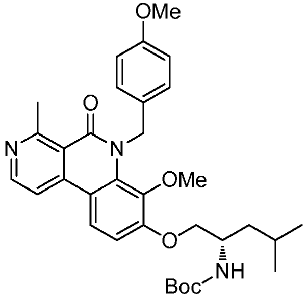

### Part H. (S)-tert-butyl 1-(7-methoxy-6-(4-methoxybenzyl)-4-methyl-5-oxo-5,6-dihydrobenzo[c][2,7]naphthyridin-8-yloxy)-4-methylpentan-2-ylcarbamate

Prepared as described in Example 16, Part H to afford the title compound (325 mg, 0.119 mmol, 49% crude yield) as a white solid. The material was carried forward without purification. LC/MS (ESI) m/e 576.3 [(M+H)⁺, calcd for C₃₃H₄₂N₃O₆ 576.3]; LC/MS retention time (method D): *t*_{R} = 0.99 min.

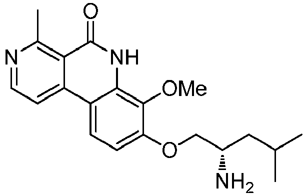

### Part I. (S)-8-(2-amino-4-methylpentyloxy)-7-methoxy-4-methylbenzo[c][2,7]naphthyridin-5(6H)-one

Prepared as described in Example 2, Part E to afford *(S)*-8-(2-amino-4-methylpentyloxy)-7-methoxy-4-methylbenzo[*c*][2,7]naphthyridin-5(*6H*)-one (12.3 mg, 0.033 mmol, 28% yield) as a white solid. LC/MS (ESI) m/e 356.2 [(M+H)⁺, calcd for C₂₀H₂₆N₃O₃ 356.2]; LC/MS retention time (method C): *t*_{R} = 1.49 min; HPLC retention time (method A): t_{R} = 7.36 min; HPLC retention time (method B): t_{R} = 8.52 min; ¹H NMR (400MHz, METHANOL-*d₄*) δ ppm 8.59 (d, *J*=5.5 Hz, 1H), 8.14 (d, *J*=5.5 Hz, 1H), 8.10 (d, *J*=9.0 Hz, 1H), 7.11 (d, *J*=9.0 Hz, 1H), 4.18 (dd, *J*=9.3, 4.3 Hz, 1H), 4.08 - 4.03 (m, 1H), 4.02 (s, 3H), 4.01 - 3.96 (m, 1H), 3.10 (s, 3H), 1.91 - 1.80 (m, 1H), 1.57 - 1.40 (m, 2H), 1.03 (d, *J*=6.5 Hz, 3H), 1.01 (d, *J*=7.0 Hz, 3H).

### Example 100

### (S)-8-(2-amino-4-methylpentyloxy)-9-bromo-7-fluoro-4-methylbenzo[c][2,7]naphthyridin-5(6H)-one

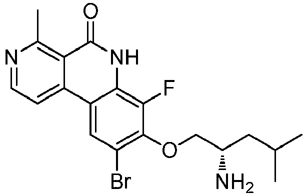

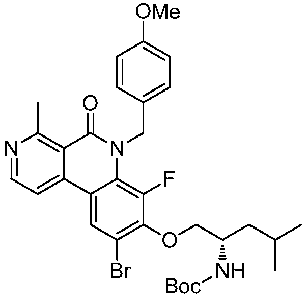

### Part A: (S)-tert-butyl 1-(9-bromo-7-fluoro-6-(4-methoxybenzyl)-4-methyl-5-oxo-5,6-dihydrobenzo[c][2,7]naphthyridin-8-yloxy)-4-methylpentan-2-ylcarbamate

Prepared from *(S)*-*tert*-butyl 1-(7-fluoro-6-(4-methoxybenzyl)-4-methyl-5-oxo-5,6-dihydrobenzo[*c*][2,7]naphthyridin-8-yloxy)-4-methylpentan-2-ylcarbamate prepared as described in Example 82, Part C, using the method described in Example 3, Part A to afford title compound (120 mg, 0.065 mmol, 44% yield). LC/MS (ESI) m/e 642.4 [(M+H)⁺, calcd for C₃₂H₃₈BrFN₃O₅ 642.2]; LC/MS retention time (method E): *t*_{R} = 1.26 min.

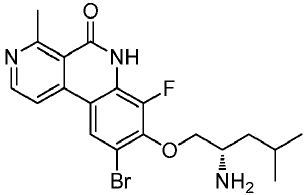

### Part B: (S)-8-(2-amino-4-methylpentyloxy)-9-bromo-7-fluoro-4-methylbenzo[c][2,7]naphthyridin-5(6H)-one

Prepared as described in Example 2, Part E to afford *(S)*-8-(2-amino-4-methylpentyloxy)-9-bromo-7-fluoro-4-methylbenzo[*c*][2,7]naphthyridin-5(*6H*)-one (3.5 mg, 8.22 µmοl, 13 % yield) as an off-white solid. LC/MS (ESI) m/e 423.0 [(M+H)⁺, calcd for C₁₉H₂₂BrFN₃O₂ 423.3]; LC/MS retention time (method C): *t*_{R} = 2.10 min; HPLC retention time (method A): t_{R} = 8.58 min; HPLC retention time (method B): t_{R} = 9.08 min; ¹H NMR (400MHz, METHANOL-*d₄*) δ ppm 8.73 (d, *J*=6.0 Hz, 1H), 8.57 (d, *J*=2.0 Hz, 1H), 8.40 (d, *J*=6.0 Hz, 1H), 4.51 (dd, *J*=10.3, 2.5 Hz, 1H), 4.38 (dd, *J*=10.4, 5.6 Hz, 1H), 3.78 - 3.73 (m, 1H), 3.17 (s, 3H), 1.94 - 1.82 (m, 2H), 1.73 - 1.60 (m, 1H), 1.08 (d, *J*=2.5 Hz, 3H), 1.06 (d, *J*=2.8 Hz, 3H).

### Example 101

### (S)-8-(2-amino-4-methylpentyloxy)-9-fluoro-4-methylbenzo[c][2,7]naphthyridin-5(6H)-one

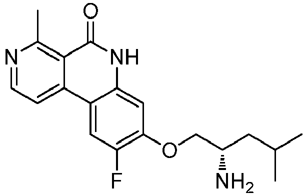

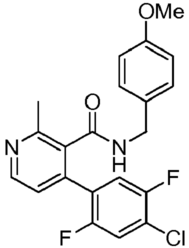

### Part A: 4-(4-chloro-2,5-difluorophenyl)-N-(4-methoxybenzyl)-2-methylnicotinamide

Prepared as described in Example 82, Part A from 4-(4-chloro-2,5-difluorophenyl)-2-methylnicotinic acid (prepared as described in Example 70, Part D) to afford title compound (440 mg, 1.015 mmol, 87 % yield) as an off-white solid. LC/MS (ESI) m/e 403.1 [(M+H)⁺, calcd for C₂₁H₁₈ClF₂N₂O₂ 403.1]; LC/MS retention time (method E): *t*_{R} = 0.93 min.

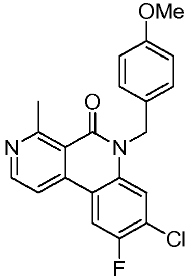

### Part B: 8-chloro-9-fluoro-6-(4-methoxybenzyl)-4-methylbenzo[c][2,7]naphthyridin-5(6H)-one

Prepared as described in Example 73, Part B to afford 8-chloro-9-fluoro-6-(4-methoxybenzyl)-4-methylbenzo[*c*][2,7]naphthyridin-5(*6H*)-one (260mg, 0.669 mmol, 61 % yield) as a white solid. LC/MS (ESI) m/e 383.0 [(M+H)⁺, calcd for C₂₁H₁₇ClFN₂O₂ 383.1]; LC/MS retention time (method C): *t*_{R} = 2.04 min.

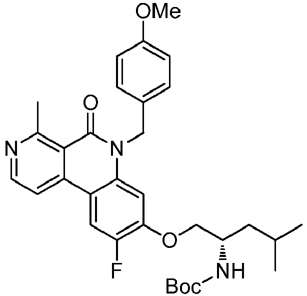

### Part C: (S)-tert-butyl 1-(9-fluoro-6-(4-methoxybenzyl)-4-methyl-5-oxo-5,6-dihydrobenzo[c][2,7]naphthyridin-8-yloxy)-4-methylpentan-2-ylcarbamate

Prepared as described in Example 16, Part H to afford *(S)*-*tert*-butyl 1-(9-fluoro-6-(4-methoxybenzyl)-4-methyl-5-oxo-5,6-dihydrobenzo[*c*][2,7]naphthyridin-8-yloxy)-4-methylpentan-2-ylcarbamate (0.2 g, 0.244 mmol, 62% yield). LC/MS (ESI) m/e 564.4 [(M+H)⁺, calcd for C₃₂H₃₉FN₃O₅ 564.3]; LC/MS retention time (method E): *t*_{R} = 1.25 min.

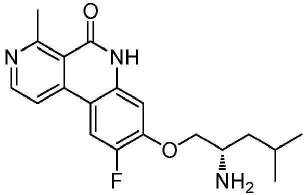

### Part D: (S)-8-(2-amino-4-methylpentyloxy)-9-fluoro-4-methylbenzo[c][2,7]naphthyridin-5(6H)-one

Prepared as described in Example 2, Part E to afford title compound (55 mg, 0.158 mmol, 87 % yield) as a white solid. LC/MS (ESI) m/e 344.0 [(M+H)⁺, calcd for C₁₉H₂₃FN₃O₂ 344.2]; LC/MS retention time (method C): *t*_{R} = 1.76 min; HPLC retention time (method A): t_{R} = 7.88 min; HPLC retention time (method B): t_{R} = 7.92 min; ¹H NMR (400MHz, METHANOL-*d₄*) δ ppm 8.60 (d, *J*=5.8 Hz, 1H), 8.15 (d, *J*=12.0 Hz, 1H), 8.08 (d, *J*=5.8 Hz, 1H), 6.99 (d, *J*=7.5 Hz, 1H), 4.22 - 4.12 (m, 1H), 4.06 - 3.92 (m, 1H), 3.40 (br. s., 1H), 3.10 (s, 3H), 1.92 - 1.80 (m, 1H), 1.59 - 1.41 (m, 2H), 1.03 (d, *J*=6.5 Hz, 3H), 1.01 (d, *J*=6.5 Hz, 3H).

### Example 102

### 8-(2-amino-2,4-dimethylpentyloxy)-9-fluoro-4-methylbenzo[c][2,7]naphthyridin-5(6H)-one

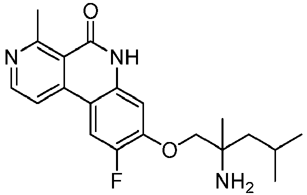

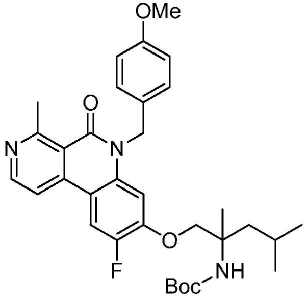

### Part A. tert-butyl 1-(9-fluoro-6-(4-methoxybenzyl)-4-methyl-5-oxo-5,6-dihydrobenzo[c][2,7]naphthyridin-8-yloxy)-2,4-dimethylpentan-2-ylcarbamate

Prepared from coupling of 8-chloro-9-fluoro-6-(4-methoxybenzyl)-4-methylbenzo[*c*][2,7]naphthyridin-5(*6H*)-one (synthesis described in Example 101, Part C) following the procedure described in Example 16, Part H to afford title compound (0.130 g, 0.123 mmol, 47% yield) as a brown oil. LC/MS (ESI) m/e 578.5 [(M+H)⁺, calcd for C₃₃H₄₁FN₃O₅ 578.3]; LC/MS retention time (method E): *t*_{R} = 1.28 min.

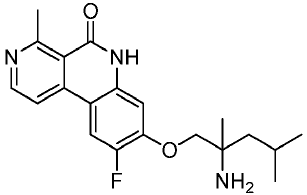

### Part B. 8-(2-amino-2,4-dimethylpentyloxy)-9-fluoro-4-methylbenzo[c][2,7]naphthyridin-5(6H)-one

Prepared as described in Example 2, Part E to afford title compound (10 mg, 0.017 mmol, 22% yield) as a pale yellow solid. LC/MS (ESI) m/e 358.0 [(M+H)⁺, calcd for C₂₀H₂₅FN₃O₂ 358.2]; LC/MS retention time (method C): *t*_{R} = 1.69 min; HPLC retention time (method A): t_{R} = 7.87 min; HPLC retention time (method B): t_{R} = 8.59 min; ¹H NMR (400MHz, METHANOL-*d₄*) δ ppm 8.67 (d, *J*=6.3 Hz, 1H), 8.39 (d, *J*=6.3 Hz, 1H), 8.32 (d, *J*=12.0 Hz, 1H), 7.08 (d, *J*=7.3 Hz, 1H), 4.44 - 4.15 (m, 2H), 3.37 - 3.35 (m, 1H), 3.19 (s, 3H), 1.97 - 1.83 (m, 2H), 1.81 - 1.70 (m, 1H), 1.56 (s, 2H), 1.10 (d, *J*=6.3 Hz, 3H), 1.06 (d, *J*=6.3 Hz, 3H).

### Example 103

### (S)-8-(2-amino-4-methylpentyloxy)-9-chloro-7-fluoro-4-methylbenzo[c][2,7]naphthyridin-5(6H)-one

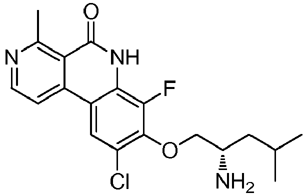

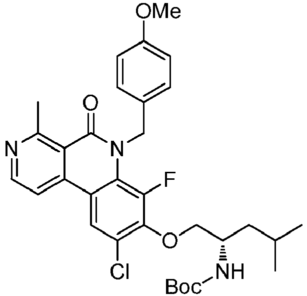

### Part A: (S)-tert-butyl 1-(9-chloro-7-fluoro-6-(4-methoxybenzyl)-4-methyl-5-oxo-5,6-dihydrobenzo[c][2,7]naphthyridin-8-yloxy)-4-methylpentan-2-ylcarbamate

Prepared as described in Example 41, Part A from (*S*)-*tert*-butyl (1-((7-fluoro-6-(4-methoxybenzyl)-4-methyl-5-oxo-5,6-dihydrobenzo[*c*][2,7]naphthyridin-8-yl)oxy)-4-methylpentan-2-yl)carbamate (synthesis described in Example 82, Part C) to afford title compound (2135 mg, 0.063 mmol, 60% crude yield) as a yellow solid. The material was carried forward without purification. LC/MS (ESI) m/e 598.3 [(M+H)⁺, calcd for C₃₂H₃₈ClFN₃O₅ 598.2]; LC/MS retention time (method E): *t*_{R} = 1.32 min.

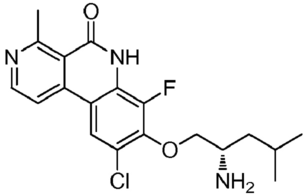

### Part B. (S)-8-(2-amino-4-methylpentyloxy)-9-chloro-7-fluoro-4-methylbenzo[c][2,7]naphthyridin-5(6H)-one

To a solution of *(S)*-*tert*-butyl (1-((9-chloro-7-fluoro-6-(4-methoxybenzyl)-4-methyl-5-oxo-5,6-dihydrobenzo[*c*][2,7]naphthyridin-8-yl)oxy)-4-methylpentan-2-yl)carbamate (.08 g, 0.037 mmol) in TFA (8 mL) cooled to 0 °C was added methanesulfonic acid (0.243 µl, 3.75 µmol). The reaction mixture was then heated at 70 °C for 2h. The volatiles were evaporated. The residue was taken up in water and neutralized with 10% aqueous NaHCO3. The solution was extracted with EtOAc (3x15 mL). The combined organic layers were washed with brine (1x15 mL), dried over Na2SO4, filtered and concentrated under reduced pressure. The residue was purified by preparative HPLC (Column:X-Bridge(19X150mm) 5µm, Flow rate:15ml/min; Solvent A:0.01% TFA, Solvent B:ACN) to affored (*S*)-8-((2-amino-4-methylpentyl)oxy)-9-chloro-7-fluoro-4-methylbenzo[*c*][2,7]naphthyridin-5(6*H*)-one, 3 TFA (9.5mg, 0.013 mmol, 34.4 % yield) as an off-white solid. LC/MS (ESI) m/e 378.0 [(M+H)⁺, calcd for C₁₉H₂₂ClFN₃O₂ 378.1]; LC/MS retention time (method C): *t*_{R} = 2.02 min; HPLC retention time (method A): t_{R} = 7.47 min; HPLC retention time (method B): t_{R} = 8.41 min; ¹H NMR (400MHz, METHANOL-*d*₄) δ ppm 8.70 (d, *J*=6.0 Hz, 1H), 8.40 (d, *J*=2.0 Hz, 1H), 8.35 (d, *J*=6.3 Hz, 1H), 4.49 (dd, *J*=10.7, 2.6 Hz, 1H), 4.35 (dd, *J*=10.5, 5.8 Hz, 1H), 3.75 - 3.69 (m, 1H), 3.14 (s, 3H), 1.90 - 1.74 (m, 2H), 1.70 - 1.56 (m, 1H), 1.04 (d, *J*=2.3 Hz, 3H), 1.03 (d, *J*=2.5 Hz, 3H).

### Example 104

### (S)-8-(2-amino-4-methylpentyloxy)-9-(methoxymethyl)-4,6-dimethylbenzo[c][2,7]naphthyridin-5(6H)-one

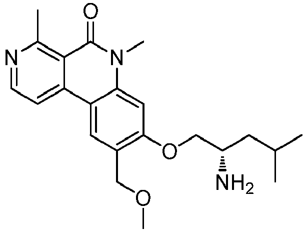

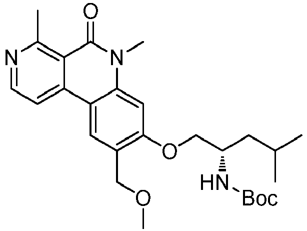

### Part A. (S)-tert-butyl 1-(9-(methoxymethyl)-4,6-dimethyl-5-oxo-5,6-dihydrobenzo[c][2,7]naphthyridin-8-yloxy)-4-methylpentan-2-ylcarbamate

Prepared as described in Example 16, Part H to afford title compound *(S)-tert*-butyl (1-((9-(methoxymethyl)-4,6-dimethyl-5-oxo-5,6-dihydrobenzo[*c*][2,7]naphthyridin-8-yl)oxy)-4-methylpentan-2-yl)carbamate (100 mg, 0.066 mmol, 84 % yield). LC/MS (ESI) m/e 484.4 [(M+H)⁺, calcd for C₂₇H₃₈N₃O₅ 484.3]; LC/MS retention time (method B): *t*_{R} = 1.65 min.

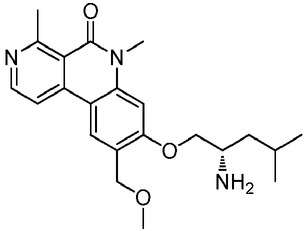

### Part B. (S)-8-(2-amino-4-methylpentyloxy)-9-(methoxymethyl)-4,6-dimethylbenzo[c][2,7]naphthyridin-5(6H)-one

Prepared as described in Example 2, Part E to afford title compound (9 mg, 0.022 mmol, 33 % yield) as a yellow solid. LC/MS (ESI) m/e 384.2 [(M+H)⁺, calcd for C₂₂H₃₀N₃O₃ 384.2]; LC/MS retention time (method C): *t*_{R} = 1.66 min; HPLC retention time (method A): t_{R} = 7.96 min; HPLC retention time (method B): t_{R} = 9.01 min; ¹H NMR (400MHz, METHANOL-d₄) δ ppm 8.54 (d, *J*=5.9 Hz, 1H), 8.33 (s, 1H), 8.11 (d, *J*=5.8 Hz, 1H), 7.00 (s, 1H), 4.73 - 4.55 (m, 2H), 4.33 (dd, *J*=10.0, 3.5 Hz, 1H), 4.13 (dd, *J*=9.9, 6.8 Hz, 1H), 3.75 (s, 3H), 3.59 - 3.50 (m, 1H), 3.47 (s, 3H), 3.06 (s, 3H), 1.97 - 1.77 (m, 1H), 1.69 - 1.45 (m, 2H), 1.04 (d, *J*=4.7 Hz, 3H), 1.02 (d, *J*=4.7 Hz, 3H).

### Example 105

### 8-(2-amino-2,4-dimethylpentyloxy)-7-fluoro-4-methylbenzo[c][2,7]naphthyridin-5(6H)-one

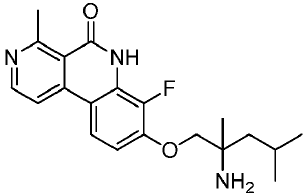

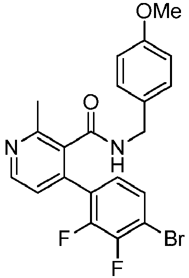

### Part A. 4-(4-bromo-2,3-difluorophenyl)-N-(4-methoxybenzyl)-2-methylnicotinamide

Prepared as described in Example 82, Part A to afford 4-(4-bromo-2,3-difluorophenyl)-*N*-(4-methoxybenzyl)-2-methylnicotinamide (0.320 g, 0.693 mmol, 97 % yield) as an off-white solid. LC/MS (ESI) m/e 447.0 [(M+H)⁺, calcd for C₂₁H₁₈BrF₂N₂O₂ 447.0]; LC/MS retention time (method E): *t*_{R} = 0.95 min.

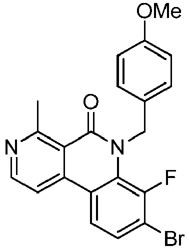

### Part B. 8-bromo-7-fluoro-6-(4-methoxybenzyl)-4-methylbenzo[c][2,7]naphthyridin-5(6H)-one

Prepared as described in Example 2, Part C to afford 8-bromo-7-fluoro-6-(4-methoxybenzyl)-4-methylbenzo[*c*][2,7]naphthyridin-5(*6H*)-one (0.12 g, 0.269 mmol, 90 % yield) as an off-white solid. LC/MS (ESI) m/e 427.1 [(M+H)⁺, calcd for C₂₁H₁₇BrFN₂O₂ 427.0]; LC/MS retention time (method E): *t*_{R} = 1.15 min.

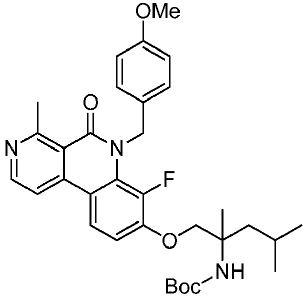

### Part C. tert-butyl 1-(7-fluoro-6-(4-methoxybenzyl)-4-methyl-5-oxo-5,6-dihydrobenzo[c][2,7]naphthyridin-8-yloxy)-2,4-dimethylpentan-2-ylcarbamate

Prepared as described in Example 16, Part H to afford (R)-*tert*-butyl (1-((7-fluoro-6-(4-methoxybenzyl)-4-methyl-5-oxo-5,6-dihydrobenzo[*c*][2,7]naphthyridin-8-yl)oxy)-2,4-dimethylpentan-2-yl)carbamate (0.025 g, 0.042 mmol, 13 % yield). LC/MS (ESI) m/e 578.5 [(M+H)⁺, calcd for C₃₃H₄₁FN₃O₅ 578.3]; LC/MS retention time (method E): *t*_{R} = 1.28 min.

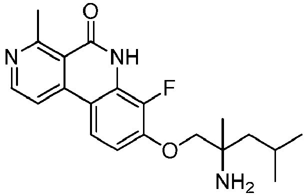

### Part D. 8-(2-amino-2,4-dimethylpentyloxy)-7-fluoro-4-methylbenzo[c][2,7]naphthyridin-5(6H)-one

Prepared as described in Example 17, Part I to afford 8-(2-amino-2,4-dimethylpentyloxy)-7-fluoro-4-methylbenzo[*c*][2,7]naphthyridin-5(6*H*)-one (0.013 g, 0.030 mmol, 69 % yield) as an off-white solid. LC/MS (ESI) m/e 358.0 [(M+H)⁺, calcd for C₂₀H₂₅FN₃O₂ 358.2]; HPLC retention time (method A): t_{R} = 7.71 min; HPLC retention time (method B): t_{R} = 8.57 min; ¹H NMR (400MHz, METHANOL-*d*₄) δ ppm 8.75 - 8.70 (m, 1H), 8.70 - 8.63 (m, 1H), 8.36 (dd, *J*=9.3, 1.8 Hz, 1H), 7.34 (dd, *J*=9.3, 7.8 Hz, 1H), 4.47 - 4.39 (m, 1H), 4.38 - 4.30 (m, 1H), 3.26 (s, 3H), 1.96 - 1.85 (m, 2H), 1.81 - 1.70 (m, 1H), 1.55 (s, 3H), 1.10 (d, *J*=6.5 Hz, 3H), 1.06 (d, *J*=6.0 Hz, 3H).

### Example 106

### (S)-8-((2-amino-5,5-difluoropent-4-en-1-yl)oxy)-4,6-dimethyl-5-oxo-5,6-dihydrobenzo[c][2,7]naphthyridine-9-carbonitrile

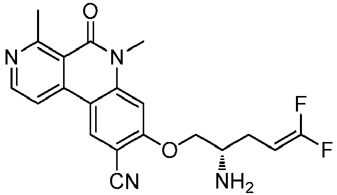

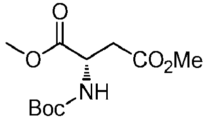

### Part A: (S)-dimethyl 2-((tert-butoxycarbonyl)amino)succinate

A 0 °C suspension of L-aspartic acid (10.1 g, 76.0 mmol) in anhydrous methanol (100 mL) was treated with trimethylsilyl chloride (24.7 g, 228 mmol) via rapid dropwise addition. The cooling bath was removed and the resulting solution stirred at ambient temperature overnight (16 h) and then concentrated under reduced pressure. The resulting oil was taken up in dichloromethane (100 mL), treated with di-*t*-butyl dicarbonate (17.4 g, 80.1 mmol) and diisopropylethylamine (26.5 mL, 152 mmol) and stirred at ambient temperature overnight (16 h). The resulting solution was washed with 0.25 N aqueous hydrochloric acid (3x50 mL), 0.25 N aqueous sodium hydroxide (3x50 mL), and brine (1x50 mL); dried over MgSO₄ and concentrated under reduced pressure to afford *(S)*-dimethyl 2-((*tert-*butoxycarbonyl)amino)succinate (15.9 g, 60.9 mmol, 80% crude yield) as a near colorless oil. The crude product was used in the next step without further purification. ¹H NMR (400MHz, *CDCl₃*) δ ppm 5.50 (br. s., 1H), 4.60 (br. s., 1H), 3.78 (s, 3H), 3.71 (s, 3H), 3.02 (dd, *J*=16.9, 4.4 Hz, 1H), 2.84 (dd, *J*=16.8, 4.8 Hz, 1H), 1.47 (s, 9H).

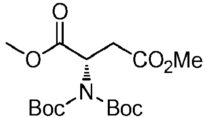

### Part B: (S)-dimethyl 2-[bis(tert-butoxycarbonyl)amino]butane-1,4-dioate

A 0 °C solution of *(S)*-dimethyl 2-((*tert*-butoxycarbonyl)amino)succinate (15.8 g, 60.5 mmol) in acetonitrile (200 mL) was treated with di-*t*-butyl dicarbonate (13.9 g, 63.5 mmol) and 4-dimethylaminopyridine (1.48 g, 12.1 mmol). The cooling bath was removed and the reaction solution stirred at ambient temperature overnight (18 h). The resulting solution was concentrated under reduced pressure. The residue was dissolved in ethyl acetate (200 mL), washed with 0.25 N aqueous hydrochloric acid (2x50 mL), 0.25N aqueous sodium hydroxide (2x50 mL), and brine (1x50 mL); dried over magnesium sulfate and concentrated under reduced pressure to afford *(S)-*dimethyl 2-[bis(*tert*-butoxycarbonyl)amino]butane-1,4-dioate (18.8 g, 52.0 mmol, 86% crude yield) as a pale yellow oil. The crude product was used without further purification. ¹H NMR (400MHz, *CDCl₃*) δ ppm 5.46 (t, *J*=6.8 Hz, 1H), 3.72 (s, 3H), 3.74 (s, 3H), 3.26 (dd, *J*=16.4, 7.2 Hz, 1H), 2.75 (dd, *J*=16.3, 6.5 Hz, 1H), 1.52 (s, 18H).

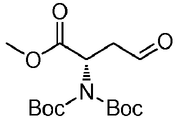

### Part C: (S)-methyl-2-{(tert-butoxy)-N-[(tert-butyl)oxycarbonyl]carbonylamino}-4-oxobutanoate

A -78 °C solution of *(S)*-dimethyl 2-[bis(*tert*-butoxycarbonyl)amino]butane-1,4-dioate (9.07 g, 25.1 mmol) in diethyl ether (100 mL) was treated dropwise with 1 M diisobutyl aluminum hydride in dichloromethane (37.6 mL, 37.6 mmol). The resulting solution was stirred for 15 min and then quenched sequentially in 15 minutes intervals with water (3.3 mL), IN aqueous sodium hydroxide (10 mL), and again with water (3.3 mL). The resulting suspension was filtered through diatomaceous earth (Celite®) and concentrated under reduced pressure to afford *(S)-*methyl-2-{(*tert*-butoxy)-*N*-[(*tert*-butyl)oxycarbonyl]carbonylamino}-4-oxobutanoate (8.26 g, 24.9 mmol, 99% crude yield) as a colorless oil. The crude product was used without further purification. ¹H NMR (400MHz, *CDCl₃*) δ ppm 9.81 (s, 1H), 5.55 (t, *J*=6.4 Hz, 1H), 3.75, (s, 3H), 3.43 (ddd, *J*=18.0, 5.9, 1.0 Hz, 1H), 2.85 (ddd, *J*=17.9, 6.0, 1.0 Hz, 1H), 1.52 (s, 18H).

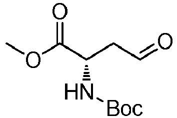

### Part D: (S)-methyl 2-((tert-butoxycarbonyl)amino)-4-oxobutanoate

A solution of *(S)*-methyl-2-{(*tert*-butoxy)-*N*-[(*tert-*butyl)oxycarbonyl]carbonylamino}-4-oxobutanoate (29.2 g, 88.0 mmol) in acetonitrile (300 mL) was treated with lithium bromide (11.5 g, 132 mmol), heated to reflux for 2 h, and then concentrated under reduced pressure. The residue was dissolved in ethyl acetate (300 mL), washed with water (1x50 mL) and brine (1x50 mL); dried over magnesium sulfate, and concentrated under reduced pressure to a dark amber oil. The crude material was purified over SiO₂ (20-100 % ethyl acetate/hexanes gradient elution) to afford *(S)*-methyl 2-((*tert-*butoxycarbonyl)amino)-4-oxobutanoate (12.5 g, 54.1 mmol, 62% crude yield) as a pale amber oil. ¹H NMR (400MHz, *CDCl₃*) δ ppm 9.76 (s, 1H), 5.41 (br. s., 1H), 4.62 (dt, *J*=8.1, 4.4 Hz, 1H), 3.77 (s, 3H), 3.19 - 2.94 (m, 2H), 1.46 (s, 9H).

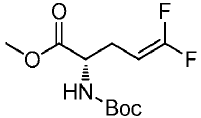

### Part E: (S)-methyl 2-((tert-butoxycarbonyl)amino)-5,5-difluoropent-4-enoate

A solution of *(S)*-methyl 2-((*tert*-butoxycarbonyl)amino)-4-oxobutanoate (5.97 g, 25.8 mmol), sodium 2-chloro-2,2-difluoroacetate (11.8 g, 77.0 mmol), triphenylphosphine (20.3 g, 77.0 mmol), and *N,N*-dimethylformamide (50 mL) was charged to a 500 mL 3-necked flask and heated to 115 °C for 15 min. The resulting mixture was filtered through diatomaceous earth (Celite®) and concentrated under reduced pressure. The crude material was purified over SiO₂ (5-40 % ethyl acetate/hexanes gradient elution) to afford *(S)*-methyl 2-((*tert-*butoxycarbonyl)amino)-5,5-difluoropent-4-enoate (0.91 g, 3.43 mmol, 13% yield) as a colorless oil. ¹H NMR (400MHz, *CDCl₃*) δ ppm 5.25 - 5.06 (br. s., 1H), 4.42 (br. s., 1H), 4.24 - 4.10 (m, 1H), 3.78 (s, 3H), 2.59 (dd, *J*=14.4, 6.7 Hz, 1H), 2.51 - 2.31 (m, 1H), 1.47 (s, 9H).

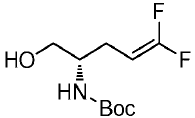

### Part F: (S)-tert-butyl (5,5-difluoro-1-hydroxypent-4-en-2-yl)carbamate

An ambient temperature solution of *(S)*-methyl 2-((*tert-*butoxycarbonyl)amino)-5,5-difluoropent-4-enoate (1.91 g, 7.20 mmol) in tetrahydrofuran (25 mL) was treated with lithium borohydride (0.31 g, 14 mmol) and stirred for 1 h. The resulting solution was cooled to 5 °C, quenched with 0.1 N aqueous hydrochloric acid (50 mL), and extracted with ethyl acetate (3x25 mL). The pooled organic extracts were washed with brine (1x20 mL), dried over MgSO₄, and concentrated under reduced pressure. The crude material was purified over SiO₂ (20-100 % ethyl acetate/hexanes gradient elution) to afford *(S)-tert-*butyl (5,5-difluoro-1-hydroxypent-4-en-2-yl)carbamate (1.4 g, 5.90 mmol, 82% yield) as a colorless oil. ¹H NMR (400MHz, CHLOROFORM-d) δ ppm 4.75 (d, *J*=6.0 Hz, 1H), 4.35 - 4.05 (m, 1H), 3.82 - 3.51 (m, 3H), 2.46 - 2.16 (m, 3H), 1.47 (s, 9H).

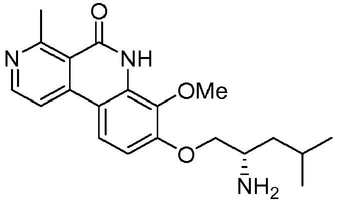

### Part G: (S)-tert-butyl (1-((4,6-dimethyl-5-oxo-5,6-dihydrobenzo[c][2,7]naphthyridin-8-yl)oxy)-5,5-difluoropent-4-en-2-yl)carbamate

A suspension of 8-chloro-4,6-dimethylbenzo[*c*][2,7]naphthyridin-5(*6H*)-one (1.26 g, 4.88 mmol), prepared as in Example 16, Part G, *(S)-tert-*butyl (5,5-difluoro-1-hydroxypent-4-en-2-yl)carbamate (1.39 g, 5.86 mmol), 2-di-*t*-butylphosphino-2',4',6'-triisopropylbiphenyl (0.42 g, 0.98 mmol), Pd(OAc)₂ (0.11 g, 0.49 mmol), Cs₂CO₃ (3.18 g, 9.76 mmol), and anhydrous toluene (15 mL) was purged with nitrogen for 10 minutes and heated to 90 °C overnight (15 h). After cooling, the reaction mixture was filtered through diatomaceous earth (Celite®), concentrated under reduced pressure and dissolved in ethyl acetate (50 mL). The organic layer was washed with brine (1x100 mL) and water (1x100 mL); dried over Na₂SO₄ and concentrated under reduced pressure to afford an amber oil. The crude material was purified over SiO₂ (1-5 % 2M ammonia in methanol/dichloromethane gradient elution) to afford *(S)-tert-*butyl (1-((4,6-dimethyl-5-oxo-5,6-dihydrobenzo[*c*][2,7]naphthyridin-8-yl)oxy)-5,5-difluoropent-4-en-2-yl)carbamate (1.04 g, 2.26 mmol, 46% yield) as a tan solid. LC/MS, (ESI) *m*/*z* 460.0 [(M+H)⁺, calcd for C₂₄H₂₈F₂N₃ O₄, 460.2]; ¹H NMR (400MHz, *CDCl₃*) δ ppm 8.59 (d, *J*=5.5 Hz, 1H), 8.05 (d, *J*=8.8 Hz, 1H), 7.73 (d, *J*=5.8 Hz, 1H), 6.83 (dd, *J*=8.8, 1.8 Hz, 1H), 6.79 (br. s., 1H), 5.13 - 4.89 (m, 1H), 4.39 - 4.21 (m, 1H), 4.20 - 3.96 (m, 3H), 3.66 (s, 3H), 3.12 (s, 3H), 2.61 - 2.31 (m, 2H), 1.48 (s, 9H).

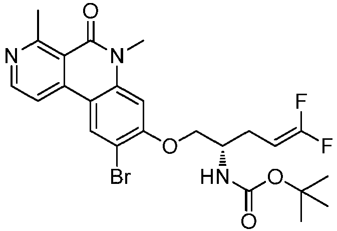

### Part H: (S)-tert-butyl (1-((9-bromo-4,6-dimethyl-5-oxo-5,6-dihydrobenzo[c][2,7]naphthyridin-8-yl)oxy)-5,5-difluoropent-4-en-2-yl)carbamate

A solution of *(S)-tert-*butyl (1-((4,6-dimethyl-5-oxo-5,6-dihydrobenzo[*c*][2,7]naphthyridin-8-yl)oxy)-5,5-difluoropent-4-en-2-yl)carbamate (1.03 g, 2.24 mmol) in acetonitrile (22 mL) was treated with *N*-bromosuccinimide (0.44 g, 2.5 mmol), heated to 80 °C for 45 min, and then concentrated under reduced pressure. The crude material was purified over SiO₂ (1-4 % 2M ammonia in methanol/dichloromethane gradient elution) to afford *(S)-tert*-butyl (1-((9-bromo-4,6-dimethyl-5-oxo-5,6-dihydrobenzo[*c*][2,7]naphthyridin-8-yl)oxy)-5,5-difluoropent-4-en-2-yl)carbamate (0.81 g, 1.51 mmol, 67% yield) as a white solid. LC/MS, (ESI) *m*/*z* 537.9 [(M+H)⁺, calcd for C₂₄H₂₇BrF₂N₃O₄, 538.1]; ¹H NMR (400MHz, *CDCl₃*) δ ppm 8.62 (d, *J*=5.8 Hz, 1H), 8.22 (s, 1H), 7.65 (d, *J*=5.5 Hz, 1H), 6.75 (s, 1H), 5.01 (d, *J*=8.3 Hz, 1H), 4.41 - 4.26 (m, 1H), 4.24 - 4.04 (m, 3H), 3.67 (s, 3H), 3.12 (s, 3H), 2.64 - 2.34 (m, 2H), 1.48 (s, 9H).

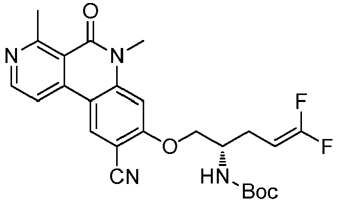

### Part I: (S)-tert-butyl (1-((9-cyano-4,6-dimethyl-5-oxo-5,6-dihydrobenzo[c][2,7]naphthyridin-8-yl)oxy)-5,5-difluoropent-4-en-2-yl)carbamate

A suspension of *(S)-tert*-butyl (1-((9-bromo-4,6-dimethyl-5-oxo-5,6-dihydrobenzo[*c*][2,7]naphthyridin-8-yl)oxy)-5,5-difluoropent-4-en-2-yl)carbamate (0.48 g, 0.892 mmol), zinc cyanide (0.115 g, 0.981 mmol), 1,1'-bis(diphenylphosphino)ferrocene (0.074 g, 0.134 mmol), Pd2(dba)3 (0.041 g, 0.045 mmol), *N,N-*dimethylformamide (3 mL), and water (0.3 mL) was charged to a 20 mL pressure rated vial and bubbled with a stream of nitrogen for 10 minutes. The vial was sealed, purged of oxygen, and stirred under nitrogen in a pre-heated reaction block at 115 °C overnight. After cooling, the reaction mixture was diluted with ethyl acetate (35 mL) and filtered through diatomaceous earth (Celite®). The filtrate was washed with brine (3x50 mL), dried over sodium sulfate, and concentrated under reduced pressure. The crude material was purified over SiO₂ (0-4% 2M ammonia in methanol/dichloromethane gradient elution) to afford *(S)-tert-*butyl (1-((9-cyano-4,6-dimethyl-5-oxo-5,6-dihydrobenzo[*c*][2,7]naphthyridin-8-yl)oxy)-5,5-difluoropent-4-en-2-yl)carbamate (0.15 g, 0.280 mmol, 32% yield) as a tan solid. LC/MS, (ESI) *m*/*z* 485.1 [(M+H)⁺, calcd for C₂₅H₂₇F₂N₄O₄, 485.2]; ¹H NMR (400MHz, *CDCl₃*) δ ppm 8.75 (d, *J*=5.5 Hz, 1H), 8.45 (s, 1H), 7.81 (d, *J*=5.8 Hz, 1H), 7.00 (s, 1H), 4.85 (d, *J*=10.0 Hz, 1H), 4.40 - 4.21 (m, 3H), 4.07 (br. s., 1H), 3.80 (s, 3H), 3.17 (s, 3H), 2.62 - 2.52 (m, 1H), 2.49 - 2.38 (m, 1H), 1.49 (s, 9H).

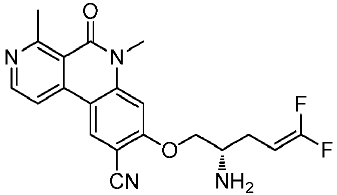

### Part J: (S)-8-((2-amino-5,5-difluoropent-4-en-1-yl)oxy)-4,6-dimethyl-5-oxo-5,6-dihydrobenzo[c][2,7]naphthyridine-9-carbonitrile

An ambient temperature solution of *(S)-tert*-butyl (1-((9-cyano-4,6-dimethyl-5-oxo-5,6-dihydrobenzo[*c*][2,7]naphthyridin-8-yl)oxy)-5,5-difluoropent-4-en-2-yl)carbamate (60 mg, 0.124 mmol) in methanol (1 mL) was treated with 4M HCl in 1,4-dioxane (1.3 mL, 5.20 mmol) and held overnight. The resulting solution was concentrated under reduced pressure. The crude material was purified via preparative LC/MS with the following conditions: Column: XBridge C18, 19 x 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 10-50% B over 20 minutes, then a 5-minute hold at 100% B; Flow: 20 mL/min. Fractions containing the desired were combined and concentrated via centrifugal evaporation to afford *(S)*-8-((2-amino-5,5-difluoropent-4-en-1-yl)oxy)-4,6-dimethyl-5-oxo-5,6-dihydrobenzo[*c*][2,7]naphthyridine-9-carbonitrile (7.6 mg, 0.020 mmol, 16% yield) as a white solid. LC/MS, (ESI) *m*/*z* 385.3 [(M+H)⁺, calcd for C₂₀H₁₉F₂N₄O₂, 385.1]; ¹H NMR (500MHz, *DMSO*) δ ppm 8.82 (s, 1H), 8.60 (d, *J*=5.5 Hz, 1H), 8.18 (d, *J*=5.8 Hz, 1H), 6.97 (s, 1H), 4.74 - 4.59 (m, 1H), 4.22 - 4.05 (m, 2H), 3.62 (s, 3H), 3.19 - 3.10 (m, 1H), 2.93 (s, 3H), 2.36 - 2.25 (m, 1H), 2.19 - 2.08 (m, 1H).

### Example 107

### (S)-8-((2-amino-4-methylpentyl)oxy)-9-chloro-7-fluoro-4,6-dimethylbenzo[c][2,7]naphthyridin-5(6H)-one

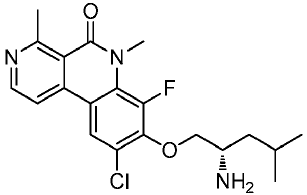

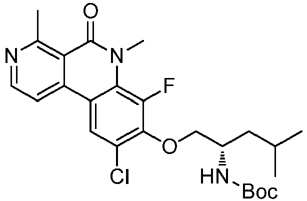

### Part A: (S)-tert-butyl (1-((9-chloro-7-fluoro-4,6-dimethyl-5-oxo-5,6-dihydrobenzo[c][2,7]naphthyridin-8-yl)oxy)-4-methylpentan-2-yl)carbamate

*(S)-tert*-butyl (1-((7-fluoro-4,6-dimethyl-5-oxo-5,6-dihydrobenzo[*c*][2,7]naphthyridin-8-yl)oxy)-4-methylpentan-2-yl)carbamate (0.08 g, 0.175 mmol), prepared as described in Example 29, Part G, was subjected to chlorination using NCS to afford *(S)-tert*-butyl (1-((9-chloro-7-fluoro-4,6-dimethyl-5-oxo-5,6-dihydrobenzo[*c*][2,7]naphthyridin-8-yl)oxy)-4-methylpentan-2 yl)carbamate (80 mg, 0.104 mmol, 60% crude yield) as semi-solid. The material was carried forward without further purification. LC/MS (ESI) *m*/*e* 492.2 [(M+H)⁺, calcd for C₂₅H₃₂ClFN₃O₄ 492.2].

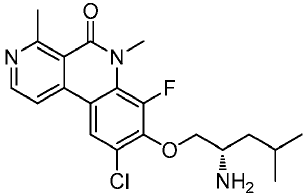

### Part B: (S)-8-((2-amino-4-methylpentyl)oxy)-9-chloro-7-fluoro-4,6-dimethylbenzo[c][2,7]naphthyridin-5(6H)-one

*(S)-tert*-Butyl (1-((9-chloro-7-fluoro-4,6-dimethyl-5-oxo-5,6-dihydrobenzo[*c*][2,7]naphthyridin-8-yl)oxy)-4-methylpentan-2-yl)carbamate (0.08 g, 0.104 mmol)) was subjected to deprotection of the Boc group as described in Example 2, Part E. The crude material was purified via reverse pahse HPLC (10MM ammonium acetate in water/AcCN) to provide *(S)*-8-((2-amino-4-methylpentyl)oxy)-9-chloro-7-fluoro-4,6-dimethylbenzo[*c*][2,7]naphthyridin-5(*6H*)-one (0.006 g, 0.015 mmol, 14% yield) as off-white solid. LC/MS (ESI) *m*/*e* 392.2 [(M+H)⁺, calcd for C₂₀H₂₄ClFN₃O₂ 392.1]; LC/MS retention time (LC/MS Method C) *t*_{R} = 2.11 min. HPLC retention time (method A): *t*_{R} = 8.96 min; HPLC retention time (method B): *t*_{R} = 5.15 min. ¹H NMR (400MHz, METHANOL-d₄) 8.62 (d, *J*=5.8 Hz, 1H), 8.32 (d, *J*=2.0 Hz, 1H), 8.12 (d, *J*=5.8 Hz, 1H), 4.26 (ddd, *J*=9.3, 3.8, 1.0 Hz, 1H), 4.06 (dd, *J*=8.7, 7.2 Hz, 1H), 3.87 (d, *J*=9.3 Hz, 3H), 3.67 - 3.64 (m, 1H), 3.08 (s, 3H), 1.92 - 1.78 (m, 1H), 1.56 - 1.37 (m, 2H), 1.02 (d, *J*=5.3 Hz, 3H), 1.01 (d, *J*=5.3 Hz, 3H).

### DESCRIPTION OF THE SPECIFIC EMBODIMENTS

In the following examples, proton NMR spectra were recorded on either a Bruker 400 or 500 MHz NMR spectrometer. Chemical shifts are reported in δ values relative to tetramethylsilane. LC/MS were run on a Shimadzu LC coupled to a Waters Micromass ZQ. HPLC retention times were obtained using at least one of the following methods:
LC-MS methods:
   LC/MS Method A: Column : PUROSPHER@star RP-18 (4X55mm), 3µm; Buffer : 20mM NH₄OAC IN WATER; Mphase A : Buffer + ACN(90+10); Mphase B : Buffer + MeCN(10+90); Flow : 2.5 mL/min)
   LC/MS Method B: Column : ZORBAX SB C18 (46X50mm), 5µm; Positive mode Mphase A : 10% MeOH - 90% H₂O - 0.1% TFA; Mphase B : 90% MeOH - 10% H₂O - 0.1% TFA; Flow : 5 mL/min)
   LC/MS Method C: Column - Ascentis Express C8 (5X2.1mm), 2.7µm; Mphase A : 2%MeCN - 98%H₂O - 10mM NH₄COOH; Mphase B : 98%ACN - 2%H2O - 10 mM NH₄COOH; Flow : 1 mL/min)
   LC/MS Method D: Column -ACQUITY UPLC BEH C18 (2.1 X 50 mm), 1.7µm; Mphase A :0.1% TFA in water; Mphase B : 1% TFA in ACN; Flow : 1/min) LC/MS Method E: Column -ACQUITY UPLC BEH C18 (2.1 X 50 mm), 1.7µm; Mphase A : 5 mM NH₄OAc:ACN (95:5); Mphase B : 5 mM NH₄OAc: ACN (5:95); Flow : 1/min)
   LC/MS Method F: Column : X-Bridge BEH C18 (50 X 2.1 mm), 2.5µm; Mphase A : ACN + H₂O (2+98) + 0.1% TFA; Mphase B : ACN + H₂O (98+2) + 0.05% TFA; Flow : 1.2 mL/min.
   LC-MS method G: Column : Kinetex C18 (50 X 2.1 mm), 2.6um: Mphase A: 2%ACN - 98%H₂O - 10mM; Mphase B : 98%ACN - 2%H₂O - 10mM NH₄COOH; Flow : 1/min).
   LC-MS method H: Column : BEH C18 (50 X 3.0 mm), 1.7 µm: Mphase A: 5%ACN - 95%H₂O - 10mM; Mphase B : 95%ACN - 5%H₂O - 10mM NH₄COOH; Flow : 1.2/min).
   LC-MS method I: Column : Ace Excel 2 C18 (50 X 3.0 mm), 2.0 µm: Mphase A: 2%ACN - 98%H₂O - 10mM; Mphase B : 98%ACN - 2%H₂O - 10mM NH₄COOH; Flow : 1.2/min).
Chiral HPLC methods:
   Method A: CHIRALCEL OJH (250x4.6) mm 5 micron Mob. phase: 0.2% DEA in n-hexane : ethanol (80:20)
   Method B: CHIRALPAK AD-H (250x4.6) mm 5 micron Mob. Phase A: 0.2% DEA in n-hexane (70) B: ethanol (30)
   Method C: CHIRALPAK- ASH (250x4.6) mm 5 micron Mob. Phase A: 0.2% DEA in n-hexane : ethanol (90:10)
   Method D: CHIRALPAK- ODH (250x4.6) mm 5 micron Mob. Phase: n-Hexane :Ethanol / methanol (50:50) (80:20); 50 min run
   Method E: Chiralpak-IA (250x4.6) mm 5 micron Mob. Phase: 35% (0.3% DEA in Methanol) in CO₂; Flow rate: CO₂: 2.6 g/min; 0.3% DEA in Methanol: 1.4 mL/min; 10 min run
   Method F: CHIRALPAK AD-H (250x4.6) mm 5 micron Mob. Phase: 40% (0.3% DEA in Methanol) in CO₂; : 4.0 g/min; 12 min run
Analytical HPLC methods:
   Method A: Waters analytical C18 sunfire column (4.6 x 150 mm, 3.5 µm); mobile phase:
      Buffer: 0.05% TFA in H₂O pH = 2.5 adjusted with ammonia
      A = buffer and acetonitrile (95:5), B = acetonitrile and buffer (95:5); 0 - 15 min, 0% B → 50% B; 15 - 18 min, 50% B → 100% B; 18 - 23 min, 100% B; flow rate = 1 mL/min; λ = 254 nm and 220 nm; run time = 28 min.
   Method B: Waters analytical phenyl xbridge column (4.6 x 150 mm, 3.5 µm), mobile phase: Buffer: 0.05% TFA in H₂O pH = 2.5 adjusted with ammonia
      A = buffer and acetonitrile (95:5), B = acetonitrile and buffer (95:5); 0 - 15 min, 0% B → 50% B; 15 - 18 min, 50% B → 100% B; 18 - 23 min, 100% B; flow rate = 1 mL/min; λ = 254 nm and 220 nm; run time = 28 min.
   Method C: Waters analytical C18 sunfire column (3.0 x 150 mm, 3.5 µm); mobile phase: A = 10mM amm. bicarbonate (pH=9.5)/95% H₂O/5% methanol, B = 10mM amm. bicarbonate (pH=9.5)/5% H₂O/95% methanol; 0 - 15 min, 0% B → 100% B; 15 - 18 min, 100% B; flow rate = 1 mL/min; λ = 254 nm; run time = 18 min.
   Method D: Waters analytical phenyl xbridge column (3.0 x 150 mm, 3.5 µm), mobile phase: A = 10mM amm. bicarbonate (pH=9.5)/95% H₂O/5% methanol, B = 10mM amm. bicarbonate (pH=9.5)/5% H₂O/95% methanol; 0 - 15 min, 0% B → 100% B; 15 - 18 min, 100% B; flow rate = 1 mL/min; λ = 254 nm; run time = 18 min.
   Method E: Waters analytical C18 sunfire column (4.6 x 150 mm, 3.5 µm); mobile phase: A = 0.05% TFA in water : Acetonitrile (95:5), B = Acetonitrile : 0.05% TFA in water (95:5); 0 - 15 min, 10% B → 100% B; flow rate = 1 mL/min; λ = 254 nm and 220 nm; run time = 15 min.
   Method F: Waters analytical phenyl xbridge column (4.6 x 150 mm, 3.5 µm), mobile phase: A = 0.05% TFA in water : Acetonitrile (95:5), B = Acetonitrile : 0.05% TFA in water (95:5); 0 - 15 min, 0% B → 50% B; 15 - 18 min, 50% B → 100% B, 18 - 23 min, 100% B; flow rate = 1 mL/min; λ = 254 nm and 220 nm; run time = 15 min.
   Method G: Waters analytical phenyl xbridge column (4.6 x 150 mm, 3.5 µm), mobile phase: A = 0.05% TFA in water : Acetonitrile (95:5), B = Acetonitrile : 0.05% TFA in water (95:5); 0 - 12 min, 10% B → 100% B; 12 - 15 min, 100% B; flow rate = 1 mL/min; λ = 254 nm and 220 nm; run time = 15 min.
   Method H: Waters analytical C18 sunfire column (4.6 x 150 mm, 3.5 µm); mobile phase: A = 0.05% TFA in water : Acetonitrile (95:5), B = Acetonitrile : 0.05% TFA in water (95:5); 0 - 15 min, 10% B → 50% B; 15 - 18 min, 50% B → 100% B; flow rate = 1 mL/min; λ = 254 nm and 220 nm; run time = 18 min.
   Method I: Waters analytical C18 sunfire column (4.6 x 150 mm, 3.5 µm); mobile phase: A = 0.05% TFA in water : Acetonitrile (95:5), B = Acetonitrile : 0.05% TFA in water (95:5); 0 - 12 min, 10% B → 100% B; 12 - 15 min, 100% B; flow rate = 1 mL/min; λ = 254 nm and 220 nm; run time = 15 min.
   Method J: Eclipse XDB C18 column (4.6 x 150 mm, 3.5 µm); mobile phase: A = 10mM NH4OAc in water, B = Methanol; 0 - 15 min, 15% B → 100% B; 15 - 18 min, 100% B; flow rate = 1 mL/min; λ = 254 nm and 220 nm; run time = 15 min.
   Method K: Waters analytical C18 sunfire column (3.0 x 150 mm, 3.5 µm); mobile phase: A = 0.1% TFA/95% H₂O/5% acetonitrile, B = 0.1% TFA/5% H₂O/95% acetonitrile; 0 - 15 min, 10% B → 100% B; 100% B; flow rate = 1 mL/min; λ = 220/254 nm; run time = 18 min.
   Method L: Waters analytical phenyl xbridge column (3.0 x 150 mm, 3.5 µm), mobile phase: A = 0.1% TFA/95% H₂O/5% acetonitrile, B = 0.1% TFA/5% H₂O/95% acetonitrile; 0 - 15 min, 10% B → 100% B; 100% B; flow rate = 1 mL/min; λ = 220/254 nm; run time = 18 min.
   Method M: Waters analytical C18 sunfire column (4.6 x 150 mm, 3.5 µm); mobile phase: A = 0.05% TFA in water : Acetonitrile (95:5), B = Acetonitrile : 0.05% TFA in water (95:5); 0 - 25 min, 10% B → 100% B; flow rate = 1 mL/min; λ = 254 nm and 220 nm; run time = 25 min.

### Example 108 (invention)

### (S)-8-((2-amino-4-methylpentyl)oxy)-9-bromobenzo[c][2,7]naphthyridin-5(6H)-one

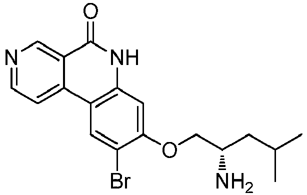

Prepared as described in Example 3: (*S*)-8-((2-amino-4-methylpentyl)oxy)-9-bromobenzo[*c*][2,7]naphthyridin-5(6*H*)-one (280 mg, 0.717 mmol, 89% yield) as a pale yellow solid. LC/MS (ESI) *m*/*e* 390.0, 392.0 Br pattern [(M+H)⁺, calcd for C₁₈H₂₁BrN₃O₂ 390.1]; HPLC retention time (method E): *t*_{R} = 9.32 min; HPLC retention time (method F): *t*_{R} = 9.43 min; ¹H NMR (400MHz, DMSO-d₆) δ 11.84 (bs, 1H), 9.37 (s, 1H), 8.84 (d, *J*= 5.6 Hz, 1H), 8.71 (s, 1H), 8.39 (d, *J*=5.6 Hz, 1H), 7.00 (s, 1H), 3.84-3.95 (m, 2H), 3.17 (m, 1H), 1.85 (m, 1H), 1.38 (m, 1H), 1.30 (m, 1H), 0.89-0.96 (m, 6H).

### Example 109 (invention)

### (S)-8-((2-amino-4-methylpentyl)oxy)-9-methylbenzo[c][2,7]naphthyridin-5(6H)-one

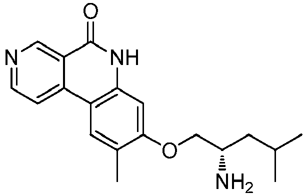

Prepared as described in Example 4: (*S*)-8-((2-amino-4-methylpentyl)oxy)-9-methylbenzo[*c*][2,7]naphthyridin-5(6*H*)-one (30 mg, 0.092 mmol, 33% yield) as an off-white solid. LC/MS (ESI) *m*/*e* 326.2 [(M+H)⁺, calcd for C₁₉H₂₄N₃O₂ 326.2]; HPLC retention time (method F): *t*_{R} = 8.35 min; HPLC retention time (method A): *t*_{R} = 7.86 min; ¹H NMR (400 MHz, MeOD(: δ 9.46 (s, 1H), 8.80 (d, *J* = 5.20 Hz, 1H),. 8.28 (d, *J* = 4.00 Hz, 1H), 8.21 (s, 1H), 6.91 (s, 1H), 4.31-4.33 (m, 1H), 4.17-4.18 (m, 1H), 3.51-3.70 (m, 1H), 2.43 (s, 3H), 1.82-1.87 (m, 1H), 1.69-1.74 (m, 2H), 1.07-1.31 (m, 6H).

### Example 110 (invention)

### (S)-8-((2-amino-4-methylpentyl)oxy)-9-isobutyl-6-methylbenzo[c][2,7]naphthyridin-5(6H)-one

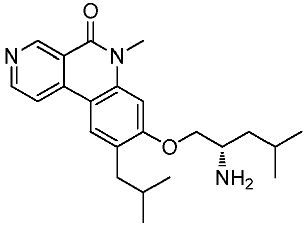

Prepared as described in Example 4: (*S*)-8-((2-amino-4-methylpentyl)oxy)-9-isobutyl-6-methylbenzo[*c*][2,7]naphthyridin-5(6*H*)-one (12 mg, 0.029 mmol, 35% yield) as an off-white solid. LC/MS (ESI) *m*/*e* 382.2 [(M+H)⁺, calcd for C₂₃H₃₂N₃O₂ 382.2]; HPLC retention time (method E): *t*_{R} = 6.15 min; HPLC retention time (method F): *t*_{R} = 5.23 min; ¹H NMR (400 MHz, MeOD): δ 9.44 (s, 1H), 8.74 (d, *J* = 6.0 Hz, 1H), 8.23 (d, *J* = 5.6 Hz, 1H), 8.14 (s, 1H), 7.01 (s, 1H), 4.18-4.15 (m, 1H), 4.09-4.05 (m, 1H), 3.81 (s, 3H), 3.36-3.27 (m, 1H), 2.75-2.62 (m, 2H), 2.03-1.90 (m, 1H), 1.89-1.87 (m, 1H), 1.63-1.51 (m, 1H), 1.49-1.44 (m, 1H), 1.03-0.94 (m, 12H).

### Example 111 (invention)

### 8-((2-amino-5,5,5-trifluoropentyl)oxy)-6-methylbenzo[c][2,7]naphthyridin-5(6H)-one

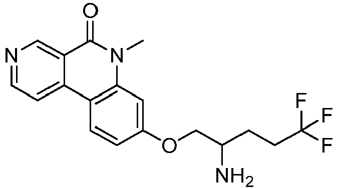

The material from Example 15 was separated into the corresponding enantiomers via chiral SFC (Chiralpak-AD-H (250 X 21 mm), 5u: mobile phase: 60% CO₂, 40% (0.5% DEA in Methanol); Flow rate: 60 g/min). First eluting enantiomer: 8-((2-amino-5,5,5-trifluoropentyl)oxy)-6-methylbenzo[*c*][2,7]naphthyridin-5(6*H*)-one (22.3 mg, 0.061 mmol, 38% yield) as a white solid. The absolute stereochemistry was not determined. LC/MS (ESI) *m*/*e* 366.2 [(M+H)⁺, calcd for C₁₈H₁₉F₃N₃O₂ 366.1]; HPLC retention time (method F): *t*_{R} = 8.24 min; Chiral HPLC (method A): *t*_{R} = 3.75 min; ¹H NMR (400 MHz, MeOD): δ 9.48 (d, *J* = 0.4 Hz, 1H), 8.78 (d, *J* = 5.6 Hz, 1H), 8.44 (d, *J* = 8.8 Hz, 1H), 8.27 (d, *J* = 5.6 Hz, 1H), 7.17 (d, *J* = 2.0 Hz, 1H), 7.12 (dd, *J* = 8.8, 2.4 Hz, 1H), 4.20 (dd, *J*= 9.2, 4.8 Hz, 1H), 4.09 (dd, *J* = 9.2, 6.0 Hz, 1H), 3.82 (s, 3H), 3.29-3.28 (m, 1H), 2.50-2.33 (m, 2H), 2.00-1.93 (m, 1H), 1.82-1.74 (m, 1H).

### Example 112

### 8-((2-amino-5,5,5-trifluoropentyl)oxy)-6-methylbenzo[c][2,7]naphthyridin-5(6H)-one

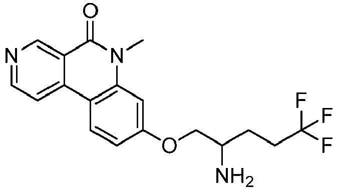

The material from Example 15 was separated into the corresponding enantiomers via chiral SFC (Chiralpak-AD-H (250 X 21 mm), 5u: mobile phase: 60% CO2, 40% (0.5% DEA in Methanol); Flow rate: 60 g/min). Second eluting enantiomer: 8-((2-amino-5,5,5-trifluoropentyl)oxy)-6-methylbenzo[*c*][2,7]naphthyridin-5(6*H*)-one (20.5 mg, 0.056 mmol, 35% yield) as a white solid. The absolute stereochemistry was not determined. LC/MS (ESI) *m*/*e* 366.2 [(M+H)⁺, calcd for C₁₈H₁₉F₃N₃O₂ 366.1]; HPLC retention time (method F): *t*_{R} = 8.25 min; Chiral HPLC (method A): *t*_{R} = 5.80 min; ¹H NMR (400 MHz, MeOD_: δ 9.48 (d, *J* = 0.4 Hz, 1H), 8.77 (d, *J* = 5.6 Hz, 1H), 8.43 (d, *J* = 8.8 Hz, 1H), 8.27 (d, *J* = 5.6 Hz, 1H), 7.17 (d, *J* = 2.0 Hz, 1H), 7.12 (dd, *J* = 8.8, 2.4 Hz, 1H), 4.19 (dd, *J* = 9.2, 4.4 Hz, 1H), 4.09 (dd, *J* = 9.2, 6.0 Hz, 1H), 3.82 (s, 3H), 3.29-3.25 (m, 1H), 2.46-2.34 (m, 2H), 2.00-1.93 (m, 1H), 1.81-1.76 (m, 1H).

### Example 113 (invention)

### 8-((2-amino-5,5,5-trifluoropentyl)oxy)-9-bromo-6-methylbenzo[c][2,7]naphthyridin-5(6H)-one

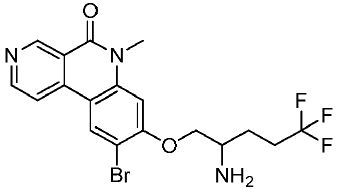

Prepared as described in Example 3: 8-((2-amino-5,5,5-trifluoropentyl)oxy)-9-bromo-6-methylbenzo[*c*][2,7]naphthyridin-5(6*H*)-one (24 mg, 0.053 mmol, 48% yield) as a pale yellow solid. LC/MS (ESI) *m*/*e* 444.0, 446.0 Br pattern [(M+H)⁺, calcd for C₁₈H₁₈BrF₃N₃O₂ 444.1]; HPLC retention time (method G): *t*_{R} = 5.45 min; HPLC retention time (method A): *t*_{R} = 9.24 min; ¹H NMR (400 MHz, MeOD): δ 7.97 (s, 1H), 7.28 (d, *J* = 6.0 Hz, 1H), 7.13 (s, 1H), 6.74 (d, *J* = 5.6 Hz, 1H), 5.62 (s, 1H), 2.76-2.68 (m, 2H), 2.31 (s, 3H), 1.83-1.81 (m, 1H), 1.07-0.90 (m, 2H). 0.59-0.22 (m, 2H).

### Example 114

### 8-((2-amino-5,5,5-trifluoropentyl)oxy)-9-bromo-6-methylbenzo[c][2,7]naphthyridin-5(6H)-one

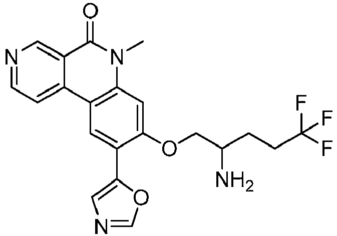

Prepared as described in Example 4: 8-((2-amino-5,5,5-trifluoropentyl)oxy)-6-methyl-9-(oxazol-5-yl)benzo[*c*][2,7]naphthyridin-5(6*H*)-one (14 mg, 0.043 mmol, 72% yield) as a pale yellow solid. LC/MS (ESI) *m*/*e* 433.2 [(M+H)⁺, calcd for C₂₁H₂₀F₃N₄O₃ 433.1]; HPLC retention time (method H): *t*_{R} = 9.04 min; HPLC retention time (method F): *t*_{R} = 9.12 min; ¹H NMR (400 MHz, MeOD): δ 9.54 (s, 1H), 8.86 (d, *J* = 6.0 Hz, 1H), 8.85 (s, 1H), 8.48 (d, *J* = 6.0 Hz, 1H), 8.41 (s, 1H), 7.68 (s, 1H), 7.29 (s, 1H), 4.70-4.58 (m, 2H), 4.03-4.00 (m, 1H), 3.89 (s, 3H), 2.59-2.52 (m, 2H). 2.27-2.15 (m, 2H).

### Example 115 (invention)

### 8-((2-amino-5,5,5-trifluoropentyl)oxy)-9-chloro-6-methylbenzo[c][2,7]naphthyridin-5(6H)-one

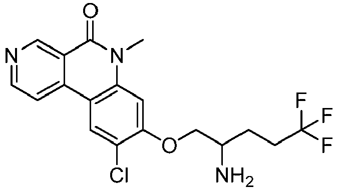

Prepared as described in Example 3: 8-((2-amino-5,5,5-trifluoropentyl)oxy)-9-chloro-6-methylbenzo[*c*][2,7]naphthyridin-5(6*H*)-one (30 mg, 0.044 mmol, 39% yield) as a pale yellow solid. LC/MS (ESI) *m*/*e* 400.6 [(M+H)⁺, calcd for C₁₈H₁₈ClF₃N₃O₂ 400.1]; HPLC retention time (method A): *t*_{R} = 8.94 min; HPLC retention time (method B): *t*_{R} = 10.01 min; ¹H NMR (400 MHz, MeOD): δ 9.54 (s, 1H), 8.85 (d, *J* = 5.6 Hz, 1H), 8.62 (s, 1H), 8.36 (d, *J* = 5.6 Hz, 1H), 7.28 (s, 1H), 4.61-4.48 (m, 2H), 3.71-3.82 (m, 4H), 2.57-2.50 (m, 2H), 2.30-2.16 (m, 2H).

### Example 116

### 8-((2-amino-5,5,5-trifluoropentyl)oxy)-9-chloro-6-methylbenzo[c][2,7]naphthyridin-5(6H)-one

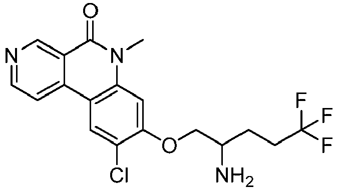

The material from Example 115 was separated into the corresponding enantiomers via chiral SFC (Chiralpak-AD-H (250 X 21 mm), 5u: mobile phase: 60% CO2, 40% (0.5% DEA in Methanol); Flow rate: 60 g/min). First eluting enantiomer: 8-((2-amino-5,5,5-trifluoropentyl)oxy)-9-chloro-6-methylbenzo[*c*][2,7]naphthyridin-5(6*H*)-one (40 mg, 0.094 mmol, 8% yield) as a pale pink solid. The absolute stereochemistry was not determined. LC/MS (ESI) *m*/*e* 400.0 [(M+H)⁺, calcd for C₁₈H₁₈ClF₃N₃O₂ 400.1]; HPLC retention time (method A): *t*_{R} = 8.87 min; HPLC retention time (method B): *t*_{R} = 9.84 min; Chiral HPLC (method B with 0.3%DEA): *t*_{R} = 7.15 min; ¹H NMR (400 MHz, CDCl3): δ 9.68 (s, 1H), 8.85 (d, *J* = 5.6 Hz, 1H), 8.23 (s, 1H), 7.87 (d, *J* = 5.6 Hz, 1H), 6.85 (s, 1H), 4.14 - 3.98 (m, 2H), 3.78 (s, 3H), 3.36 - 3.33 (m, 1H), 2.49 - 2.24 (m, 2H), 1.99 - 1.70 (m, 2H).

### Example 117

### 8-((2-amino-5,5,5-trifluoropentyl)oxy)-9-chloro-6-methylbenzo[c][2,7]naphthyridin-5(6H)-one

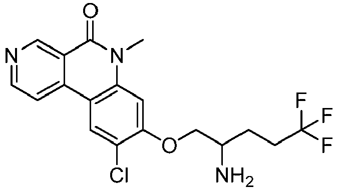

The material from Example 115 was separated into the corresponding enantiomers via chiral SFC (Chiralpak-AD-H (250 X 21 mm), 5u: mobile phase: 60% CO₂, 40% (0.5% DEA in Methanol); Flow rate: 60 g/min). Second eluting enantiomer: 8-((2-amino-5,5,5-trifluoropentyl)oxy)-9-chloro-6-methylbenzo[*c*][2,7]naphthyridin-5(6*H*)-one (61 mg, 0.146 mmol, 13% yield) as a pale yellow solid. The absolute stereochemistry was not determined. LC/MS (ESI) *m*/*e* 400.0 [(M+H)⁺, calcd for C₁₈H₁₈ClF₃N₃O₂ 400.1]; HPLC retention time (method A): *t*_{R} = 8.86 min; HPLC retention time (method B): *t*_{R} = 9.84 min; Chiral HPLC (method B with 0.3%DEA): *t*_{R} = 8.98 min; ¹H NMR (400 MHz, CDCl3): δ 9.67 (s, 1H), 8.85 (d, *J* = 5.6 Hz, 1H), 8.23 (s, 1H), 7.87 (d, *J* = 5.6 Hz, 1H), 6.85 (s, 1H), 4.14 - 3.98 (m, 2H), 3.78 (s, 3H), 3.37 - 3.33 (m, 1H), 2.45 - 2.27 (m, 2H), 1.99 - 1.72 (m, 2H).

### Example 118 (invention)

### 4-amino-8-((2-amino-5,5,5-trifluoropentyl)oxy)-6-methylbenzo[c][2,7]naphthyridin-5(6H)-one

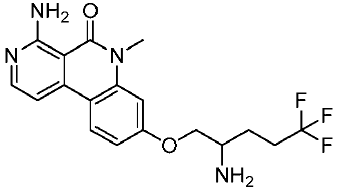

Prepared as described in Example 17: 4-amino-8-((2-amino-5,5,5-trifluoropentyl)oxy)-6-methylbenzo[*c*][2,7]naphthyridin-5(6*H*)-one (4.0 mg, 0.004 mmol, 37% yield) as a colorless solid. LC/MS (ESI) *m*/*e* 381.2 [(M+H)⁺, calcd for C₁₈H₂₀F₃N₄O₂ 381.2]; HPLC retention time (method A): *t*_{R} = 7.83 min; HPLC retention time (method B): *t*_{R} = 9.02 min; ¹H NMR (400 MHz, MeOD): δ 8.28 (d, *J* = 8.8 Hz, 1H), 8.11 (d, *J* = 5.6 Hz, 1H), 7.37 (d, *J* = 6.0 Hz, 1H), 7.09 (s, 1H), 7.06 (d, *J* = 8.8 Hz, 1H), 4.35-4.19 (m, 2H), 3.76 (s, 3H), 3.56-3.52 (m, 1H), 2.50-2.41 (m, 2H), 2.07-1.93 (m, 2H).

### Example 119

### 4-amino-8-((2-amino-5,5,5-trifluoropentyl)oxy)-6-methylbenzo[c][2,7]naphthyridin-5(6H)-one

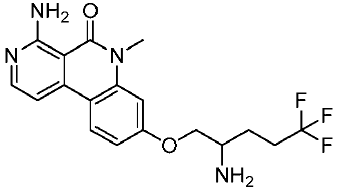

The material from Example 118 was separated into the corresponding enantiomers via chiral SFC (Chiralpak-AD-H (250 X 21 mm), 5u: mobile phase: 60% CO₂, 40% (0.3% DEA in Methanol); Flow rate: 60 g/min). First eluting enantiomer: 4-amino-8-((2-amino-5,5,5-trifluoropentyl)oxy)-6-methylbenzo[*c*][2,7]naphthyridin-5(6*H*)-one (143 mg, 0.365 mmol, 22% yield) as a colorless solid. The absolute stereochemistry was not determined. LC/MS (ESI) *m*/*e* 381.2 [(M+H)⁺, calcd for C₁₈H₂₀F₃N₄O₂ 381.2]; HPLC retention time (method A): *t*_{R} = 7.81 min; HPLC retention time (method B): *t*_{R} = 8.90 min; Chiral HPLC (method B with 0.3%DEA): *t*_{R} = 5.45 min; ¹H NMR (400 MHz, MeOD): δ 8.23 (d, *J* = 8.8 Hz, 1H), 8.09 (d, *J* = 5.6 Hz, 1H), 7.34 (d, *J* = 6.0 Hz, 1H), 7.05 - 7.02 (m, 2H), 4.16 (dd, *J* = 9.2, 4.8 Hz, 1H), 4.06 (dd, *J* = 9.2, 6.4 Hz, 1H), 3.74 (s, 3H), 3.32 - 3.20 (m, 1H), 2.49 - 2.33 (m, 2H), 1.99 - 1.92 (m, 1H), 1.81 - 1.72 (m, 1H).

### Example 120

### 4-amino-8-((2-amino-5,5,5-trifluoropentyl)oxy)-6-methylbenzo[c][2,7]naphthyridin-5(6H)-one

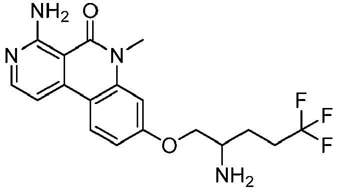

The material from Example 118 was separated into the corresponding enantiomers via chiral SFC (Chiralpak-AD-H (250 X 21 mm), 5µ: mobile phase: 60% CO₂, 40% (0.3% DEA in Methanol); Flow rate: 60 g/min). Second eluting enantiomer: 4-amino-8-((2-amino-5,5,5-trifluoropentyl)oxy)-6-methylbenzo[*c*][2,7]naphthyridin-5(6*H*)-one (143 mg, 0.365 mmol, 22% yield) as a colorless solid. The absolute stereochemistry was not determined. LC/MS (ESI) *m*/*e* 381.2 [(M+H)⁺, calcd for C₁₈H₂₀F₃N₄O₂ 381.2]; HPLC retention time (method A): *t*_{R} = 7.79 min; HPLC retention time (method B): *t*_{R} = 8.84 min; Chiral HPLC (method B with 0.3%DEA): *t*_{R} = 10.39 min; ¹H NMR (400 MHz, MeOD): δ 8.23 (d, *J* = 8.8 Hz, 1H), 8.09 (d, *J* = 5.6 Hz, 1H), 7.34 (d, *J* = 6.0 Hz, 1H), 7.05 - 7.02 (m, 2H), 4.16 (dd, *J* = 9.2, 4.8 Hz, 1H), 4.06 (dd, *J* = 9.2, 6.4 Hz, 1H), 3.74 (s, 3H), 3.34 - 3.20 (m, 1H), 2.50 - 2.38 (m, 2H), 1.99 - 1.70 (m, 2H).

### Example 121 (invention)

### (S)-8-((2-amino-4-methylpentyl)oxy)-9-(difluoromethyl)-6-methylbenzo[c][2,7]naphthyridin-5(6H)-one

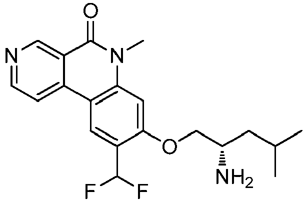

Prepared as described in Example 27: (*S*)-8-((2-amino-4-methylpentyl)oxy)-9-(difluoromethyl)-6-methylbenzo[*c*][2,7]naphthyridin-5(6*H*)-one (5 mg, 0.013 mmol, 44% yield) as an off-white solid. LC/MS (ESI) *m*/*e* 376.2 [(M+H)⁺, calcd for C₂₀H₂₄F₂N₃O₂ 376.2]; HPLC retention time (method A): *t*_{R} = 9.22 min; HPLC retention time (method B): *t*_{R} = 9.75 min; ¹H NMR (400 MHz, MeOD): δ 9.52 (s, 1H) 8.83 (d, *J*=5.77 Hz, 1H) 8.63 (s, 1H) 8.33 (d, *J*=5.52 Hz, 1H) 6.98 - 7.37 (m, 2H) 4.36 (dd, *J*=9.54, 3.51 Hz, 1H) 4.19 (d, *J*=6.53 Hz, 1H) 3.87 (s, 3H) 3.51 (d, *J*=1.51 Hz, 1H) 1.87 (s, 1H) 1.48 - 1.59 (m, 2H) 1.05 (d, *J*=6.6 Hz, 3H), 1.03 (d, *J*=6.6 Hz, 3H).

### Example 122 (invention)

### (S)-8-((2-amino-4-methylpentyl)oxy)-9-bromo-4,6-dimethylbenzo[c][2,7]naphthyridin-5(6H)-one

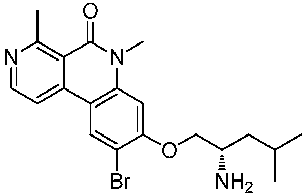

Prepared as described in Example 3: (*S*)-8-((2-amino-4-methylpentyl)oxy)-9-bromo-4,6-dimethylbenzo[*c*][2,7]naphthyridin-5(6*H*)-one (15 mg, 0.035 mmol, 36% yield) as an off-white solid. LC/MS (ESI) *m*/*e* 418.0, 420.0 Br pattern [(M+H)⁺, calcd for C₂₀H₂₅BrN₃O₂ 418.1]; HPLC retention time (method I): *t*_{R} = 4.52 min; HPLC retention time (method G): *t*_{R} = 5.19 min; ¹H NMR (300 MHz, DMSO-*d*₆) δ ppm 8.70 (s, 1H), 8.62 (d, *J*=5.67 Hz, 1H), 8.25 (d, *J*=5.67 Hz, 1H), 7.06 (s, 1 H), 4.16 - 4.07 (m, 1H), 4.05 - 3.96 (m, 1H), 3.69 (s, 3H), 3.19 - 3.11 (m, 1H), 2.99 (s, 3H), 1.90 - 1.71 (m, 1H), 1.42 - 1.27 (m, 2H), 0.95 - 0.90 (m, 6H).

### Example 123 (invention)

### 8-((2-amino-5,5,5-trifluoropentyl)oxy)-4,6-dimethylbenzo[c][2,7]naphthyridin-5(6H)-one

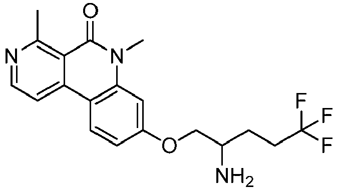

Prepared as described in Example 15: 8-((2-amino-5,5,5-trifluoropentyl)oxy)-4,6-dimethylbenzo[*c*][2,7]naphthyridin-5(6*H*)-one (10.0 mg, 0.026 mmol, 25% yield) as an off-white solid. LC/MS (ESI) *m*/*e* 380.2 [(M+H)⁺, calcd for C₁₉H₂₁F₃N₃O₂ 380.2]; HPLC retention time (method A): *t*_{R} = 7.81 min; HPLC retention time (method B): *t*_{R} = 8.96 min; ¹H NMR (400 MHz, DMSO-*d*₆) δ ppm 8.59 (d, *J*=5.77 Hz, 1H), 8.41 (d, *J*=8.53 Hz, 1H), 8.15 (d, *J*=5.77 Hz, 1H), 6.96 (d, *J*=2.26 Hz, 2H), 4.06 - 3.92 (m, 2H), 3.64 (s, 3H), 3.05 (dd, *J*=8.91, 4.39 Hz, 1H), 2.97 (s, 3H), 2.40 - 2.28 (m, 1H), 1.86 - 1.71 (m, 2H), 1.54 - 1.43 (m, 1H).

### Example 124 (invention)

### 8-((2-amino-5,5,5-trifluoropentyl)oxy)-9-chloro-4,6-dimethylbenzo[c][2,7]naphthyridin-5(6H)-one

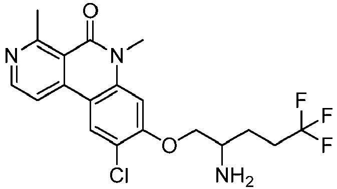

Prepared as described in Example 3: 8-((2-amino-5,5,5-trifluoropentyl)oxy)-9-chloro-4,6-dimethylbenzo[*c*][2,7]naphthyridin-5(6*H*)-one (2.5 mg, 0.006 mmol, 20% yield) as an off-white solid. LC/MS (ESI) *m*/*e* 414.0 [(M+H)⁺, calcd for C₁₉H₂₀ClF₃N₃O₂ 414.1]; HPLC retention time (method B): *t*_{R} = 8.22 min; HPLC retention time (method I): *t*_{R} = 8.24 min; ¹H NMR (400 MHz, DMSO-*d*₆) δ ppm 8.66 - 8.59 (m, 2H), 8.28 (s, 1H), 7.15 - 7.12 (m, 1H), 4.17 - 4.12 (m, 2H), 3.71 (s, 3H), 3.11 - 3.19 (m, 1H), 3.01 (s, 3H), 2.36 - 2.45 (m, 1H), 1.89 - 1.79 (m, 2H), 1.62 - 1.53 (m, 1H).

### Example 125 (invention)

### (S)-8-((2-amino-4-methylpentyl)oxy)-9-cyclopropyl-4,6-dimethylbenzo[c][2,7]naphthyridin-5(6H)-one

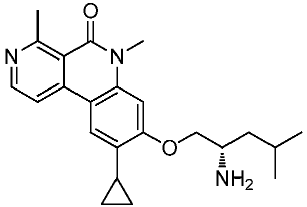

Prepared as described in Example 4: (*S*)-8-((2-amino-4-methylpentyl)oxy)-9-cyclopropyl-4,6-dimethylbenzo[*c*][2,7]naphthyridin-5(6*H*)-one (4 mg, 0.010 mmol, 35% yield) as an off-white solid. LC/MS (ESI) *m*/*e* 379.9 [(M+H)⁺, calcd for C₂₃H₃₀N₃O₂ 380.2]; HPLC retention time (method A): *t*_{R} = 9.56 min; HPLC retention time (method G): *t*_{R} = 5.45 min; ¹H NMR (400 MHz, DMSO-*d*₆) δ ppm 8.58 (d, *J*=5.65 Hz, 1H), 8.26 (d, *J*=5.65 Hz, 1H), 7.88 (s, 1H), 6.94 (s, 1H), 4.09-3.92 (m, 2H), 3.69 (s, 3H), 3.18 - 3.12 (m, 1H), 2.99 (s, 3H), 2.21 - 2.14 (m, 1H), 1.92 - 1.82 (m, 1H), 1.43 - 1.20 (m, 2H), 0.96 - 0.89 (m, 8H), 0.87 - 0.82 (m, 2H).

### Example 126 (invention)

### 8-((2-amino-5,5,5-trifluoropentyl)oxy)-6-methyl-5-oxo-5,6-dihydrobenzo[c][2,7]naphthyridine-9-carbonitrile

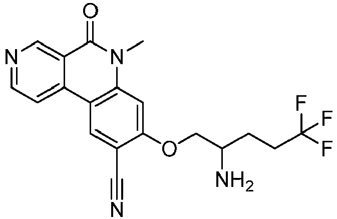

The material from Example 56 was separated into the corresponding enantiomers via chiral SFC (CHIRALPAK- ODH (250x21) mm 5 micron; mobile phase: n-Hexane :Ethanol : methanol (50:50) (80:20) over 50 min); Flow rate: 60 g/min). First eluting enantiomer: 8-((2-amino-5,5,5-trifluoropentyl)oxy)-6-methyl-5-oxo-5,6-dihydrobenzo[*c*][2,7]naphthyridine-9-carbonitrile (25 mg, 0.063 mmol, 14% yield) as a colorless solid. The absolute stereochemistry was not determined. LC/MS (ESI) *m*/*e* 391.2 [(M+H)⁺, calcd for C₁₉H₁₈F₃N₄O₂ 391.2]; HPLC retention time (method B): *t*_{R} = 9.88 min; HPLC retention time (method J): *t*_{R} = 7.93 min; Chiral HPLC (method D): *t*_{R} = 33.7 min; ¹H NMR (400MHz, METHANOL-d₄) δ ppm 9.51 (d, *J*=0.8 Hz, 1H), 8.70 (s, 1H), 8.84 (d, *J*=6.0 Hz, 1H), 8.33 (d, *J*=5.6 Hz, 1H), 7.22 (s, 1H), 4.37 - 4.24 (m, 2H), 3.86 (s, 3H), 3.38 - 3.34 (m, 1H), 2.57 - 2.31 (m, 2H), 2.06 - 1.83 (m, 2H).

### Example 127

### 8-((2-amino-5,5,5-trifluoropentyl)oxy)-6-methyl-5-oxo-5,6-dihydrobenzo[c][2,7]naphthyridine-9-carbonitrile

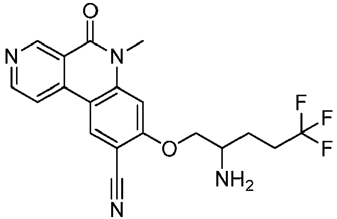

The material from Example 56 was separated into the corresponding enantiomers via chiral SFC (CHIRALPAK- ODH (250x421) mm 5 micron; mobile phase: n-Hexane :Ethanol : methanol (50:50) (80:20) over 50 min); Flow rate: 60 g/min). Second eluting enantiomer: 8-((2-amino-5,5,5-trifluoropentyl)oxy)-6-methyl-5-oxo-5,6-dihydrobenzo[*c*][2,7]naphthyridine-9-carbonitrile (21 mg, 0.053 mmol, 12% yield) as a colorless solid. The absolute stereochemistry was not determined. LC/MS (ESI) *m*/*e* 391.2 [(M+H)⁺, calcd for C₁₉H₁₈F₃N₄O₂ 391.2]; HPLC retention time (method A): *t*_{R} = 9.86 min; HPLC retention time (method J): *t*_{R} = 7.92 min; Chiral HPLC (method E): *t*_{R} = 41.6 min; ¹H NMR (400MHz, METHANOL-d₄) δ ppm 9.51 (d, *J*=0.8 Hz, 1H), 8.73 (s, 1H), 8.84 (d, *J*=6.0 Hz, 1H), 8.33 (d, *J*=5.6 Hz, 1H), 7.23 (s, 1H), 4.38 - 4.24 (m, 2H), 3.86 (s, 3H), 3.38 - 3.34 (m, 1H), 2.57 - 2.31 (m, 2H), 2.06 - 1.83 (m, 2H).

### Example 128 (invention)

### 8-((2-amino-5,5,5-trifluoropentyl)oxy)-9-bromo-4,6-dimethylbenzo[c][2,7]naphthyridin-5(6H)-one

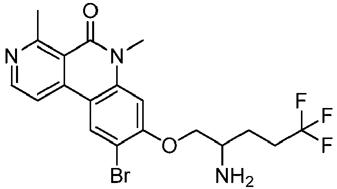

Prepared as described in Example 3: 8-((2-amino-5,5,5-trifluoropentyl)oxy)-9-bromo-4,6-dimethylbenzo[*c*][2,7]naphthyridin-5(6*H*)-one (7 mg, 0.015 mmol, 56% yield) as an off-white solid. LC/MS (ESI) *m*/*e* 458.4, 460.4 Br pattern [(M+H)⁺, calcd for C₁₉H₂₀BrF₃N₃O₂ 458.4]; HPLC retention time (method A): *t*_{R} = 7.89 min; HPLC retention time (method B): *t*_{R} = 8.80 min; ¹H NMR (400 MHz, METHANOL-d₄) δ ppm 8.62 (s, 1H), 8.58 (d, *J*=6.0 Hz, 1H), 8.13 (d, *J*=5.6 Hz, 1H), 7.09 (s, 1H), 4.86-4.19 (m, 2H), 3.78 (s, 3H), 3.51 - 3.41 (m, 1H), 3.09 (s, 3H), 2.52 - 2.40 (m, 2H), 2.09 - 2.03 (m, 1H), 1.97 - 1.86 (m, 1H).

### Example 129 (invention)

### (S)-8-((2-amino-4-methylpentyl)oxy)-9-isopropyl-4,6-dimethylbenzo[c][2,7]naphthyridin-5(6H)-one

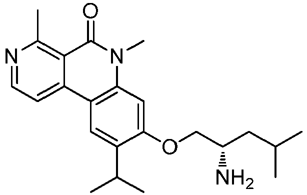

Prepared as described in Example 4: (S)-8-((2-amino-4-methylpentyl)oxy)-9-isopropyl-4,6-dimethylbenzo[*c*][2,7]naphthyridin-5(6*H*)-one (22 mg, 0.054 mmol, 66% yield) as an off-white solid. LC/MS (ESI) *m*/*e* 382.2[(M+H)⁺, calcd for C₂₃H₃₂N₃O₂ 382.2]; HPLC retention time (method A): *t*_{R} = 9.37 min; HPLC retention time (method B): *t*_{R} = 10.82 min; ¹H NMR (300 MHz, DMSO-*d*₆) δ ppm 8.60 (d, *J*=6.0 Hz, 1H), 8.25 (d, *J*=6.0 Hz, 1H), 8.20 (s, 1 H), 6.95 (s, 1H), 4.06 - 4.02 (m, 1H), 4.00 - 3.96 (m, 1H), 3.70 (s, 3H), 3.42 - 3.35 (m, 1H), 3.16 - 3.14 (m, 1H), 3.01 (s, 3H), 1.88 - 1.82 (m, 1H), 1.40 - 1.24 (m, 2H), 1.25 (d, *J* = 6.4 Hz, 6H), 0.95 (d, *J* = 6.4 Hz, 3H), 0.90 (d, *J* = 6.4 Hz, 3H).

### Example 130

### (S)-8-((2-amino-4-methylpentyl)oxy)-9-isopropyl-4,6-dimethylbenzo[c][2,7]naphthyridin-5(6H)-one

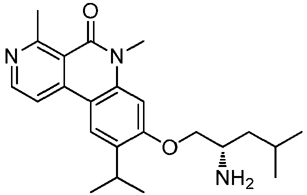

Prepared as described in Example 44: (*S*)-8-((2-amino-4-methylpentyl)oxy)-9-isopropyl-4,6-dimethylbenzo[*c*][2,7]naphthyridin-5(6*H*)-one (25 mg, 0.066 mmol, 77% yield) as an off-white solid. LC/MS (ESI) *m*/*e* 382.2 [(M+H)⁺, calcd for C₂₃H₃₂N₃O₂ 382.2]; HPLC retention time (method A): *t*_{R} = 6.99 min; HPLC retention time (method B): *t*_{R} = 7.54 min; ¹H NMR (400 MHz, METHANOL-d₄) δ ppm 8.56 (d, *J*=6.0 Hz, 1H), 8.37 (d, *J*=9.2 Hz, 1H), 8.13 (d, *J*=5.6 Hz, 1H), 7.23 (d, *J*=2.4 Hz, 1H), 7.06 (d, *J*=2.4 Hz, 1H), 7.05 (d, *J*=9.2 Hz, 1H), 4.51 (t, *J*=6.4 Hz, 2H), 4.24 (dd, *J*=9.6, 3.6 Hz, 1H), 4.05 (dd, *J*=9.6, 6.8 Hz, 1H), 3.94 (t, *J*=6.4 Hz, 2H), 3.46 - 3.43 (m, 1H), 3.46 (s, 3H), 3.08 (s, 3H), 1.88 - 1.84 (m, 1H), 1.57 - 1.50 (m, 2H), 1.05 (d, *J* = 6.4 Hz, 3H), 1.03 (d, *J* = 6.4 Hz, 3H).

### Example 131 (invention)

### (S)-8-((2-amino-4-methylpentyl)oxy)-9-bromo-6-(2-methoxyethyl)-4-methylbenzo[c][2,7]naphthyridin-5(6H)-one

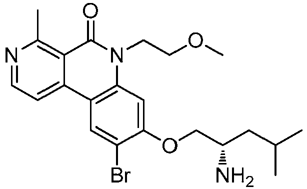

Prepared as described in Examples 3 and 12: (*S*)-8-((2-amino-4-methylpentyl)oxy)-9-bromo-6-(2-methoxyethyl)-4-methylbenzo[*c*][2,7]naphthyridin-5(6*H*)-one (10 mg, 0.021 mmol, 25% yield) as a pale brown solid. LC/MS (ESI) *m*/*e* 462.2, 464.2 Br pattern [(M+H)⁺, calcd for C₂₂H₂₈BrN₃O₃ 462.2]; HPLC retention time (method A): *t*_{R} = 9.25 min; HPLC retention time (method B): *t*_{R} = 11.97 min; ¹H NMR (400 MHz, METHANOL-d₄) δ ppm 8.64 (s, 1H), 8.59 (d, *J*=5.6 Hz, 1H), 8.14 (d, *J*=5.6 Hz, 1H),7.33 (s, 1H), 4.00 (t, *J*=5.6 Hz, 2H), 4.25 - 4.06 (m, 2H), 3.84 (t, *J*=5.6 Hz, 2H), 3.39 (s, 3H), 3.09 (s, 3H), 1.91 - 1.84 (m, 1H), 1.61 - 1.46 (m, 2H), 1.04 (d, *J* = 6.4 Hz, 3H), 1.02 (d, *J* = 6.4 Hz, 3H).

### Example 132 (invention)

### (S)-8-((2-amino-4-methylpentyl)oxy)-9-chloro-6-(2-hydroxyethyl)-4-methylbenzo[c][2,7]naphthyridin-5(6H)-one

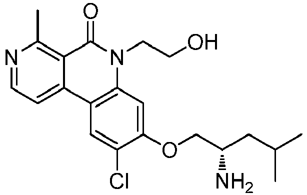

Prepared as described in Example 44: (*S*)-8-((2-amino-4-methylpentyl)oxy)-9-chloro-6-(2-hydroxyethyl)-4-methylbenzo[*c*][2,7]naphthyridin-5(6*H*)-one (6.2 mg, 0.015 mmol, 35% yield) as a colorless solid. LC/MS (ESI) *m*/*e* 404.2[(M+H)⁺, calcd for C₂₁H₂₇ClN₃O₃ 404.2]; HPLC retention time (method I): *t*_{R} = 8.02 min; HPLC retention time (method B): *t*_{R} = 8.91 min; ¹H NMR (300 MHz, METHANOL-d₄) δ ppm 8.56 (d, *J*=6.0 Hz, 1H), 8.44 (s, 1H), 8.10 (d, *J*=7.6 Hz, 1H), 7.32 (s, 1H), 4.51 (t, *J*=6.0 Hz, 2H), 4.29 (dd, *J*=9.6, 3.9 Hz, 1H), 4.11 (dd, *J*=9.6, 3.9 Hz, 1H), 3.95 (t, *J*=6.0 Hz, 2H), 3.36 - 3.10 (m, 1H), 3.07 (s, 3H), 1.88 - 1.81 (m, 1H), 1.62 - 1.48 (m, 2H), 1.02 (d, *J* = 6.4 Hz, 3H), 1.00 (d, *J* = 6.4 Hz, 3H).

### Example 133 (invention)

### (R)-8-((2-amino-4-methylpentyl)oxy)-9-bromo-4,6-dimethylbenzo[c][2,7]naphthyridin-5(6H)-one

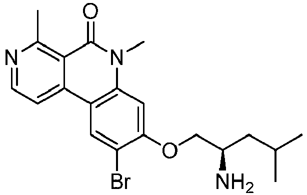

Prepared as described in Example 3: (*R*)-8-((2-amino-4-methylpentyl)oxy)-9-bromo-4,6-dimethylbenzo[*c*][2,7]naphthyridin-5(6*H*)-one (13 mg, 0.030 mmol, 46% yield) as an off-white solid. LC/MS (ESI) *m*/*e* 418.2, 420.2 Br pattern [(M+H)⁺, calcd for C₂₀H₂₅BrN₃O₂ 418.1]; HPLC retention time (method A): *t*_{R} = 8.27 min; HPLC retention time (method B): *t*_{R} = 9.51 min; ¹H NMR (400 MHz, METHANOL-d₄) δ ppm 8.59 (s, 1H), 8.56 (d, *J*=5.6 Hz, 1H), 8.10 (d, *J*=6.0 Hz, 1H), 7.05 (s, 1H), 4.29 (dd, *J*=9.6, 4.0 Hz, 1H), 4.09 (dd, *J*=9.6, 6.4 Hz, 1H), 3.68 (s, 3H), 3.45 - 3.30 (m, 1H), 3.06 (s, 3H), 1.90 - 1.83 (m, 1H), 1.62 - 1.45 (m, 2H), 1.04 (d, *J=* 6.4 Hz, 3H), 1.01 (d, *J=* 6.4 Hz, 3H).

### Example 134 (invention)

### (S)-8-((2-amino-4-methylpentyl)oxy)-9-bromo-7-fluoro-4,6-dimethylbenzo[c][2,7]naphthyridin-5(6H)-one

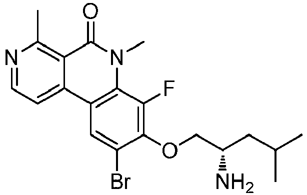

Prepared as described in Example 3: (*S*)-8-((2-amino-4-methylpentyl)oxy)-9-bromo-7-fluoro-4,6-dimethylbenzo[*c*][2,7]naphthyridin-5(6*H*)-one (5 mg, 0.011 mmol, 14% yield) as an off-white solid. LC/MS (ESI) *m*/*e* 436.0, 438.0 Br pattern [(M+H)⁺, calcd for C₂₀H₂₄BrFN₃O₂ 436.1]; HPLC retention time (method A): *t*_{R} = 9.16 min; HPLC retention time (method G): *t*_{R} = 5.28 min ¹H NMR (400 MHz, METHANOL-d₄) δ ppm 8.61 (d, *J*=5.6 Hz, 1H), 8.44 (d, *J*=2.0 Hz, 1H), 8.12 (d, *J*=5.6 Hz, 1H), 4.27 - 4.24 (m, 1H), 4.09 - 4.05 (m, 1H), 3.70 - 3.62 (m, 1H), 3.36 (s, 3H), 3.07 (s, 3H), 1.81 - 1.84 (m, 1H), 1.52 - 1.31 (m, 2H), 1.02 (d, *J* = 6.4 Hz, 3H), 1.01 (d, *J* = 6.4 Hz, 3H).

### Example 135 (invention)

### 8-(2-amino-4,4,4-trifluorobutoxy)-6-methylbenzo[c][2,7]naphthyridin-5(6H)-one

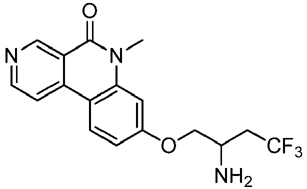

The material from Example 93 was separated into the corresponding enantiomers via chiral SFC (Chiralpak-IA(250 X 21 mm), 5 micron; mobile phase: 60% CO₂ / 40% (0.5% DEA in Methanol); Flow rate: 60 g/min). First eluting enantiomer: 8-(2-amino-4,4,4-trifluorobutoxy)-6-methylbenzo[c][2,7]naphthyridin-5(6*H*)-one (8 mg, 0.022 mmol, 10% yield) as an off-white solid. The absolute stereochemistry was not determined. LC/MS (ESI) m/e 352.0 [(M+H)⁺, calcd for C₁₇H₁₇F₃N₃O₂ 352.1]; HPLC retention time (method B): *t_{R}* = 7.19 min; HPLC retention time (method A): *t_{R}* = 6.80 min; Chiral HPLC (method E): *t*_{R} = 4.28 min; ¹H NMR (400 MHz, MeOD): δ 9.40 (s, 1H), 8.72 (d, *J*=6.0 Hz, 1H), 8.32 (d, *J*=8.8 Hz, 1H), 8.17 (d, *J*=5.6 Hz, 1H), 7.08 (d, *J*=2.4 Hz, 1H), 7.06 (dd, *J*=8.8, 2.4 Hz, 1H), 4.20 - 4.11 (m, 2H), 3.75 (s, 3H), 3.65 - 3.55 (m, 1H), 2.70 - 2.62 (m, 1H), 2.49 - 2.40 (m, 1H).

### Example 136

### 8-(2-amino-4,4,4-trifluorobutoxy)-6-methylbenzo[c][2,7]naphthyridin-5(6H)-one

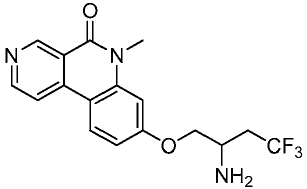

The material from Example 93 was separated into the corresponding enantiomers via chiral SFC (Chiralpak-IA (250 X 21 mm), 5 micron; mobile phase: 60% CO₂ / 40% (0.5% DEA in Methanol); Flow rate: 60 g/min). Second eluting enantiomer: 8-(2-amino-4,4,4-trifluorobutoxy)-6-methylbenzo[*c*][2,7]naphthyridin-5(6*H*)-one (8 mg, 0.022 mmol, 10% yield) as an off-white solid. The absolute stereochemistry was not determined. LC/MS (ESI) m/e 352.0 [(M+H)⁺, calcd for C₁₇H₁₇F₃N₃O₂ 352.1]; HPLC retention time (method B): *t_{R}* = 7.2 min; HPLC retention time (method A): *t_{R}* = 6.79 min; Chiral HPLC (method E): *t*_{R} = 6.09 min; ¹H NMR (400 MHz, MeOD): δ 9.47 (s, 1H), 8.77 (d, *J*=6.0 Hz, 1H), 8.41 (d, *J*=8.8 Hz, 1H), 8.25 (d, *J*=5.6 Hz, 1H), 7.14 (d, *J*=2.4 Hz, 1H), 7.10 (dd, *J*=8.8, 2.4 Hz, 1H), 4.22 - 4.13 (m, 2H), 3.80 (s, 3H), 3.66 - 3.58 (m, 1H), 2.70 - 2.62 (m, 1H), 2.48 - 2.42 (m, 1H).

### Example 137 (invention)

### 8-(2-amino-4,4,4-trifluorobutoxy)-9-bromo-4,6-dimethylbenzo[c][2,7]naphthyridin-5(6H)-one

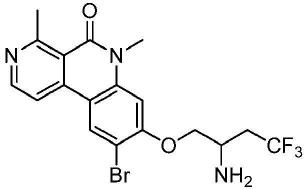

Prepared as described in Example 93: 8-(2-amino-4,4,4-trifluorobutoxy)-9-bromo-4,6-dimethylbenzo[*c*][2,7]naphthyridin-5(6*H*)-one (20 mg, 0.044 mmol, 69% yield) as an off-white solid. LC/MS (ESI) m/e 443.9, 445.9 Br pattern [(M+H)⁺, calcd for C₁₈H₁₈BrF₃N₃O₂ 444.1]; HPLC retention time (method B): *t_{R}* = 8.64 min; HPLC retention time (method A): *t_{R}* = 7.95 min; ¹H NMR (400MHz, METHANOL-d₄) δ 8.57 (d, *J*=5.5 Hz, 1H), 8.55 (s, 1H), 8.08 (d, *J*=6.0 Hz, 1H), 7.02 (s, 1H), 4.32 - 4.16 (m, 2H), 3.74 (s, 3H), 3.69 - 3.61 (m, 1H), 3.07 (s, 3H), 2.81 - 2.64 (m, 1H), 2.55 - 2.39 (m, 1H).

### Example 138

### 8-(2-amino-4,4,4-trifluorobutoxy)-9-bromo-4,6-dimethylbenzo[c][2,7]naphthyridin-5(6H)-one

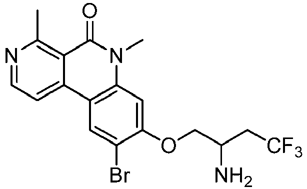

The material from Example 137 was separated into the corresponding enantiomers via chiral SFC ((Chiralpak-AD-H (250 X 21 mm), 5u: mobile phase: 60% CO₂, 40% (0.3% DEA in Methanol); Flow rate: 60 g/min). First eluting enantiomer: 8-(2-amino-4,4,4-trifluorobutoxy)-9-bromo-4,6-dimethylbenzo[*c*][2,7]naphthyridin-5(6*H*)-one (8 mg, 0.018 mmol, 32% yield) as an off-white solid. The absolute stereochemistry was not determined. LC/MS (ESI) m/e 443.9, 445.9 Br pattern [(M+H)⁺, calcd for C₁₈H₁₈BrF₃N₃O₂ 444.1]; HPLC retention time (method B): *t_{R}* = 8.63 min; HPLC retention time (method A): *t_{R}* = 8.10 min; Chiral HPLC (method F): *t*_{R} = 2.38 min; ¹H NMR (400MHz, METHANOL-d₄) δ 9.45 (d, *J*=1.0 Hz, 1H), 8.78 (d, *J*=6.0 Hz, 1H), 8.59 (s, 1H), 8.20 (d, *J*=5.0 Hz, 1H), 7.08 (s, 1H), 4.36 - 4.19 (m, 2H), 3.79 (s, 3H), 3.64 (dq, *J*=8.0, 5.0 Hz, 1H), 2.85 - 2.64 (m, 1H), 2.58 - 2.36 (m, 1H).

### Example 139

### 8-(2-amino-4,4,4-trifluorobutoxy)-9-bromo-4,6-dimethylbenzo[c][2,7]naphthyridin-5(6H)-one

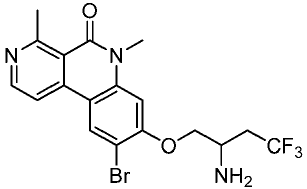

The material from Example 137 was separated into the corresponding enantiomers via chiral SFC ((Chiralpak-AD-H (250 X 21 mm), 5µ: mobile phase: 60% CO₂, 40% (0.3% DEA in Methanol); Flow rate: 60 g/min). Second eluting enantiomer: 8-(2-amino-4,4,4-trifluorobutoxy)-9-bromo-4,6-dimethylbenzo[*c*][2,7]naphthyridin-5(6*H*)-one (8 mg, 0.018 mmol, 32% yield) as an off-white solid. The absolute stereochemistry was not determined. LC/MS (ESI) m/e 443.9, 445.9 Br pattern [(M+H)⁺, calcd for C₁₈H₁₈BrF₃N₃O₂ 444.1]; HPLC retention time (method B): *t_{R}* = 8.63 min; HPLC retention time (method A): *t_{R}* = 8.10 min; Chiral HPLC (method F): *t*_{R} = 4.85 min; ¹H NMR (400MHz, METHANOL-d₄) δ 9.47 (s, 1H), 8.79 (d, *J*=6.0 Hz, 1H), 8.48 (s, 1H), 8.23 (d, *J*=5.5 Hz, 1H), 7.15 (s, 1H), 4.33 - 4.17 (m, 2H), 3.81 (s, 3H), 3.71 - 3.61 (m, 1H), 2.71 (ddd, *J*=15.2, 11.4, 5.0 Hz, 1H), 2.46 (dqd, *J*=15.0, 11.2, 7.5 Hz, 1H).

### Example 140 (invention)

### 8-(2-amino-4,4,4-trifluorobutoxy)-9-bromo-6-methylbenzo[c][2,7]naphthyridin-5(6H)-one

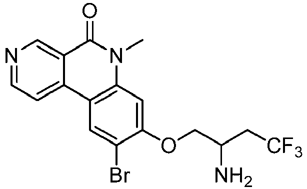

Prepared as described in Examples 93 and 3: 8-(2-amino-4,4,4-trifluorobutoxy)-9-bromo-6-methylbenzo[*c*][2,7]naphthyridin-5(6*H*)-one (8 mg, 0.018 mmol, 32% yield) as an off-white solid. LC/MS (ESI) m/e 429.9, 431.9 Br pattern [(M+H)⁺, calcd for C₁₇H₁₆BrF₃N₃O₂ 430.0]; HPLC retention time (method A): *t_{R}* = 8.66 min; HPLC retention time (method B): *t_{R}* = 8.97 min; ¹H NMR (400MHz, METHANOL-d₄) δ 8.60 - 8.55 (m, 2H), 8.10 (d, *J*=6.0 Hz, 1H), 7.05 (s, 1H), 4.35 - 4.17 (m, 2H), 3.76 (s, 3H), 3.69 - 3.58 (m, 1H), 3.07 (s, 3H), 2.83 - 2.61 (m, 1H), 2.46 (dqd, *J*=15.0, 11.1, 7.8 Hz, 1H).

### Example 141 (invention)

### 8-(2-amino-4,4,4-trifluorobutoxy)-9-chloro-6-methylbenzo[c][2,7]naphthyridin-5(6H)-one

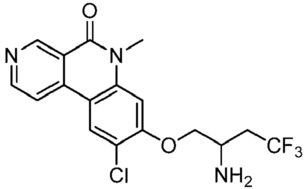

Prepared as described in Examples 93 and 3: 8-(2-amino-4,4,4-trifluorobutoxy)-9-chloro-6-methylbenzo[c][2,7]naphthyridin-5(6H)-one (15 mg, 0.038 mmol, 37% yield) as an off-white solid. LC/MS (ESI) m/e 386.0 [(M+H)⁺, calcd for C₁₇H₁₆ClF₃N₃O₂ 386.1]; HPLC retention time (method A): *t_{R}* = 8.37 min; HPLC retention time (method B): *t_{R}* = 8.63 min; ¹H NMR (400MHz, METHANOL-d₄) δ 8.62 - 8.54 (m, 2H), 8.10 (d, *J*=5.5 Hz, 1H), 7.05 (s, 1H), 4.33 - 4.12 (m, 2H), 3.76 (s, 3H), 3.69 - 3.59 (m, 1H), 3.07 (s, 3H), 2.84 - 2.61 (m, 1H), 2.47 (dqd, *J* =15.0, 11.2, 7.5 Hz, 1H).

### Example 142 (invention)

### (S)-8-((2-amino-4-methylpentyl)oxy)-9-chlorobenzo[c][2,7]naphthyridin-5(6H)-one

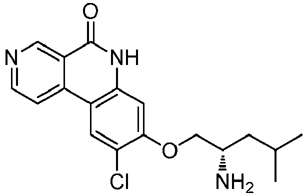

Prepared as described in Example 3: (*S*)-8-((2-amino-4-methylpentyl)oxy)-9-chlorobenzo[*c*][2,7]naphthyridin-5(6*H*)-one (10 mg, 0.717 mmol, 89% yield) as a pale yellow solid. LC/MS (ESI) *m*/*e* 346.0 [(M+H)⁺, calcd for C₁₈H₂₁ClN₃O₂ 346.1]; HPLC retention time (method A): *t*_{R} = 8.13 min, HPLC retention time (method B): *t*_{R} = 9.00 min; ¹H NMR (400MHz, METHANOL-d₄) δ 8.66 (d, *J*=6.0 Hz, 1H), 8.56 (s, 1H), 8.41 (d, *J*=6.0 Hz, 1H), 7.02 (s, 1H), 4.46 (dd, *J*=10.3, 3.3 Hz, 1H), 4.31 (dd, *J* =10.5, 5.5 Hz, 1H), 3.87 - 3.80 (m, 1H), 3.18 (s, 3H), 1.90 - 1.69 (m, 3H), 1.08 (d, *J* =3.5 Hz, 3H), 1.07 (d, *J*=3.5 Hz, 3H).

### Example 143 (invention)

### (S)-8-((2-amino-4-methylpentyl)oxy)-9-bromo-6-(2-hydroxyethyl)-4-methylbenzo[c][2,7]naphthyridin-5(6H)-one

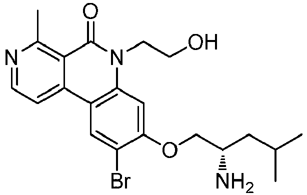

Prepared as described in Example 44: (*S*)-8-((2-amino-4-methylpentyl)oxy)-9-bromo-6-(2-hydroxyethyl)-4-methylbenzo[*c*][2,7]naphthyridin-5(6*H*)-one (8 mg, 0.016 mmol, 24% yield) as a colorless solid. LC/MS (ESI) *m*/*e* 448.0, 450.0 Br pattern [(M+H)⁺, calcd for C₂₁H₂₇BrN₃O₃ 448.1]; HPLC retention time (method A): *t*_{R} = 8.66 min; HPLC retention time (method B): *t*_{R} = 10.05 min; ¹H NMR (300 MHz, METHANOL-d₄) δ ppm 8.62 (s, 1H), 8.57 (d, *J*=5.7 Hz, 1H), 8.13 (d, *J=6.0* Hz, 1H), 7.31 (s, 1H), 4.52 (t, *J*=5.7 Hz, 2H), 4.34 (dd, *J*=9.6, 3.6 Hz, 1H), 4.15 (dd, *J*=9.6, 6.3 Hz, 1H), 3.96 (t, *J*=5.7 Hz, 2H), 3.55 - 3.52 (m, 1H), 3.07 (s, 3H), 1.90 - 1.81 (m, 1H), 1.69 - 1.52 (m, 2H), 1.04 (d, *J* = 6.4 Hz, 3H), 1.02 (d, *J* = 6.4 Hz, 3H).

### Example 144 (invention)

### 8-methoxy-4,6-dimethylbenzo[c][2,7]naphthyridin-5(6H)-one

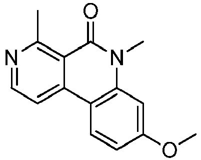

Prepared as described in Example 2: 8-methoxy-4,6-dimethylbenzo[*c*][2,7]naphthyridin-5(6*H*)-one (40 mg, 0.155 mmol, 80% yield) as a colorless solid. LC/MS (ESI) *m*/*e* 255.2 [(M+H)⁺, calcd for C₁₅H₁₅N₂O₂ 255.1]; HPLC retention time (method A): *t*_{R} = 8.91 min; HPLC retention time (method B): *t*_{R} = 10.52 min; ¹H NMR (400MHz, DMSO-d₆) δ 8.61 (d, *J*=5.5 Hz, 1H), 8.43 (d, *J*=8.5 Hz, 1H), 8.17 (d, *J*=5.5 Hz, 1H), 7.04 - 6.90 (m, 2H), 3.94 (s, 3H), 3.67 (s, 3H), 3.00 (s, 3H).

### Example 145 (invention)

### (S)-1-(8-((2-amino-4-methylpentyl)oxy)-9-fluoro-6-methyl-5-oxo-5,6-dihydrobenzo[c][2,7]naphthyridin-4-yl)-3-methylurea

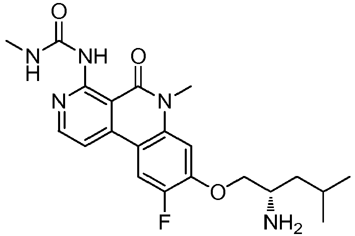

Prepared as described in Example 18: (*S*)-1-(8-((2-amino-4-methylpentyl)oxy)-9-fluoro-6-methyl-5-oxo-5,6-dihydrobenzo[*c*][2,7]naphthyridin-4-yl)-3-methylurea, TFA (147 mg, 0.264 mmol, 26% yield) as a pale yellow solid. LC/MS (ESI) *m*/*e* 416.2 [(M+H)⁺, calcd for C₂₁H₂₇FN₅O₃ 416.2]; HPLC retention time (method K): *t*_{R} = 5.53 min; HPLC retention time (method L): *t*_{R} = 5.61 min; ¹H NMR (400MHz, METHANOL-d₄) δ 8.21 (d, J=6.3 Hz, 1H), 8.00 (d, J=11.8 Hz, 1H), 7.57 (br. s., 1H), 7.20 (d, J=7.3 Hz, 1H), 4.57 (dd, J=10.5, 3.0 Hz, 1H), 4.44 (dd, J=10.7, 6.4 Hz, 1H), 3.88 - 3.79 (m, 1H), 3.76 (s, 3H), 2.92 (s, 3H), 1.89 (dt, J=13.3, 6.7 Hz, 1H), 1.84 - 1.67 (m, 2H), 1.09 (d, J=4.0 Hz, 3H), 1.07 (d, J=4.0 Hz, 3H).

### Example 146 (invention)

### 8-(2-hydroxy-3-(isopropylamino)propoxy)-6-methylbenzo[c][2,7]naphthyridin-5(6H)-one

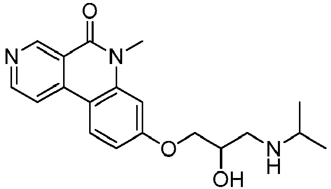

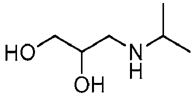

### Part A: 3-(isopropylamino)propane-1,2-diol

To propan-2-amine (0.399 g, 6.75 mmol) in ethanol (2 mL) was added oxiran-2-ylmethanol (0.5 g, 6.75 mmol) slowly. The mixture was stirred at room temperature for 5 h. The crude reaction mixture was concentrated under reduced pressure to afford 3-(isopropylamino)propane-1,2-diol (0.82 g, 6.16 mmol, 91 % crude yield) as a brownish color semi-solid which was carried forward without further purification. LC/MS (ESI) *m*/*e* 133.9 [(M+H)⁺, calcd for C₆H₁₆NO₂ 134.2]; ¹H NMR (400MHz, CDCl₃) δ 3.76 - 3.49 (m, 3H), 2.89 - 2.60 (m, 3H), 2.04 (br s, 3H), 1.08 (d, *J*=6.4 Hz, 3H), 1.06 (d, *J*=6.4 Hz, 3H).

### Part B: 8-(2-hydroxy-3-(isopropylamino)propoxy)-6-methylbenzo[c][2,7]naphthyridin-5(6H)-one

Prepared as described in Example 2 using 3-(isopropylamino)propane-1,2-diol (0.082 g, 0.616 mmol) and 8-chloro-6-methylbenzo[*c*][2,7]naphthyridin-5(6*H*)-one (0.1 g, 0.409 mmol) to afford 8-(2-hydroxy-3-(isopropylamino)propoxy)-6-methylbenzo[*c*][2,7]naphthyridin-5(6*H*)-one (55 mg, 0.159 mmol, 39% yield) as a colorless solid. LC/MS (ESI) *m*/*e* 342.2 [(M+H)⁺, calcd for C₁₉H₂₄N₃O₃ 342.2]; HPLC retention time (method A): *t*_{R} = 5.48 min; HPLC retention time (method B): *t*_{R} = 6.17 min; ¹H NMR (400MHz, DMSO-d₆) δ 9.41 (s, 1H), 8.82 (d, *J*=6.0 Hz, 1H), 8.48 (d, *J*=9.5 Hz, 1H), 8.37 (d, *J*=5.5 Hz, 1H), 7.14 - 7.01 (m, 2H), 4.28 - 4.09 (m, 3H), 3.70 (s, 3H), 3.35 (dt, *J*=12.9, 6.3 Hz, 2H), 3.10 - 2.98 (m, 1H), 1.26 (d, *J*=4.0 Hz, 3H), 1.24 (d, *J*=4.0 Hz, 3H).

### Example 147 (invention)

### 8-(2-hydroxy-3-(isopropylamino)propoxy)-4,6-dimethylbenzo[c][2,7]naphthyridin-5(6H)-one

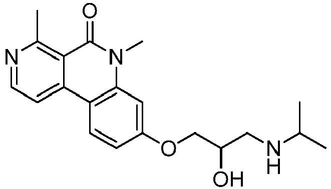

Prepared as described in Example 146: 8-(2-hydroxy-3-(isopropylamino)propoxy)-4,6-dimethylbenzo[*c*][2,7]naphthyridin-5(6*H*)-one (62 mg, 0.172 mmol, 45% yield) as a colorless solid. LC/MS (ESI) *m*/*e* 356.2 [(M+H)⁺, calcd for C₂₀H₂₆N₃O₃ 356.2]; HPLC retention time (method A): *t*_{R} = 5.91 min; HPLC retention time (method B): *t*_{R} = 6.64 min; ¹H NMR (400MHz, METHANOL-d₄) δ 8.67 - 8.63 (m, 2H), 8.55 (d, *J*=8.0 Hz, 1H), 7.20 - 7.16 (m, 2H), 4.38 - 4.30 (m, 3H), 3.81 (s, 3H), 3.52 - 3.50 (m, 1H), 3.38 - 3.36 (m, 1H), 3.26 (s, 3H), 3.24 - 3.22 (m, 1H), 1.42 (d, *J*=6.4 Hz, 3H), 1.41 (d, *J*=6.4 Hz, 3H).

### Example 148 (invention)

### 9-bromo-8-(2-hydroxy-3-(isopropylamino)propoxy)-6-methylbenzo[c][2,7]naphthyridin-5(6H)-one

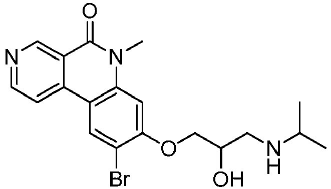

Prepared as described in Example 3: 9-bromo-8-(2-hydroxy-3-(isopropylamino)propoxy)-6-methylbenzo[*c*][2,7]naphthyridin-5(6*H*)-one (12 mg, 0.028 mmol, 24% yield) as a colorless solid. LC/MS (ESI) *m*/*e* 420.0, 422.0 Br pattern [(M+H)⁺, calcd for C₁₉H₂₃BrN₃O₃ 420.1]; HPLC retention time (method A): *t*_{R} = 7.21 min; HPLC retention time (method B): *t*_{R} = 8.13 min; ¹H NMR (400 MHz, METHANOL-d₄) δ ppm 8.50 (br s, 1H), 8.83 (bs, 1H), 8.63 (s, 1H), 8.28 (d, *J*=4.64 Hz, 1 H), 7.11 (s, 1H), 4.25 - 4.45 (m, 3H), 3.81 (s, 3H), 3.51-3.58 (m, 1H), 3.32-3.49 (m, 2H), 1.44 (d, *J*=6.4 Hz, 3H), 1.43 (d, *J*=6.4 Hz, 3H).

### Example 149 (invention)

### 9-bromo-8-(2-hydroxy-3-(isopropylamino)propoxy)-4,6-dimethylbenzo[c][2,7]naphthyridin-5(6H)-one

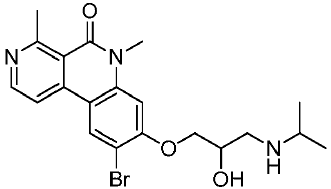

Prepared as described in Example 3: 9-bromo-8-(2-hydroxy-3-(isopropylamino)propoxy)-4,6-dimethylbenzo[c][2,7]naphthyridin-5(6*H*)-one (10 mg, 0.021 mmol, 19% yield) as a colorless solid. LC/MS (ESI) *m*/*e* 434.0, 436.0 Br pattern [(M+H)⁺, calcd for C₂₀H₂₅BrN₃O₃ 434.1]; HPLC retention time (method A): *t*_{R} = 6.78 min; HPLC retention time (method B): *t*_{R} = 7.77 min; ¹H NMR (400 MHz, METHANOL-*d*₄) δ ppm 8.61 (s, 1H), 8.59 (d, *J*=5.6 Hz, 1H), 8.12 (d, *J*=6.0 Hz, 1H), 7.09 (s, 1 H), 4.39 - 4.29 (m, 3H), 3.78 (s, 3H), 3.40 - 3.18 (m, 3H), 3.09 (s, 3H), 1.37 (d, *J*=6.4 Hz, 3H), 1.36 (d, *J*=6.4 Hz, 3H).

### Example 150 (invention)

### 8-((2,4-dimethylpentyl)oxy)-6-methylbenzo[c][2,7]naphthyridin-5(6H)-one

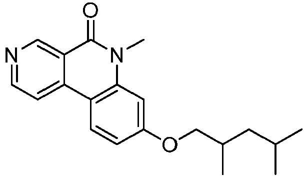

Prepared as described in Example 2: 8-((2,4-dimethylpentyl)oxy)-6-methylbenzo[*c*][2,7]naphthyridin-5(6*H*)-one (8.5 mg, 0.026 mmol, 13% yield) as a brown solid. LC/MS (ESI) *m*/*e* 325.2 [(M+H)⁺, calcd for C₂₀H₂₅N₂O₂ 325.2]; HPLC retention time (method G): *t*_{R} = 10.12 min; HPLC retention time (method I): *t*_{R} = 9.41 min; ¹H NMR (400MHz, METHANOL-d₄) δ 9.47 (s, 1H), 8.76 (d, *J*=6.0 Hz, 1H), 8.41 (d, *J*=10.0 Hz, 1H), 8.35 (d, *J*=6.0 Hz, 1H), 7.11 - 7.02 (m, 2H), 4.14 - 4.02 (m, 1H), 4.01 - 3.94 (m, 1H), 3.79 (s, 3H), 2.23 - 2.03 (m, 1H), 1.87 - 1.71 (m, 1H), 1.45 (ddd, *J*=13.6, 8.0, 5.5 Hz, 1H), 1.28 - 1.17 (m, 1H), 1.10 (d, *J*=6.5 Hz, 3H), 0.99 (d, *J*=6.5 Hz, 3H), 0.95 (d, *J*=6.5 Hz, 3H).

### Example 151 (invention)

### 6-methyl-8-((4-methylpentyl)oxy)benzo[c][2,7]naphthyridin-5(6H)-one

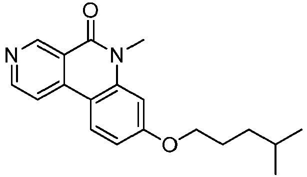

Prepared as described in Example 2: 6-methyl-8-((4-methylpentyl)oxy)benzo[*c*][2,7]naphthyridin-5(6*H*)-one (3.2 mg, 0.009 mmol, 5% yield) as a pale yellow solid. LC/MS (ESI) *m*/*e* 311.2 [(M+H)⁺, calcd for C₁₉H₂₃N₂O₂ 311.2]; HPLC retention time (method G): *t*_{R} = 9.62 min; HPLC retention time (method I): *t*_{R} = 8.70 min; ¹H NMR (400MHz, METHANOL-d₄) δ 9.47 (d, *J*=1.0 Hz, 1H), 8.76 (d, *J*=5.5 Hz, 1H), 8.41 (d, *J*=9.0 Hz, 1H), 8.26 (d, *J*=5.5 Hz, 1H), 7.11 - 7.09 (m, 1H), 7.07 (dd, *J*=9.0, 2.5 Hz, 1H), 4.19 (t, *J*=6.5 Hz, 2H), 3.81 (s, 3H), 1.98 - 1.83 (m, 2H), 1.67 (dt, *J*=13.4, 6.6 Hz, 1H), 1.50 - 1.41 (m, 2H), 0.98 (d, *J*=6.5 Hz, 6H).

### Example 152 (invention)

### (S)-8-((2-hydroxy-4-methylpentyl)oxy)-4,6-dimethylbenzo[c][2,7]naphthyridin-5(6H)-one

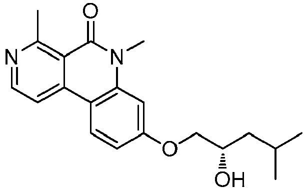

Prepared as described in Example 2: (S)-8-((2-hydroxy-4-methylpentyl)oxy)-4,6-dimethylbenzo[*c*][2,7]naphthyridin-5(6*H*)-one (26 mg, 0.074 mmol, 19% yield) as a colorless solid. LC/MS (ESI) *m*/*e* 341.0 [(M+H)⁺, calcd for C₂₀H₂₅N₂O₃ 341.2]; HPLC retention time (method M): *t*_{R} = 6.06 min; HPLC retention time (method G): *t*_{R} = 6.95 min; ¹H NMR (400MHz, METHANOL-d₄) δ 8.54 (d, *J*=6.0 Hz, 1H), 8.36 (d, *J*=8.5 Hz, 1H), 8.13 (d, *J*=6.0 Hz, 1H), 7.13 - 6.97 (m, 2H), 4.25 - 3.98 (m, 3H), 3.75 (s, 3H), 3.08 (s, 3H), 2.05 - 1.84 (m, 1H), 1.65 - 1.52 (m, 1H), 1.50 - 1.38 (m, 1H), 1.01 (m, 6H).

### Example 153 (invention)

### 8-(2-hydroxy-3-isopropoxypropoxy)-4,6-dimethylbenzo[c][2,7]naphthyridin-5(6H)-one

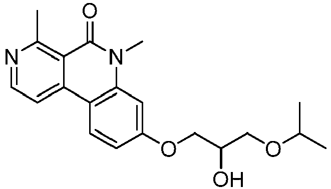

Prepared as described in Example 146: 8-(2-hydroxy-3-isopropoxypropoxy)-4,6-dimethylbenzo[*c*][2,7]naphthyridin-5(6*H*)-one (11 mg, 0.031 mmol, 23% yield) as a colorless solid. LC/MS (ESI) *m*/*e* 357.1 [(M+H)⁺, calcd for C₂₀H₂₅N₂O₄ 357.2]; HPLC retention time (method B): *t*_{R} = 10.82 min; HPLC retention time (method I): *t*_{R} = 9.66 min; ¹H NMR (400MHz, METHANOL-d₄) δ 7.04 (d, *J*=5.5 Hz, 1H), 6.75 (d, *J*=9.5 Hz, 1H), 6.60 (d, *J*=5.5 Hz, 1H), 5.47 (dd, *J*=9.0, 2.5 Hz, 1H), 5.33 (d, *J*=2.5 Hz, 1H), 2.43 - 2.26 (m, 2H), 1.83 - 1.81 (m, 1H), 1.55 (s, 3H), 0.37 - 0.24 (m, 1H), 0.11 - -0.05 (m, 2H), -0.25 (s, 3H), -0.50 (d, *J*=6.5 Hz, 3H), -0.53 (d, *J*=6.5 Hz, 3H).

### Example 154 (invention)

### 9-bromo-8-((2,4-dimethylpentyl)oxy)-6-methylbenzo[c][2,7]naphthyridin-5(6H)-one

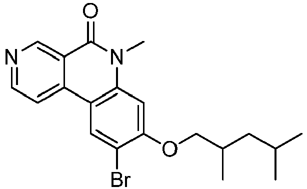

Prepared as described in Example 3: 9-bromo-8-((2,4-dimethylpentyl)oxy)-6-methylbenzo[*c*][2,7]naphthyridin-5(6*H*)-one (15 mg, 0.037 mmol, 60% yield) as an off-white solid. LC/MS (ESI) *m*/*e* 402.18, 404.8 Br pattern [(M+H)⁺, calcd for C₂₀H₂₄BrN₂O₂ 403.1]; HPLC retention time (method G): *t*_{R} = 10.69 min; HPLC retention time (method I): *t*_{R} = 11.69 min; ¹H NMR (400MHz, METHANOL-d₄) δ 9.48 (s, 1H), 8.78 (d, *J*=6.0 Hz, 1H), 8.65 (s, 1H), 8.26 (d, *J*=5.5 Hz, 1H), 7.09 (s, 1H), 4.22 - 4.02 (m, 2H), 3.83 (s, 3H), 2.18 (dq, *J*=13.4, 6.6 Hz, 1H), 1.89 - 1.74 (m, 1H), 1.53 (ddd, *J*=13.4, 7.9, 5.8 Hz, 1H), 1.30 - 1.20 (m, 1H), 1.15 (d, *J*=6.5 Hz, 3H), 1.00 (d, *J*=6.5 Hz, 3H), 0.97 (d, *J*=6.5 Hz, 3H).

### Example 155 (invention)

### 8-((2,4-dimethylpentyl)oxy)-4,6-dimethylbenzo[c][2,7]naphthyridin-5(6H)-one

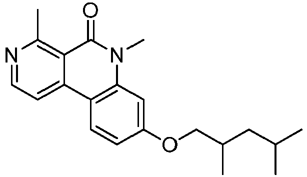

Prepared as described in Example 2: 8-((2,4-dimethylpentyl)oxy)-4,6-dimethylbenzo[*c*][2,7]naphthyridin-5(6*H*)-one (40 mg, 0.118 mmol, 12% yield) as an off-white solid. LC/MS (ESI) *m*/*e* 339.0 [(M+H)⁺, calcd for C₂₁H₂₇N₂O₂ 339.2]; HPLC retention time (method G): *t*_{R} = 9.62 min; HPLC retention time (method I): *t*_{R} = 8.27 min; ¹H NMR (400MHz, METHANOL-d₄) δ 8.54 (d, *J*=6.0 Hz, 1H), 8.34 (d, *J*=10.0 Hz, 1H), 8.12 (d, *J*=6.0 Hz, 1H), 7.06 - 6.93 (m, 2H), 4.08 - 3.99 (m, 1H), 3.98 - 3.90 (m, 1H), 3.75 (s, 3H), 3.08 (s, 3H), 2.25 - 2.04 (m, 1H), 1.87 - 1.70 (m, 1H), 1.44 (ddd, *J*=13.7, 8.2, 5.8 Hz, 1H), 1.27 - 1.17 (m, 1H), 1.10 (d, *J*=6.5 Hz, 3H), 0.99 (d, *J*=6.5 Hz, 3H), 0.95 (d, *J*=6.5 Hz, 3H).

### Example 156 (invention)

### 4,6-dimethyl-8-((4-methylpentyl)oxy)benzo[c][2,7]naphthyridin-5(6H)-one

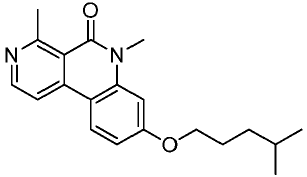

Prepared as described in Example 2: 4,6-dimethyl-8-((4-methylpentyl)oxy)benzo[*c*][2,7]naphthyridin-5(6*H*)-one (32 mg, 0.098 mmol, 34% yield) as an off-white solid. LC/MS (ESI) *m*/*e* 325.0 [(M+H)⁺, calcd for C₂₂H₂₅N₂O₂ 325.2]; HPLC retention time (method G): *t*_{R} = 9.13 min; HPLC retention time (method I): *t*_{R} = 7.80 min; ¹H NMR (400MHz, METHANOL-d₄) δ 8.52 (d, *J*=5.5 Hz, 1H), 8.36 - 8.23 (m, 1H), 8.08 (d, *J*=5.5 Hz, 1H), 7.03 - 6.91 (m, 2H), 4.15 (t, *J*=6.5 Hz, 2H), 3.72 (s, 3H), 3.06 (s, 3H), 1.94 - 1.81 (m, 2H), 1.67 (dquin, *J*=13.4, 6.6 Hz, 1H), 1.50 - 1.37 (m, 2H), 0.98 (d, *J*=7.0 Hz, 6H).

### Example 157 (invention)

### 8-((2,4-dimethylpentyl)oxy)-6-methylbenzo[c][2,7]naphthyridin-5(6H)-one

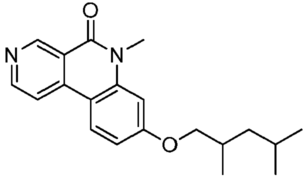

Prepared as described in Example 2: 8-((2,4-dimethylpentyl)oxy)-6-methylbenzo[*c*][2,7]naphthyridin-5(6*H*)-one (18 mg, 0.055 mmol, 7% yield) as a colorless solid. LC/MS (ESI) *m*/*e* 325.0 [(M+H)⁺, calcd for C₂₀H₂₅N₂O₂ 325.2]; HPLC retention time (method G): *t*_{R} = 9.54 min; HPLC retention time (method I): *t*_{R} = 9.44 min; ¹H NMR (400MHz, METHANOL-d₄) δ 9.47 (s, 1H), 8.76 (d, *J*=6.0 Hz, 1H), 8.41 (d, *J*=10.0 Hz, 1H), 8.35 (d, *J*=6.0 Hz, 1H), 7.13 - 6.96 (m, 2H), 4.14 - 4.02 (m, 1H), 4.01 - 3.93 (m, 1H), 3.79 (s, 3H), 2.26 - 2.03 (m, 1H), 1.88 - 1.70 (m, 1H), 1.45 (ddd, *J*=13.6, 8.0, 5.5 Hz, 1H), 1.27 - 1.17 (m, 1H), 1.10 (d, *J*=6.5 Hz, 3H), 0.99 (d, *J*=6.5 Hz, 3H), 0.95 (d, *J*=6.5 Hz, 3H).

### Example 158

### 8-((2,4-dimethylpentyl)oxy)-6-methylbenzo[c][2,7]naphthyridin-5(6H)-one

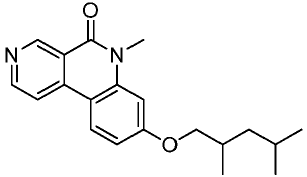

Prepared as described in Example 2: 8-((2,4-dimethylpentyl)oxy)-6-methylbenzo[*c*][2,7]naphthyridin-5(6*H*)-one (20 mg, 0.061 mmol, 7% yield) as a colorless solid. LC/MS (ESI) *m*/*e* 325.0 [(M+H)⁺, calcd for C₂₀H₂₅N₂O₂ 325.2]; HPLC retention time (method G): *t*_{R} = 9.54 min; HPLC retention time (method I): *t*_{R} = 9.44 min; ¹H NMR (400MHz, METHANOL-d₄) δ 9.47 (s, 1H), 8.76 (d, *J*=6.0 Hz, 1H), 8.45 - 8.39 (m, 1H), 8.35 (d, *J*=6.0 Hz, 1H), 7.22 - 6.89 (m, 2H), 4.13 - 4.02 (m, 1H), 4.01 - 3.92 (m, 1H), 3.79 (s, 3H), 2.19 - 2.04 (m, 1H), 1.89 - 1.66 (m, 1H), 1.45 (ddd, *J*=13.6, 8.0, 5.5 Hz, 1H), 1.28 - 1.17 (m, 1H), 1.10 (d, *J*=6.5 Hz, 3H), 0.99 (d, *J*=6.5 Hz, 3H), 0.95 (d, *J*=6.5 Hz, 3H).

### Methods

### AAK1 Kinase Assay

The assays were performed in U-bottom 384-well plates. The final assay volume was 30 µl prepared from 15 µl additions of enzyme and substrates (fluoresceinated peptide (5-FAM)-Aha-KEEQSQITSQVTGQIGWR-NH2 and ATP) and test compounds in assay buffer (10 mM Tris-HCL pH 7.4, 10 mM MgCl₂ 0.01% Tween-20 and 1.0 mM DTT). The reactions were initiated by the combination of bacterially expressed, GST-Xa-hAAK1 with substrates and test compounds. The reactions were incubated at room temperature for 3 hours and terminated by adding 60 µl of 35 mM EDTA buffer to each sample. The reactions were analyzed on the Caliper LabChip 3000 (Caliper, Hopkinton, MA) by electrophoretic separation of the fluorescent substrate and phosphorylated product. Inhibition data were calculated by comparison to EDTA quenched control reactions for 100% inhibition and vehicle-only reactions for 0% inhibition. The final concentration of reagents in the assays are ATP, 22 µM; (5-FAM)-Aha-KEEQSQITSQVTGQIGWR-NH2, 1.5 µM; GST-Xa-hAAK1, 3.5 nM; and DMSO, 1.6%. Dose response curves were generated to determine the concentration required inhibiting 50% of kinase activity (IC₅₀). Compounds were dissolved at 10 mM in dimethylsulfoxide (DMSO) and evaluated at eleven concentrations. IC₅₀ values were derived by non-linear regression analysis.

### HEK281 Cell-Based Assay

HEK293F cells were cultured in media containing DMEM (Gibco, cat. #11965), 10% FBS (SAFC Biosciences, cat. #12103C), 1X GPS (glutamine, penicillin and streptomycin). On day one, cells were plated on a 10cm dish so that they are ∼80% confluent at time of transfection. Roughly 12 million cells were in a 10cm dish at time of transfection. On day two, each dish was transfected with 48 ug DNA and 144 ul Lipofectamine 2000 (Invitrogen, cat.# 11668-019). The DNA was comprised of a mixture (per 10cm dish) containing 3 ug AAK1/HA/pIRES (full length human, NCBI accession no. NP_055726.2), 45 µg Flag/AP2MI/pcDNA (full length human), and 1.5 ml OPTI-MEM. The Lipofectamine 2000 is made up of a mixture (per 10cm dish) containing 144 µl Lipofectamine 2000 and 1.5 ml OPTI-MEM. Each mixture was transferred to individual 15ml tubes and incubated at RT for 5 minutes, and then the two mixes were combined and incubated at RT for 20 minutes. Growth media was then aspirated from each 10cm plate and replaced with 10ml of DMEM+10% FBS (no GPS). Finally, 3 ml DNA/Lipofectamine mix was added to each 10cm dish and mix gently followed by incubate of plate overnight at 37°C and 5% CO₂.

On day three, compounds were diluted in 100% DMSO at 1000X final concentration, followed by 3-fold serial dilutions for a total of 5 concentrations tested. Four compounds were tested per 10cm dish. One ul of each compound dilution was then pipetted into a deep-well, 96-well plate, followed by addition of 500 µl DMEM + 0.5% FBS into each well for a 2X final concentration of each compound. Cells were resuspended in a 10cm dish by simple pipetting (HEK293 cells come off the plate that easy at this point) and then transferred to a 50 ml conical tube and pelleted by centrifugation at 1000rpm for 5 min. Cell pellets were then resuspended in 2.75 ml DMEM + 0.5% FBS per 10cm dish and 100 µl of cell suspension transferred into each well of 96-well TC plate. Finally, 100 µl of 2X compound diluted in DMEM + 0.5% FBS was then added into wells containing cell suspension for a 1X final concentration. Plates were then incubated at 37°C and 5% CO₂ for 3 hours followed by transferring of cell suspensions from each well into 12-tube PCR strips. The PCR strips were spun in a tip rack at 1000rpm for 5 minutes to pellet cells and media was then removed by pipetting without disturbing the cell pellet.

To prepare for Western Blot analysis, cell pellets were resuspend in 40ul 1X LDS-PAGE sample buffer (Invitrogen, cat.# NP0008) + 2X Halt phophatase and protease inhibitor cocktail (Thermo Scientific, cat.# 1861284), followed by sonicating each with microtip sonicator set at 5 for 8-10 seconds. Five ul of 10X NuPage Sample Reducing Agent (with 50 mM DTT) was to each sample followed by heat denaturing at 70C for 10 min on PCR machine. A total of 10µl per sample was loaded into each lane of a 4-20% Tris-Glycine Criterion 26-well gel (Biorad, cat.# 345-0034) for the phospho-mu2 blot and 10µl per lane in a 4-12% Bis-Tris (+MES buffer) NuPAGE 26-well gel (Invitrogen, cat.# WG1403BX10) for the mu2 blot. For controls, 2ng of phospho-mu2 or 20ng mu2/Flag proteins were loaded in the last well of each gel. After SDS-PAGE, samples on each gel were transferred to PVDF membrane using an iBlot and membranes were blocked for one hour in TBST + 5% milk, followed by wash 3X for 5-10 min with TBST. Criterion gels were probed with rabbit anti-phospho-mu2 (1:5000; a rabbit polyclonal antibody produced by New England Peptide and affinity purified at Lexicon) in TBST + 5% BSA, whereas, NuPAGE gels were probed with mouse anti-Flag (1:500; Sigma, cat.# F1804) in TBST + 5% milk, and these primary antibodies were incubated overnight at 4°C on a rocker.

On day four, Western blots were washed 3X for 5-10 minutes with TBST, probe with anti-rabbit-HRP (1:2000; BioRad, cat.# 170-6515) or anti-mouse-HRP (1:2000; Biorad, cat.# 170-6516) in TBST + 5% milk for 1 hour at RT, washed 3X for 10 minutes with TBST, and developed with ECL reagent (GE Healthcare, cat.# RPN2132) on a Versadoc. Finally, the camera was set up to take a picture every 30 seconds for 10 minutes and the best image saved for each blot with no saturated signal (when the signal is saturated, the bands will be highlighted red). A volume analysis on each band was performed to obtain density values. Percent inhibition was calculated for each sample by first normalizing to total Mu2 expression levels and then comparing to 0% and 100% controls. IC₅₀ values were then calculated using Excel fitting software.

### AAK1 Knockout Mice

Mice homozygous (-/-) for the disruption of the AAK1 gene were prepared by two methods; gene trapping and homologous recombination.

Gene trapping is a method of random insertional mutagenesis that uses a fragment of DNA coding for a reporter or selectable marker gene as a mutagen. Gene trap vectors have been designed to integrate into introns or genes in a manner that allows the cellular splicing machinery to splice vector encoded exons to cellular mRNAs. Commonly, gene trap vectors contain selectable marker sequences that are preceded by strong splice acceptor sequences and are not preceded by a promoter. Thus, when such vectors integrate into a gene, the cellular splicing machinery splices exons from the trapped gene onto the 5' end of the selectable marker sequence. Typically, such selectable marker genes can only be expressed if the vector encoding the gene has integrated into an intron. The resulting gene trap events are subsequently identified by selecting for cells that can survive selective culture.

Embryonic stem cells (Lex-1 cells from derived murine strain A129), were mutated by a process involving the insertion of at least a portion of a genetically engineered vector sequence into the gene of interest, the mutated embryonic stem cells were microinjected into blastocysts which were subsequently introduced into pseudopregnant female hosts and carried to term using established methods. *See, e.g.,* "Mouse Mutagenesis", 1998, Zambrowicz et al., eds., Lexicon Press, The Woodlands, TX. The resulting chimeric animals were subsequently bred to produce offspring capable of germline transmission of an allele containing the engineered mutation in the gene of interest.

AAK1-gene disrupted mice were also made by homologous recombination. In this case, the second coding exon of the murine AAK1 gene (see GenBank Accession Number NM_177762) was removed by methods known in the art. *See, e.g.,* U.S. Patent Nos. 5,487,992, 5,627,059, and 5,789,215.

Mice homozygous (-/-) for the disruption of the AAK1 gene were studied in conjunction with mice heterozygous (+/-) for the disruption of the AAK1 gene, and wild-type (+/+) litter mates. During this analysis, the mice were subject to a medical work-up using an integrated suite of medical diagnostic procedures designed to assess the function of the major organ systems in a mammalian subject. Homozygous (-/-) "knockout" mice were studied in conjunction with their heterozygous (+/-) and wild-type (+/+) litter mates. Disruption of the AAK1 gene was confirmed by Southern analysis. Expression of the murine homolog of AAK1 was detected by RT-PCR in murine brain; spinal cord; eye; thymus; spleen; lung; kidney; liver; skeletal muscle; bone; stomach, small intestine and colon; heart; adipose; asthmatic lung; LPS liver; blood; banded heart; aortic tree; prostate; and mammary gland (5 week virgin, mature virgin, 12 DPC, 3 day post-partum (lactating), 3 day post-weaning (early involution), and 7 day post-weaning (late involution)).

AAK1 homozygous (-/-) and their wild-type (+/+) littermates were tested using the formalin paw test in order to assess their acute and tonic nociceptive responses. For these tests, Automatic Nociception Analyzers (purchased from the Ozaki lab at University of California, San Diego) were used. A metal band was placed around the left hind paw of each mouse 30 minutes prior to testing. After the 30-minute acclimation period, 20 µl of 5% formalin is subcutaneously injected in the dorsal surface of the left hind paw. Mice were individually housed in cylindrical chambers for 45 minutes. Fresh 5 % formalin solution was prepared by diluting formaldehyde (Formalde-fresh 20%, Fisher Scientific, Fair Lawn, NJ) with distilled water. Investigatory compounds were administered 30 minutes prior to formalin injection.

A computer recorded flinches per minute, total flinches for phase I (acute phase = first 8 minutes), and total flinches for phase II (tonic phase = time between minutes 20 - 40) through an electromagnetic field. *See* Yaksh TL, Ozaki G, McCumber D, Rathbun M, Svensson C, Malkmus S, Yaksh MC. An automated flinch detecting system for use in the formalin nociceptive bioassay. J Appl Physiol., 2001; 90:2386-402. As shown in Figure 1, phase 1 and phase 2 data were obtained using homozygous (-/-) mice females (n = 16), wild-type females (n = 15), homozygous (-/-) mice males (n = 9), and wild-type males (n = 18). In all groups and in both phases, the AAK1 homozygous (-/-) mice exhibited significantly less recorded paw flinching than their wild-type (+/+) littermates.

Studies of AAK1 knockout mice showed that disruption of the AAK1 gene affects pain response as measured using the formalin paw test described above. The same test was used to confirm that the administration of an AAK1 inhibitor can also affect pain response.

A compound of the disclosure was tested in this assay at different doses. Gabapentin and pregabalin were used as positive controls. Results are shown below in Table 2, wherein the effect of gabapentin at 200 mg/kg is considered a 100% response, the % response for the other compounds is relative to the 200 mg/kg dose of gabapentin, "sc" means subcutaneous administration; "po" means oral administration.

**Table 2**

| *Compound* | *Dose (mg*/*kg)* | *Response* |
|---|---|---|
| Gabapentin | 50 sc | 60% |
| Gabapentin | 200 sc | 100% |
| Pregabalin | 50 sc | 90% |
| Example 14: (*S*)-8-((2-amino-4-methylpentyl)oxy)-9-fluoro-6-methylbenzo[*c*][2,7]naphthyridin-5(6*H*)-one | 30 po | 62% |

## Claims

1. A compound selected from
(S)-8-((2-amino-4-methylpentyl)oxy)-9-bromobenzo[c][2,7]naphthyridin-5(6H)-one;
(S)-8-((2-amino-4-methylpentyl)oxy)-9-methylbenzo[c][2,7]naphthyridin-5(6H)-one;
(S)-8-((2-amino-4-methylpentyl)oxy)-9-isobutyl-6-methylbenzo[c][2,7]naphthyridin-5(6H)-one;
8-((2-amino-5,5,5-trifluoropentyl)oxy)-6-methylbenzo[c][2,7]naphthyridin-5(6H)-one;
8-((2-amino-5,5,5-trifluoropentyl)oxy)-9-bromo-6-methylbenzo[c][2,7]naphthyridin-5(6H)-one;
8-((2-amino-5,5,5-trifluoropentyl)oxy)-9-chloro-6-methylbenzo[c][2,7]naphthyridin-5(6H)-one;
4-amino-8-((2-amino-5,5,5-trifluoropentyl)oxy)-6-methylbenzo[c][2,7]naphthyridin-5(6H)-one;
(S)-8-((2-amino-4-methylpentyl)oxy)-9-(difluoromethyl)-6-methylbenzo[c][2,7]naphthyridin-5(6H)-one;
(S)-8-((2-amino-4-methylpentyl)oxy)-9-bromo-4,6-dimethylbenzo[c][2,7]naphthyridin-5(6H)-one;
8-((2-amino-5,5,5-trifluoropentyl)oxy)-4,6-dimethylbenzo[c][2,7]naphthyridin-5(6H)-one;
8-((2-amino-5,5,5-trifluoropentyl)oxy)-9-chloro-4,6-dimethylbenzo[c][2,7]naphthyridin-5(6H)-one;
(S)-8-((2-amino-4-methylpentyl)oxy)-9-cyclopropyl-4,6-dimethylbenzo[c][2,7]naphthyridin-5(6H)-one;
8-((2-amino-5,5,5-trifluoropentyl)oxy)-6-methyl-5-oxo-5,6-dihydrobenzo[c][2,7]naphthyridine-9-carbonitrile;
8-((2-amino-5,5,5-trifluoropentyl)oxy)-9-bromo-4,6-dimethylbenzo[c][2,7]naphthyridin-5(6H)-one;
(S)-8-((2-amino-4-methylpentyl)oxy)-9-isopropyl-4,6-dimethylbenzo[c][2,7]naphthyridin-5(6H)-one;
(S)-8-((2-amino-4-methylpentyl)oxy)-9-bromo-6-(2-methoxyethyl)-4-methylbenzo[c][2,7]naphthyridin-5(6H)-one;
(S)-8-((2-amino-4-methylpentyl)oxy)-9-chloro-6-(2-hydroxyethyl)-4-methylbenzo[c][2,7]naphthyridin-5(6H)-one;
(R)-8-((2-amino-4-methylpentyl)oxy)-9-bromo-4,6-dimethylbenzo[c][2,7]naphthyridin-5(6H)-one;
(S)-8-((2-amino-4-methylpentyl)oxy)-9-bromo-7-fluoro-4,6-dimethylbenzo[c][2,7]naphthyridin-5(6H)-one;
8-(2-amino-4,4,4-trifluorobutoxy)-6-methylbenzo[c][2,7]naphthyridin-5(6H)-one;
8-(2-amino-4,4,4-trifluorobutoxy)-9-bromo-4,6-dimethylbenzo[c][2,7]naphthyridin-5(6H)-one;
8-(2-amino-4,4,4-trifluorobutoxy)-9-bromo-6-methylbenzo[c][2,7]naphthyridin-5(6H)-one;
8-(2-amino-4,4,4-trifluorobutoxy)-9-chloro-6-methylbenzo[c][2,7]naphthyridin-5(6H)-one;
(S)-8-((2-amino-4-methylpentyl)oxy)-9-chlorobenzo[c][2,7]naphthyridin-5(6H)-one;
(S)-8-((2-amino-4-methylpentyl)oxy)-9-bromo-6-(2-hydroxyethyl)-4-methylbenzo[c][2,7]naphthyridin-5(6H)-one;
8-methoxy-4,6-dimethylbenzo[c][2,7]naphthyridin-5(6H)-one;
(S)-1-(8-((2-amino-4-methylpentyl)oxy)-9-fluoro-6-methyl-5-oxo-5,6-dihydrobenzo[c][2,7]naphthyridin-4-yl)-3-methylurea;
8-(2-hydroxy-3-(isopropylamino)propoxy)-6-methylbenzo[c][2,7]naphthyridin-5(6H)-one;
8-(2-hydroxy-3-(isopropylamino)propoxy)-4,6-dimethylbenzo[c][2,7]naphthyridin-5(6H)-one;
9-bromo-8-(2-hydroxy-3-(isopropylamino)propoxy)-6-methylbenzo[c][2,7]naphthyridin-5(6H)-one;
9-bromo-8-(2-hydroxy-3-(isopropylamino)propoxy)-4,6-dimethylbenzo[c][2,7]naphthyridin-5(6H)-one;
8-((2,4-dimethylpentyl)oxy)-6-methylbenzo[c][2,7]naphthyridin-5(6H)-one;
6-methyl-8-((4-methylpentyl)oxy)benzo[c][2,7]naphthyridin-5(6H)-one;
(S)-8-((2-hydroxy-4-methylpentyl)oxy)-4,6-dimethylbenzo[c][2,7]naphthyridin-5(6H)-one;
8-(2-hydroxy-3-isopropoxypropoxy)-4,6-dimethylbenzo[c] [2,7]naphthyridin-5(6H)-one;
9-bromo-8-((2,4-dimethylpentyl)oxy)-6-methylbenzo [c] [2,7]naphthyridin-5(6H)-one;
8-((2,4-dimethylpentyl)oxy)-4,6-dimethylbenzo[c][2,7]naphthyridin-5(6H)-one;
4,6-dimethyl-8-((4-methylpentyl)oxy)benzo[c][2,7]naphthyridin-5(6H)-one; and
8-((2,4-dimethylpentyl)oxy)-6-methylbenzo[c][2,7]naphthyridin-5(6H)-one; or a pharmaceutically acceptable salt thereof.

2. A composition comprising a pharmaceutically acceptable amount of a compound of claim 1, or a pharmaceutically acceptable salt thereof, and a pharmaceutically acceptable carrier.

3. A compound according to claim 1 or a pharmaceutically acceptable salt thereof or composition according to claim 2 for use as a medicament.

4. A compound according to claim 1 or a pharmaceutically acceptable salt thereof or composition according to claim 2 for use in treating or managing a disease or a disorder mediated by AAK1 activity wherein the disease or disorder is selected from Alzheimer's disease, bipolar disorder, pain, Parkinson's disease, and schizophrenia.

5. A compound or pharmaceutically acceptable salt thereof or composition for use according to claim 4 wherein the pain is neuropathic pain.

6. A compound or pharmaceutically acceptable salt thereof or composition for use according to claim 5 wherein the neuropathic pain is fibromyalgia or peripheral neuropathy.

## Patentansprüche

1. Verbindung, ausgewählt aus:
(S)-8-((2-Amino-4-methylpentyl)oxy)-9-brombenzo[c][2,7]naphthyridin-5(6H)-on;
(S)-8-((2-Amino-4-methylpentyl)oxy)-9-methylbenzo[c][2,7]naphthyridin-5(6H)-on;
(S)-8-((2-Amino-4-methylpentyl)oxy)-9-isobutyl-6-methylbenzo[c][2,7]naphthyridin-5(6H)-on;
8-((2-Amino-5,5,5-trifluorpentyl)oxy)-6-methylbenzo[c][2,7]naphthyridin-5(6H)-on;
8-((2-Amino-5,5,5-trifluorpentyl)oxy)-9-brom-6-methylbenzo[c][2,7]naphthyridin-5(6H)-on;
8-((2-Amino-5,5,5-trifluorpentyl)oxy)-9-chlor-6-methylbenzo[c][2,7]naphthyridin-5(6H)-on;
4-Amino-8-((2-amino-5,5,5-trifluorpentyl)oxy)-6-methylbenzo[c][2,7]naphthyridin-5(6H)-on;
(S)-8-((2-Amino-4-methylpentyl)oxy)-9-(difluormethyl)-6-methylbenzo[c][2,7]naphthyridin-5(6H)-on;
(S)-8-((2-Amino-4-methylpentyl)oxy)-9-brom-4,6-dimethylbenzo[c][2,7]naphthyridin-5(6H)-on;
8-((2-Amino-5,5,5-trifluorpentyl)oxy)-4,6-dimethylbenzo[c][2,7]naphthyridin-5(6H)-on;
8-((2-Amino-5,5,5-trifluorpentyl)oxy)-9-chlor-4,6-dimethylbenzo[c][2,7]naphthyridin-5(6H)-on;
(S)-8-((2-Amino-4-methylpentyl)oxy)-9-cyclopropyl-4,6-dimethylbenzo[c][2,7]naphthyridin-5(6H)-on;
8-((2-Amino-5,5,5-trifluorpentyl)oxy)-6-methyl-5-oxo-5,6-dihydrobenzo[c][2,7]naphthyridin-9-carbonitril;
8-((2-Amino-5,5,5-trifluorpentyl)oxy)-9-brom-4,6-dimethylbenzo[c][2,7]naphthyridin-5(6H)-on;
(S)-8-((2-Amino-4-methylpentyl)oxy)-9-isopropyl-4,6-dimethylbenzo[c][2,7]naphthyridin-5(6H)-on;
(S)-8-((2-Amino-4-methylpentyl)oxy)-9-brom-6-(2-methoxyethyl)-4-methylbenzo[c][2,7]naphthyridin-5(6H)-on;
(S)-8-((2-Amino-4-methylpentyl)oxy)-9-chlor-6-(2-hydroxyethyl)-4-methylbenzo[c][2,7]naphthyridin-5(6H)-on;
(R)-8-((2-Amino-4-methylpentyl)oxy)-9-brom-4,6-dimethylbenzo[c][2,7]naphthyridin-5(6H)-on;
(S)-8-((2-Amino-4-methylpentyl)oxy)-9-brom-7-fluor-4,6-dimethylbenzo[c][2,7]naphthyridin-5(6H)-on;
8-(2-Amino-4,4,4-trifluorbutoxy)-6-methylbenzo[c][2,7]naphthyridin-5(6H)-on;
8-(2-Amino-4,4,4-trifluorbutoxy)-9-brom-4,6-dimethylbenzo[c][2,7]naphthyridin-5(6H)-on;
8-(2-Amino-4,4,4-trifluorbutoxy)-9-brom-6-methylbenzo[c][2,7]naphthyridin-5(6H)-on;
8-(2-Amino-4,4,4-trifluorbutoxy)-9-chlor-6-methylbenzo[c][2,7]naphthyridin-5(6H)-on;
(S)-8-((2-Amino-4-methylpentyl)oxy)-9-chlorbenzo[c][2,7]naphthyridin-5(6H)-on;
(S)-8-((2-Amino-4-methylpentyl)oxy)-9-brom-6-(2-hydroxyethyl)-4-methylbenzo[c][2,7]naphthyridin-5(6H)-on;
8-methoxy-4,6-dimethylbenzo[c][2,7]naphthyridin-5(6H)-on;
(S)-1-(8-((2-Amino-4-methylpentyl)oxy)-9-fluor-6-methyl-5-oxo-5,6-dihydrobenzo[c][2,7]naphthyridin-4-yl)-3-methylharnstoff;
8-(2-Hydroxy-3-(isopropylamino)propoxy)-6-methylbenzo[c][2,7]naphthyridin-5(6H)-on;
8-(2-Hydroxy-3-(isopropylamino)propoxy)-4,6-dimethylbenzo[c][2,7]naphthyridin-5(6H)-on;
9-Brom-8-(2-hydroxy-3-(isopropylamino)propoxy)-6-methylbenzo[c][2,7]naphthyridin-5(6H)-on;
9-Brom-8-(2-hydroxy-3-(isopropylamino)propoxy)-4,6-dimethylbenzo[c][2,7]naphthyridin-5(6H)-on;
8-((2,4-Bimethylpentyl)oxy)-6-methylbenzo[c][2,7]naphthyridin-5(6H)-on;
6-Methyl-8-((4-methylpentyl)oxy)benzo[c][2,7]naphthyridin-5(6H)-on;
(S)-8-((2-Hydroxy-4-methylpentyl)oxy)-4,6-dimethylbenzo[c][2,7]naphthyridin-5(6H)-on;
8-(2-Hydroxy-3-isopropoxypropoxy)-4,6-dimethylbenzo[c][2,7]naphthyridin-5(6H)-on;
9-Brom-8-((2,4-dimethylpentyl)oxy)-6-methylbenzo[c][2,7]naphthyridin-5(6H)-on;
8-((2,4-Dimethylpentyl)oxy)-4,6-dimethylbenzo[c][2,7]naphthyridin-5(6H)-on;
4,6-Dimethyl-8-((4-methylpentyl)oxy)benzo[c][2,7]naphthyridin-5(6H)-on; und
8-((2,4-Dimethylpentyl)oxy)-6-methylbenzo[c][2,7]naphthyridin-5(6H)-on; oder ein pharmazeutisch verträgliches Salz davon.

2. Zusammensetzung, umfassend eine pharmazeutisch verträgliche Menge einer Verbindung nach Anspruch 1 oder ein pharmazeutisch verträgliches Salz davon und einen pharmazeutisch verträglichen Träger.

3. Verbindung nach Anspruch 1 oder ein pharmazeutisch verträgliches Salz davon oder Zusammensetzung nach Anspruch 2 zur Verwendung als ein Medikament.

4. Verbindung nach Anspruch 1 oder ein pharmazeutisch verträgliches Salz davon oder Zusammensetzung nach Anspruch 2 zur Verwendung in der Behandlung oder Bewältigung einer Erkrankung oder einer Störung, die durch AAK1-Aktivität vermittelt wird, wobei die Erkrankung oder Störung ausgewählt ist aus Alzheimer Krankheit, bipolarer Störung, Schmerz, Parkinson Krankheit und Schizophrenie.

5. Verbindung oder pharmazeutisch verträgliches Salz davon oder Zusammensetzung zur Verwendung nach Anspruch 4, wobei der Schmerz ein neuropathischer Schmerz ist.

6. Verbindung oder pharmazeutisch verträgliches Salz davon oder Zusammensetzung zur Verwendung nach Anspruch 5, wobei der neuropathische Schmerz Fibromyalgie oder periphere Neuropathie ist.

## Revendications

1. Composé choisi parmi:
(S)-8-((2-amino-4-méthylpentyl)oxy)-9-bromobenzo[c][2,7]naphtyridin-5(6H)-one;
(S)-8-((2-amino-4-méthylpentyl)oxy)-9-méthylbenzo[c][2,7]naphtyridin-5(6H)-one;
(S)-8-((2-amino-4-méthylpentyl)oxy)-9-isobutyl-6-méthylbenzo[c][2,7]naphtyridin-5(6H)-one;
8-((2-amino-5,5,5-trifluoropentyl)oxy)-6-méthylbenzo[c][2,7]naphtyridin-5(6H)-one;
8-((2-amino-5,5,5-trifluoropentyl)oxy)-9-bromo-6-méthylbenzo[c][2,7]naphtyridin-5(6H)-one;
8-((2-amino-5,5,5-trifluoropentyl)oxy)-9-chloro-6-méthylbenzo[c][2,7]naphtyridin-5(6H)-one;
4-amino-8-((2-amino-5,5,5-trifluoropentyl)oxy)-6-méthylbenzo[c][2,7]naphtyridin-5(6H)-one;
(S)-8-((2-amino-4-méthylpentyl)oxy)-9-(difluorométhyl)-6-méthylbenzo[c][2,7]naphtyridin-5(6H)-one;
(S)-8-((2-amino-4-méthylpentyl)oxy)-9-bromo-4,6-diméthylbenzo[c][2,7]naphtyridin-5(6H)-one;
8-((2-amino-5,5,5-trifluoropentyl)oxy)-4,6-diméthylbenzo[c][2,7]naphtyridin-5(6H)-one;
8-((2-amino-5,5,5-trifluoropentyl)oxy)-9-chloro-4,6-diméthylbenzo[c][2,7]naphtyridin-5(6H)-one;
(S)-8-((2-amino-4-méthylpentyl)oxy)-9-cyclopropyl-4,6-diméthylbenzo[c][2,7]naphtyridin-5(6H)-one;
8-((2-amino-5,5,5-trifluoropentyl)oxy)-6-méthyl-5-oxo-5,6-dihydrobenzo[c][2,7]naphtyridine-9-carbonitrile;
8-((2-amino-5,5,5-trifluoropentyl)oxy)-9-bromo-4,6-diméthylbenzo[c][2,7]naphtyridin-5(6H)-one;
(S)-8-((2-amino-4-méthylpentyl)oxy)-9-isopropyl-4,6-diméthylbenzo[c][2,7]naphtyridin-5(6H)-one;
(S)-8-((2-amino-4-méthylpentyl)oxy)-9-bromo-6-(2-méthoxyéthyl)-4-méthylbenzo[c][2,7]naphtyridin-5(6H)-one;
(S)-8-((2-amino-4-méthylpentyl)oxy)-9-chloro-6-(2-hydroxyéthyl)-4-méthylbenzo[c][2,7]naphtyridin-5(6H)-one;
(R)-8-((2-amino-4-méthylpentyl)oxy)-9-bromo-4,6-diméthylbenzo[c][2,7]naphtyridin-5(6H)-one;
(S)-8-((2-amino-4-méthylpentyl)oxy)-9-bromo-7-fluoro-4,6-diméthylbenzo[c][2,7]naphtyridin-5(6H)-one;
8-(2-amino-4,4,4-trifluorobutoxy)-6-méthylbenzo[c][2,7]naphtyridin-5(6H)-one;
8-(2-amino-4,4,4-trifluorobutoxy)-9-bromo-4,6-diméthylbenzo[c][2,7]naphtyridin-5(6H)-one;
8-(2-amino-4,4,4-trifluorobutoxy)-9-bromo-6-méthylbenzo[c] [2,7]naphtyridin-5(6H)-one;
8-(2-amino-4,4,4-trifluorobutoxy)-9-chloro-6-méthylbenzo[c][2,7]naphtyridin-5(6H)-one;
(S)-8-((2-amino-4-méthylpentyl)oxy)-9-chlorobenzo[c][2,7]naphtyridin-5(6H)-one;
(S)-8-((2-amino-4-méthylpentyl)oxy)-9-bromo-6-(2-hydroxyéthyl)-4-méthylbenzo[c][2,7]naphtyridin-5(6H)-one;
8-méthoxy-4,6-diméthylbenzo[c][2,7]naphtyridin-5(6H)-one;
(S)-1-(8-((2-amino-4-méthylpentyl)oxy)-9-fluoro-6-méthyl-5-oxo-5,6-dihydrobenzo[c][2,7]naphtyridin-4-yl)-3-méthylurée;
8-(2-hydroxy-3-(isopropylamino)propoxy)-6-méthylbenzo[c][2,7]naphtyridin-5(6H)-one;
8-(2-hydroxy-3-(isopropylamino)propoxy)-4,6-diméthylbenzo[c][2,7]naphtyridin-5(6H)-one;
9-bromo-8-(2-hydroxy-3-(isopropylamino)propoxy)-6-méthylbenzo[c][2,7]naphtyridin-5(6H)-one;
9-bromo-8-(2-hydroxy-3-(isopropylamino)propoxy)-4,6-diméthylbenzo[c][2,7]naphtyridin-5(6H)-one;
8-((2,4-diméthylpentyl)oxy)-6-méthylbenzo[c][2,7]naphtyridin-5(6H)-one;
6-méthyl-8-((4-méthylpentyl)oxy)benzo[c][2,7]naphtyridin-5(6H)-one;
(S)-8-((2-hydroxy-4-méthylpentyl)oxy)-4,6-diméthylbenzo[c][2,7]naphtyridin-5(6H)-one;
8-(2-hydroxy-3-isopropoxypropoxy)-4,6-diméthylbenzo[c][2,7]naphtyridin-5(6H)-one;
9-bromo-8-((2,4-diméthylpentyl)oxy)-6-méthylbenzo[c][2,7]naphtyridin-5(6H)-one;
8-((2,4-diméthylpentyl)oxy)-4,6-diméthylbenzo[c][2,7]naphtyridin-5(6H)-one;
4,6-diméthyl-8-((4-méthylpentyl)oxy)benzo[c][2,7]naphtyridin-5(6H)-one; et
8-((2,4-diméthylpentyl)oxy)-6-méthylbenzo[c][2,7]naphtyridin-5(6H)-one; ou un de leurs sels pharmaceutiquement acceptables.

2. Composition comprenant une quantité pharmaceutiquement acceptable d'un composé selon la revendication 1, ou d'un de ses sels pharmaceutiquement acceptables, et un véhicule pharmaceutiquement acceptable.

3. Composé selon la revendication 1 ou un de ses sels pharmaceutiquement acceptables ou composition selon la revendication 2 pour une utilisation en tant que médicament.

4. Composé selon la revendication 1 ou un de ses sels pharmaceutiquement acceptables ou composition selon la revendication 2 pour une utilisation dans le traitement ou la gestion d'une maladie ou d'un trouble induit par l'activité de AAK1, où la maladie ou le trouble est choisi parmi une maladie d'Alzheimer, un trouble bipolaire, une douleur, une maladie de Parkinson et une schizophrénie.

5. Composé ou un de ses sels pharmaceutiquement acceptables ou composition pour une utilisation selon la revendication 4, où la douleur est une douleur neuropathique.

6. Composé ou un de ses sels pharmaceutiquement acceptables ou composition pour une utilisation selon la revendication 5, où la douleur neuropathique est une fibromyalgie ou une neuropathie périphérique.
